# EUROPEAN PATENT SPECIFICATION

(11) **EP 1 470 156 B1**
(45) Date of publication and mention of the grant of the patent: **18.04.2007**
(21) Application number: 02781695.8
(22) Date of filing: 20.12.2002
(51) Int. Cl.: C07K 14/47, C12Q 1/68, C12N 15/63, G01N 33/50

(54) **POLYMORPHISMS OF THE OCTN1 AND OCTN2 CATION TRANSPORTERS ASSOCIATED WITH INFLAMMATORY BOWEL DISEASE**
POLYMORPHISMEN DER KATIONENTRANSPORTER OCTN1 UND OCTN2 WELCHE MIT ENTZÜNDLICHEN DARMERKRANKUNGEN IN ZUSAMMENHANG STEHEN
POLYMORPHISMES DES TRANSPORTEURS DE CATIONS OCTN1 ET OCTN2 LIES AUX TROUBLES INTESTINAUX INFLAMMATOIRES

(30) Priority: 21.12.2001 US 343338 P; 08.03.2002 US 362700 P; 08.03.2002 US 362717 P; 19.11.2002 US 427529 P
(43) Date of publication of application: 27.10.2004
(73) Proprietor: Ellipsis Biotherapeutics Corporation, Toronto, Ontario M5G 1Z6 (CA)
(72) Inventor: PELTEKOVA, Vanya D., Ellipsis Biotherapeutics Corp, Toronto, Ontario M5G 1Z6 (CA); WINTLE, Richard F., Ellipsis Biotherapeutics Corp., Toronto, Ontario M5G 1Z6 (CA); RUBIN, Laurence A., Ellipsis Biotherapeutics Corp., Toronto, Ontario M5G 1Z6 (CA); ST. GEORGE-HYSLOP, Peter H., Toronto, Ontario M5G 1Z6 (CA); SIMINOVITCH, Katherine, A., Toronto, Ontario M5G 1Z6 (CA)
(74) Representative: HOFFMANN EITLE
(86) International application number: PCT/IB2002/005560
(87) International publication number: WO 2003/054011

(56) References cited:
- EP-A- 1 020 518
- EP-A- 1 111 041
- WO-A-01/42511
- IKUMI TAMAI ET AL: "CLONING AND CHARACTERIZATION OF A NOVEL HUMAN PH-DEPENDENT ORGANIC CATION TRANSPORTER OCTN1" FEBS LETTERS, ELSEVIER SCIENCE PUBLISHERS, AMSTERDAM, NL, vol. 419, no. 1, 1997, pages 107-111, XP002911182 ISSN: 0014-5793 cited in the application -& DATABASE EMBL [Online] 22 December 1998 (1998-12-22) retrieved from EMBL Database accession no. AB007448 XP002253476
- NEZU J-I ET AL: "PRIMARY SYSTEMIC CARNITINE DEFICIENCY IS CAUSED BY MUTATIONS IN A GENE ENCODING SODIUM ION-DEPENDENT CARNITINE TRANSPORTER" NATURE GENETICS, NEW YORK, NY, US, vol. 21, no. 1, January 1999 (1999-01), pages 91-94, XP002928509 ISSN: 1061-4036
- WU X ET AL: "CDNA SEQUENCE, TRANSPORT FUNCTION, AND GENOMIV ORGANIZATION OF HUMAN OCTN2, A NEW MEMBER OF THE ORGANIC CATION TRANSPORTER FAMILY" BIOCHEMICAL AND BIOPHYSICAL RESEARCH COMMUNICATIONS, ACADEMIC PRESS INC. ORLANDO, FL, US, vol. 246, no. 3, 1998, pages 589-595, XP002920229 ISSN: 0006-291X
- RIOUX J D ET AL: "Genetic variation in the 5q31 cytokine gene cluster confers susceptibility to Crohn disease." NATURE GENETICS. UNITED STATES OCT 2001, vol. 29, no. 2, October 2001 (2001-10), pages 223-228, XP001154441 ISSN: 1061-4036 cited in the application
- LAHJOUJI K ET AL: "Carnitine transport by organic cation transporters and systemic carnitine deficiency." MOLECULAR GENETICS AND METABOLISM. UNITED STATES AUG 2001, vol. 73, no. 4, August 2001 (2001-08), pages 287-297, XP009016382 ISSN: 1096-7192
- LAMHONWAH ANNE-MARIE ET AL: "A third human carnitine/organic cation transporter (OCTN3) as a candidate for the 5q31 Crohn's disease locus (IBD5)." BIOCHEMICAL AND BIOPHYSICAL RESEARCH COMMUNICATIONS. UNITED STATES 31 JAN 2003, vol. 301, no. 1, 31 January 2003 (2003-01-31), pages 98-101, XP001154459 ISSN: 0006-291X

## Description

### FIELD OF THE INVENTION

This invention relates generally to the analysis of biological material. In particular, the invention relates to diagnostic analyses for markers that are associated with inflammatory disorders of the gastrointestinal tract, and to their uses for the development of novel therapeutic treatments for inflammatory disorders.

### BACKGROUND OF THE INVENTION

Crohn's Disease (CD) is an idiopathic condition characterized by chronic, relapsing intestinal inflammation, with most patients developing symptoms in early adulthood. It is a subset of the Inflammatory Bowel Diseases (IBD) which include Crohn's Disease, indeterminate colitis (IC) and Ulcerative Colitis (UC).

These conditions affect a relatively large portion of the population, with varying estimates of prevalence around 300,000 in North America. Inflammatory Bowel Diseases have a high economic impact, with costs running into the billions of dollars.

Genetic studies have indicated the presence of several loci predisposing to Inflammatory-Bowel Diseases, the most widely replicated of which are on chromosomes 16 (caused by mutations in the *NOD2*/*CARD15* gene), 12, 6 and 14. A locus at chromosome 5q31, termed *IBD5,* which predisposes to early-onset Crohn's Disease (with age of onset 16 years or earlier) has been described by Rioux JD *et al., Nature Genet.* 29, 223-228 (2001). This locus is marked by a risk haplotype of 11 Single Nucleotide Polymorphisms (SNPs) over a region of approximately 250-kb. The region contains multiple candidate genes including the genes for organic cation transporters (*OCTN1*/*SLC22A4* and *OCTN2*/*SLC22A5),* the gene for a LIM-domain-containing protein (*RIL*/*PDLIM3*), the gene for the α2 subunit of proline-4-hydroxylase (*P4HA2*) and a gene of unknown function (NCBI UniGene identifier Hs.70932). WO 01/42511 discloses several single nucleotide polymorphisms which are associated with inflammatory bowel diseases. A Crohn's disease susceptibility locus was identified in the 5q31-33 region. Tamai I. et al., FEBS Letters, 419, 107 to 111 (1997) discloses the amino acid sequence of OCTN1. EP 1 020 518 discloses the nucleotide sequences of cDNAs of human OCTN1 and human OCTN2. EP 1 111 041 discloses that mutations in the OCTN2 gene are associated with carnitine deficiency. Nezu J. et al., Nature Genetics, 21, 91 - 94 (1999) also discloses the link between mutations in OCTN2 and systemic carnitine deficiency. Because of extensive linkage disequilibrium (LD) in this region, it had not previously been possible to further refine the SNP map and unambiguously identify a single susceptibility gene.

Thus, there is a continuing need in the medical arts for genetic markers of Crohn's Disease and guidance for the use of such markers.

### SUMMARY OF THE INVENTION

The invention provides a method for diagnosing Inflammatory Bowel Diseases, using genetic markers that are implicated in severe, early-onset Crohn's Disease (CD). The invention also provides coding sequence mutations in the *OCTN1* gene (the human OCTN1 gene, also known as "*Homo sapiens* solute carrier family 22 (organic cation transporter), member 4 (SLC22A4)") that significantly reduces its ability to transport the organic cation carnitine. The invention further provides mutations in the promoter region of *OCTN2* (the human OCTN2 gene, also known as "*Homo sapiens* solute carrier family 22 (organic cation transporter), member 5 (SLC22A5)") that downregulates both basal transcription and transcription induced either by heat shock or arachidonic acid. This transcription difference is apparently due to the disruption of a binding site for heat shock transcription factor 1 (HSF1). The invention further provides a haplotype of these two mutations in combination. The two genes, *OCTN1* and *OCTN2,* show significant downregulation in inflamed Crohn's Disease tissue.

The identified OCTN1 and OCTN2 sequence variations have diagnostic and pharmacogenetic utility and reveal a disease-related molecular pathway that can be targeted for therapeutic intervention. The OCTN1 and OCTN2 genes are differentially expressed in inflamed and non-inflamed colon tissue from CD patients. Both genes contain disease-associated DNA sequence variations (polymorphisms). The identified polynucleotide polymorphisms are thus the basis for a diagnostic and prognostic test describing susceptibility to Inflammatory Bowel Diseases in certain individuals. The invention also provides for the identification and use of these polynucleotides and encoded polypeptide sequences as targets for the development of therapeutic compounds intended to be useful in the treatment of Inflammatory Bowel Diseases and inflammation generally.

The invention also provides a model for Crohn's Disease, wherein the combined effect of these two mutations results in overall poor carnitine transport, particularly in response to inflammation. As carnitine is a cofactor required for the uptake of long-chain fatty acids into the mitochondria for subsequent β-oxidation, the effect of this mutation haplotype leads to metabolic stress, which contributes to inflammatory damage to gastrointestinal tract tissue in Crohn's Disease patients. In addition, the invention provides evidence of an association between the OCTN1 and OCTN2 genes and generalized inflammatory responses. This model provides the basis for the therapeutic treatment of Inflammatory Bowel Diseases. The invention also provides a model for Crohn's Disease, wherein the effect of mutations in OCTN1 results in overall poor efflux of molecules from the cell. These molecules could include toxic metabolites, bacterial endotoxins, xenobiotics, pharmaceutical compounds used to treat inflammation, or free radical species.

### BRIEF DESCRIPTION OF THE DRAWINGS

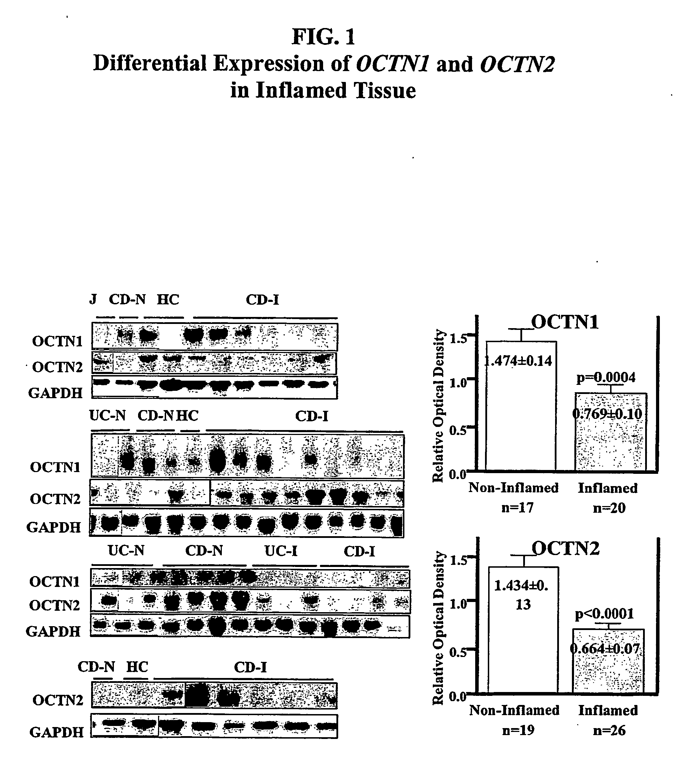

FIG. 1 is a set of Northern blots showing a differential expression of *OCTN1* and *OCTN2* in inflamed tissue. Northern blots probed with *OCTN1, OCTN2* or *GAPDH* are shown at the left of FIG. 1. Bar graphs of the relative optical density (normalized to *GAPDH*) corresponding to *OCTN1* expression (right side, above) or *OCTN2* expression (right side, below) are shown at the right, with value ± SEM labeled on the bar. p-values of significance are shown above the bar corresponding to inflamed tissue. HC: normal human colon. J: Jurkat T-cell leukemia cells. UC-N: non-inflamed tissue from Ulcerative Colitis patient. UC-I: inflamed tissue from Ulcerative Colitis patient. CD-N: non-inflamed tissue from Crohn's Disease patient. CD-I: inflamed tissue from Crohn's Disease patient.

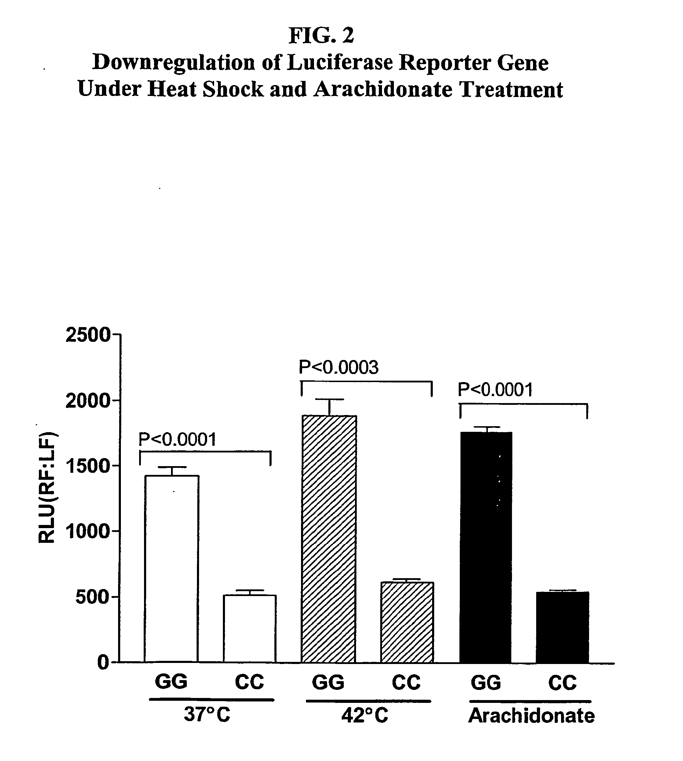

FIG. 2 is a set of bar graphs showing that a G-207C mutation in the heat shock enhancer (HSE) of the promoter region of OCTN2 gene results in downregulation of luciferase reporter gene under heat shock and arachidonate treatment. HeLa cells transfected with constructs containing the luciferase reporter gene fused to the 2.7-kb region of OCTN2 promoter with wild-type (G-207) or mutant (G-207C) alleles. Relative luminescence of transfected *OCTN2* promoter constructs was measured in untreated control cells (37°C), cells exposed to a standard heat shock (42°C) and to 20 µM arachidonic acid (arachidonate). Values are means of three independent experiments. Error bars are the SEM. G: nonrisk allele at position -207 of the OCTN2 promoter; C: risk allele.

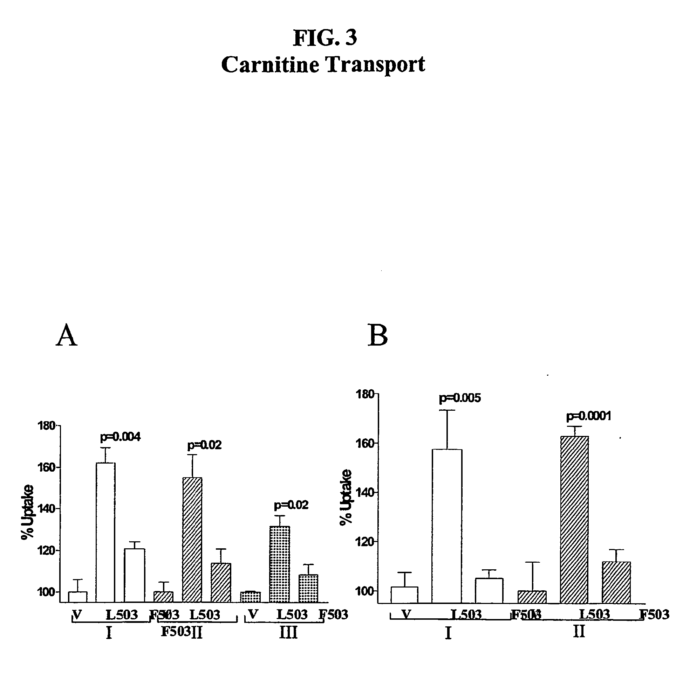

FIG. 3 is a set of bar graphs showing the carnitine transport by human HeLa (FIG. 3A) and human OCTN2 deficient cell line (FIG. 3B), transfected with OCTN1 cDNA with L503 or the mutant L1503F allele. Cells transfected with pcDNA3 vector alone served as a control. Uptake of [³H]-carnitine (20 nM) was measured at pH 7.5 in a media containing NaCl. Carnitine uptake was calculated as cpm/mg protein/min, values were corrected for transfection efficiency and the uptake was normalized with respect to the value of the mock transfected cells. Points are means ± SE of three (FIG. 3A) to six (FIG. 3B) determinations in three (FIG. 3A) or two (FIG. 3B) independent experiments.

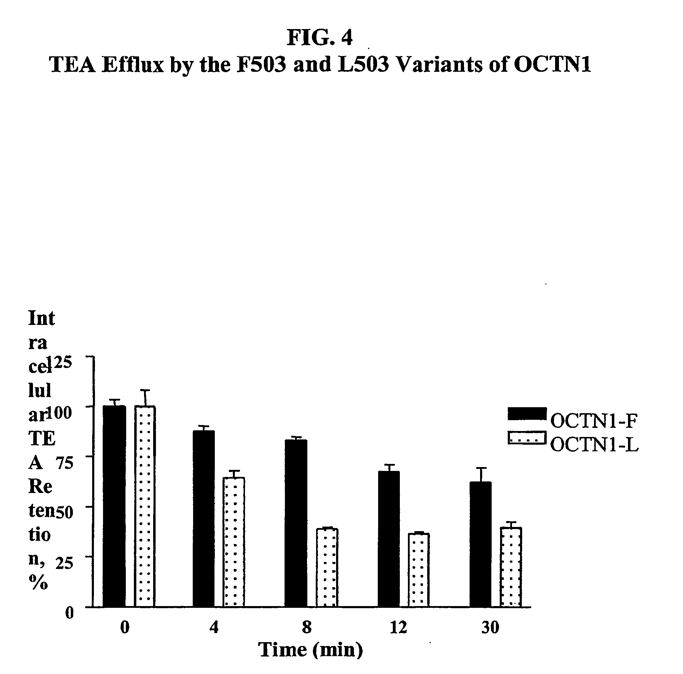

FIG. 4 is a set of bar graphs showing TEA efflux by the mutant 503F and wild-type 503L variants of OCTN1. Intracellular retention of radiolabeled TEA is shown as a percentage of the maximally retained amount at time zero. OCTN1-F: L503F variant of OCTN1. OCTN1-L: wild-type 503L variant of OCTN1. Points are means ± SE of three determinations.

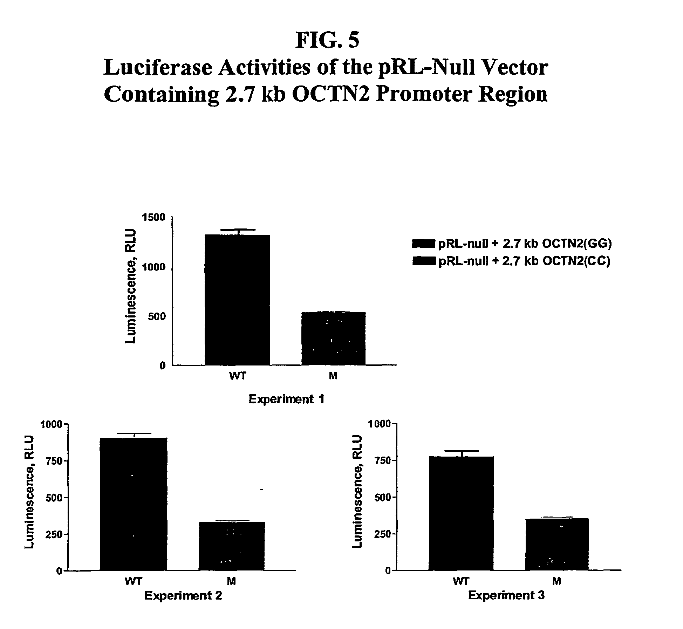

FIG. 5 is a set of bar graphs showing the luciferase activities of the pRL-null vector containing 2.7 kb OCTN2 promoter region.

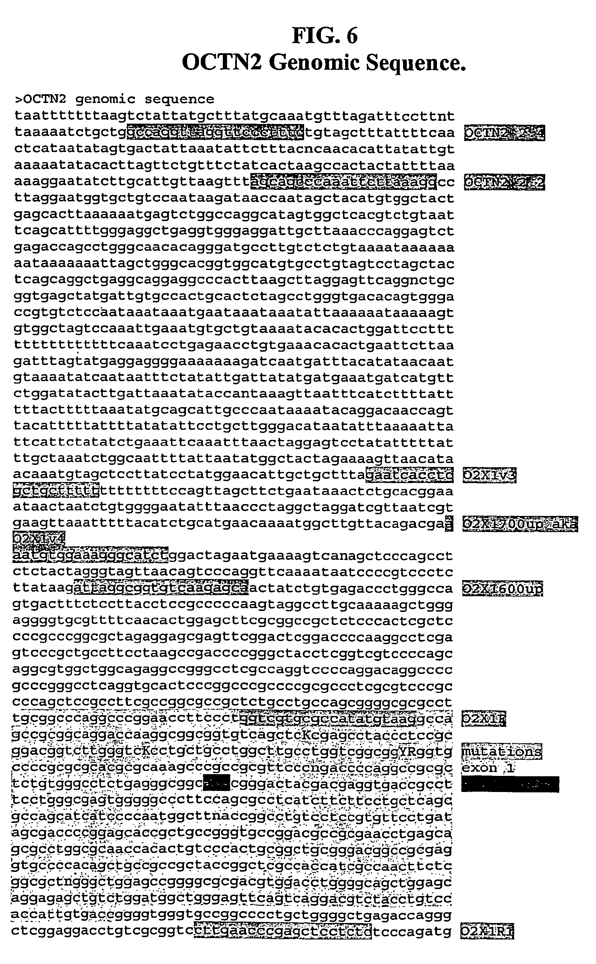

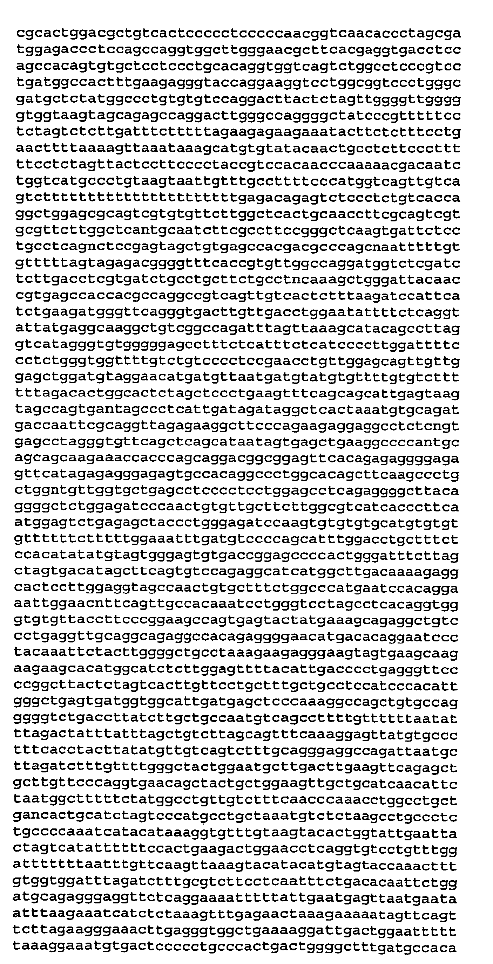

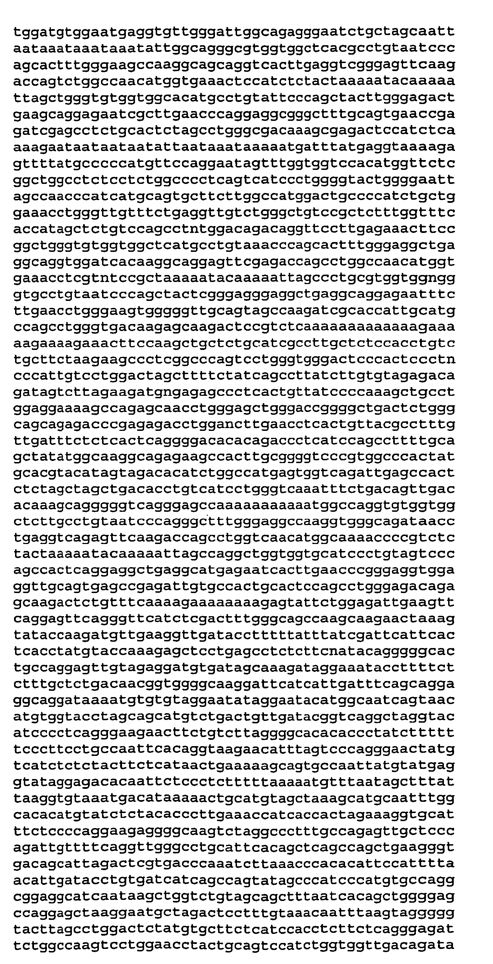

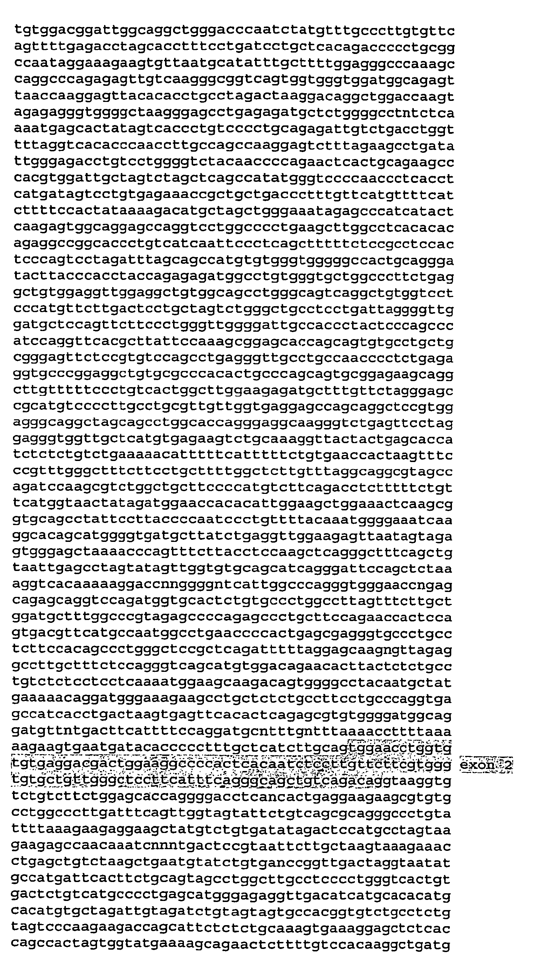

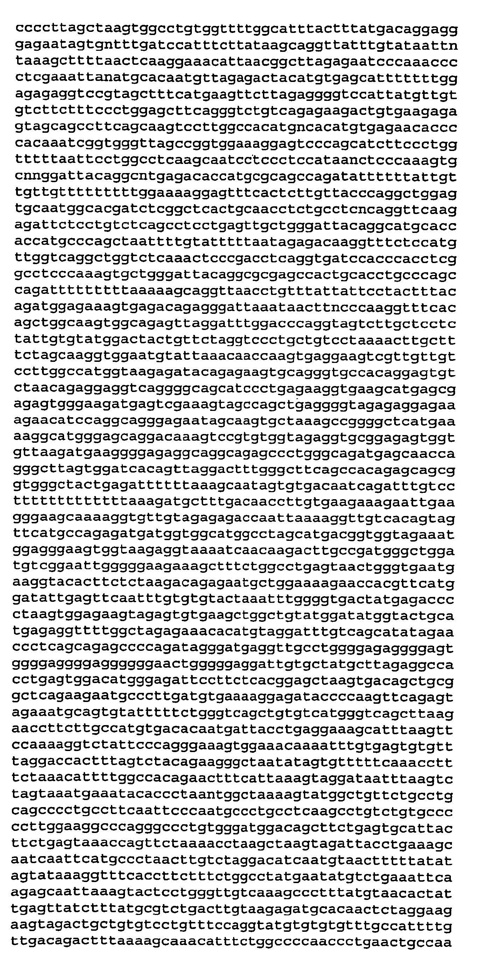

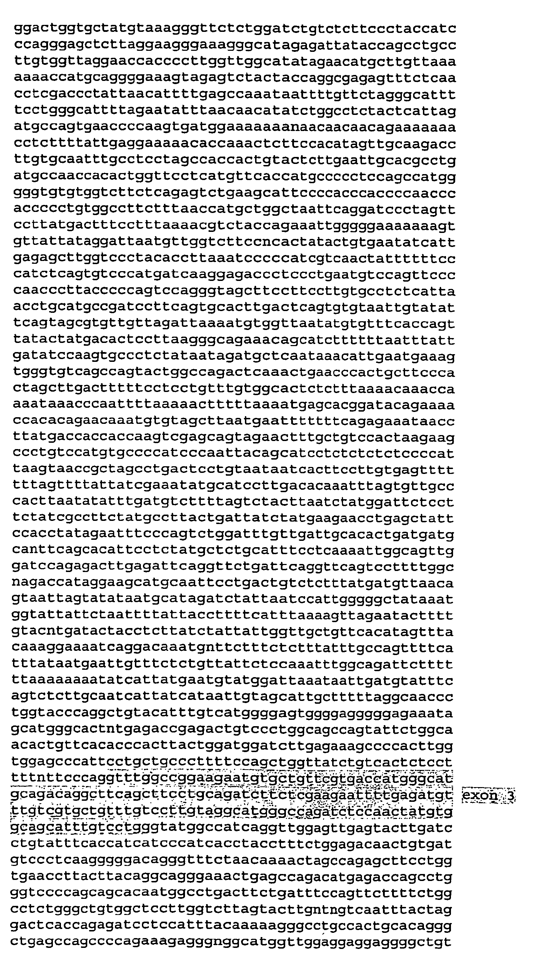

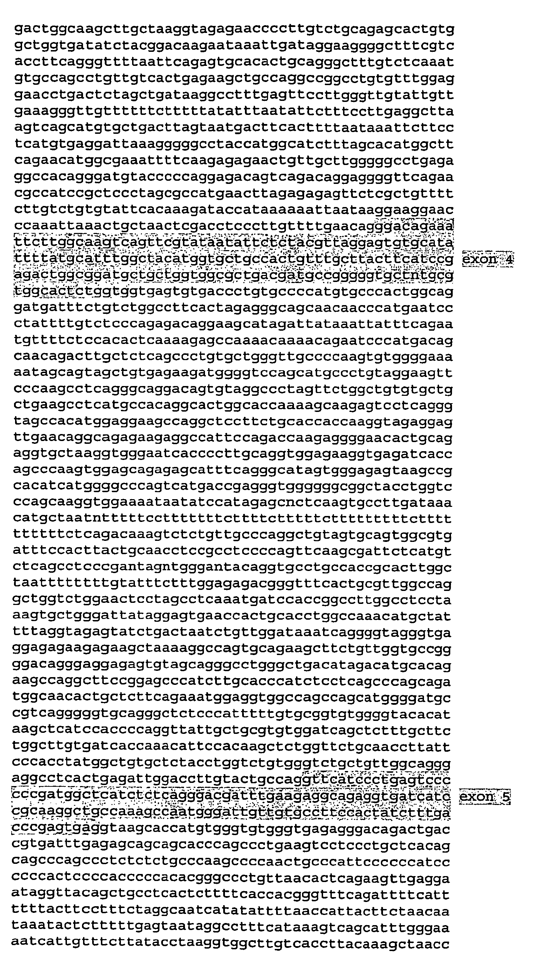

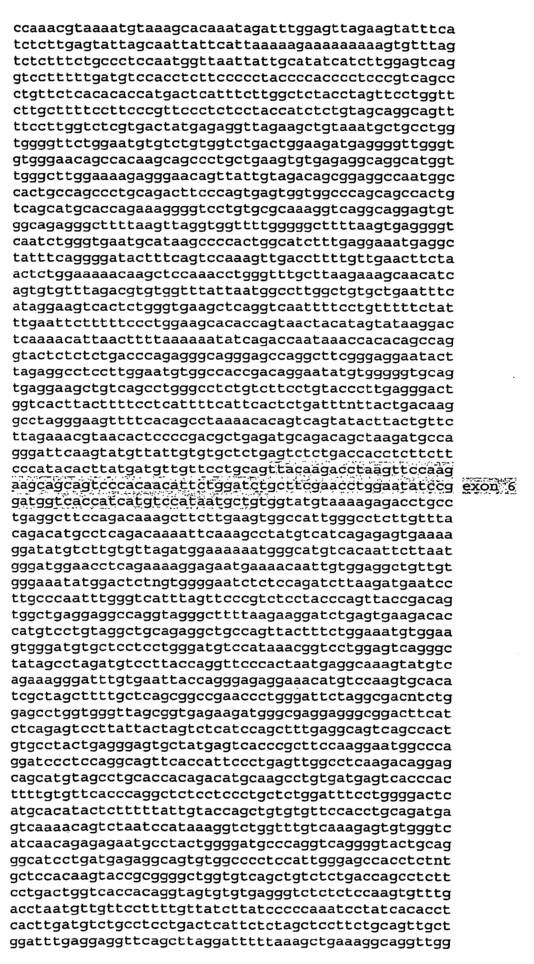

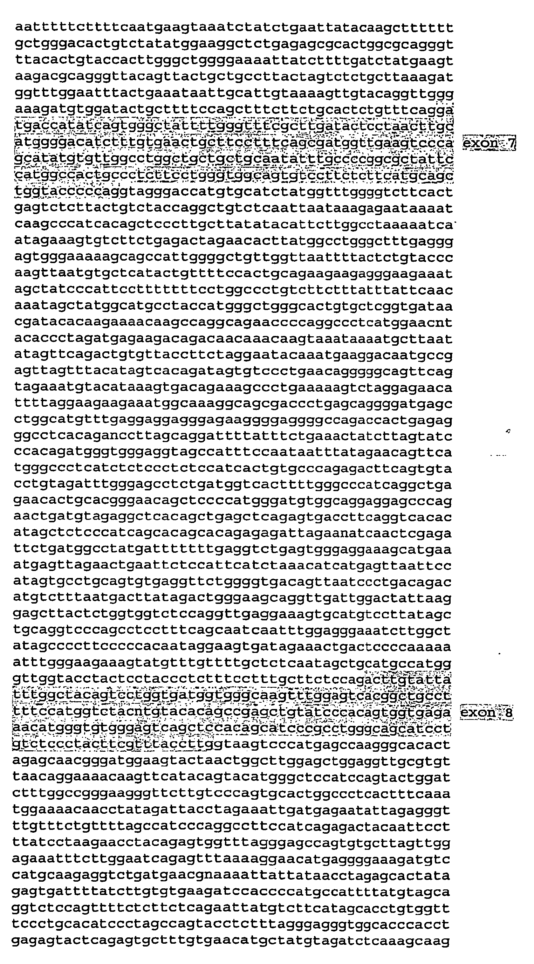

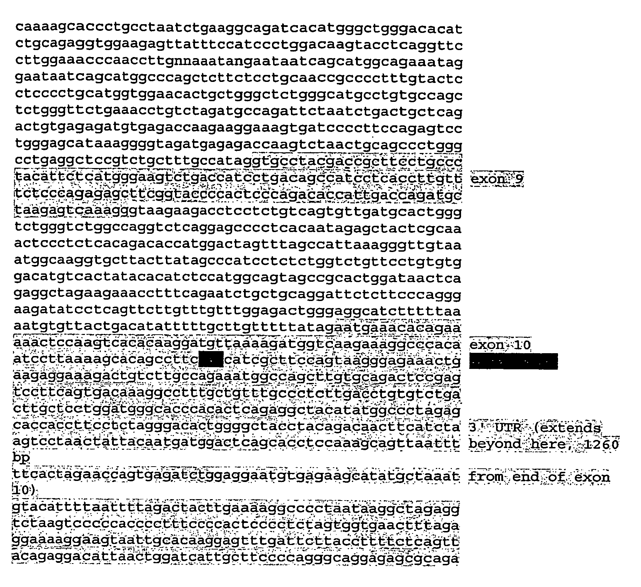

FIG. 6 shows the genomic DNA sequence including the OCTN2 gene. Locations of exons are highlighted in yellow. Locations of primer sequences used are highlighted in green (note that the O2X1F primer is located within exon 1, and that O2X1R is the reverse complement of the sequence as shown). Start and stop codons of the coding sequence are highlighted in red. Mutations within the 5' region of the gene are highlighted and are shown with standard DNA nomenclature for polymorphic bases, *i.e.* "K" meaning G or T, "Y" meaning C or T, "R" meaning A or G, and "S" meaning C or G.

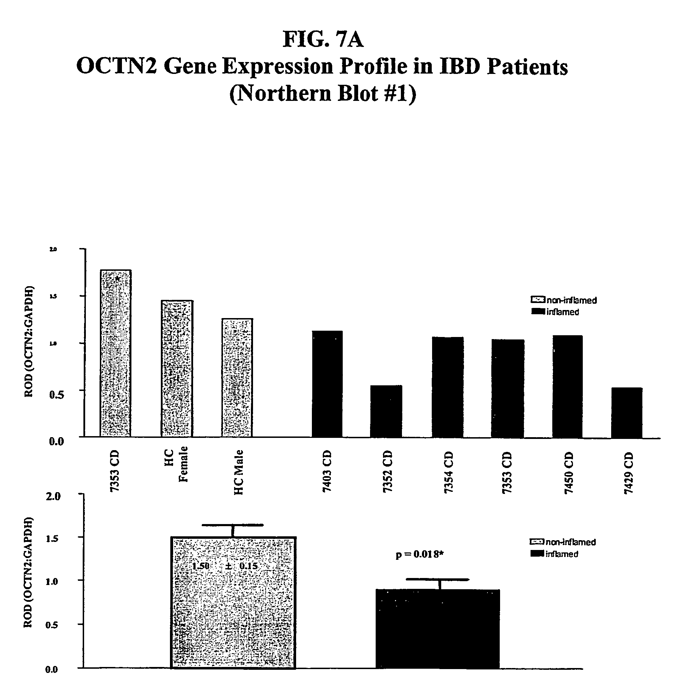

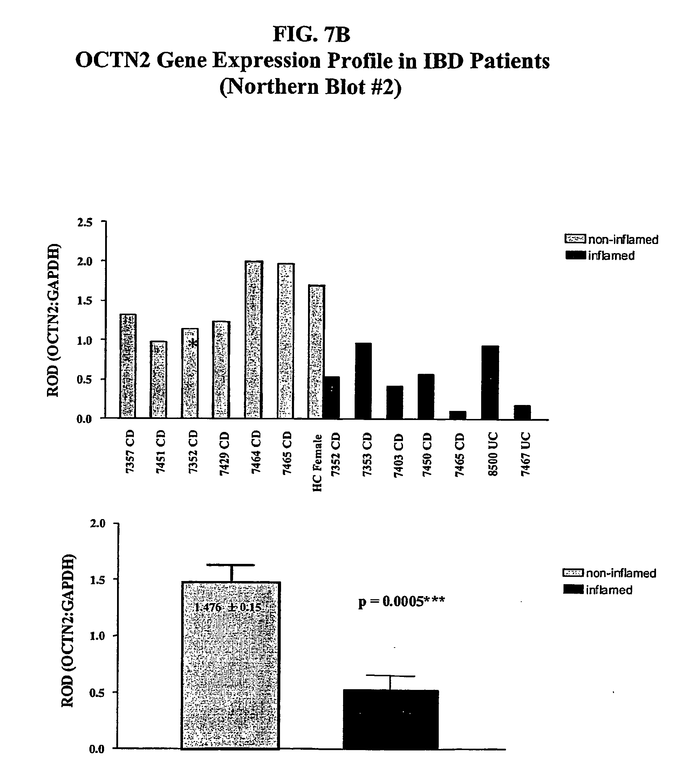

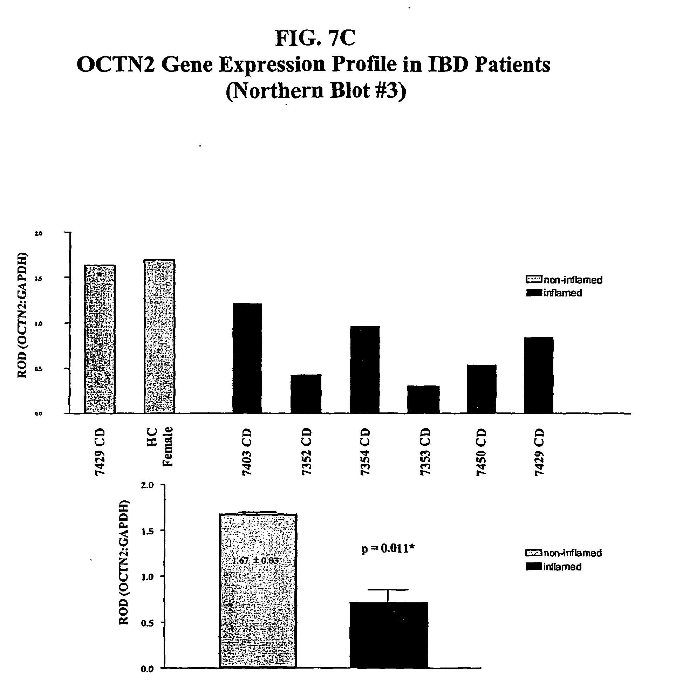

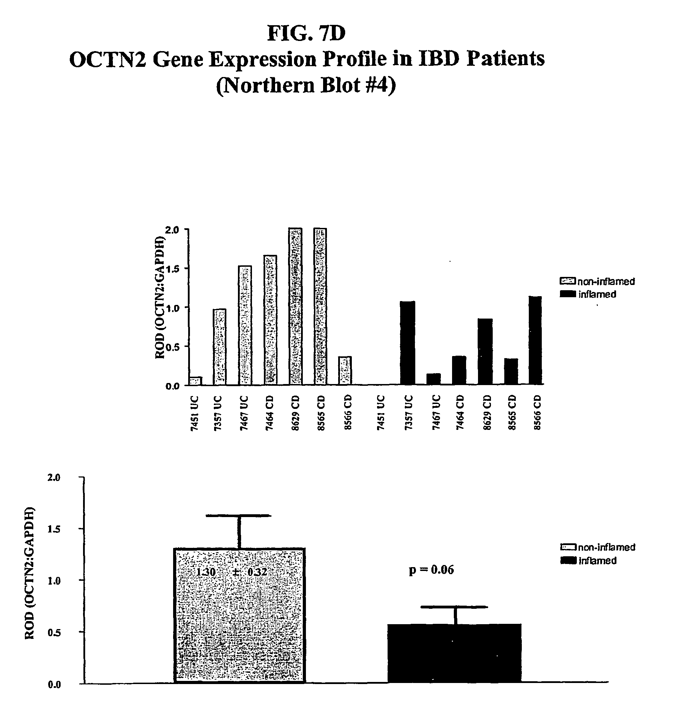

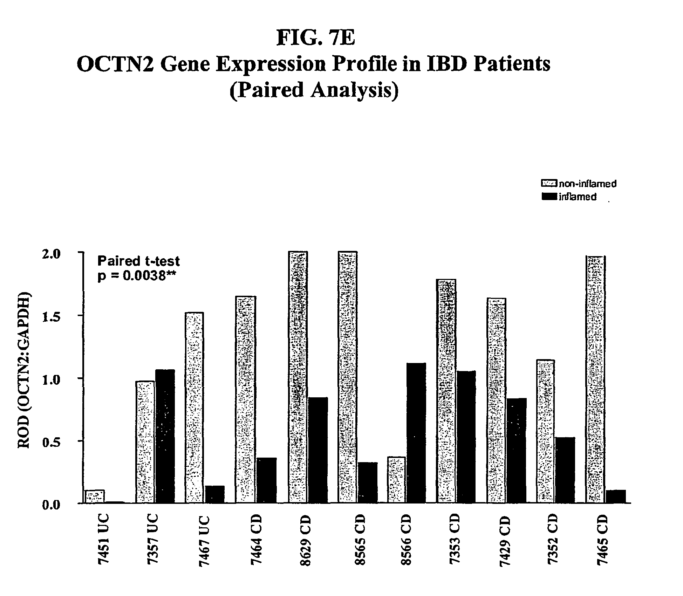

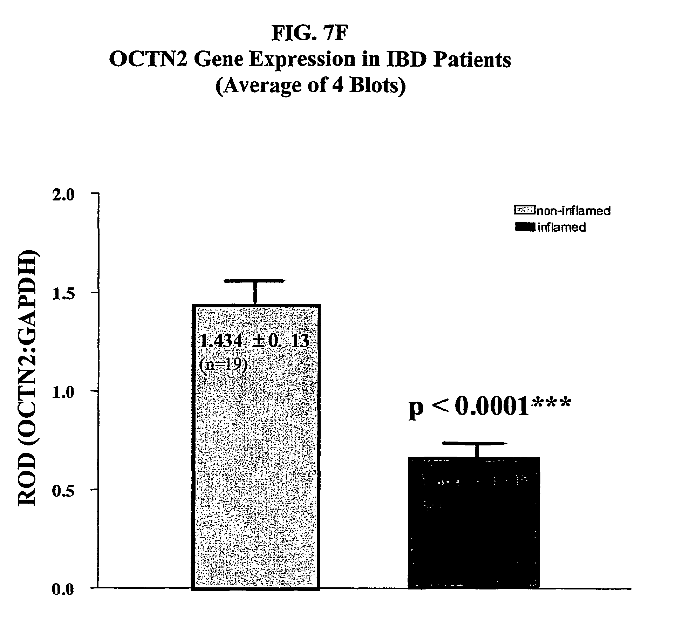

FIG. 7 shows in graphical form the expression level of the OCTN2 gene, expressed as relative optical density ("ROD") of autoradiographic signal from the OCTN2 gene as compared to the glyceraldehyde-3-phosphate dehydrogenase (GAPDH) gene. Blue bars represent expression level from RNA extracted from surgically removed non-inflamed GI tract tissue, and red bars represent expression level from RNA extracted from surgically removed inflamed GI tract tissue. Individual RNA samples are identified below the bars with a unique identifier number (*e.g*. 7403) and patient's diagnosis ("UC" for Ulcerative Colitis, "CD" for Crohn's Disease). Control unaffected samples are labeled as "HC male" and "HC female" and are from individuals without Inflammatory Bowel Disease. FIGS. 7A-D each represent data from a single "Northern blot" (*i.e*. DNA:RNA hybridization experiment); for each blot, individual sample results are presented in the top panel and averaged results for inflamed and non-inflamed samples are presented in the bottom panel. In the bottom panel, each bar is labeled with its mean value and standard error of the mean (SEM). Statistical p-values are also presented in the bottom panel. FIG. 7E shows individual sample results for pairs of inflamed and non-inflamed tissue samples from the same individual. Individual identifier numbers and diagnoses, values and standard errors are indicated as in FIGS. 5A-F shows the averaged results for all affected and unaffected samples. Individual identifier numbers and diagnoses, values and standard errors are indicated as in FIGS. 7A-D.

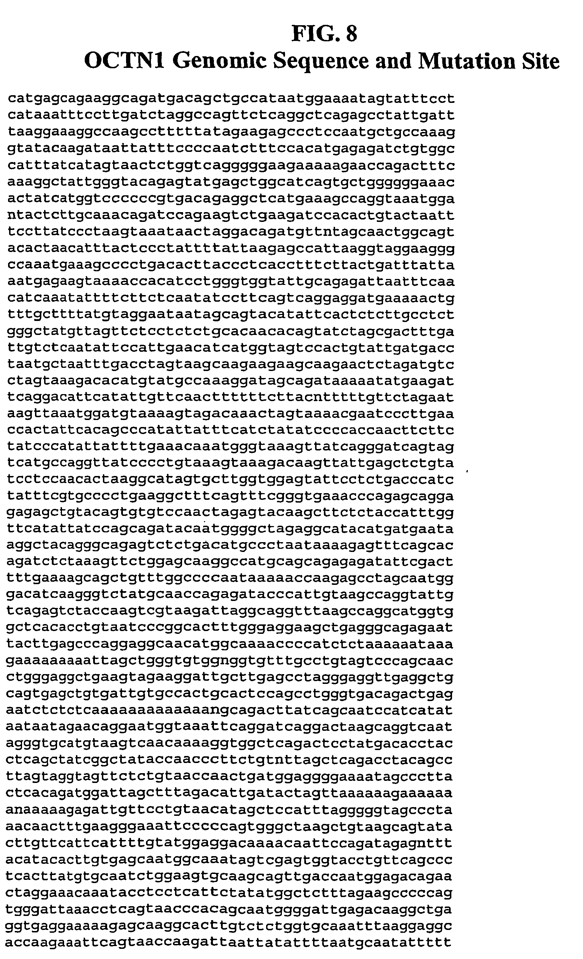

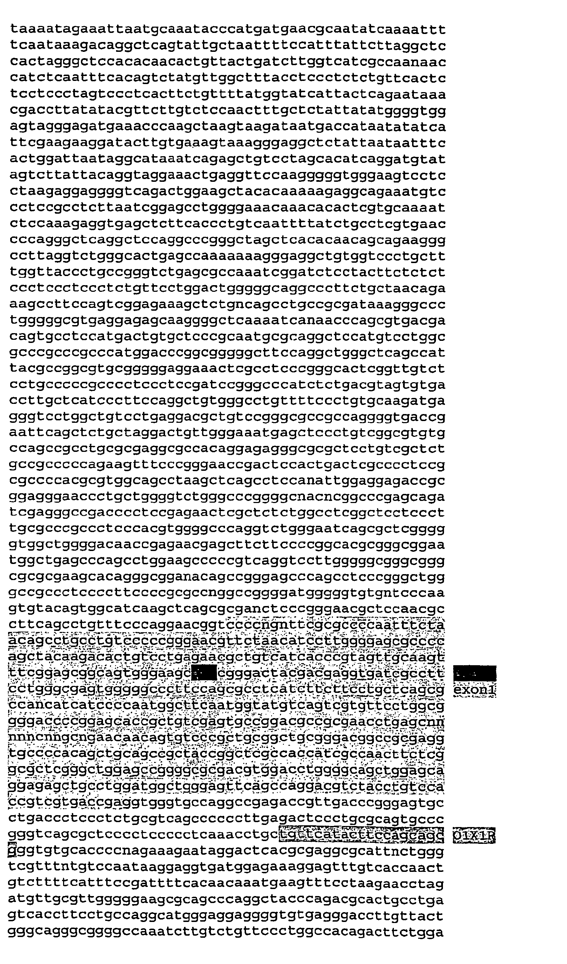

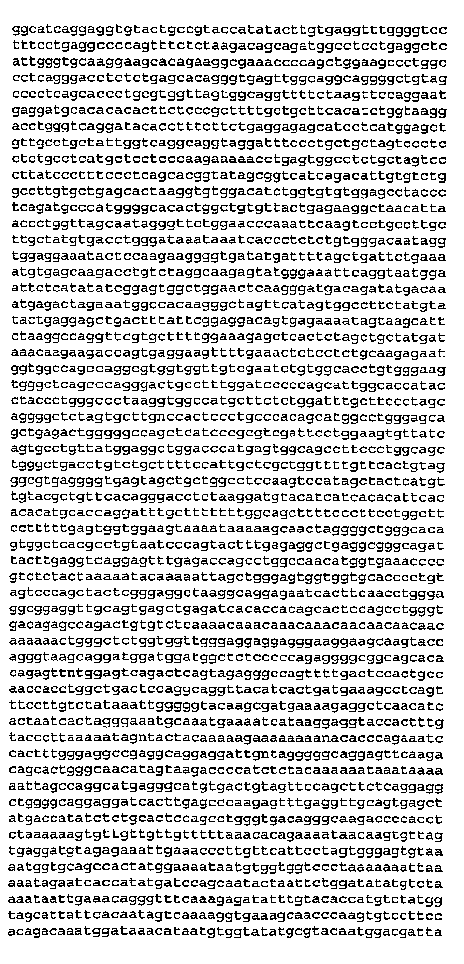

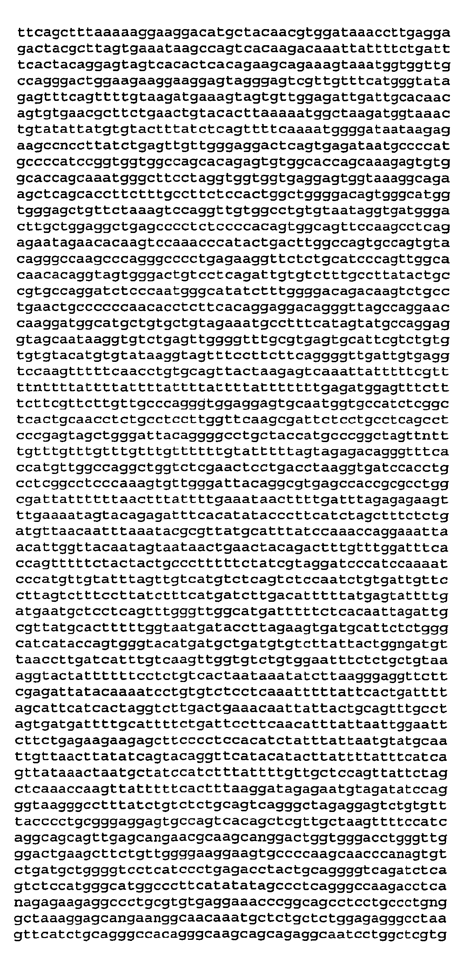

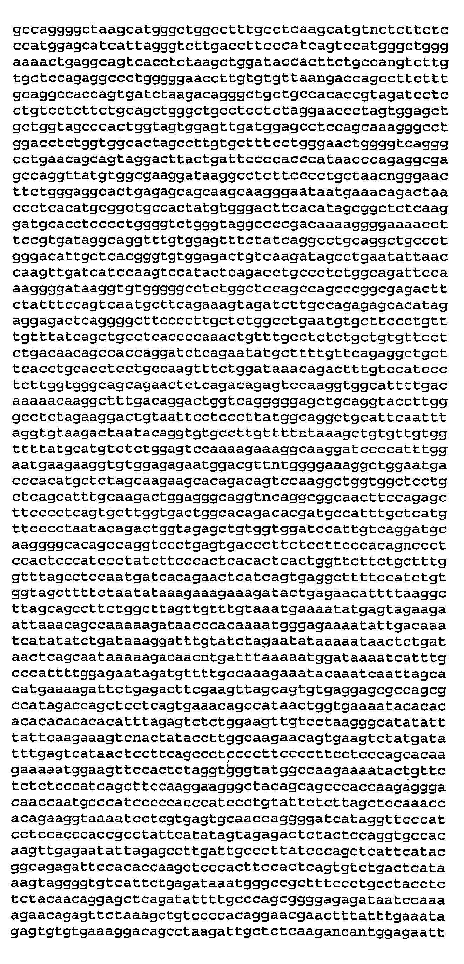

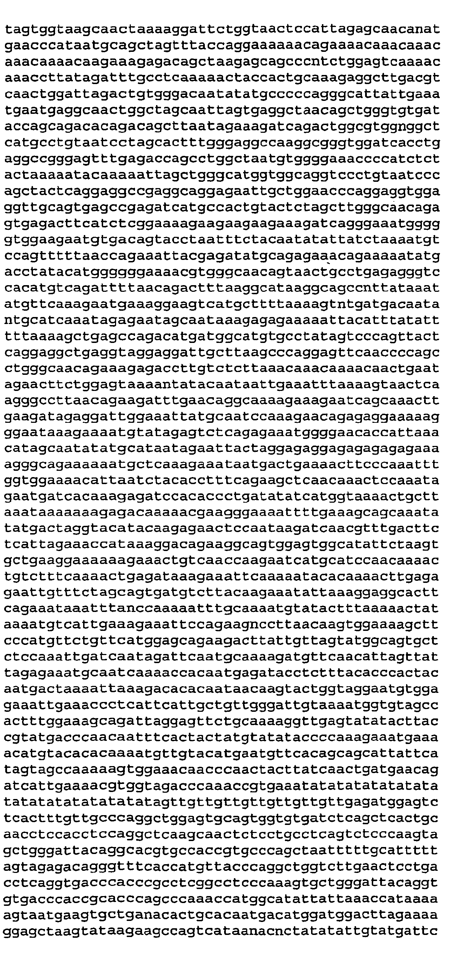

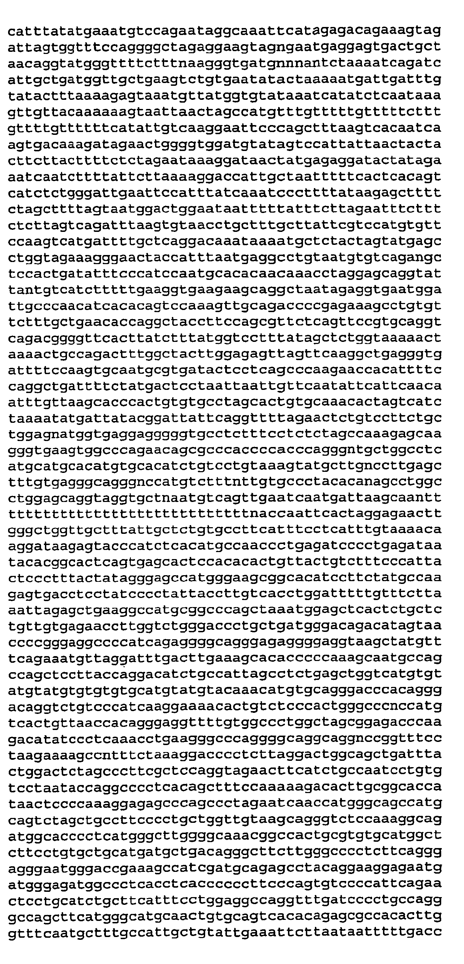

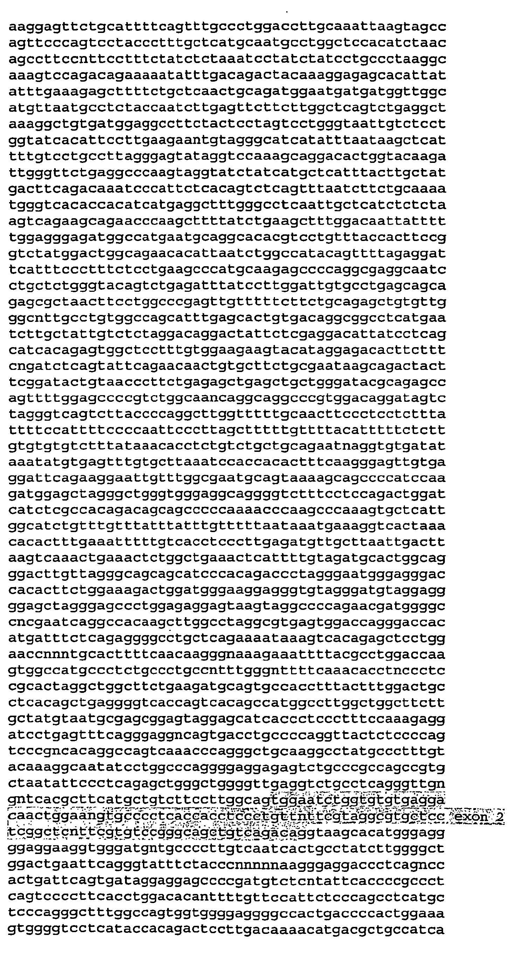

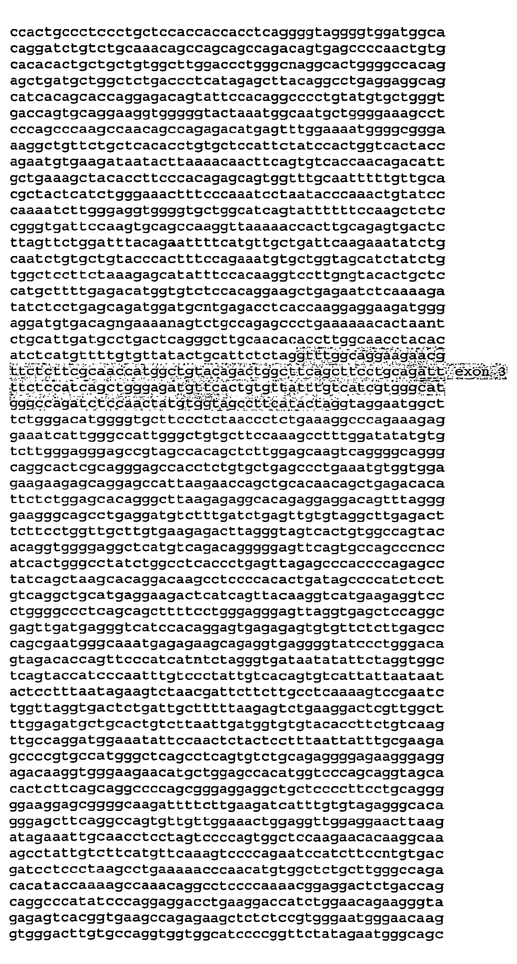

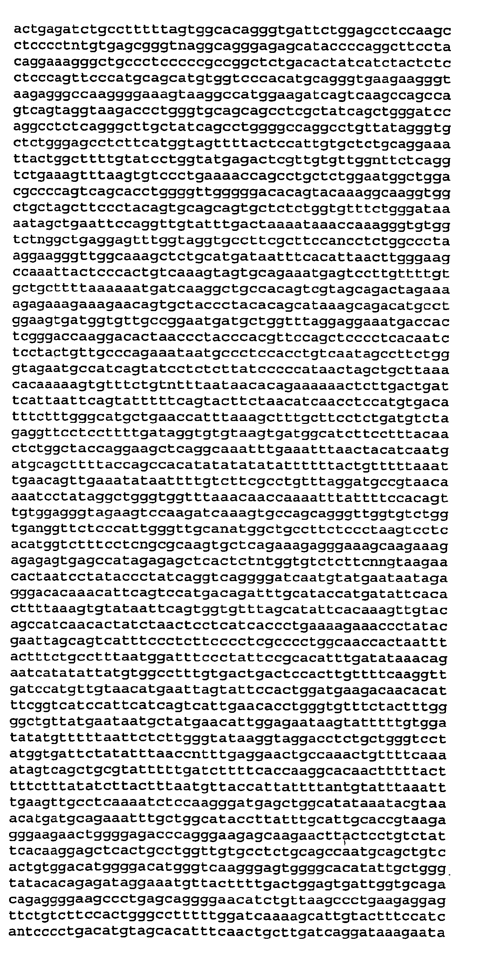

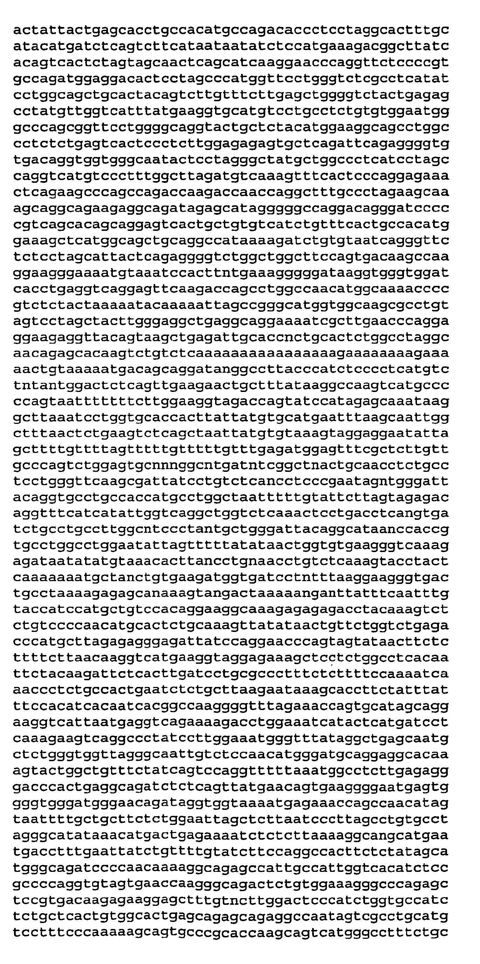

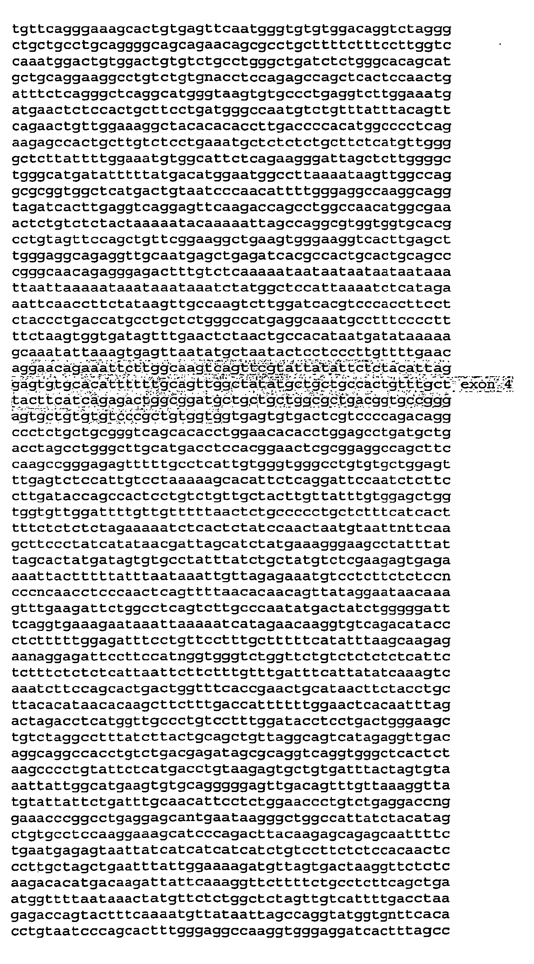

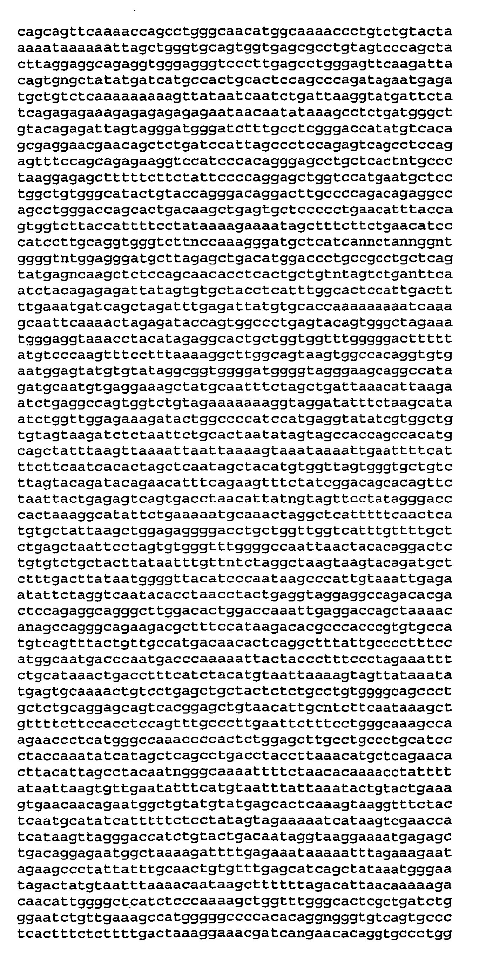

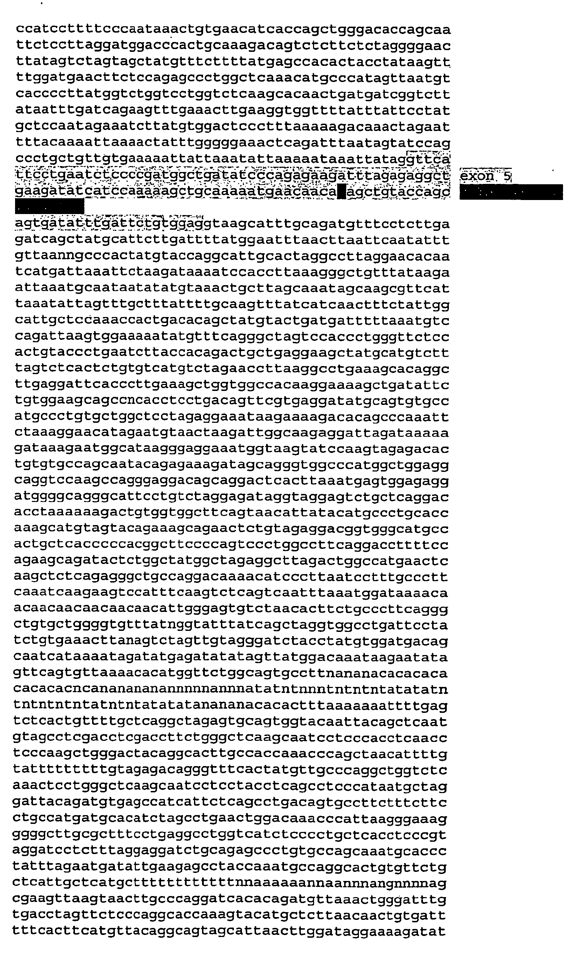

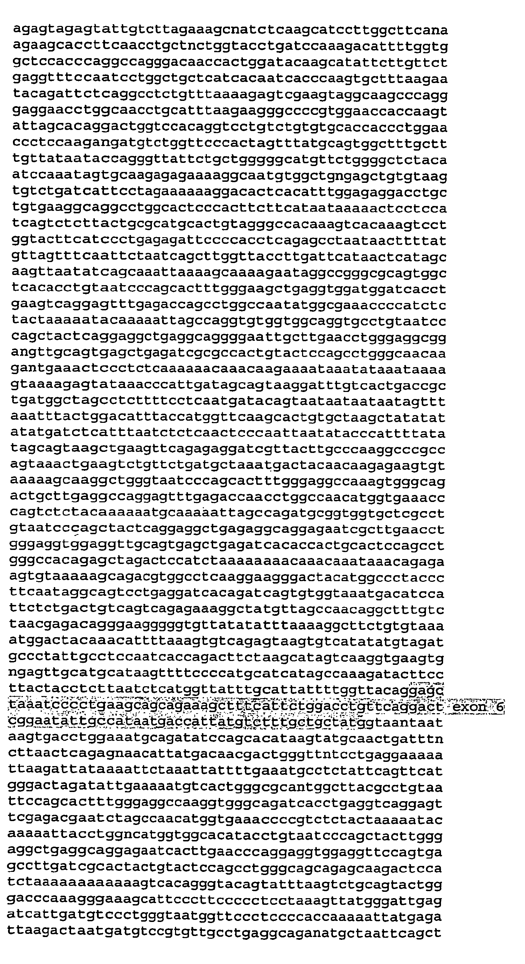

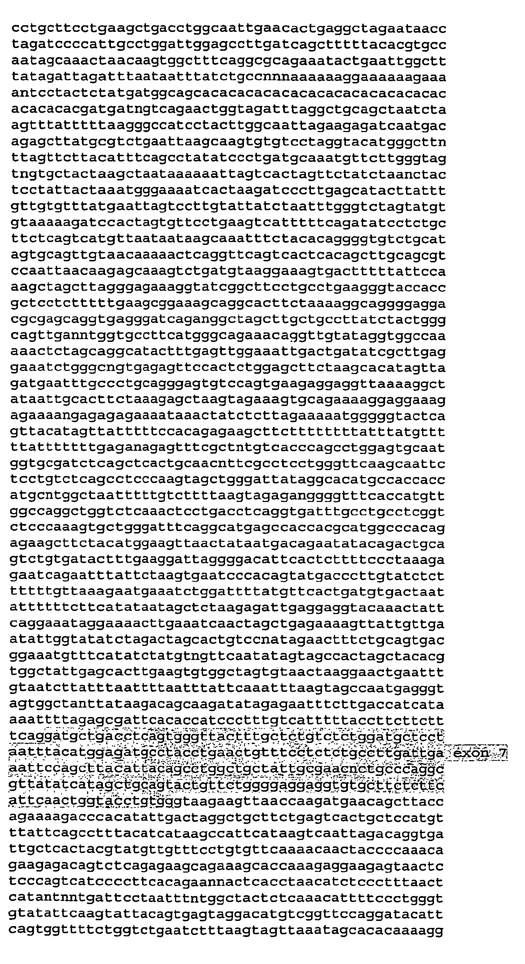

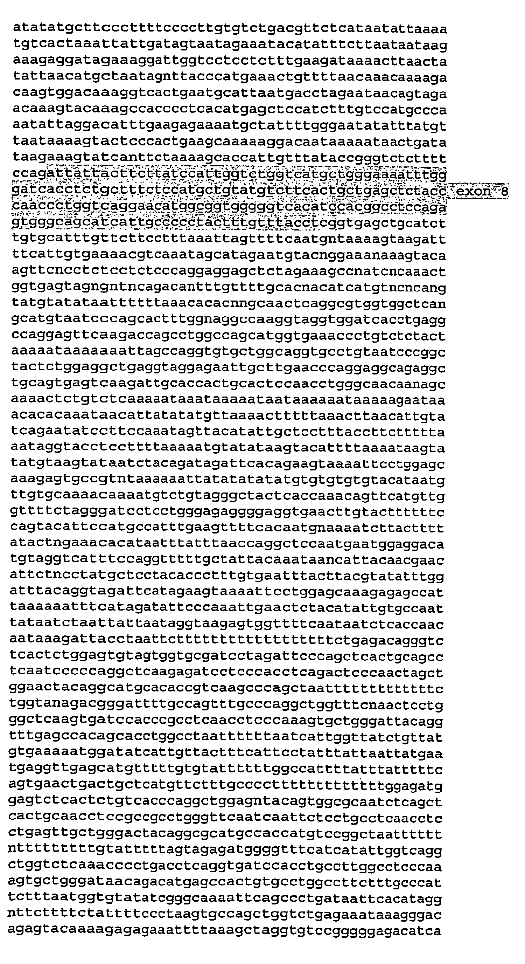

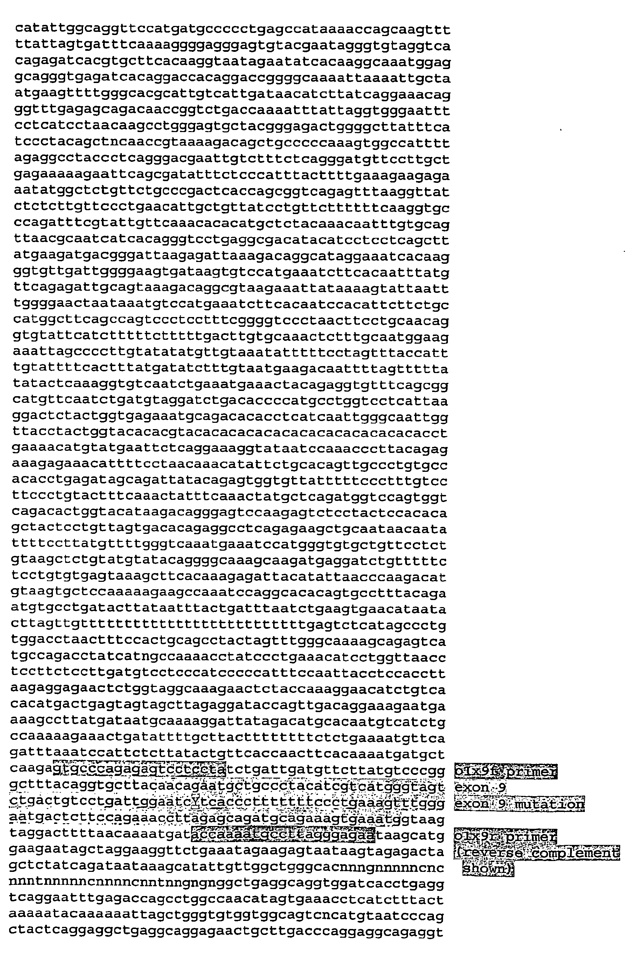

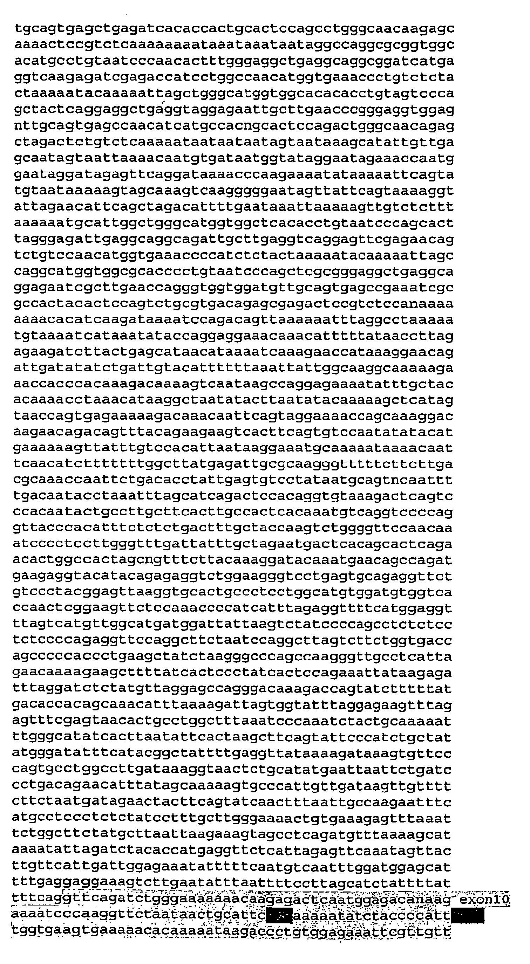

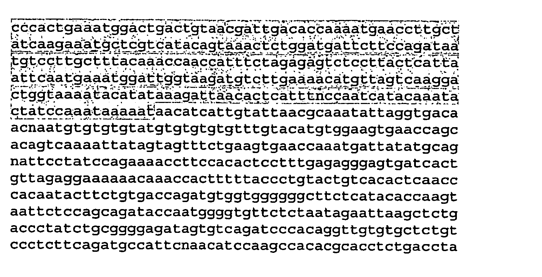

FIG. 8 shows the genomic DNA sequence including the OCTN1 gene. Locations of exons are highlighted in yellow. Locations of oligonucleotide primer sequences used are highlighted in green (note that the O1X9R primer is the reverse complement of the sequence as shown). Start and stop codons of the coding sequence are highlighted in red. The mutation within exon 9 of the gene is highlighted and is shown with standard DNA nomenclature for polymorphic bases, *i.e.* "Y" meaning C or T.

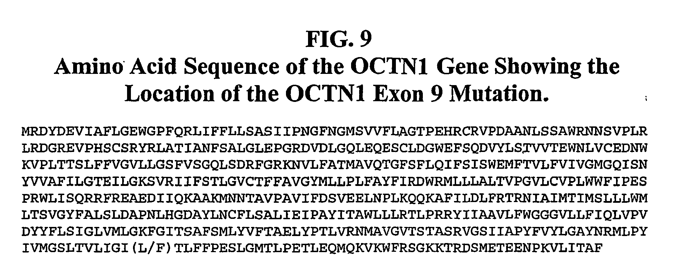

FIG. 9 shows the amino acid sequence as determined from known mRNA sequences for OCTN1. Each letter represents a single amino acid as defined by standard nomenclature. The location of the amino acid change caused by the OCTN1 exon 9 mutation is shown as "(L/F)", meaning that where the "C" allele is present in genomic DNA, Leucine ("L") is the corresponding amino acid in the OCTN1 protein, and where the "T" allele is present in genomic DNA, Phenylalanine ("F") is the corresponding amino acid in the OCTN1 protein.

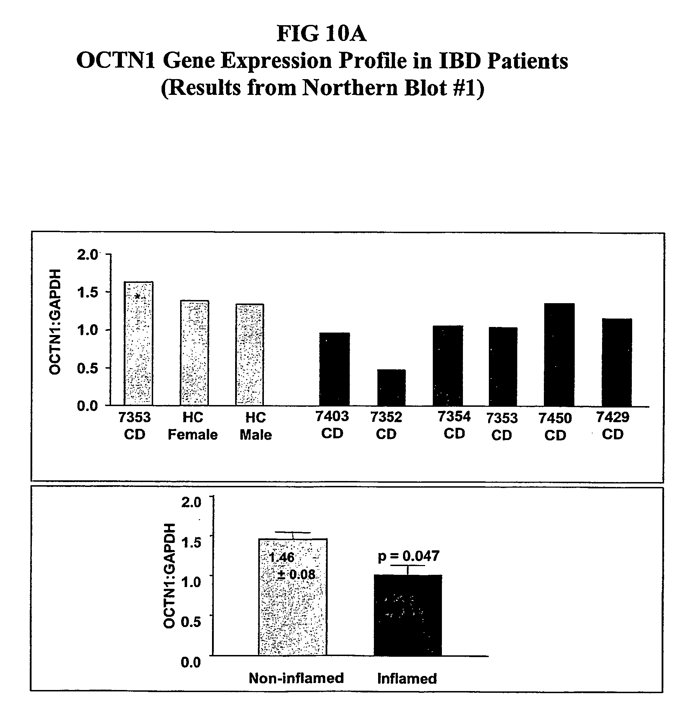

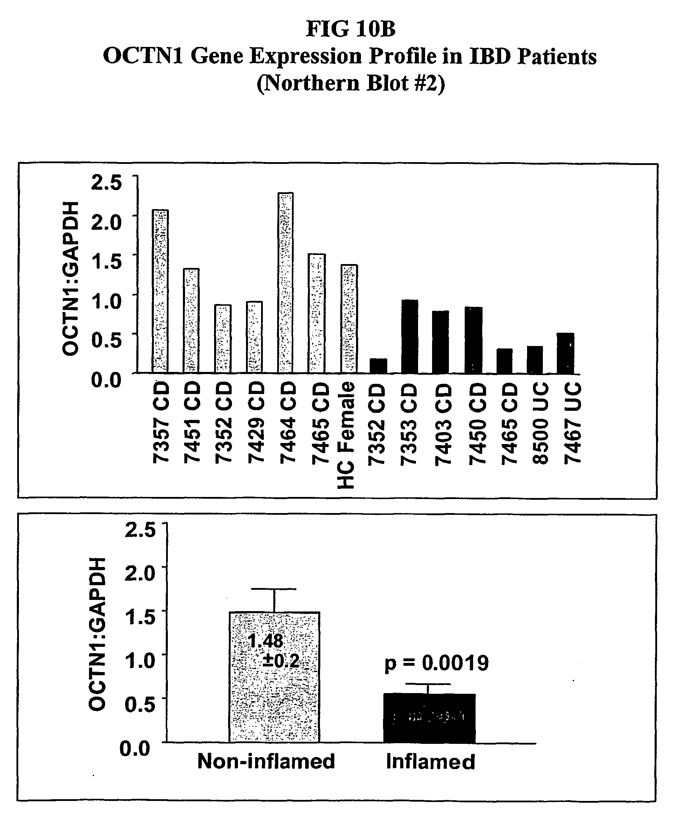

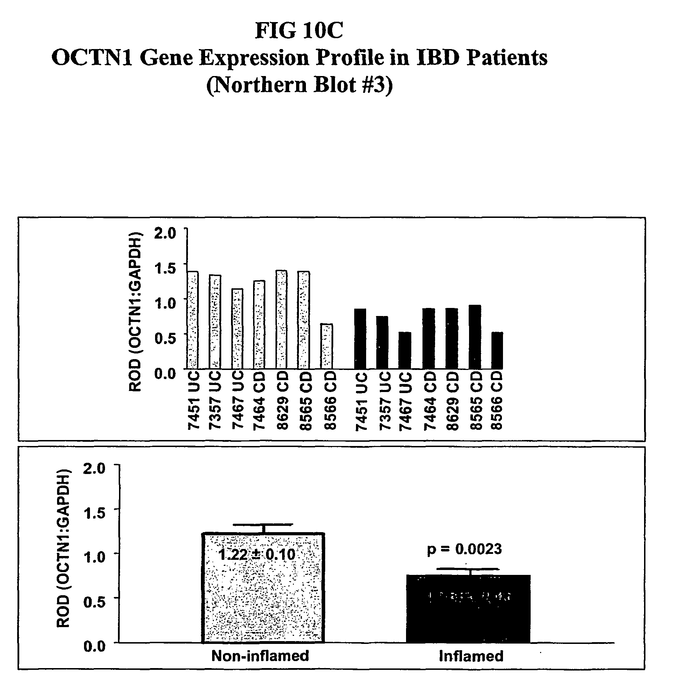

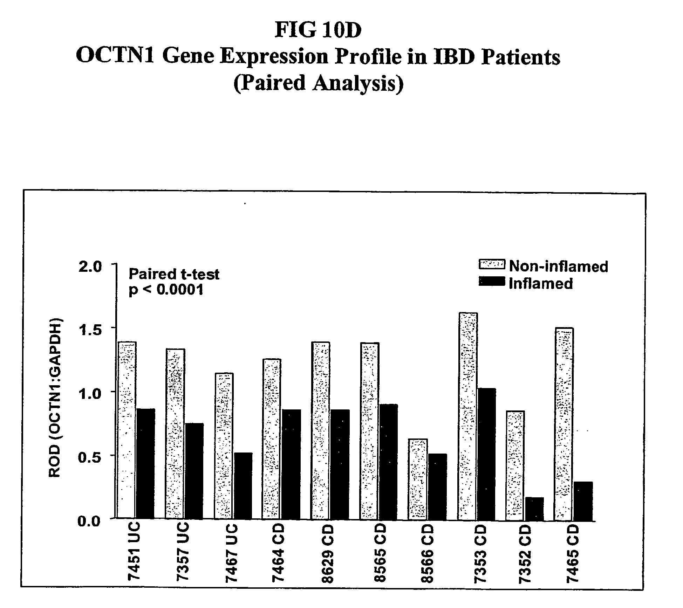

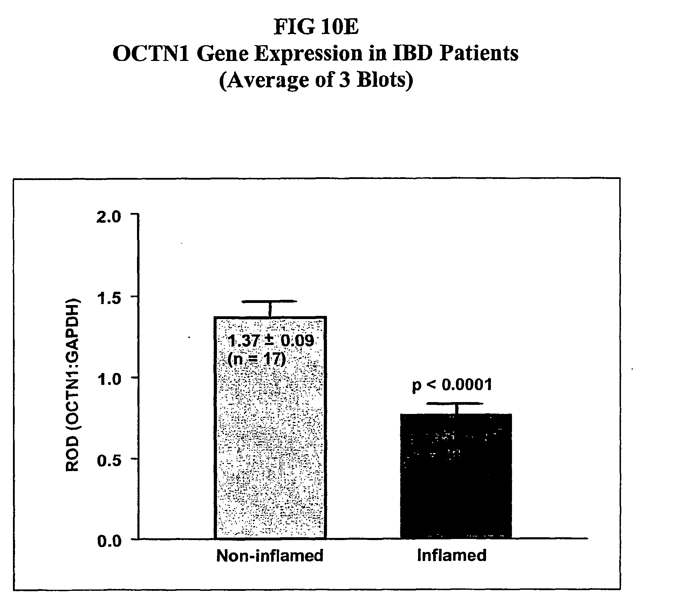

FIG. 10 shows in graphical form the expression level of the OCTN1 gene, expressed as relative optical density ("ROD") of autoradiographic signal from the OCTN1 gene as compared to the glyceraldehyde 3-phosphate dehydrogenase (GAPDH) gene. Blue bars represent expression level from RNA extracted from surgically removed non-inflamed GI tract tissue, and red bars represent expression level from RNA extracted from surgically removed inflamed GI tract tissue. Individual RNA samples are identified below the bars with a unique identifier number (*e.g.* 7403) and patient's diagnosis ("UC" for Ulcerative Colitis, "CD" for Crohn's Disease). Control unaffected samples are labeled as "HC male" and "HC female" and are from individuals without Inflammatory Bowel Disease. FIGS. 10A-C each represent data from a single "Northern blot" (*i.e*. DNA:RNA hybridization results); for each blot, individual sample results are presented in the top panel and averaged results for inflamed and non-inflamed samples are presented in the bottom panel. In the bottom panel, each bar is labeled with its mean value and standard error of the mean (SEM). Statistical p-values are also presented in the bottom panel. FIG. 10D shows individual sample results for pairs of inflamed and non-inflamed tissue samples from the same individual. Individual identifier numbers and diagnoses are indicated as in FIGS. 10A-C. FIG. 10E shows averaged results for all affected and unaffected samples. Individual identifier numbers and diagnoses, values and standard errors are indicated as in FIGS. 10A-C.

### DETAILED DESCRIPTION OF THE INVENTION

*Summary.* A functional analysis and in-depth mutation screening of candidate genes was performed in the 250 kb region of chromosome 5q31, described by Rioux JD *et al., Nature Genet.* 29, 223-228 (2001). Also examined were genes in the region for expression level differences in inflamed and non-inflamed gastrointestinal (GI) tract tissue from Inflammatory Bowel Disease patients. Two genes, *OCTN1* (SEQ ID NO:1) and *OCTN2* (SEQ ID NO:3), showed significant downregulation in inflamed Crohn's Disease tissue. A coding sequence mutation in the *OCTN1* gene that significantly reduces its ability to transport the organic cation carnitine and the prototypical organic cation substrate tetraethyl ammonium (TEA) was detected. Additionally, a mutation in the promoter region of *OCTN2* was found to downregulate both basal transcription and transcription induced either by heat shock or arachidonic acid. This transcription difference is apparently due to the disruption of a binding site for heat shock transcription factor 1 (HSF1).

*Mutation detection.* For mutation detection and analysis, bidirectional sequencing of polymerase chain reaction (PCR) amplified exons of the *OCTN1* and *OCTN2* genes was performed on ABI 377 or 3700 sequencers. The detected mutations were verified by visual inspection of both forward and reverse electropherograms. PCR primers for genomic DNA were designed based on the genomic sequences of human OCTN1 and OCTN2 reported in GenBank (loci AC008599 and AC004628 on chromosome 5q). Primers were ~60 bp upstream or downstream of each exon, allowing sequencing of splice donor and splice acceptor sequences and of the lariat branch site. In the case of the *OCTN2* promoter region, PCR amplification of 1.3 kb fragment using PCR primers, two primers, OCTN2 Promoter Forward (O2PF): 5'-CTGCACGGAAATAACTAATCTGTG-3' (SEQ ID NO:5) and OCTN2 Promoter Reverse (O2PR): 5'-GAGAGGAGCTCGGGTTCAAG-3' (SEQ ID NO:6) were used. PCR was performed using PCRx Enhancer System (Invitrogen, La Jolla, Calif., USA) with addition of 1x final PCRx Enhancer solution concentration, to overcome the GC-rich nature of this region, and with Platinum Taq DNA Polymerase High Fidelity (Invitrogen, La Jolla, Calif., USA). The PCR protocol includes 30 cycles of 94°C (for 40 sec), 54°C (for 1 min), and 72°C (for 2 min) with a 5-min extension at 98°C at the first cycle and a 10-min extension at 72°C of the final cycle.

SNP risk haplotypes were determined from data previously described SNP by Rioux JD *et al., Nature Genet.* 29: 223-228 (2001), and *OCTN1* and *OCTN2* mutation data were phased with SNP haplotypes by visual inspection of pedigrees. Significance levels for allele frequency differences between groups (*see,* TABLE 1, below) were calculated with a two by two chi-squared contingency table. Relative risk calculations (formally, odds ratios) were calculated as described by Bland JM & Altman DG, *BMJ* 320: 1468 (2000).

Mutations were initially detected by sequencing of exons, splice junctions and promoter regions of the *OCTN1* and *OCTN2* genes from a panel of 16 unrelated patients either heterozygous or homozygous for the Crohn's Disease SNP risk haplotype at 5q31.

Two mutations that co-segregated with the SNP risk haplotype were identified, a C to T change in exon 9 of *OCTN1* that changes the leucine residue at position 503 to phenylalanine (L503F; SEQ ID NO:7), and a G to C change 207 bp 5' of the start codon of *OCTN2* (G-207C; SEQ ID NO:8). The G-207C mutation alters a consensus heat shock transcription factor 1 (HSF1) binding site. Sequencing of other candidate genes in the region did not reveal any mutations consistently found on the risk haplotype background.

We then sequenced exon 9 of *OCTN1* and the promoter region of *OCTN2* in individuals from 46 families that were used for previous SNP analysis. Rioux JD *et al., Nature Genet.* 29, 223-228 (2001). Forty-one of these families segregate at least one copy of the SNP haplotype, and five segregate no copies of this haplotype. Examination of SNP haplotypes in unrelated children from these families revealed that the previously defined SNP risk haplotype always carried both the phenylalanine variant of L503F and the C allele of G-207C.

We next genotyped these two mutations in a panel of healthy controls, and in a panel of patients known to carry no copies of the SNP risk haplotype. The L503F (SEQ ID NO:7) and G-207C (SEQ ID NO:8) mutant alleles were significantly more common in unrelated patients from these families than in healthy controls, and were almost never found in patients homozygous for the nonrisk haplotype (only one L503F allele in 25 patients (50 chromosomes) and one G-207C allele in 16 patients (32 chromosomes). Allele frequencies in the control group did not deviate from Hardy-Weinberg equilibrium. Allele frequencies are presented in TABLE 1.

**TABLE 1**

| ALLELE FREQUENCIES OF *OCTN1* AND *OCTN2* MUTATIONS | | |
|---|---|---|
| Group | *OCTN1* L503F | *OCTN2* G-207C |
| healthy controls | F allele: 74 (48%) | C allele: 93 (50%) |
| | I allele: 80 (52%) | G allele: 93 (50%) |
| CD patients from risk haplotype families | F allele: 42 (78%) | C allele: 37 (77%) |
| | I allele: 12 (22%) | G allele: 11 (23%) |
| | p=0.0003 | p=0.0014 |
| CD patients without risk haplotype | F allele: 1 (0%) | C allele: 1 (3%) |
| | I allele: 49 (98%) | G allele: 31 (97%) |
| | p<0.0000001 | p=0.000002 |

Numbers of chromosomes are shown for each allele, and the significance level for difference from the healthy control group is shown for the two Crohn's Disease patient groups.

Odds ratios for Crohn's Disease susceptibility are presented in TABLE 2.

**TABLE 2**

| RELATIVE RISK FOR CROHN'S DISEASE | | |
|---|---|---|
| Mutation | One copy | Two copies |
| *OCTN1* L503F | 5.52 | 18.67 |
| | 95% C.I. (0.66 - 46.20) | 95% C.I. (2.25 -154.92) |
| *OCTN2* G-207C | 4.04 | 14.00 |
| | 95% C.I. (0.48 - 33.88) | 95% C.I. (1.69 -115.84) |

*Northern blot analysis.* Northern blot analysis of RNA from gastrointestinal tract tissue of Ulcerative Colitis and Crohn's Disease patients revealed a significant ' downregulation of both *OCTN1* and *OCTN2* in actively inflamed tissue as compared with uninflamed tissue. To control for variability in expression level between individuals, we also examined matched inflamed and non-inflamed tissue sample pairs from the same patient. The results showed that both *OCTN1* and *OCTN2* had significant downregulation in inflamed tissue. *See,* FIG. 1.

Surgical tissue samples were obtained from patients undergoing scheduled surgical resection for either Crohn's Disease or Ulcerative Colitis. Ethics approval and informed consent were obtained prior to sample collection. Samples were preserved in RNAlater (Ambion) prior to RNA extraction. An adjacent section of tissue was used for standard pathological examination to verify the diagnosis (Crohn's Disease, UC), region of the gastrointestinal tract, and status (inflamed, non-inflamed) of tissue. Control colon RNA from unaffected individuals was purchased from Clontech. Total RNA was extracted from tissue using TRIZOL (Gibco BRL). RNA samples (15 µg per lane) were separated in 1% agarose-3.7% formaldehyde denaturing gel, transferred onto MSI nylon transfer membrane (Osmonics Laboratory Products), and cross-linked by ultraviolet irradiation.

The membranes were hybridized with ³²P-labeled complementary probes for OCTN1 and OCTN2. OCTN1 transcripts were detected with a 0.8-kb *Not*I/*EcoR*I fragment of OCTN1 (ResGen) containing the 5' part of the coding region of the human OCTN1 gene. OCTN2 transcripts were detected with 2.2 kb *Not*I/*Sal*I fragment of OCTN1 (ResGen) containing the 3' part of the coding region of the human OCTN1 gene. Probes were radiolabeled with [α³²P]-dCTP using random priming (Roche Diagnostics GmbH). The unincorporated radiolabeled nucleotides were removed by ProbeQuant G-50 Micro column (Amersham Pharmacia Biotech). The Northern blots containing total RNA from human tissues were hybridized in QuickHyb hybridization solution (Stratagene, La Jolla, Calif., USA) at 68°C for 2 hrs. The blots were then washed twice in 2x SSC containing 0.1% SDS at room temperature for 15 min and once at 60°C for 30 min with 0.1x SSC containing 0.1% SDS.

Densitometry of Northern blots was performed using Scion Imaging Software (Scion Corporation), normalized to G3PDH, and expressed as relative optical density units. Data were analyzed with GraphPad Prism.

*Tissue expression analysis.* For expression analysis, RT-PCR of multiple tissue expression panels (Clontech) was performed according to the manufacturer's directions. First-strand cDNA preparations from human gastrointestinal (GI) tract and from human immune system, normalized against several housekeeping genes were used to assess tissue specificity and relative abundance of OCTN1 and OCTN2 mRNA. 5 µl (0.2 ng/µl) of each cDNA were used as a template for RT-PCR using gene-specific primers.

For OCTN1, the forward primer was 5'-ACCATCGCCAACTTCTCGGC-3' (SEQ ID NO:9) and the reverse primer was 5'-CTTTCTGCTGCTTCAGGGGA-3' (SEQ ID NO:10).

For OCTN2, the forward primer was 5'-CCATCGCCAACTTCTCGG-3' (SEQ ID NO:11) and the reverse primer was 5'-AATGTTGTGGGACTGCTGCTTC-3' (SEQ ID NO:12).

After 32 cycles, 5 µl sample of each PCR product was run on 2% agarose/ethidium bromide gel. G3PDH PCR primers and a control cDNA were used as a positive control.

RT-PCR expression analysis of multiple tissue panels from the gastrointestinal (GI) tract and immune system of healthy control individuals demonstrated widespread low levels of expression of *OCTN1* and *OCTN2,* with *OCTN1* highest in ascending colon and fetal liver, and *OCTN2* highest in colon and placenta. RT-PCR results are summarized in TABLE 3.

**TABLE 3**

| RT-PCR EXPRESSION ANALYSIS OF OCTN1 AND OCTN2 | | |
|---|---|---|
| Gene | GI tract panel | Immune system panel |
| *OCTN1* | low, highest in ascending colon | high in fetal liver |
| *OCTN2* | low, medium in colon | low, but high in placenta |

*Transient expression of OCTN1 (transporter assays) and OCT2 (luciferase promoter assays).* For transient expression of wild-type or mutant (L503F; SEQ ID NO:7), HeLa cells were plated out at a density 1.5 x 10⁶ - 10⁵ cells/dish in 100-mm plastic culture dishes (Falcon) and carnitine deficient human fibroblasts, (GM 10665) were plated out at a density 2.5 x 10⁵ cells/well in 6-well plastic culture dishes (Falcon) 24 hr prior transfection with LipofectAMINE PLUS^{™} Reagent (Invitrogen, La Jolla, Calif., USA) as recommended by the manufacturer.

For HeLa cell culture, human HeLa cells were grown in D-MEM supplemented with 10% fetal bovine serum, 2 mM L-glutamine, 100 U/ml penicillin, and 100 µg/ml streptomycin. For culture of carnitine deficient human fibroblasts from a patient with primary carnitine deficiency, described by Scaglia F. *et al., Genet. Med.* 1,34-39 (1998) (see description of the patient GM 10665 in National Institute of General Medical Sciences catalog) were obtained from the National Institute of General Medical Sciences Human Genetic Mutant ell Repository, Coriell Cell Repositories (Camden, New Jersey, USA). Fibroblasts were grown in D-MEM supplemented with 15 % fetal bovine serum, 2 mM L-glutamine, 100 U/ml penicillin, and 100 µg/ml streptomycin and 1x concentration of non-essential amino acids. Cells were cultured in 37°C humidified incubator with a mixture of 5% CO₂ and 95% air.

Forty-eight hr after transfection the media was removed and replaced with Earle's balanced salt solution containing D-glucose (5.5 mM) and supplemented with 0.5% BSA.

*For the OCTN1 transporter assays,* carnitine transport measurements were made at room temperature using L-[³H]carnitine as substrate, (specific activity 81 Ci/mmol, Moravek Biochemicals Inc.). The transport buffer was composed of 25 mM HEPES/Tris pH 7.5, supplemented with 140 mM NaCl, 5.4 mM KCl, 1.8 mM CaCl₂, 0.8 mM MgSO4, and 5 mM glucose. The transport medium containing the radiolabeled carnitine were preincubated at 37°C and then added to the cells. After incubation for 30 min at 37°C, transport was terminated by aspiration of the buffer followed by four washes with ice-cold transport buffer. The cells were then solubilized with 1x cell culture lysis reagent (Promega), and the associated radioactivity was quantitated in a liquid scintillation counter. Cellular protein content was determined according to the bicinchoninic acid method of Smith PK *et al., Anal. Biochem.* 150: 76-85 (1985), using BCA standard protein assay reagent kit (Pierce). To normalize for the transfection efficiency, cells were co-transfected with 20-fold less firefly luciferase plasmid pGL3-P vector. The luciferase activity was measured on manual luminometer (Lumat LB 9501, Berthold). The cells transfected with empty vector under similar conditions served as control. All assays were carried out in triplicate.

*For the OCTN2 luciferase promoter assays,* a Dual-Luciferase Reporter Assay System (Promega) was used to study the effect of the mutation on the OCTN2 promoter region. The OCTN2-promoter luciferase reporter gene constructs were generated by PCR amplification of approximately 2.7 kb OCTN2-promoter fragments using genomic DNA from a patient homozygous for the G allele as a template and ligation into *Bgl*II/*Mlu*I site of the promoterless luciferase vector pRL-null (Promega) that uses luciferase from *Renilla reniformis* as a reporter. For PCR primers, two primers, OCTN2-BglII: 5'-GAAGATCTTGGAAGGCTCAGGTGGGAGG-3' (SEQ ID NO:13) and OCTN2-MluI: 5'-CGACGCGTGGCAGCAGGCGACCCAAGAC-3' (SEQ ID NO:14) were used. PCR was performed using PCRx Enhancer System (Invitrogen, La Jolla, Calif., USA) with addition of 1x final PCRx Enhancer solution concentration and with Platinum Taq DNA Polymerase High Fidelity (Invitrogen, La Jolla, Calif., USA), according to a standard protocol with 30 cycles of 94°C (for 40 sec), 61°C (for 1 min), and 72°C (for 3 min) with a 5-min extension at 98°C at the first cycle and a 10-min extension at 72°C of the final cycle.

The C mutant construct was then created by replacing the 600 bp fragment encompassing the position -207 by digesting it with *EcoN*I/*Nde*I*,* followed by ligation of the corresponding fragment amplified from a patient homozygous for the C allele.

Constructs were fully sequenced to exclude the possibility of PCR induced errors:

To normalize the activity of the experimental reporter, constructs were cotransfected into HeLa cells with 20-fold less firefly luciferase plasmid pGL3-P as an internal control LipofectAMINE PLUS^{™} Reagent (Invitrogen, La Jolla, Calif., USA) as per the manufacturer's protocol. 24 hr after transfection, HeLa cells that were undergoing arachidonate treatment were starved for 6 hr in serum-free medium. After starvation, cells were treated at 37°C with 20 µM arachidonate for 30 min, followed by 2x wash with PBS. Control cells were treated with an equivalent concentration of ethanol. After that the cells were placed in incubator for additional 18 hr in complete media. Cells that were heat shocked 24 hr after transfection were incubated at 42°C for 2 hr. Cells were then placed back in 37°C-incubator for additional 18 hr. The luminescence from (*Renilla* luciferase reaction ("experimental" reporter *a*:) was measured simultaneously with the activity of firefly luciferase ("control" reporter) using Dual-Luciferase Reporter Assay System (Promega). The instrumentation used was a manual luminometer (Lumat LB 9501, Berthold). The cells transfected with empty vector under similar conditions served as control. All assays were carried out in triplicate.

Transfection of the luciferase construct with the C allele of the G-207C mutation of a 2.7kb fragment of the *OCTN2* promoter into HeLa cells resulted in an average 2.8 fold decrease in basal expression as compared with the wild-type (G) allele. Transfection after heat-shock at 42°C resulted in 3.1 fold reduction of heat shock-induced expression. Transfection after treatment with 20 µM of arachidonic acid at 37°C also resulted in decreased expression of 3.3 fold as compared to the arachidonate-induced level. Results are presented in FIG. 2.

*For CTN2 gel shift assays,* human HeLa cells were grown in D-MEM supplemented with 10% fetal bovine serum, 2 mm L-glutamate, 100 U/ml penicillin, and 100 µg/ml streptomycin at 37°C in T-75 tissue culture flasks (Sarstedt). Heat-shocked cells were prepared by incubating the flasks in tissue culture incubator at 42°C for 3 hr. For arachidonate experiment, the day before, the HeLa cells were starved over night with in serum-free D-MEM. The next day cells were washed twice with PBS. Cells were treated then with for 30 min with 20 mM arachidonate at 37°C. The working concentrations were prepared from sodium arachidonate (Sigma) and stored in cold absolute ethanol as a 100 mM stock solution. Control cells were treated with an equivalent concentration of ethanol. Nuclear extracts were prepared by NP-40 based fractionation. This procedure is a modification of the procedure described by Baler R, Dahl G & Voellmy R, *Mol. Cell. Biol.* 13: 2486-2496 (1993). Briefly, 2.5 x 10⁶ cells were pelleted, washed twice in 1x PBS and resuspended in 1 ml low salt buffer (A) containing 10 mM Tris HCl, pH 7.5, 10 mM NaCl, 3 mM MgCl₂, 0.5% Nonidet P-40 (NP-40), 0.5 mM phenylmethylsulfonyl fluoride (PMSF). The nuclei were pellet by centrifugation at 7,000g for 10 min at 4°C. The supernatant was removed and the pellet (the crude nuclei) was suspended in 50 µl high salt buffer (B), containing 20 mM HEPES, pH 7.5, 5mM MgCl₂, 0.2 mM EDTA, 1 mM dithiothreitol (DTT), 20% glycerol, 300 mM NaCl, 0.5 mM PMSF. After incubation for 15 min at 4°C the sample was sonicated for 5 sec, centrifuged at 20,000g for 10 min and the resulting extracts was the nuclear fraction. The nuclear extracts were stored at -80°C. The protein concentration of the extracts was measured using the Bradford assay (Bio-Rad).

For gel mobility-shift assays (EMSA), the binding reactions were carried out in a 20 µl volume containing 10 µg of nuclear extract, 0.5 ng ³²P-labeled oligonucleotide probe, 1 µg of poly (dI-dC) and 10 µg of BSA, in 10 mM Tris HCl, pH 7.5, 50 mM NaCl, 1 mM DTT, 1 mM EDTA, 5% glycerol for 2 hr on ice. The antibodies supershift experiments were performed by incubating 2 µl of HSF1 and HSF2 antibodies (TransCruzTM Gel Supershift Antibodies), prior addition of the labeled probes. For the competition experiments a 100-fold molar excess of the unlabeled oligonucleotides was added to each reaction. The competitors, used as positive controls were as follows: WT-OCTN2 (P) Antisense: 5'-CAGGCCCGGAACCTTCCCTGGTCGT-3' (SEQ ID NO:15); WT-OCTN2 (P) Sense: 5'-ACGACCAGGGAAGGTTCCGGGCCTG-3' (SEQ ID NO:16); Mutant OCTN2 (P) Antisense: 5'-CAGGCCCGCAACCTTCCCTGGTCGT-3' (SEQ ID NO:17); and Mutant OCTN2 (P) Sense: 5'-ACGACCAGGGAAGGTTGCGGGCCTG-3' (SEQ ID NO:18).

The design of the oligonucleotides for the typical HSE for human HSP70 were as follows: Sense 5'-TCGGCTG**GAATATTC**CCGACCTGGCAGCCGA-3' (SEQ ID NO:19) and Antisense 5'-TCGGCTGCCAGGTCGG**GAATATTC**CAGCCGA-3' (SEQ ID NO:20).

The reaction products were separated electrophoretically on 4% polyacrylamide gel for 3 h at 200 V, dried and exposed to film and detected by autoradiography.

Gel shift assays of the region surrounding the OCTN2 G-207C promoter mutation demonstrated a specific interaction between a nuclear factor and an oligonucleotide containing the region surrounding the wild-type G allele. Binding to this oligo was specific, in that it could be competed by excess wild-type oligo but not by an oligo containing the mutant C allele, nor by a randomly chosen control oligo unrelated to the *OCTN2* promoter. Binding of this factor was induced after incubation at 42°C and arachidonic acid, but not at 37°C, and was identical in mobility to a factor binding to an oligo corresponding to the heat stress element (HSE) from the *HSP70* gene. Binding to the G allele of the *OCTN2* promoter was also competed by excess *HSP70* HSE oligo, further suggesting that the factor was a heat shock transcription factor. Since the G-207C mutation alters a consensus HSF1 binding site, we hypothesized that this factor was HSF1. Anti-HSF1, but not Anti-HSF2, antibody supershifted the complex, positively identifying the unknown factor as HSF1. HSF1 binding to the *OCTN2* promoter was completely abolished by the presence of the C allele under all tested conditions.

*OCTN1 transporter assays.* For OCTN1 transport assays, full length *OCTN1* cDNA (2.24 kb) was obtained as a full-length genoscope clone ID CSODJ 003YB03 (ResGen, Invitrogen, La Jolla, Calif., USA). The cDNA clone was subcloned into *EcoR*I/*Not*I sites of the pcDNA3 vector. The construct was fully sequenced and found to contain the leucine allele at position 503 (L503). The construct was used as a template for PCR-based site-directed mutagenesis (QuikChange^{™}, Stratagene, La Jolla, Calif., USA) to create the phenylalanine allele (L503F). Parental DNA strands were removed by *Dpn*I digestion, and the nicked circular DNA was used to transform XL-10 Gold supercompetent cells (Stratagene, La Jolla, Calif., USA).

The mutagenic primers used to prepare the L503F mutant were as follows: 5'-GACTGTCCTGATTGGAATCTTCACCCTTTTTTTCCCTGA-3' (forward) (SEQ ID NO:21) and 5'-TCAGGGAAAAAAAGGGTGAAGATTCCAATCAGGACAGTC-3' (reverse) (SEQ ID NO:22).

The phenylalanine allele of the L503F mutation resulted in a 3.6 fold reduction toward the vector-alone baseline in uptake of radiolabeled carnitine after transient transfection into HeLa cells (FIG. 3A), as compared with an identical construct containing the leucine allele. In a cell line from an individual lacking OCTN2 expression, the OCTN1 L503F mutation resulted in a 5.2-fold reduction toward vector baseline in uptake of radiolabeled carnitine as compared with the leucine allele (FIG. 3B).

*TEA efflux assays:* Measurement of efflux of radiolabeled TEA demonstrated that there was a significant decrease in efflux by the CD-associated phenylalanine (L503F) variant of OCTN1 as compared with the leucine variant (L503). *See,* FIG. 4.

Transfected Phoenix mouse fibroblast cells were loaded with ¹⁴C-labeled TEA for 30 min. at 37°C, washed twice in cold transport buffer, and then resuspended in transport medium. Efflux was initiated at 37°C and measured at 0, 4, 8, 12 and 30 minutes. Measurements were taken by washing twice in cold transport buffer, solubilizing with 1x cell culture lysis reagent (Promega) and quantifying the 14C retention from 150 µL aliquots. Cellular protein content was determined according to the BCA method. For sodium-free experiments, cells were suspended in medium with sodium replaced isotonically with N-methyl-D-glucamine.

*Discussion.* The two mutations described here co-segregate with the previously described SNP haplotype, are not found on non-risk haplotypes, and contribute to approximately the same degree of genetic susceptibility to Crohn's Disease (TABLE 2). Apparently, Rioux JD *et al., Nature Genet.* 29: 223-228 (2001) slightly underestimated the genetic risk (*i.e*., 2-fold genome relative risk for one SNP haplotype, 6-fold for two), likely because the allele frequency of the risk haplotype was previously estimated from non-transmitted parental chromosomes and not from an independent control set, as we have done here.

The mutation causing the L503F change in OCTN1 has been previously identified as a SNP of unknown function (dbSNP identifier rs1050152), although no allele frequency or functional data was previously available. The L503F mutation is common in the general population. This is likely why the phenylalanine variant (which we show here to be associated with Crohn's Disease susceptibility) was initially reported as the wild-type form of the transporter. Tamai I *et al., FEBS Lett.* 419, 107-111 (1997). Prior functional assays of the OCTN1 transporter have been performed using the phenylalanine variant, without consideration of other variants at this position, as shown herein.

For wild-type variants, the amino acid corresponding to position 503 of OCTN1 is either leucine, isoleucine, methionine or valine in all other known organic cation transporters from mouse, rat or human. Burckhardt G & Wolff NA, *Am. J. Physiol. Renal. Physiol.* 278, F853-F866 (2000). A change to a bulky phenylalanine residue might have a structural effect on the protein. This residue is located in transmembrane domain 11 of OCTN1, in a region previously shown to be involved in carnitine transport. Our transporter assay results show that the leucine variant of OCTN1 is significantly better at transporting carnitine than the phenylalanine variant. This result disagrees with previous functional analysis of OCTN1, which used the phenylalanine variant (Tamai I *et al., FEBS Lett.* 419, 107-111 (1997)) and which suggested that the *in vivo* function of OCTN1 may actually be as a carnitine transporter rather than simply as a polyspecific cation transporter.

In respect of *OCTN2,* these data shows that basal transcription is downregulated by the C allele of G-207C, and that this allele disrupts the ability of *OCTN2* to be upregulated in response to heat shock or arachidonic acid as a result of impaired HSF1 binding and subsequent transcriptional activation.

The mechanism by which these organic cation transporters contribute to the pathology of Crohn's Disease relate to the *in vivo* metabolic importance of carnitine. Carnitine facilitates transport of long chain fatty acids across the mitochondrial inner membrane for subsequent β-oxidation, and is also important in the maintenance of cellular CoA levels. Carnitine uptake into lymphocytes, along with a corresponding decrease in plasma levels, is a physiological response to inflammation. Symptoms of the related condition Ulcerative Colitis may be due to an energy deficiency in colonic epithelium secondary to poor mitochondrial function due to decreased long-chain fatty acid transport to the mitochondria. Roediger WE, *Lancet* 2*,* 712-715 (1980). The haplotype of mutations in Crohn's Disease patients provided by this invention might affect cellular metabolic energy levels in inflamed tissue by combining impaired OCTN1 transporter function with downregulation and inability to respond to heat or inflammatory stress by the *OCTN2* gene.

Heat shock proteins and arachidonic acids are involved in response to a variety of cellular stresses, including sepsis, metabolic stress and ischaemia. The heat shock response modulates inflammation through modulation of NF-κB activation. OCTN2 has not previously been described as a heat stress inducible protein, but from the results of this invention OCTN2 is a heat stress inducible protein. Thus, the *OCTN2* gene is normally upregulated in response to inflammation through binding of HSF1 protein to its promoter. This in turn mobilizes carnitine and bolster metabolism in the inflamed tissue. Impaired OCTN1 carnitine transporter activity and lowered *OCTN2* expression level results in reduced metabolism and either trigger or worsen cellular stress in areas of inflammation.

The two OCTN transporters may therefore function in the inflammatory pathology of Crohn's Disease. Intriguingly, there are reports that topical irrigation of the colon with propyonil-L-carnitine (PLC) can improve some symptoms of Ulcerative Colitis (Giancaterini A *et al., Am. J. Gastroenterol.* 96, 2275-2276 (2001)), and PLC also inhibits inflammation in various models of vascular inflammation in rodents. Caruso A *et al., Pharmacol. Res.* 31, 67-72 (1995); Amico-Roxas M *et al., Drugs Exp. Clin. Res.* 19, 213-217 (1993). Additionally, OCTN1 is a polyspecific cation transporter, and might have a role in the uptake of drugs used to treat CD from the gut. Mutations such as L503F might therefore serve to worsen the condition by reducing bioavailability of therapeutic compounds, or by decreasing the ability of the cell to remove by efflux toxic compounds such as free radicals, toxic metabolites or bacterial toxins.

*Mutations of the OCTN2 gene and uses in diagnosing inflammation.* As described above, OCTN2 is a transporter protein with the ability to transport carnitine in a sodium-dependent manner. Missense mutations and nonsense mutations in the organic cation transporter OCTN2 had previously been identified in patients with primary Systemic Carnitine Deficiency (SCD; (OMIM 212 140)), an autosomal recessive disorder characterized by progressive cardiomyopathy, skeletal myopathy, hypoglycemia and hyperammonemia. By contrast, the invention provides polynucleotide polymorphisms in the OCTN2 gene upstream from the start codon in exon 1 (*see,* FIG. 6), which are shown here to be related to inflammatory bowel diseases (IBDs), Crohn's disease (CD) and ulcerative colitis (UC). The G-207C mutation of *OCTN2,* identified above, although it reduces basal transcription level also, is most relevant to transcription induced by heat stress or inflammation. In combination with the accompanying *OCTN1* mutation, this haplotype of mutations results in susceptibility to Crohn's Disease.

The invention provides polynucleotide polymorphisms as shown in the following SEQ. ID. NO:23:

The invention also provides other polynucleotide having sequences that are fragments of SEQ ID NO:23. For example, the fragments can have sequences that that are 20 bases long (or multiples thereof).

Moreover, the invention provides uses for polynucleotides containing the 410 mutation (for example, taagccga(c/g)cccgggcta, SEQ ID NO:24). Alternatively, the invention provides uses for polynucleotides containing the G-207C mutation (for example, ccaggcccg(c/g)aaccttccc, SEQ ID NO:25). Other polynucleotides provided by the invention include cggcggtgtcagctc(t/g)cgagcctaccctccgc (SEQ ID NO:26); ggacggtcttgggtc(t/g)cctgctgcctggcttg (SEQ ID NO:27); and cctggtcggcgg(cg/ta)ggtgccccgcgcgcacgc ("TA"; SEQ ID NO:28). Other polynucleotides can be determined from observation of FIG. 6.

*OCTN2 Promoter Region.* TABLE 2 shows the allele frequencies of the OCTN2 G-207C promoter region mutation. Data on G-207C polymorphism allele frequencies had not previously been obtained in any populations (healthy or diseased).

Alleles are listed in TABLE 4 by number (1 or 2) and the DNA nucleotide present at that position (C or G). The number of chromosomes carrying each allele is shown for either unrelated healthy control individuals ("healthy" column) or Crohn's Disease patients from families with genetic risk conferred by the chromosome 5 locus previously described ("CD" column). The frequency of each allele as a percentage of the total is shown in brackets, and the total number of chromosomes ("n") is shown at the bottom of each column. The results of a 2 by 2 contingency table chi-squared test are presented at the bottom of TABLE 4, demonstrating a significant difference in allele frequency between the "healthy" and "CD" classes (chi-squared statistic of 10.2640, p value with one degree of freedom 0.0014).

**TABLE 4**

| ALLELE FREQUENCY DATA FOR OCTN2 G-207C MUTATION | | |
|---|---|---|
| allele | healthy | CD |
| 1 (C) | 93 (50%) | 37 (77%) |
| 2 (G) | 93 (50%) | 11 (23%) |
| | n=186 | n=48 |
| | | chi-squared =10.2640 |
| | | p (1df) =0.0014 |

TABLE 5 shows allele frequency data for three polymorphisms of the OCTN2 gene (specifically, the 410, G-207C and "TA" polymorphisms). Allele frequencies are expressed as the number of chromosomes carrying a specific allele followed by the percentage of total chromosomes ("n") carrying that allele (*e.g.*, for the G-207C mutation in healthy controls, 52 of 110 chromosomes (*i.e.,* 47%) carry the "C" allele). The populations studied are as follows: *healthy controls ("healthy"):* DNA samples from venous blood of individuals with no history of IBD; *RA sibs ("RA"):* DNA samples from venous blood of healthy siblings of patients with rheumatoid arthritis but no history of IBD; *Wegener's Granulomatosis ("WG"):* DNA samples from venous blood of patients with Wegener's Granulomatosis but no history of IBD; *surgical cancer*/*polyp ("cancer"):* DNA samples from venous blood of patients undergoing surgery for either colon cancer or colonic polyps but no history of IBD; *parents of chr(5) patients:* DNA samples from venous blood or cultured white blood cells of parents of CD patients from families showing linkage to chromosome 5; *unrelated chr(5) Crohn's Disease (CD) patients ("chr(5) CD"):* DNA samples from venous blood or cultured white blood cells of unrelated patients from families showing linkage to chromosome 5; *surgical CD:* DNA samples from venous blood of patients undergoing surgical removal of bowel tissue as a result of CD; and *surgical UC:* DNA samples from venous blood of patients undergoing surgical removal of colon tissue as a result of Ulcerative Colitis (UC).

**TABLE 5**

| ALLELE FREQUENCIES OF OCTN2 POLYMORPHISMS IN VARIOUS POPULATIONS | | | | | |
|---|---|---|---|---|---|
| | | | CONTROL SAMPLES | | |
| mutation | allele | healthy controls | R.A sibs | Wegener's Granulomatosis | surgical cancer / polyp |
| G-207C | 1 (C) | 52 (47%) | 8 (44%) | 19 (41%) | 14 (54%) |
| | 2 (G) | 58 (53%) | 10 (56%) | 27 (59%) | 12 (46%) |
| | | n=110 | n=18 | n=46 | n=26 |
| 410 | 1 (C) | 34 (31%) | 3 (21%) | 12 (33%) | 5 (23%) |
| | 2 (G) | 76 (69%) | 11 (79%) | 24 (67%) | 17 (77%) |
| | | n=110 | n=14 | n=36 | n=22 |
| TA | 1 (CG) | 105 (94%) | 18 (100)% | 42 (96%) | 19 (79%) |
| | 2 (TA) | 7 (6%) | 0 (0%) | 2 (4%) | 5 (21%) |
| | | n=112 | n=18 | n=44 | n=24 |

| | | CD FAMILY SAMPLES | | SURGICAL SAMPLES | |
|---|---|---|---|---|---|
| mutation | allele | parents of chr(5) patients | unrelated chr(5) CD patients | surgical CD | surgical UC |
| G-207C | 1 (C) | 27 (56%) | 35 (76%) | 13 (54%) | 13 (41%) |
| | 2 (G) | 21 (44%) | 11 (24%) | 11 (46%) | 19 (59%) |
| | | n=48 | n=46 | n=24 | n=32 |
| 410 | 1 (C) | 10 (21%) | 7 (29%) | 11 (42%) | 14 (47%) |
| | 2 (G) | 38 (79%) | 17 (71%) | 15 (58%) | 16 (53%) |
| | | n=48 | n=24 | n=26 | n=30 |
| TA | 1 (CG) | 2 (100%) | 18 (90%) | 15 (94%) | 25 (89%) |
| | 2 (TA) | 0 (0%) | 2 (10%) | 1 (6%) | 3 (11%) |
| | | n=2 | n=20 | n=16 6 | n=28 |

TABLE 6 shows statistical chi-squared analysis of allele frequencies in the populations as described in TABLE 5. The number of alleles present in these populations is as in TABLE 5. For each test, results are shown for the 410, G-207C and "TA" polymorphisms. Compared populations are indicated along the top of each 2 by 2 contingency table (*e.g.* "healthy", "c5 patients"), individual alleles are listed to the left and the total number of counted alleles for each polymorphism is shown below the table (*e.g.* 110 for the G-207C polymorphism in the first such table). To the right of each table ("stats" column) are listed the chi-squared value (top) and the associated p value based on the one-tailed chi-squared distribution with one degree of freedom (*e.g*. chi-squared for the first such table is 9.7806, and the associated p value is 0.0018). Some populations are grouped together for these analyses. Where this has been done the nomenclature of TABLE 5 has been followed except for the following: "all CD" means "chr(5) CD and surgical CD combined"; and "all IBD" means "chr(5) CD and surgical CD and surgical UC combined".

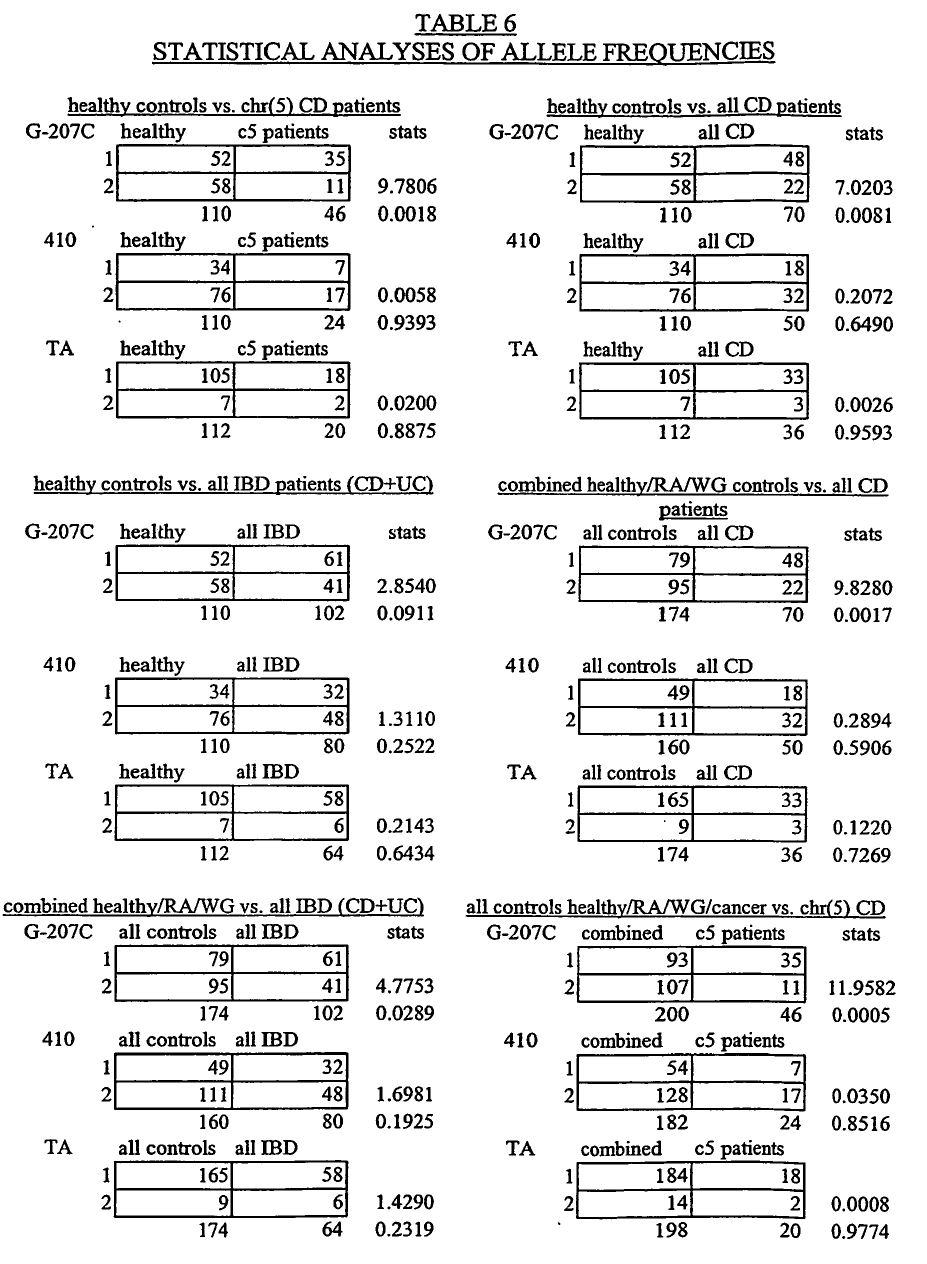

The most common OCTN2 DNA sequence variation (mutation) identified is significantly increased in frequency in Crohn's Disease patients. This observation is statistically highly significant (p=0.0014). The risk allele is nearly always found on the Single Nucleotide Polymorphism (SNP) risk haplotype background in these patients, fitting our genetic model of Crohn's Disease susceptibility in this region.

In addition, we have detected an association between the OCTN2 gene and generalized inflammatory responses. We performed a gel shift assay that showed a connection between a mutation in the OCTN2 gene, the specific protein that binds to the promoter region of the OCTN2 gene (HSF1; heat shock transcription factor 1, NCBI UniGene identified Hs.1499), and an HSF1 involvement in inflammation. The specific interaction of the Hsf1 protein with the OCTN2 promoter region is abolished by the presence of the G-207C "C" allele.

No complexes with wild-type or mutant oligos (with the OCTN2 promoter region abolished by the presence of the G-207C "C" allele) were detected (by hybridization with ³²P-labeled probes) in the absence of nuclear extract. No complexes were formed between wild-type oligo and nuclear extract from cells that are not heat shocked. Wild-type oligo formed a complex with nuclear extract from heat-shocked cells that is specifically competed for by cold (50x excess over ³²P- labeled test oligonucleotide) wild-type oligo and by cold oligo containing a known heat shock enhancer (HSE). This complex was not competed for by cold mutant oligo or cold unrelated oligo. This complex was identical in gel mobility to the complex between labeled oligo containing a known HSE and nuclear extract. This complex is supershifted, *i.e.* further reduced in mobility, upon forming a higher-order complex with anti-HSF1 antibody, indicating that the protein component of the nuclear extract that binds to the complex is Hsf1. No complexes are seen between labeled mutant oligo and nuclear extract, regardless of the presence of competitor oligo or anti-Hsfl antibody.

Thus, the OCTN2 G-207C mutation forms specific complexes with the HSF1 protein and that the binding of HSF1 is fully abolished by the presence of the G-207C mutant ("C") allele.

Thus, disclosed is a method for identifying an anti-inflammatory agent. A complex is formed, *in vivo* or *in vitro,* between the heat shock factor 1 (HSF1) protein and a polynucleotide containing a mutation in the OCTN2 promoter region. For example, the mutation can be the G-207C mutation in the OCTN2 promoter region. The complex is contacted with an agent, such as a drug, suspected of dissociating the complex. Detecting the dissociation of the complex identifies the agent as being an agent that is an anti-inflammatory agent.

Primers useful for PCR amplification parts of the OCTN2 gene include OCTN2 2.4: gccaggttaggttccctttc (SEQ ID NO:29); OCTN2 2.2: agcagcccaaattcttaaagg (SEQ ID NO:30); 02X1v3: gaatcacctcgctgcttttt (SEQ ID NO:31); 02X1v4: aaatgtggaaagggcatct (SEQ ID NO:32); O2X1600up: attaggcggtgtcaagagca (SEQ ID NO:33); O2X1F: ggtcgtgcgccatatgtaag (SEQ ID NO:34); and O2X1R1: gagaggagctcgggttcaag (SEQ ID NO:35).

*Mutations of the OCTN1 gene and uses in diagnosing inflammation.* As described above, OCTN1 is a transporter protein with the ability to transport carnitine in a sodium-dependent manner. OCTN1, which has been cloned and characterized in mouse (553 amino acids) and in human fetal liver (551 amino acids), carries a nucleotide binding site motif. OCTN1 is strongly expressed in adult kidney, trachea, bone marrow, and fetal liver, and several tumor cells, but not in adult human liver. OCTN1 mediates uptake of TEA in a pH-dependent manner.

FIG. 8 shows the nucleotide sequence of the OCTN1 gene, including the single nucleotide polymorphism in exon 9. This polymorphism is represented in dbSNP database as rs1050152. The allele frequency of this SNP had not been determined in any population (control or disease related). The nucleotide sequence of exon 9 is provided as: gtgcttacaacagaatgctgccctacatcgtcatgggtagtctgactgtcctgattggaatcYtcaccctttttttccctgaaagtttggga atgactcttccagaaaccttagagcagatgcagaaagtgaaatg (SEQ ID NO:36).

The nucleotide sequence of the spliced exons is provided as:

FIG. 9 shows the amino acid sequence deduced from the OCTN1 gene including position of the amino acid change caused by the OCTN1 exon 9 mutation (SEQ ID NO:38). Oligonucleotide primers for amplification of OCTN1 exon 9 mutation site include O1X9F: gtgcccagagagtcctccta (SEQ ID NO:39) and O1X9R: ttctccctaaggcattttggt (SEQ ID NO:40).

TABLE 7 shows the allele frequencies of the OCTN1 exon 9 mutation *(see,* FIG. 8 and FIG. 9). Alleles are listed by number (1 or 2), the DNA nucleotide present at that position (C or T), and the amino acid encoded by the codon including that nucleotide (Phe for phenylalanine or Leu for leucine). The number of chromosomes carrying each allele is shown for either unrelated healthy control individuals ("healthy" column) or Crohn's Disease patients from families with genetic risk conferred by the chromosome 5 locus previously described ("CD" column). The frequency of each allele as a percentage of the total is shown in brackets, and the total number of chromosomes ("n") is shown at the bottom of each column. The results of a 2 by 2 contingency table chi-squared test are presented at the bottom of the table demonstrating a significant difference in allele frequency between the "healthy" and "CD" classes (chi-squared statistic of 13.1422, p value with one degree of freedom 0.0003).

**TABLE 7**

| ALLELE FREQUENCY DATA FOR OCTN1 EXON 9 MUTATION | | |
|---|---|---|
| allele | healthy | CD |
| 1 (T, Phe) | 74(48%) | 42(78%) |
| 2 (C, Leu) | 80 (52%) | 12 (22%) |
| | n=154 | n=54 |
| | | chi-squared =13.1422 |
| | | p (1df) =0.0003 |

The description also discloses other polynucleotide having sequences that are fragments of SEQ ID NOS:37 or 38. For example, the fragments can have sequences that that are 20 bases long (or multiples thereof).

In addition, there may be an association between the OCTN1 gene and generalized inflammatory responses. A specific protein (HSF1) may bind to the promoter region of the OCTN1 gene. Accordingly, inflammatory disease in general is within the scope of the present invention.

Thus disclosed is a method for identifying an anti-inflammatory agent. A complex is formed, either *in vivo* or *in vitro* between the heat shock factor 1 (HSF1) protein and a polynucleotide containing the OCTN1 promoter region. The complex with an agent, such as a drug, suspected of being able to dissociate the complex. Detecting the dissociation of the complex identifies the agent as being an agent that is an anti-inflammatory agent.

*OCTN1 and OCTN2 Polynucleotides.* The invention provides polymorphisms of OCTN1 and OCTN2 as defined in the claims. The term "polynucleotide" encompasses RNA and DNA, including cDNA, genomic DNA, and synthetic (*e.g.*, chemically synthesized) DNA. The polynucleotide may be double-stranded or single-stranded. Where single-stranded, the polynucleotide may be the sense strand or the antisense strand. The polynucleotides disclosed thus provide material for making OCTN1 or OCTN2 polypeptides.

The polynucleotide can be used as the basis of drug development diagnostics for Inflammatory Bowel Diseases. In addition to the structural information provided by the polynucleotide of the invention is the associated information provided by the genetic analysis. Methods for using the polynucleotide of the invention in conjunction with further genetic analyses of complex genetic traits are known in the art (*see,* Lander E. & Kruglyak L., *Nature Genet.* 11: 241-247 (1995); Ott, J., *Nature* 379: 772-773 (1996)); as are methods for assessing the relative risk of Crohn's disease for individuals who are heterozygous, homozygous, or compound heterozygous for the identified genetic loci (*see,* for example, Hugot J.-P., *et al., Nature* 411: (2001); IBD International Genetics Consortium, *Am. J. Hum. Genet.* 68: 1165-1171 (2001)).

By "isolated polynucleotide" is meant DNA that is not immediately contiguous with both of the coding sequences with which it is immediately contiguous (one on the 5' end and one on the 3' end) in the naturally occurring genome of the organism from which it is derived. Thus, a recombinant polynucleotide could include some or all of the 5' non-coding (*e.g.,* promoter) sequences which are immediately contiguous to the coding sequence. The term therefore includes, for example, a recombinant DNA which is incorporated into a vector; into an autonomously replicating plasmid or virus, such as a retrovirus; or into the genomic DNA of a prokaryote or eukaryote, or which exists as a separate molecule (*e.g.,* a cDNA or a genomic DNA fragment produced by PCR or restriction endonuclease treatment) independent of other sequences. It also includes a recombinant DNA that is part of a hybrid gene encoding additional polypeptide sequence.

By "substantially identical" is meant a polypeptide or polynucleotide having a sequence that is at least 85%, preferably 90%, and more preferably 95% or more identical to the sequence of the reference amino acid or polynucleotide sequence. For polypeptides, the length of the reference polypeptide sequence will generally be at least 16 amino acids, preferably at least 20 amino acids, more preferably at least 25 amino acids, and most preferably 35 amino acids. For polynucleotides, the length of the reference polynucleotide sequence will generally be at least 50 nucleotides, preferably at least 60 nucleotides, more preferably at least 75 nucleotides, and most preferably at least 110 nucleotides.

To determine the percent identity of two polynucleotides, the sequences are aligned for optimal comparison purposes (*e.g.,* gaps can be introduced in the sequence of a first amino acid or polynucleotide sequence for optimal alignment with a second amino or polynucleotide sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (*i.e.,* % identity = # of identical positions/total # of positions (*e.g.,* overlapping positions) x 100). Preferably, the two sequences are the same length.

The determination of percent homology between two sequences can be accomplished using a mathematical algorithm. A preferred, non-limiting example of a mathematical algorithm utilized for the comparison of two sequences is the algorithm of Karlin & Altschul, *Proc. Natl. Acad. Sci. USA* 87:2264-2268 (1990), modified as in Karlin & Altschul, *Proc. Natl. Acad Sci. USA* 90:5873-5877 (1993). Such an algorithm is incorporated into the BLASTX and BLASTN programs of Altschul *et al., J. Mol. Biol.* 215:403-410 (1990). BLAST nucleotide searches can be performed with the NBLAST program, score=100, wordlength=12 to obtain nucleotide sequences homologous to OCTN2 polynucleotide molecules of the invention. BLAST protein searches can be performed with the BLASTX program, score=50, wordlengkh=3 to obtain amino acid sequences homologous to OCTN2 protein molecules of the invention. To obtain gapped alignments for comparison purposes, Gapped BLAST can be utilized as described in Altschul *et al., Nucleic Acids Res.* 25: 3389-3402 (1997). Alternatively, PSI-Blast can be used to perform an iterated search that detects distant relationships between molecules. *Id.* When utilizing BLAST, Gapped BLAST, and PSI-Blast programs, the default parameters of the respective programs (*e.g.,* BLASTX and BLASTN) can be used. *See* <http://www.ncbi.nlm.nih.gov>. Another preferred, non-limiting example of a mathematical algorithm used for the comparison of sequences is the algorithm of Myers and Miller, (1988) *CABIOS* 4:11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The percent identity between two sequences can be determined using techniques similar to those described above, with or without allowing gaps. In calculating percent identity, only exact matches are counted.

Disclosed are also polynucleotides that hybridize under stringent conditions to a polynucleotide encoding an OCTN1 or OCTN2 polypeptide. Examples of stringent conditions include: 1) hybridization at 50°C in Church buffer (7% SDS, 0.5% NaHPO4, 1 mM EDTA, 1% BSA) and washing at 50°C in 2x SSC; and 2) hybridization in 6x sodium chloride/sodium citrate (SSC) at about 45°C, followed by one or more washes in 0.2 x SSC, 0.1% SDS at 50-65°C. Other stringent conditions are known to those skilled in the art and can be found *in Current Protocols in Molecular Biology,* (John Wiley & Sons, N.Y. 1989), 6.3.1-6.3.6. The hybridizing portions of the hybridizing polynucleotides are preferably 20, 30, 50, or 70 bases long. Preferably, the hybridizing portion of the hybridizing polynucleotide is 95% or even 98% identical to the sequence of a portion of a polynucleotide encoding an OCTN2 polypeptide. Hybridizing polynucleotides of the type described above can be used as a cloning probe, a primer (*e.g.,* a PCR primer), or a diagnostic probe. Preferred hybridizing polynucleotides encode a polypeptide having some or all of the biological activities possessed by naturally-occurring OCTN1 or OCTN2. Hybridizing polynucleotides can be splice variants encoded by one of the OCTN1 or OCTN2 genes described herein. Thus, they may encode a protein that is shorter or longer than the various forms of OCTN1 or OCTN2 described herein. Hybridizing polynucleotides may also encode proteins that are related to OCTN1 or OCTN2 (*e.g.* proteins encoded by genes which include a portion having a relatively high degree of identity to an OCTN1 or OCTN2 gene described herein).

The description also features isolated polynucleotide sequences that encode a portion of OCTN1 or OCTN2. Examples of detectable markers include β-lactamase, chloramphenicol acetyltransferase (CAT), alkaline phosphatase (AP), adenosine deaminase (ADA), aminoglycoside phosphotransferase (neo^{r}, G418^{r}), dihydrofolate reductase (DHFR), hygromycin-B-phosphotransferase (HPH), thymidine kinase (TK), β-galactosidase, and xanthine guanine phosphoribosyl-transferase (XGPRT).

The polypeptides include recombinant polypeptides, natural polypeptides produced from polynucleotides of the invention having a polymorphism disclosed herein, and synthetic polypeptides as well as polypeptides which are preproteins or proproteins. The polypeptides can be expressed fused to another polypeptide, *e.g.,* a marker polypeptide or fusion partner. For example, the polypeptide can be fused to a hexa-histidine tag to facilitate purification of bacterially expressed protein or a hemagglutinin tag to facilitate purification of protein expressed in eukaryotic cells.

The description features transformed cells harbouring a polynucleotide. The description also features vectors that include a polynucleotide of the invention that is properly positioned for expression. For example, the vector can be an expression vector, and can include one or more regulatory elements. Regulatory elements that can influence the expression of the polynucleotide inserted into the vector, such as regulatory elements that direct tissue-specific expression, are well known to those of skill in the art. Examples of regulatory elements include the cytomegalovirus hCMV immediate early gene, the early promoter of SV40 adenovirus, the late promoter of SV40 adenovirus, the lac system, the trp system, the TAC system, the TRC system, the major operator and promoter regions of phage lambda, the control regions of fd coat protein, the promoter for 3-phosphoglycerate kinase, the promoters of acid phosphatase, and the promoters of the yeast α-mating factors. The vector can be a plasmid, or a virus, such as a retrovirus.

By "transformed cell" is meant a cell into which (or into an ancestor of which) has been introduced, by means of recombinant DNA techniques, a DNA molecule encoding (as used herein) an OCTN1 or OCTN2 polypeptide.

By "positioned for expression" is meant that the selected DNA molecule is positioned adjacent to one or more sequence elements which direct transcription and/or translation of the sequence such that the sequence elements can control transcription and/or translation of the selected DNA (*i.e.,* the selected DNA is operably associated with the sequence elements). Such operably associated elements can be used to facilitate the production of an OCTN1 or OCTN2 polypeptide.

Antagonists can inhibit one or more of the functions of OCTN1 or OCTN2. Suitable antagonists can include large or small molecules (*e.g.*, organic molecules), antibodies to OCTN1 or OCTN2, and OCTN1 or OCTN2 polypeptides that compete with a native form of OCTN1 or OCTN2. Agonists of OCTN1 or OCTN2 will enhance or facilitate one or more of the functions of OCTN1 or OCTN2. Suitable agonists can include, for example, large or small molecules (*e.g.*, organic molecules), and antibodies to OCTN1 or OCTN2. Also within the invention are polynucleotide molecules that can be used to interfere with OCTN1 or OCTN2 expression, *e.g.,* antisense molecules and ribozymes.

The description also features a cell that harbours a recombinant polynucleotide encoding an OCTN1 or OCTN2 polypeptide; a vector which includes a polynucleotide encoding a OCTN2 polypeptide.

*Methods.* Disclosed is a method for detecting Inflammatory Bowel Diseases. This method includes: (a) obtaining a biological sample; (b) contacting the sample with probe which selectively binds an OCTN1 or OCTN2 polynucleotide; and (c) determining the amount of the probe selectively bound to said biological sample as a measure of the level of expression of v. This level, in turn, serves as a biological marker for disease activity and/or progression in individuals with Inflammatory Bowel Diseases. Thus, the invention provides a method to track or monitor expression level changes in Inflammatory Bowel Diseases as a diagnostic or prognostic tool.

In general, OCTN1 or OCTN2 proteins and fusion proteins according to the invention can be produced using the polynucleotide of the invention by transformation (transfection, transduction, or infection) of a host cell with all or part of an OCTN1-encoding or OCTN2-encoding DNA fragment (*e.g.*, the cDNA described herein) in a suitable expression vehicle. Suitable expression vehicles include: plasmids, viral particles, and phage. For insect cells, baculovirus expression vectors are suitable. The entire expression vehicle, or a part thereof, can be integrated into the host cell genome. In some circumstances, it is desirable to employ an inducible expression vector, *e.g.,* the LACSWITCH^{™} Inducible Expression System (Stratagene; La Jolla, California, USA).

Those skilled in the field of molecular biology will understand that any of a wide variety of expression systems can be used to provide the recombinant protein. The precise host cell used is not critical to the invention. The OCTN1 or OCTN2 protein can be produced in a prokaryotic host (*e.g., E. coli* or *B. subtilis*) or in a eukaryotic host (*e.g., Saccharomyces* or *Pichia;* mammalian cells, *e.g.,* COS, NIH 3T3, CHO, BHK, 293, or HeLa cells; or insect cells).

Plant cells can also produce proteins and polypeptides. For plant cells viral expression vectors (*e.g.*, cauliflower mosaic virus and tobacco mosaic virus) and plasmid expression vectors (*e.g.,* Ti plasmid) are suitable. Such cells are available from a wide range of sources (*e.g.,* the American Type Culture Collection (ATCC), Manassas, VA, USA.; *see also, e.g.,* Ausubel *et al., Current Protocols in Molecular Biology,* (John Wiley & Sons, New York, 1994)). The methods of transformation or transfection and the choice of expression vehicle will depend on the host system selected. Transformation and transfection methods are described, *e.g*., in Ausubel *et al. Current Protocols in Molecular Biology,* (John Wiley & Sons, New York, 1994); expression vehicles may be chosen from those provided, *e.g*., in *Cloning Vectors: A Laboratory Manual,* P. H. Pouwels *et al.* (1985, Supp. 1987).

The host cells harboring the expression vehicle can be cultured in conventional nutrient media adapted as needed for activation of a chosen gene, repression of a chosen gene, selection of transformants, or amplification of a chosen gene.

OCTN1 or OCTN2 polypeptides can be produced as fusion proteins. For example, the expression vector pUR278 (Ruther *et al., EMBO J.* 2:1791,1983), can be used to create *lac*Z fusion proteins. The pGEX vectors can be used to express foreign polypeptides as fusion proteins with glutathione S-transferase (GST). In general, such fusion proteins are soluble and can be easily purified from lysed cells by adsorption to glutathione-agarose beads followed by elution in the presence of free glutathione. The pGEX vectors are designed to include thrombin or factor Xa protease cleavage sites so that the cloned target gene product can be released from the GST moiety.

In an insect cell expression system, *Autographa californica* nuclear polyhidrosis virus (AcNPV), which grows in *Spodoptera frugiperda* cells, is used as a vector to express foreign genes. An OCTN1 or OCTN2 coding sequence can be cloned individually into non-essential regions (for example the polyhedrin gene) of the virus and placed under control of an AcNPV promoter, *e.g*., the polyhedrin promoter. Successful insertion of a gene encoding an OCTN1 or OCTN2 polypeptide or protein will result in inactivation of the polyhedrin gene and production of non-occluded recombinant virus (*i.e.,* virus lacking the proteinaceous coat encoded by the polyhedrin gene). These recombinant viruses are then used to infect *Spodoptera frugiperda* cells in which the inserted gene is expressed (see, *e.g.,* Smith *et al., J. Virol.* 46:584, 1983; Smith, U.S. Pat. No. 4,215,051).

In mammalian host cells, a number of viral-based expression systems can be utilized. In cases where an adenovirus is used as an expression vector, the OCTN1 or OCTN2 polynucleotide sequence can be ligated to an adenovirus transcription/translation control complex, *e.g.*, the late promoter and tripartite leader sequence. This chimeric gene can then be inserted into the adenovirus genome by *in vitro* or *in vivo* recombination. Insertion into a non-essential region of the viral genome (*e.g.*, region E1 or E3) will result in a recombinant virus that is viable and capable of expressing an OCTN1 or OCTN2 gene product in infected hosts. *See, e.g.,* Logan, *Proc. Natl. Acad. Sci. USA* 81:3655 (1984).

Specific initiation signals may also be required for efficient translation of inserted polynucleotide sequences. These signals include the ATG initiation codon and adjacent sequences. In cases where an entire native OCTN1 or OCTN2 gene or cDNA, including its own initiation codon and adjacent sequences, is inserted into the appropriate expression vector, no additional translational control signals may be needed. In other cases, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators. Bittner *et al., Methods in Enzymol.* 153:516 (1987).

In addition, a host cell may be chosen which modulates the expression of the inserted sequences, or modifies and processes the gene product in a specific, desired fashion. Such modifications (*e.g.*, glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells that possess the cellular machinery for proper processing of the primary transcript, glycosylation, and phosphorylation of the gene product can be used. Such mammalian host cells include, but are not limited to, CHO, VERO, BHK, HeLa, COS, MDCK, 293, 3T3, WI38, and in particular, choroid plexus cell lines.

Any technique known in the art can be used to introduce a OCTN2 transgene into animals to produce the founder lines of transgenic animals. Such techniques include, but are not limited to, pronuclear microinjection (U.S. Pat. No. 4,873,191); retrovirus mediated gene transfer into germ lines (Van der Putten *et al., Proc. Natl. Acad. Sci., USA* 82:6148 (1985)); gene targeting into embryonic stem cells (Thompson *et al., Cell* 56:313 (1989)); and electroporation of embryos (Lo, Mol. Cell. Biol. 3:1803, 1983).

The present invention provides for non human transgenic animals that carry the OCTN2 transgene in all their cells, as well as animals that carry the transgene in some, but not all of their cells, *i.e*., mosaic animals. The transgene can be integrated as a single transgene or in concatamers, *e.g.*, head-to-head tandems or head-to-tail tandems. The transgene can also be selectively introduced into and activated in a particular cell type. Lasko *et al., Proc. Natl. Acad. Sci. USA* 89:6232 (1992). The regulatory sequences required for such a cell-type specific activation will depend upon the particular cell type of interest, and will be apparent to those of skill in the art.

When it is desired that the OCTN1 or OCTN2 transgene be integrated into the chromosomal site of the endogenous OCTN2 gene, gene targeting is preferred. Briefly, when such a technique is to be used, vectors containing some nucleotide sequences homologous to an endogenous OCTN1 or OCTN2 gene are designed for the purpose of integrating, via homologous recombination with chromosomal sequences, into and disrupting the function of the nucleotide sequence of the endogenous gene. The transgene also can be selectively introduced into a particular cell type, thus inactivating the endogenous OCTN1 or OCTN2 gene in only that cell type. Gu *et al., Science* 265:103 (1984). The regulatory sequences required for such a cell-type specific inactivation will depend upon the particular cell type of interest, and will be apparent to those of skill in the art.

Once non-human transgenic animals have been generated, the expression of the recombinant OCTN1 or OCTN2 gene can be assayed utilizing standard techniques. Initial screening may be accomplished by Southern blot analysis or PCR techniques to analyse animal tissues to assay whether integration of the transgene has taken place. The level of mRNA expression of the transgene in the tissues of the transgenic animals may also be assessed using techniques which include, but are not limited to, Northern blot analysis of tissue samples obtained from the animal, *in situ* hybridisation analysis, and RT-PCR. Samples of OCTN1 or OCTN2 gene-expressing tissue also can be evaluated immunocytochemically using antibodies specific for the OCTN1 or OCTN2 transgene product.

*Antisense Polynucleotides.* Antisense approaches involve the design of oligonucleotides (either DNA or RNA) that are complementary to OCTN1 or OCTN2 mRNA. The antisense oligonucleotides bind to the complementary OCTN1 or OCTN2 mRNA transcripts and prevent translation. Absolute complementarity, although preferred, is not required. A sequence "complementary" to a portion of an RNA, as referred to herein, means a sequence having sufficient complementarity to be able to hybridise with the RNA, forming a stable duplex; in the case of double-stranded antisense polynucleotides, a single strand of the duplex DNA may be tested, or triplex formation may be assayed. The ability to hybridise will depend on both the degree of complementarily and the length of the antisense polynucleotide. Generally, the longer the hybridising polynucleotide, the more base mismatches with an RNA it may contain and still form a stable duplex (or triplex, as the case may be). One skilled in the art can ascertain a tolerable degree of mismatch by use of standard procedures to determine the melting point of the hybridised complex.

Oligonucleotides that are complementary to the 5' end of the message, *e.g*., the 5' untranslated sequence up to and including the AUG initiation codon, should work most efficiently at inhibiting translation. However, sequences complementary to the 3' untranslated sequences of mRNAs recently have been shown to be effective at inhibiting translation of mRNAs as well. Wagner, *Nature* 372:333 (1984). Thus, oligonucleotides complementary to the 5'- or 3'-non-translated, non-coding regions of the OCTN1 or OCTN2 gene, *e.g*., the human genes shown in FIG. 8 and FIG. 6, could be used in an antisense approach to inhibit translation of endogenous OCTN2 mRNA. Oligonucleotides complementary to the 5' untranslated region of the mRNA should include the complement of the AUG start codon.

Antisense oligonucleotides complementary to mRNA coding regions are less efficient inhibitors of translation. Whether designed to hybridise to the 5'-, 3'-, or coding region of OCTN1 or OCTN2 mRNA, antisense polynucleotides should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

Regardless of the choice of target sequence, it is preferred that *in vitro* studies are first performed to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridisation to the target sequence.

The oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridisation, *etc.* The oligonucleotide may include other appended groups such as peptides (*e.g*., for targeting host cell receptors *in vivo*), or agents facilitating transport across the cell membrane (as described, *e.g.*, in Letsinger *et al., Proc. Natl. Acad. Sci. USA* 86:6553 (1989); Lemaitre *et al., Proc. Natl. Acad. Sci. USA* 84:648 (1987); PCT Publication No. WO 88/09810) or the blood-brain barrier (see, *e.g.*, PCT Publication No. WO 89/10134), or hybridisation-triggered cleavage agents (see, *e.g.,* Krol *et al., BioTechniques* 6:958 (1988)), or intercalating agents (*see, e.g.,* Zon, *Pharm. Res.* 5:539 (1988)). To this end, the oligonucleotide can be conjugated to another molecule, *e.g*., a peptide, hybridisation triggered cross-linking agent, transport agent, or hybridisation-triggered cleavage agent.

The antisense oligonucleotide may comprise at least one modified base moiety which is selected from the group including, but not limited to, 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethyl-aminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-uracil-5-oxyacetic acid (v), wybutoxosine, pseudouracil, queosine, 2-thiocytosine, 5-methyl-2-theouracil, 2-thiouracil, 4-thiouracil, 5-methyluracil, uracil-5-oxyacetic acid methylester, uracil-5-oxyacetic acid (v), 5-methyl-2-thiouracil, 2-(3-amino-3-N-2-carboxypropl) uracil, (acp3)w, and 2,6-diaminopurine.

The antisense oligonucleotide may also have at least one modified sugar moiety selected from the group including, but not limited to, arabinose, 2-fluoroarabinose, xylulose, and hexose.

For example, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal, or an analog of any of these backbones.

For instance, the antisense oligonucleotide is an α-anomeric oligonucleotide. An α-anomeric oligonucleotide forms specific double-stranded hybrids with complementary RNA in which, contrary to the usual β-units, the strands run parallel to each other. Gautier *et al., Nucl. Acids. Res.* 15:6625 (1987). The oligonucleotide is a 2'-0-methylribonucleotide (Inoue *et al., Nucl. Acids Res.* 15:6131 (1987)), or a chimeric RNA-DNA analog (Inoue *et al., FEBS Lett.* 215:327 (1987)).

Antisense oligonucleotides can be synthesized by standard methods known in the art, *e.g.* by use of an automated DNA synthesizer (such as are commercially available from Biosearch, Applied Biosystems, *etc*.). As examples, phosphorothioate oligonucleotides can be synthesized by the method of Stein *et al. Nucl. Acids Res.* 16:3209 (1988), and methylphosphonate oligonucleotides can be prepared by use of controlled pore glass polymer supports. Sarin *et al., Proc. Natl. Acad. Sci. USA* 85:7448 (1988).

While antisense nucleotides complementary to the OCTN1 or OCTN2 coding region sequence could be used, those complementary to the transcribed untranslated region are most preferred.

The antisense molecules can be delivered to cells that express OCTN1 or OCTN2 *in vivo, e.g.,* cells of the gastrointestinal tract and the immune system. A number of methods have been developed for delivering antisense DNA or RNA to cells; *e.g*., antisense molecules can be injected directly into the tissue site, or modified antisense molecules, designed to target the desired cells (*e.g*., antisense linked to peptides or antibodies that specifically bind receptors or antigens expressed on the target cell surface) can be administered systemically to achieve intracellular concentrations of the antisense molecule sufficient to suppress translation of endogenous mRNAs and are known in the art.

*Ribozymes.* Ribozyme molecules designed to catalytically cleave OCTN1 or OCTN2 mRNA transcripts also can be used to prevent translation of OCTN2 mRNA and expression of OCTN2 (*see, e.g.,* PCT Publication No. WO 90/11364; Saraver *et al., Science* 247:1222 (1990)). While various ribozymes that cleave mRNA at site-specific recognition sequences can be used to destroy OCTN1 or OCTN2 mRNAs, the use of hammerhead ribozymes is preferred. Hammerhead ribozymes cleave mRNAs at locations dictated by flanking regions that form complementary base pairs with the target mRNA. The sole requirement is that the target its mRNA have the following sequence of two bases: 5'-UG-3'. The construction and production of hammerhead ribozymes is well known in the art. Haseloff *et al., Nature* 334:585 (1988).

The ribozymes also include RNA endoribonucleases (hereinafter "Cech-type ribozymes"), such as the one that occurs naturally in *Tetrahymena thermophila* (known as the IVS or L-19 IVS RNA), and which has been extensively described by Cech and his collaborators. Zaug *et al., Science* 224:574 (1984); Zaug *et al., Science* 231:470 (1986); Zug *et al., Nature* 324:429 (1986); PCT Application No. WO 88/04300; and Been *et al., Cell* 47:207 (1986). The Cech-type ribozymes have an eight base-pair sequence that hybridises to a target RNA sequence, whereafter cleavage of the target RNA takes place. The invention encompasses those Cech-type ribozymes that target eight base-pair active site sequences present in OCTN2.

*Methods for Modulating OCTN1 or OCTN2 Expression.* Endogenous OCTN1 or OCTN2 gene expression can also be reduced by inactivating or "knocking out" the OCTN1 or OCTN2 gene or its promoter using targeted homologous recombination (see, *e.g*., U.S. Pat. No. 5,464,764). For example, a mutant, non-functional OCTN2 (or a completely unrelated DNA sequence) flanked by DNA homologous to the endogenous OCTN2 gene (either the coding regions or regulatory regions of the OCTN2 gene) can be used, with or without a selectable marker and/or a negative selectable marker, to transfect cells that express OCTN2 *in vivo.* Insertion of the DNA construct, via targeted homologous recombination, results in inactivation of the OCTN1 or OCTN2 gene. Such approaches are particularly suited for use in the agricultural field where modifications to ES (embryonic stem) cells can be used to generate animal offspring with an inactive OCTN1 or OCTN2. However, this approach can be adapted for use in humans, provided the recombinant DNA constructs are directly administered or targeted to the required site *in vivo* using appropriate viral vectors, *e.g*., herpes virus vectors for delivery to brain tissue; *e.g.*, the arcuate nucleus or the choroid plexus.

Modulators of OCTN1 or OCTN2 expression are identified in a method in which a cell is contacted with a candidate compound and the expression of OCTN1 or OCTN2 mRNA or protein in the cell is determined. The level of expression of OCTN1 or OCTN2 mRNA or protein in the presence of the candidate compound is compared to the level of expression of OCTN1 or OCTN2 mRNA or protein in the absence of the candidate compound. The candidate compound can then be identified as a modulator of OCTN1 or OCTN2 expression based on this comparison. For example, when expression of OCTN1 or OCTN2 mRNA or protein is greater (statistically significantly greater) in the presence of the candidate compound than in its absence, the candidate compound is identified as a stimulator of OCTN1 or OCTN2 mRNA or protein expression. Alternatively, when expression of OCTN1 or OCTN2 mRNA or protein is less (statistically significantly less) in the presence of the candidate compound than in its absence, the candidate compound is identified as an inhibitor of OCTN1 or OCTN2 mRNA or protein expression. The level of OCTN1 or OCTN2 mRNA or protein expression in the cells can be determined by methods described herein for detecting OCTN1 or OCTN2 mRNA or protein.

Examples of methods for the synthesis of molecular libraries can be found in the art, for example in: DeWitt *et al., Proc. Natl. Acad. Sci. USA* 90:6909 (1993); Erb *et al., Proc. Natl. Acad. Sci. USA* 91:11422 (1994); Zuckermann *et al., J. Med. Chem.* 37:2678 (1994); Cho *et al., Science* 261:1303 (1993); Carrell *et al., Angew. Chem. Int. Ed. Engl.* 33:2059 (1994); *Carell et al., Angew. Chem. Int. Ed. Engl.* 33:2061 (1994); and Gallop *et al., J. Med. Chem.* 37:1233 (1994).

Libraries of compounds may be presented in solution (*e.g.,* Houghten, *Bio*/*Techniques* 13:412-421 (1992)), or on beads (Lam, *Nature* 354:82-84 (1991)), chips (Fodor, *Nature* 364:555-556 (1993)), bacteria (U.S. Pat. No. 5,223,409), spores (U.S. Pat. Nos. 5,571,698; 5,403,484; and 5,223,409), plasmids (Cull *et al., Proc. Natl. Acad. Sci. USA* 89:1865-1869 (1992)) or phage (Scott & Smith, *Science* 249:386-390 (1990); Devlin, *Science* 249:404-406 (1990); Cwirla *et al., Proc. Natl. Acad. Sci. USA* 87:6378-6382 (1990); and Felici, *J. Mol. Biol.* 222:301-310 (1991).

*Diagnostic Applications.* The polynucleotides, polypeptides, and antibodies of the invention are useful for identifying those compartments of mammalian cells that contain proteins important to the function of OCTN1 or OCTN2. Antibodies specific for OCTN1 or OCTN2 may be produced as described above. The normal location of the protein is then determined either in situ or using fractionated cells by any standard immunological or immunohistochemical procedure (see, *e.g.*, Ausubel *et al., supra;* Bancroft & Stevens, *Theory and Practice of Histological Techniques,* (Churchill Livingstone, 1982).

Alternatively, OCTN1 or OCTN2 expression can be assayed by standard Northern blot analysis or can be aided by PCR (*see, e.g.,* Ausubel *et al., supra; PCR Technology: Principles and Applications for DNA Amplification,* ed., H. A. Ehrlich (Stockton Press, N.Y.). Also, see above for working examples. If desired or necessary, analysis can be carried out to detect point mutations in the OCTN2 sequence (for example, using well-known polynucleotide mismatch detection techniques). All of the above techniques are enabled by the OCTN1 or OCTN2 sequences described herein.

Accordingly, the polynucleotides, polypeptides and antibodies of the invention can be used in a method for determining whether a patient has a disorder associated with abnormal expression of OCTN1 or OCTN2. The method can be carried out by quantitating the level of expression of OCTN1 or OCTN2 in a biological sample obtained from the patient. As a control, the quantitation can be carried out using a biological sample obtained from a subject who is healthy.

OCTN1 or OCTN2 expression can be assessed at the level of gene expression, for example, by quantitating the level of OCTN1 or OCTN2 mRNA expression in the biological sample, or at the level of protein expression, by quantitating the level of OCTN1 or OCTN2 protein expressed. Quantitation can be carried out using the techniques described above, which are well within the abilities of those of skill in the art to perform.

Should it be determined that a patient has a disorder that is associated with abnormal expression or activity of OCTN1 or OCTN2, the patient can be given a compound that modulates that expression or activity. For example, the patient can receive a compound such as a small molecule, an antisense polynucleotide molecule, or a ribozyme, that inhibits the expression of OCTN2. The patient can also receive a compound that inhibits the activity of OCTN1 or OCTN2. An antibody that specifically binds OCTN1 or OCTN2 can be used for this purpose. Alternatively, the patient can receive a compound that enhances the expression or activity of OCTN2. Compounds that inhibit or enhance the expression or activity of OCTN2 can include synthetic molecules. These methods of treatment can be used to treat Inflammatory Bowel Diseases and related disorders associated with cellular proliferation.

The details of one or more embodiments of the invention are set forth in the accompanying description above. Although any methods and materials similar or equivalent to those described herein can be used in the practice or testing of the present invention, the preferred methods and materials are now described. Other features, objects, and advantages of the invention will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs.

The foregoing description has been presented only for the purposes of illustration and is not intended to limit the invention to the precise form disclosed, but by the claims appended hereto.

## Claims

1. An isolated OCTN 1 polynucleotide fragment of the nucleic acid encoding SEQ ID No. 7, wherein said fragment comprises at least 20 nucleotides in length and contains a sequence corresponding to the L503F residue of SEQ ID NO: 7.

2. The polynucleotide fragment according to claim 1 comprising SEQ ID NO: 21 or SEQ ID NO: 22.

3. An isolated OCTN 1 primer comprising SEQ ID NO: 39 or SEQ ID NO: 40

4. A vector comprising the polynucleotide of any one of claims 1-3.

5. An article of manufacture comprising
the complement of the polynucleotide according to claim 1 as a polynucleotide hybridisation probe for the diagnosis of inflammatory bowel disease that is homologous to and specifically binds under stringent hybridisation conditions to an OCTN 1 polynucleotide.

6. The article of manufacture of claim 5 further comprising a hybridisation probe homologous to and specifically binding under stringent hybridisation conditions to a polynucleotide selected from
1) SEQ ID NO: 1 or fragments of at least twenty nucleotides in length of SEQ ID NO: 1,
2) SEQ ID NO: 36 or fragments thereof of at least twenty nucleotides in length
3) SEQ ID NO: 37 or fragments thereof of at least twenty nucleotides in length
4) the polynucleotide of any one of claims 1-4,
5) SEQ ID NO: 36 comprising a nucleotide sequence encoding phenylalanine at a position corresponding to position 503 of SEQ ID NO: 7, or fragments thereof of at least twenty nucleotides in length comprising a nucleotide sequence encoding phenylalanine at a position corresponding to position 503 of SEQ ID NO: 7,
6) SEQ ID NO: 37 comprising a nucleotide sequence encoding phenylalanine at a position corresponding to position 503 of SEQ ID NO: 7, or fragments thereof of at least twenty nucleotides in length comprising a nucleotide sequence encoding phenylalanine at a position corresponding to position 503 of SEQ ID NO: 7.

7. An isolated OCTN 2 polynucleotide fragment of SEQ ID No. 8, wherein said fragment comprises at least 20 nucleotides in length and contains a sequence corresponding to the G-207C residue of SEQ ID NO: 8.

8. The polynucleotide fragment according to claim 7 comprising SEQ ID NO: 17 or SEQ ID NO: 18.

9. A vector comprising the polynucleotide of claim 7 or 8.

10. An article of manufacture comprising
the complement of the polynucleotide according to claim 7 as a polynucleotide hybridisation probe for the diagnosis of inflammatory bowel disease that is homologous to and specifically binds under stringent hybridisation conditions to an OCTN 2 polynucleotide.

11. The article of manufacture of claim 10 further comprising a hybridisation probe homologous to and specifically binding under stringent hybridisation conditions to a polynucleotide selected from
1) SEQ ID NO: 3 or fragments thereof of at least twenty nucleotides in length,
2) SEQ ID NO: 23 or fragments thereof of at least twenty nucleotides in length
3) the polynucleotide of any one of claims 7-9,
4) SEQ ID NO: 3 comprising a nucleotide sequence corresponding to the G-207C residue of SEQ ID NO: 8, or fragments thereof of at least twenty nucleotides in length, comprising a nucleotide sequence corresponding to the G-207C residue of SEQ ID NO: 8,
5) SEQ ID NO: 23 comprising a nucleotide sequence corresponding to the G-207C residue of SEQ ID NO: 8, or fragments thereof of at least twenty nucleotides in length comprising a nucleotide sequence corresponding to the G-207C residue of SEQ ID NO: 8,
6) SEQ ID NO: 25..

12. An in vitro method for determining a susceptibility to Inflammatory Bowel Disease (IBD) in a mammal, comprising:
(a) contacting a biological sample that has been obtained from a mammal with polynucleotide probe that selectively binds an OCTN1 polynucleotide or an OCTN2 polynucleotide; and
(b) determining the expression of the OCTN1 polynucleotide or the OCTN2 polynucleotide as a measure of susceptibility of the mammal to Inflammatory Bowel Disease.

13. The method of claim 12, wherein the expression of the OCTN1 polynucleotide or the OCTN2 polynucleotide in the biological sample is reduced in mammals having a susceptibility to Inflammatory Bowel Disease as compared with mammals that are not susceptible to Inflammatory Bowel Disease.

14. The method of claim 12 or 13, wherein the biological sample is selected from the group consisting of gastrointestinal tract tissue and immune system tissue.

15. The method of any of claims 12 to 14, wherein the biological sample is selected from inflamed tissue.

16. The method of any of claims 12 to 15, wherein the mammal is human.

17. The method of any of claims 12 to 16, wherein the Inflammatory Bowel Disease is selected from the group consisting of Crohn's disease (CD), indeterminate colitis (IC) and ulcerative colitis (UC).

18. The method of any of claims 12 to 17, wherein the determination of the expression is by Northern analysis.

19. The method of any of claims 12 to 18, wherein the determination of the expression is by polymerase chain reaction.

20. The method of any of claims 12 to 19, wherein the expression of both the OCTN1 polynucleotide and the OCTN2 polynucleotide in the biological sample is determined.

21. The method of any of claims 12 to 20, wherein the basal transcription of the OCTN2 gene is downregulated.

22. The method of any of claims 12 to 21, wherein the transcription of the OCTN2 gene induced either by heat shock or by arachidonic acid is downregulated.

23. The method of any of claims 12 to 22, wherein both the basal transcription of the OCTN2 gene and the transcription of the OCTN2 gene induced either by heat shock or by arachidonic acid is downregulated.

24. An in vitro method for determining a susceptibility to inflammatory bowel disease (IBD), in a human, comprising:
(a) determining the polynucleotide sequence of the OCTN1 gene or the OCTN2 gene for a human; and
(b) determining that the polynucleotide sequence of the OCTN1 gene or the OCTN2 gene for the human contains a polymorphism that results in reduced expression of OCTN1 or OCTN2 in the human.

25. The method of claim 24, wherein the polynucleotide sequences of both the OCTN1 gene and the OCTN2 gene are determined for the human.

26. The method of claim 24 or 25, wherein the polynucleotide sequence of the OCTN1 gene contains at least one L503F polymorphism.

27. The method of any of claims 24 to 26, wherein the polynucleotide sequence of the OCTN1 gene significantly reduces the ability of the encoded OCTN1 polypeptide to transport carnitine.

28. The method of any of claims 24 to 27, wherein the polynucleotide sequence of the OCTN2 gene contains at least one polymorphism selected from the group consisting of G-207C, 410 and TA.

29. The method of any of claims 24 to 28, wherein the polynucleotide sequence of the OCTN2 gene contains at least one polymorphism in the OCTN2 promoter region.

30. The method of any of claims 24 to 29, wherein the polymorphism alters the binding of heat shock transcription factor 1 (HSF1) to the OCTN2 promoter.

31. The method of any of claims 24 to 30, wherein the polymorphism of the OCTN2 gene downregulates basal transcription.

32. The method of any of claims 24 to 30, wherein the polymorphism of the OCTN2 gene downregulates transcription induced either by heat shock or by arachidonic acid.

33. The method of any of claims 24 to 30, wherein the polymorphism of the OCTN2 gene downregulates both basal transcription and transcription induced either by heat shock or by arachidonic acid.

34. A method for identifying an anti-inflammatory agent, comprising:
(a) forming a complex between the heat shock factor 1 (HSF1) protein and a polynucleotide comprising an OCTN2 promoter region;
(b) contacting the complex with an agent suspected of dissociating the complex; and
(c) detecting the dissociation of the complex, wherein the dissociation of the complex identifies the agent as being an agent that is an anti-inflammatory agent.

35. An in vitro method for detecting a nucleotide polymorphism in a mammalian OCTN1 gene or a mammalian OCTN2 gene associated with an Inflammatory Bowel Disease, which method comprises:
detecting a variation in a sequence of a gene encoding OCTN1 or OCTN2 obtained from a mammal diagnosed with or suspected of having Inflammatory Bowel Disease.

36. The method of claim 35, wherein the mammal is human.

37. The method of claim 35 or 36, wherein the expression of the OCTN1 gene is significantly downregulated in inflamed tissue in the mammal.

38. The method of any of claims 35 to 37, wherein the expression of the OCTN2 gene is significantly downregulated in inflamed tissue in the mammal.

39. A method for identifying a compound for treating an Inflammatory Bowel Disease, which method comprises:
(a) contacting a test compound with a system for measuring carnitine transport activity, which system comprises an OCTN1 or OCTN2 polypeptide or a functional fragment of an OCTN1 or OCTN2 polypeptide, and a substrate for measuring carnitine transport by the system; and
(b) detecting an increase in the carnitine transport activity in the presence of the test compound compared to the carnitine transport activity in the absence of the test compound.

40. The method of claim 39, wherein the test compound is a small molecule.

41. The method of claim 39, wherein the test compound is an intracellular factor that binds to the OCTN1 or the OCTN2 polypeptide.

42. The method of claim 39, wherein the test compound is an extracellular factor that binds to the OCTN1 or the OCTN2 polypeptide.

43. The method of any of claims 39 to 42, wherein the substrate for measuring carnitine transport is selected from the group consisting of carnitine, tetraethyl ammonium (TEA) and other compounds transported by the OCTN1 or the OCTN2 transporters.

44. The method of any of claims 39 to 43, wherein the OCTN1 polypeptide is encoded by a nucleotide sequence comprising a sequence selected from the group consisting of SEQ ID NO: 7 and functional fragments thereof containing a sequence corresponding to the L503F residue of SEQ ID NO: 7.

45. The method of any of claims 39 to 44, wherein the OCTN2 polypeptide is encoded by a nucleotide sequence comprising a sequence selected from the group consisting of SEQ ID NO: 8 and functional fragments thereof containing a sequence corresponding to the G-207C residue of SEQ ID NO: 8.

46. A method for identifying a compound for modulating carnitine transport, which method comprises:
(a) contacting a test compound with a system for measuring carnitine transport activity, which system comprises an OCTN1 or an OCTN2 polypeptide or a functional fragment of OCTN1 or an OCTN2 polypeptide, and a substrate for measuring carnitine transport by the system; and
(b) detecting a modulation in the carnitine transport activity in the presence of the test compound compared to the carnitine transport activity in the absence of the test compound.

47. The method of claim 46, wherein the compound enhances the transporter function of OCTN1.

48. The method of claim 46, wherein the compound reduces or blocks the transporter function of OCTN1.

49. The method of claim 46, wherein the compound enhances the transporter function of OCTN2.

50. The method of claim 46, wherein the compound reduces or blocks the transporter function of OCTN2.

51. A method for identifying a compound for treating an Inflammatory Bowel Disease, which method comprises:
(a) obtaining a transgenic mammal or a mutant mammal, which mammal has a nucleotide polymorphism in an OCTN1 gene or the OCTN2 gene, wherein the polymorphism in the OCTN1 gene or the OCTN2 gene reduces the tissue expression of the OCTN1 gene or the OCTN2 gene; wherein said mammal is a non-human mammal.
(b) contacting the mammal with a test compound; and
(c) detecting an improvement in a condition of the mammal in response to the test compound, wherein the condition is a symptom of an Inflammatory Bowel Disease.

52. The method of claim 51, wherein the mammal has a mutation in both the OCTN1 gene and the OCTN2 gene.

53. The method of claim 51 or 52, wherein the Inflammatory Bowel Disease is selected from the group consisting of Crohn's disease (CD), indeterminate colitis (IC) and ulcerative colitis (UC).

## Patentansprüche

1. Isoliertes OCTN 1-Polynukleotidfragment der Nukleinsäure codierend SEQ ID NO: 7, wobei das Fragment mindestens 20 Nukleotide in der Länge umfaßt und eine Sequenz enthält, die zu dem L503F Rest von SEQ ID NO: 7 korrespondiert.

2. Polynukleotidfragment gemäß Anspruch 1, umfassend SEQ ID NO: 21 oder SEQ ID NO: 22.

3. Isolierter OCTN 1-Primer, umfassend SEQ ID NO: 39 oder SEQ ID NO: 40.

4. Vektor, umfassend das Polynukleotid gemäß einem der Ansprüche 1 bis 3.

5. Herstellungsartikel, umfassend das Komplement des Polynukleotids gemäß Anspruch 1 als Polynukleotid-Hybridisierungssonde für die Diagnose einer entzündlichen Verdauungstrakterkrankung, unter stringenten Hybridisierungsbedingungen homolog zu und spezifisch bindend an ein OCTN 1-Polynukleotid.

6. Herstellungsartikel gemäß Anspruch 5, weiterhin umfassend eine Hybridisierungssonde, unter stringenten Hybridisierungsbedingungen homolog zu und spezifisch bindend an ein Polynukleotid, ausgewählt aus
1) SEQ ID NO: 1 oder Fragmenten davon von mindestens zwanzig Nukleotiden in der Länge von SEQ ID NO: 1,
2) SEQ ID NO: 36 oder Fragmenten davon von mindestens zwanzig Nukleotiden in der Länge,
3) SEQ ID NO: 37 oder Fragmenten davon von mindestens zwanzig Nukleotiden in der Länge,
4) dem Polynukleotid gemäß einem der Ansprüche 1 bis 4,
5) SEQ ID NO: 36, umfassend eine Nukleotidsequenz, codierend Phenylalanin an einer Position korrespondierend zu Position 503 von SEQ ID NO: 7 oder Fragmenten davon mit mindestens zwanzig Nukleotiden in der Länge, umfassend eine Nukleotidsequenz, codierend Phenylalanin an einer Position korrespondierend zu Position 503 von SEQ ID NO: 7,
6) SEQ ID NO: 37, umfassend eine Nukleotidsequenz, codierend Phenylalanin an einer Position korrespondierend zu Position 503 von SEQ ID NO: 7 oder Fragmenten davon mit mindestens zwanzig Nukleotiden in der Länge, umfassend eine Nukleotidsequenz, codierend Phenylalanin an einer Position korrespondierend zu Position 503 von SEQ ID NO: 7.

7. Isoliertes OCTN 2-Polynukleotidfragment gemäß SEQ ID NO: 8, wobei das Fragment mindestens zwanzig Nukleotide in der Länge umfaßt und eine Sequenz enthält, korrespondierend zu dem G-207C-Rest von SEQ ID NO: 8.

8. Polynukleotidfragment gemäß Anspruch 7, umfassend SEQ ID NO: 17 oder SEQ ID NO: 18.

9. Vektor, umfassend das Polynukleotid gemäß Anspruch 7 oder 8.

10. Herstellungsartikel, umfassend das Komplement des Polynukleotids gemäß Anspruch 7 als Polynukleotid-Hybridisierungssonde für die Diagnose einer entzündlichen Verdauungstrakterkrankung, unter stringenten Hybridisierungsbedingungen homolog zu und spezifisch bindend an ein OCTN 2-Polynukleotid.

11. Herstellungsartikel gemäß Anspruch 10, weiterhin umfassend eine Hybridisierungssonde, unter stringenten Hybridisierungsbedingungen homolog zu und spezifisch bindend an ein Polynukleotid, ausgewählt aus
1) SEQ ID NO: 3 oder Fragmenten davon von mindestens zwanzig Nukleotiden in der Länge,
2) SEQ ID NO: 23 oder Fragmenten davon von mindestens zwanzig Nukleotiden in der Länge,
3) dem Polynukleotid gemäß einem der Ansprüche 7 bis 9,
4) SEQ ID NO: 3, umfassend eine Nukleotidsequenz, korrespondierend zu dem G-207C-Rest von SEQ ID NO: 8 oder Fragmenten davon mit einer Länge von mindestens zwanzig Nukleotiden, umfassend eine Nukleotidsequenz, korrespondierend zu dem G-207C-Rest von SEQ ID NO: 8,
5) SEQ ID NO: 23, umfassend eine Nukleotidsequenz, korrespondierend zu dem G-207C-Rest von SEQ ID NO: 8 oder Fragmenten davon mit einer Länge von mindestens zwanzig Nukleotiden, umfassend eine Nukleotidsequenz, korrespondierend zu dem G-207C-Rest von SEQ ID NO: 8,
6) SEQ ID NO: 25.

12. In-vitro-Verfahren zur Bestimmung einer Empfänglichkeit gegenüber einer entzündlichen Verdauungstrakterkrankung (IBD) bei einem Säuger, umfassend:
(a) Kontaktieren einer biologischen Probe, die von einem Säuger erhalten wurde, mit einer Polynukleotidsonde, die selektiv an ein OCTN 1-Polynukleotid oder ein OCTN 2-Polynukleotid bindet, und
(b) Bestimmung der Expression des OCTN 1-Polynukleotids oder des OCTN 2-Polynukleotids als Maß der Empfänglichkeit des Säugers gegenüber einer entzündlichen Verdauungstrakterkrankung.

13. Verfahren gemäß Anspruch 12, wobei die Expression des OCTN 1-Polynukleotids oder des OCTN 2-Polynukleotids in der biologischen Probe bei Säugern mit einer Empfänglichkeit gegenüber einer entzündlichen Verdauungstrakterkrankung im Vergleich zu Säugern, die gegenüber der entzündlichen Verdauungstrakterkrankung nicht empfänglich sind, reduziert ist.

14. Verfahren gemäß Anspruch 12 oder 13, wobei die biologische Probe aus der Gruppe ausgewählt ist, bestehend aus einem Gastrointestinaltraktgewebe und einem Immunsystemgewebe.

15. Verfahren gemäß einem der Ansprüche 12 bis 14, wobei die biologische Probe von einem entzündeten Gewebe gewählt wird.

16. Verfahren gemäß einem der Ansprüche 12 bis 15, wobei der Säuger menschlich ist.

17. Verfahren gemäß einem der Ansprüche 12 bis 16, wobei die entzündliche Verdauungstrakterkrankung ausgewählt ist aus der Gruppe bestehend aus Morbus Crohn (CD), einer Colitis indeterminata (IC) und einer Colitis ulcerosa. (UC).

18. Verfahren gemäß einem der Ansprüche 12 bis 17, wobei die Bestimmung der Expression durch eine Northern-Analyse geschieht.

19. Verfahren gemäß einem der Ansprüche 12 bis 18, wobei die Bestimmung der Expression durch eine Polymerasekettenreaktion geschieht.

20. Verfahren gemäß einem der Ansprüche 12 bis 19, wobei die Expression von sowohl dem OCTN 1-Polynukleotid als auch dem OCTN 2-Polynukleotid in der biologischen Probe bestimmt wird.

21. Verfahren gemäß einem der Ansprüche 12 bis 20, wobei die Basaltranskription des OCTN 2-Gens herabreguliert ist.

22. Verfahren gemäß einem der Ansprüche 12 bis 21, wobei die Transkription des-OCTN 2-Gens, induziert entweder durch Hitzeschock oder durch Arachidonsäure, herabreguliert ist.

23. Verfahren gemäß einem der Ansprüche 12 bis 22, wobei sowohl die Basaltranskription des OCTN 2-Gens als auch die Transkription des OCTN 2-Gens, induziert entweder durch Hitzeschock oder durch Arachidonsäure, herabreguliert ist.

24. In-vitro-Verfahren zur Bestimmung einer Empfänglichkeit gegenüber einer entzündlichen Verdauungstrakterkrankung (IBD) bei einem Menschen, umfassend:
(a) Bestimmung der Polynukleotidsequenz des OCTN 1-Gens oder des OCTN 2-Gens für einen Menschen und
(b) Bestimmung, daß die Polynukleotidsequenz des OCTN 1-Gens oder des OCTN 2-Gens für den Menschen einen Polymorphismus enthält, der zu einer reduzierten Expression von OCTN 1 oder OCTN 2 beim Menschen führt.

25. Verfahren gemäß Anspruch 24, wobei die Polynukleotidsequenzen von sowohl dem OCTN 1-Gen als auch dem OCTN 2-Gen für den Menschen bestimmt werden.

26. Verfahren gemäß Anspruch 24 oder 25, wobei die Polynukleotidsequenz des OCTN 1-Gens mindestens einen L503F-Polymorphismus enthält.

27. Verfahren gemäß einem der Ansprüche 24 bis 26, wobei die Polynukleotidsequenz des OCTN 1-Gens die Fähigkeit des codierten OCTN 1-Polypeptids zum Transport von Carnitin signifikant reduziert.

28. Verfahren gemäß einem der Ansprüche 24 bis 27, wobei die Polynukleotidsequenz des OCTN 2-Gens mindestens einen Polymorphismus enthält, ausgewählt aus der Gruppe bestehend aus G-207C, 410 und TA.

29. Verfahren gemäß einem der Ansprüche 24 bis 28, wobei die Polynukleotidsequenz des OCTN 2-Gens mindestens einen Polymorphismus in der OCTN 2-Promotorregion enthält.

30. Verfahren gemäß einem der Ansprüche 24 bis 29, wobei der Polymorphismus die Bindung des Hitzeschock-Transkriptionsfaktors 1 (HSF1) an den OCTN 2-Promotor verändert.

31. Verfahren gemäß einem der Ansprüche 24 bis 30, wobei der Polymorphismus des OCTN 2-Gens die basale Transkription herabreguliert.

32. Verfahren gemäß einem der Ansprüche 24 bis 30, wobei der Polymorphismus des OCTN 2-Gens die Transkription herabreguliert, die entweder durch Hitzeschock oder durch Arachidonsäure induziert wird.

33. Verfahren gemäß einem der Ansprüche 24 bis 30, wobei der Polymorphismus des OCTN 2-Gens sowohl die basale Transkription als auch die entweder durch Hitzeschock oder durch Arachidonsäure induzierte Transkription herabreduziert.

34. Verfahren zur Identifizierung eines antientzündlichen Mittels, umfassend:
(a) Bildung eines Komplexes zwischen dem Hitzeschockfaktor 1-(HSF1)-protein und einem Polynukleotid, umfassend eine OCTN 2-Promotorregion;
(b) Kontaktieren des Komplexes mit einem Mittel, von dem man vermutet, daß es den Komplex dissoziiert; und
(c) Nachweis der Dissoziation des Komplexes, wobei die Dissoziation des Komplexes das Mittel als ein Mittel identifiziert, bei dem es sich um ein antientzündliches Mittel handelt.

35. In-vitro-Verfahren zum Nachweis eines Nukleotid-Polymorphismus bei einem Säuger OCTN 1-Gen oder einem Säuger OCTN 2-Gen, assoziiert mit einer entzündlichen Verdauungstrakterkrankung, wobei das Verfahren folgendes umfaßt:
Nachweis einer Variation in einer Sequenz eines Gens codierend OCTN 1 oder OCTN 2, erhalten von einem Säuger, der eine Diagnose einer entzündlichen Verdauungstrakterkrankung hat oder bei dem diese vermutet wird.

36. Verfahren gemäß Anspruch 35, wobei der Säuger menschlich ist.

37. Verfahren gemäß Anspruch 35 oder 36, wobei die Expression des OCTN 1-Gens in dem entzündeten Gewebe des Säugers signifikant herabreguliert ist.

38. Verfahren gemäß einem der Ansprüche 35 bis 37, wobei die Expression des OCTN 2-Gens in dem entzündeten Gewebe des Säugers signifikant herabreguliert ist.

39. Verfahren zur Identifizierung einer Verbindung zur Behandlung einer entzündlichen Verdauungstrakterkrankung, wobei das Verfahren folgendes umfaßt:
(a) Kontaktieren einer Testverbindung mit einem System zur Messung der Carnitin-Transportaktivität, wobei das System ein OCTN 1- oder OCTN 2-Polypeptid oder ein funktionelles Fragment eines OCTN 1- oder OCTN 2-Polypeptids umfaßt und ein Substrat zur Messung des Carnitin-Transports durch das System; und
(b) Nachweis eines Anstiegs der Carnitin-Transportaktivität in Gegenwart der Testverbindung im Vergleich zu der Carnitin-Transportaktivität in Abwesenheit der Testverbindung.

40. Verfahren gemäß Anspruch 39, wobei die Testverbindung ein kleines Molekül ist.

41. Verfahren gemäß Anspruch 39, wobei die Testverbindung ein intrazellulärer Faktor ist, der an das OCTN 1- oder OCTN 2-Polypeptid bindet.

42. Verfahren gemäß Anspruch 39, wobei die Testverbindung ein extrazellulärer Faktor ist, der an das OCTN 1- oder OCTN 2-Polypeptid bindet.

43. Verfahren gemäß einem der Ansprüche 39 bis 42, wobei das Substrat zur Messung des Carnitin-Transports ausgewählt ist aus der Gruppe bestehend aus Carnitin, Tetraethylammonium (TEA) und anderen Verbindungen, die durch die OCTN 1- oder OCTN 2-Transporter transportiert werden.

44. Verfahren gemäß einem der Ansprüche 39 bis 43, wobei das OCTN 1-Polypeptid durch eine Nukleotidsequenz codiert wird, umfassend eine Sequenz ausgewählt aus der Gruppe bestehend aus SEQ ID NO: 7 und funktionellen Fragmenten davon, enthaltend eine Sequenz korrespondierend zu dem L503F-Rest der SEQ ID NO: 7.

45. Verfahren gemäß einem der Ansprüche 39 bis 44, wobei das OCTN 2-Polypeptid durch eine Nukleotidsequenz codiert wird, umfassend eine Sequenz ausgewählt aus der Gruppe bestehend aus SEQ ID NO: 8 und funktionellen Fragmenten davon, enthaltend eine Sequenz korrespondierend zu dem G-207C-Rest der SEQ ID NO: 8.

46. Verfahren zur Identifizierung einer Verbindung zur Modulation des Carnitin-Transports, wobei das Verfahren folgendes umfaßt:
(a) Kontaktieren einer Testverbindung mit einem System zur Messung der Carnitin-Transportaktivität, wobei das System ein OCTN 1- oder ein OCTN 2-Polypeptid oder ein funktionelles Fragment von einem OCTN 1- oder einem OCTN 2-Polypeptid umfaßt und ein Substrat zur Messung des Carnitin-Transports durch das System; und
(b) Nachweis einer Modulation in der Carnitin-Transportaktivität in Gegenwart der Testverbindung im Vergleich zu der Carnitin-Transportaktivität in Abwesenheit der Testverbindung.

47. Verfahren gemäß Anspruch 46, wobei die Verbindung die Transporterfunktion von OCTN 1 verstärkt.

48. Verfahren gemäß Anspruch 46, wobei die Verbindung die Transporterfunktion von OCTN 1 reduziert oder blockiert.

49. Verfahren gemäß Anspruch 46, wobei die Verbindung die Transporterfunktion von OCTN 2 verstärkt.

50. Verfahren gemäß Anspruch 46, wobei die Verbindung die Transporterfunktion von OCTN 2 reduziert oder blockiert.

51. Verfahren zur Identifizierung einer Verbindung zur Behandlung einer entzündlichen Verdauungstrakterkrankung, wobei das Verfahren folgendes umfaßt:
(a) Erhalt eines transgenen Säugers oder eines mutierten Säugers, wobei der Säuger einen Nukleotid-Polymorphismus in einem OCTN 1- oder dem OCTN 2-Gen aufweist, wobei der Polymorphismus in dem OCTN 1-Gen oder dem OCTN 2-Gen die Gewebeexpression des OCTN 1-Gens oder des OCTN 2-Gens reduziert, wobei der Säuger ein nichtmenschlicher Säuger ist;
(b) Kontaktieren des Säugers mit einer Testverbindung; und
(c) Nachweis einer Verbesserung eines Zustands des Säugers als Reaktion auf die Testverbindung, wobei der Zustand ein Symptom einer entzündlichen Verdauungstrakterkrankung ist.

52. Verfahren gemäß Anspruch 51, wobei der Säuger eine Mutation sowohl in dem OCTN 1- als auch dem OCTN 2-Gen aufweist.

53. Verfahren gemäß Anspruch 51 oder 52, wobei die entzündliche Verdauungstrakterkrankung ausgewählt ist aus der Gruppe bestehend aus Morbus Crohn (CD), der Colitis indeterminata (IC) und der Colitis ulcerosa (UC).

## Revendications

1. Fragment de polynucléotide OCTN 1 isolé de l'acide nucléique codant pour SEQ ID No 7, dans lequel ledit fragment comprend au moins 20 nucléotides de long et contient une séquence correspondant au résidu L503F de SEQ ID No 7.

2. Fragment de polynucléotide selon la revendication 1, comprenant SEQ ID No: 21 ou SEQ ID No : 22.

3. Amorce OCTN 1 isolée comprenant SEQ ID No : 39 ou SEQ ID No : 40.

4. Vecteur comprenant le polynucléotide selon l'une quelconque des revendications 1 à 3.

5. Article de fabrication comprenant :
Le complément du polynucléotide selon la revendication 1 sous forme d'une sonde d'hybridation polynucléotidique pour le diagnostique d'une maladie intestinale inflammatoire, qui est homologue à et se lie spécifiquement dans des conditions d'hybridation rigoureuses à un polynucléotide OCTN 1.

6. Article de fabrication selon la revendication 5, comprenant en outre une sonde d'hybridation homologue à et se liant spécifiquement dans des conditions d'hybridation rigoureuses à un polynucléotide choisi parmi
1) SEQ ID No : 1 ou des fragments d'au moins vingt nucléotides de long de SEQ ID No : 1,
2) SEQ ID No : 36 ou des fragments de celle-ci d'au moins vingt nucléotides de long
3) SEQ ID No : 37 ou des fragments de celle-ci d'au moins vingt nucléotides de long
4) le polynucléotide selon l'une quelconque des revendications 1 à 4,
5) SEQ ID No : 36 comprenant une séquence nucléotidique codant pour la phénylalanine à une position correspondant à la position 503 de SEQ ID No : 7, ou des fragments de celle-ci d'au moins vingt nucléotides de long comprenant une séquence nucléotidique codant pour la phénylalanine à une position correspondant à la position 503 de SEQ ID No : 7,
6) SEQ ID No : 37 comprenant une séquence nucléotidique codant pour la phénylalanine à une position correspondant à la position 503 de SEQ ID No: 7, ou des fragments de celle-ci d'au moins vingt nucléotides de long comprenant une séquence nucléotidique codant pour la phénylalanine à une position correspondant à la position 503 de SEQ ID No: 7.

7. Fragment de polynucléotide OCTN 2 isolé de SEQ ID No : 8, dans lequel ledit fragment comprend au moins 20 nucléotides de long et contient une séquence correspondant au résidu G-207C de SEQ ID No : 8.

8. Fragment de polynucléotide selon la revendication 7, comprenant SEQ ID No : 17 ou SEQ ID No : 18.

9. Vecteur comprenant le polynucléotide selon la revendication 7 ou 8.

10. Article de fabrication comprenant
le complément du polynucléotide selon la revendication 7 sous forme d'une sonde d'hybridation polynucléotidique pour le diagnostique d'une maladie intestinale inflammatoire, qui est homologue à et se lie spécifiquement dans des conditions d'hybridation rigoureuses à un polynucléotide OCTN 2.

11. Article de fabrication selon la revendication 10, comprenant en outre une sonde d'hybridation homologue à et se liant spécifiquement dans des conditions d'hybridation rigoureuses à un polynucléotide choisi parmi
1) SEQ ID No : 3 ou des fragments de celle-ci d'au moins vingt nucléotides de long,
2) SEQ ID No : 23 ou des fragments de celle-ci d'au moins vingt nucléotides de long
3) le polynucléotide selon l'une quelconque des revendications 7 à 9,
4) SEQ ID No : 3 comprenant une séquence nucléotidique correspondant au résidu G-207C de SEQ ID No: 8, ou des fragments de celle-ci d'au moins vingt nucléotides de long, comprenant une séquence nucléotidique correspondant au résidu G-207C de SEQ ID No : 8,
5) SEQ ID No : 23 comprenant une séquence nucléotidique correspondant au résidu G-207C de SEQ ID No : 8, ou des fragments de celle-ci d'au moins vingt nucléotides de long comprenant une séquence nucléotidique correspondant au résidu G-207C de SEQ ID No : 8,
6) SEQ ID No : 25.

12. Procédé *in vitro* pour déterminer une susceptibilité à une maladie intestinale inflammatoire (MII) chez un mammifère, comprenant :
a) la mise en contact d'un échantillon biologique qui a été obtenu à partir d'un mammifère avec une sonde polynucléotidique qui se lie sélectivement à un polynucléotide OCTN 1 ou un polynucléotide OCTN 2 ; et
b) la détermination de l'expression du polynucléotide OCTN 1 ou du polynucléotide OCTN 2 comme mesure de la susceptibilité du mammifère à une maladie intestinale inflammatoire.

13. Procédé selon la revendication 12, dans lequel l'expression du polynucléotide OCTN 1 ou du polynucléotide OCTN 2 dans l'échantillon biologique est réduite chez les mammifères ayant une susceptibilité à une maladie intestinale inflammatoire par comparaison aux mammifères qui ne sont pas susceptibles à une maladie intestinale inflammatoire.

14. Procédé selon la revendication 12 ou 13, dans lequel l'échantillon biologique est choisi dans le groupe constitué de tissu du tractus gastro-intestinal et de tissu du système immunitaire.

15. Procédé selon l'une quelconque des revendications 12 à 14, dans lequel l'échantillon biologique est choisi parmi un tissu enflammé.

16. Procédé selon l'une quelconque des revendications 12 à 15, dans lequel le mammifère est humain.

17. Procédé selon l'une quelconque des revendications 12 à 16, dans lequel la maladie intestinale inflammatoire est choisie dans le groupe constitué de la maladie de Crohn (MC), la colite indéterminée (CI) et la colite ulcéreuse (CU).

18. Procédé selon l'une quelconque des revendications 12 à 17, dans lequel la détermination de l'expression s'effectue par une analyse de Northern.

19. Procédé selon l'une quelconque des revendications 12 à 18, dans lequel la détermination de l'expression s'effectue par une réaction en chaîne par polymérase.

20. Procédé selon l'une quelconque des revendications 12 à 19, dans lequel l'expression, à la fois du polynucléotide OCTN 1 et du polynucléotide OCTN 2 dans l'échantillon biologique est déterminée.

21. Procédé selon l'une quelconque des revendications 12 à 20, dans lequel la transcription de base du gène de l'OCTN 2 est régulée vers le bas.

22. Procédé selon l'une quelconque des revendications 12 à 21, dans lequel la transcription du gène de l'OCTN 2 induite par le choc thermique ou par l'acide arachidonique est régulée vers le bas.

23. Procédé selon l'une quelconque des revendications 12 à 22, dans lequel à la fois la transcription de base du gène de l'OCTN 2 et la transcription du gène de l'OCTN 2 induite par le choc thermique ou par l'acide arachidonique sont régulées vers le bas.

24. Procédé *in vitro* pour déterminer une susceptibilité à une maladie intestinale inflammatoire (MII), chez un humain, comprenant :
(a) déterminer la séquence polynucléotidique du gène de l'OCTN 1 ou du gène de l'OCTN 2 pour un humain ; et
(b) déterminer que la séquence polynucléotidique du gène de l'OCTN 1 ou du gène de l'OCTN 2 pour l'humain contient un polymorphisme qui aboutit à une expression réduite de l'OCTN 1 ou l'OCTN 2 chez l'humain.

25. Procédé selon la revendication 24, dans lequel les séquences polynucléotidiques du gène de l'OCTN 1 et du gène de l'OCTN 2 à la fois sont déterminées pour l'humain.

26. Procédé selon la revendication 24 ou 25, dans lequel la séquence polynucléotidique du gène de l'OCTN 1 contient au moins un polymorphisme L503F.

27. Procédé selon l'une quelconque des revendications 24 à 26, dans lequel la séquence polynucléotidique du gène de l'OCTN 1 réduit significativement la capacité du polypeptide OCTN 1 codé à transporter la carnitine.

28. Procédé selon l'une quelconque des revendications 24 à 27, dans lequel la séquence polynucléotidique du gène de l'OCTN 2 contient au moins un polymorphisme choisi dans le groupe constitué de G-207C, 410 et TA.

29. Procédé selon l'une quelconque des revendications 24 à 28, dans lequel la séquence polynucléotidique du gène de l'OCTN 2 contient au moins un polymorphisme dans la région promotrice de l'OCTN 2.

30. Procédé selon l'une quelconque des revendications 24 à 29, dans lequel le polymorphisme modifie la liaison du facteur de transcription du choc thermique 1 (HSF1) au promoteur de l'OCTN 2.

31. Procédé selon l'une quelconque des revendications 24 à 30, dans lequel le polymorphisme du gène de l'OCTN 2 régule vers le bas la transcription de base.

32. Procédé selon l'une quelconque des revendications 24 à 30, dans lequel le polymorphisme du gène de l'OCTN 2 régule vers le bas la transcription induite par le choc thermique ou par l'acide arachidonique.

33. Procédé selon l'une quelconque des revendications 24 à 30, dans lequel le polymorphisme du gène de l'OCTN 2 régule vers le bas à la fois la transcription de base et la transcription induite par le choc thermique ou par l'acide arachidonique.

34. Procédé pour identifier un agent anti-inflammatoire, comprenant :
(a) la formation d'un complexe entre la protéine facteur de choc thermique 1 (HSF1) et un polynucléotide comprenant une région promotrice de l'OCTN 2 ;
(b) la mise en contact du complexe avec un agent soupçonné de dissocier le complexe; et
(c) la détection de la dissociation du complexe, dans laquelle la dissociation du complexe identifie l'agent comme étant un agent qui est un agent anti-inflammatoire.

35. Procédé in vitro pour détecter un polymorphisme nucléotidique dans un gène de l'OCTN 1 de mammifère ou un gène de l'OCTN 2 de mammifère associé à une maladie intestinale inflammatoire, lequel procédé comprend :
la détection d'une variation dans une séquence d'un gène codant pour l'OCTN 1 ou l'OCTN 2 obtenu à partir d'un mammifère diagnostiqué avec ou soupçonné d'être atteint d'une maladie intestinale inflammatoire.

36. Procédé selon la revendication 35, dans lequel le mammifère est humain.

37. Procédé selon la revendication 35 ou 36, dans lequel l'expression du gène de l'OCTN 1 est significativement régulée vers le bas dans le tissu enflammé chez le mammifère.

38. Procédé selon l'une quelconque des revendications 35 à 37, dans lequel l'expression du gène de l'OCTN 2 est significativement régulée vers le bas dans le tissu enflammé chez le mammifère.

39. Procédé pour identifier un composé destiné à traiter une maladie intestinale inflammatoire, lequel procédé comprend :
(a) la mise en contact d'un composé test avec un système pour mesurer l'activité de transport de la carnitine, lequel système comprend un polypeptide OCTN 1 ou OCTN 2 ou un fragment fonctionnel d'un polypeptide OCTN 1 ou OCTN 2, et un substrat pour mesurer le transport de la carnitine par le système ; et
(b) la détection d'une augmentation de l'activité de transport de la carnitine en présence du composé test par rapport à l'activité de transport de la carnitine en l'absence du composé test.

40. Procédé selon la revendication 39, dans lequel le composé test est une petite molécule.

41. Procédé selon la revendication 39, dans lequel le composé test est un facteur intracellulaire qui se lie au polypeptide OCTN 1 ou OCTN 2.

42. Procédé selon la revendication 39, dans lequel le composé test est un facteur extracellulaire qui se lie au polypeptide OCTN 1 ou OCTN 2.

43. Procédé selon l'une quelconque des revendications 39 à 42, dans lequel le substrat pour mesurer le transport de la carnitine est choisi dans le groupe constitué de la carnitine, du tétraéthyl ammonium (TEA) et autres composés transportés par les transporteurs OCTN 1 ou OCTN 2.

44. Procédé selon l'une quelconque des revendications 39 à 43, dans lequel le polypeptide OCTN 1 est codé par une séquence nucléotidique comprenant une séquence choisie dans le groupe constitué de SEQ ID No : 7 et des fragments fonctionnels de celle-ci contenant une séquence correspondant au résidu L503F de SEQ ID No : 7.

45. Procédé selon l'une quelconque des revendications 39 à 44, dans lequel le polypeptide OCTN 2 est codé par une séquence nucléotidique comprenant une séquence choisie dans le groupe constitué de SEQ ID No : 8 et des fragments fonctionnels de celle-ci contenant une séquence correspondant au résidu G-207C de SEQ ID No : 8.

46. Procédé pour identifier un composé destiné à moduler le transport de la carnitine, lequel procédé comprend :
(a) la mise en contact d'un composé test avec un système pour mesurer l'activité de transport de la carnitine, lequel système comprend un polypeptide OCTN 1 ou OCTN 2 ou un fragment fonctionnel d'un polypeptide OCTN 1 ou OCTN 2, et un substrat pour mesurer le transport de la carnitine par le système ; et
(b) la détection d'une modulation de l'activité de transport de la carnitine en présence du composé test par rapport à l'activité de transport de la carnitine en l'absence du composé test.

47. Procédé selon la revendication 46, dans lequel le composé augmente la fonction de transporteur de l'OCTN 1.

48. Procédé selon la revendication 46, dans lequel le composé réduit ou bloque la fonction de transporteur de l'OCTN 1.

49. Procédé selon la revendication 46, dans lequel le composé augmente la fonction de transporteur de l'OCTN 2.

50. Procédé selon la revendication 46, dans lequel le composé réduit ou bloque la fonction de transporteur de l'OCTN 2 .

51. Procédé pour identifier un composé destiné à traiter une maladie intestinale inflammatoire, lequel procédé comprend:
(a) l'obtention d'un mammifère transgénique ou d'un mammifère mutant, lequel mammifère présente un polymorphisme nucléotidique dans un gène de l'OCTN 1 ou le gène de l'OCTN 2, dans lequel le polymorphisme dans le gène de l'OCTN 1 ou le gène de l'OCTN 2 réduit l'expression tissulaire du gène de l'OCTN 1 ou du gène de l'OCTN 2 ; dans lequel ledit mammifère est un animal non humain.
(b) la mise en contact du mammifère avec un composé test ; et
(c) la détection d'une amélioration dans un état du mammifère en réponse au composé test, dans lequel l'état est un symptôme d'une maladie intestinale inflammatoire.

52. Procédé selon la revendication 51, dans lequel le mammifère présente une mutation à la fois dans le gène de l'OCTN 1 et le gène de l'OCTN 2.

53. Procédé selon la revendication 51 ou 52, dans lequel la maladie intestinale inflammatoire est choisie dans le groupe constitué de la maladie de Crohn (MC), la colite indéterminée (CI) et la colite ulcéreuse (CU).


### SEQUENCE LISTING

<110> Ellipsis Biotherapeutics Corporation
   Peltekova, Vanya D
   Siminovitch, Katherine A
   St George-Hyslop, Peter H
   Rubin, Laurence A
   Peltekova, Vanya D
   Wintle, Richard F
<120> POLYMORPHISMS OF THE OCTN1 AND OCTN2 CATION TRANSPORTERS ASSOCIATED WITH INFLAMMATORY BOWEL DISORDERS
<130> ELLP-020PC
<140> PCT/IB02/05560
   <141> 2002-12-20
<150> 60/362,700
   <151> 2002-03-08
<150> 60/343,338
   <151> 2001-12-21
<150> 60/427,529
   <151> 2002-11-19
<150> 60/362,717
   <151> 2002-03-08
<160> 42
<170> Patent In version 3.1
<210> 1
   <211> 2214
   <212> DNA
   <213> Homo sapiens
<220>
   <221> CDS
   <222> (166)..(1821)
   <223>
<400> 1
   cctgtttccc aggaacggtc cccggcttcg cgccccaatt tctaacagcc tgcctgtccc     60
   ccgggaacgt tctaacatcc ttggggagcg ccccagctac aagacactgt cctgagaacg    120
   ctgtcatcac ccgtagttgc aagtttcgga gcggcagtgg gaagc atg cgg gac tac    177
                                                     Met Arg Asp Tyr
                                                     1
   gac gag gtg atc gcc ttc ctg ggc gag tgg ggg ccc ttc cag cgc ctc      225
   Asp Glu Val Ile Ala Phe Leu Gly Glu Trp Gly Pro Phe Gln Arg Leu
   5                   10                  15                  20
   atc ttc ttc ctg ctc agc gcc agc atc atc ccc aat ggc ttc aat ggt      273
   Ile Phe Phe Leu Leu Ser Ala Ser Ile Ile Pro Asn Gly Phe Asn Gly
                   25                  30                  35
   atg tca gtc gtg ttc ctg gcg ggg acc ccg gag cac cgc tgt cga gtg      321
   Met Ser Val Val Phe Leu Ala Gly Thr Pro Glu His Arg Cys Arg Val
               40                  45                  50
   ccg gac gcc gcg aac ctg agc agc gcc tgg cgc aac aac agt gtc ccg      369
   Pro Asp Ala Ala Asn Leu Ser Ser Ala Trp Arg Asn Asn Ser Val Pro
           55                  60                  65
   ctg cgg ctg cgg gac ggc cgc gag gtg ccc cac agc tgc agc cgc tac      417
   Leu Arg Leu Arg Asp Gly Arg Glu Val Pro His Ser Cys Ser Arg Tyr
       70                  75                  80
   cgg ctc gcc acc atc gcc aac ttc tcg gcg ctc ggg ctg gag ccg ggg      465
   Arg Leu Ala Thr Ile Ala Asn Phe Ser Ala Leu Gly Leu Glu Pro Gly
   85                  90                  95                  100
   cgc gac gtg gac ctg ggg cag ctg gag cag gag agc tgc ctg gat ggc      513
   Arg Asp Val Asp Leu Gly Gln Leu Glu Gln Glu Ser Cys Leu Asp Gly
                   105                 110                 115
   tgg gag ttc agc cag gac gtc tac ctg tcc acc gtc gtg acc gag tgg      561
   Trp Glu Phe Ser Gln Asp Val Tyr Leu Ser Thr Val Val Thr Glu Trp
               120                 125                 130
   aat ctg gtg tgt gag gac aac tgg aag gtg ccc ctc acc acc tcc ctg      609
   Asn Leu Val Cys Glu Asp Asn Trp Lys Val Pro Leu Thr Thr Ser Leu
           135                 140                 145
   ttc ttc gta ggc gtg ctc ctc ggc tcc ttc gtg tcc ggg cag ctg tca      657
   Phe Phe Val Gly Val Leu Leu Gly Ser Phe Val Ser Gly Gln Leu Ser
       150                 155                 160
   gac agg ttt ggc agg aag aac gtt ctc ttc gca acc atg gct gta cag      705
   Asp Arg Phe Gly Arg Lys Asn Val Leu Phe Ala Thr Met Ala Val Gln
   165                 170                 175                 180
   act ggc ttc agc ttc ctg cag att ttc tcc atc agc tgg gag atg ttc      753
   Thr Gly Phe Ser Phe Leu Gln Ile Phe Ser Ile Ser Trp Glu Met Phe
                   185                 190                 195
   act gtg tta ttt gtc atc gtg ggc atg ggc cag atc tcc aac tat gtg      801
   Thr Val Leu Phe Val Ile Val Gly Met Gly Gln Ile Ser Asn Tyr Val
               200                 205                 210
   gta gcc ttc ata cta gga aca gaa att ctt ggc aag tca gtt cgt att      849
   Val Ala Phe Ile Leu Gly Thr Glu Ile Leu Gly Lys Ser Val Arg Ile
           215                 220                 225
   ata ttc tct aca tta gga gtg tgc aca ttt ttt gca gtt ggc tat atg      897
   Ile Phe Ser Thr Leu Gly Val Cys Thr Phe Phe Ala Val Gly Tyr Met
       230                 235                 240
   ctg ctg cca ctg ttt gct tac ttc atc aga gac tgg cgg atg ctg ctg      945
   Leu Leu Pro Leu Phe Ala Tyr Phe Ile Arg Asp Trp Arg Met Leu Leu
   245                 250                 255                 260
   ctg gcg ctg acg gtg ccg gga gtg ctg tgt gtc ccg ctg tgg tgg ttc      993
   Leu Ala Leu Thr Val Pro Gly Val Leu Cys Val Pro Leu Trp Trp Phe
                   265                 270                 275
   att cct gaa tct ccc cga tgg ctg ata tcc cag aga aga ttt aga gag      1041
   Ile Pro Glu Ser Pro Arg Trp Leu Ile Ser Gln Arg Arg Phe Arg Glu
               280                 285                 290
   gct gaa gat atc atc caa aaa gct gca aaa atg aac aac ata gct gta      1089
   Ala Glu Asp Ile Ile Gln Lys Ala Ala Lys Met Asn Asn Ile Ala Val
           295                 300                 305
   cca gca gtg ata ttt gat tct gtg gag gag cta aat ccc ctg aag cag      1137
   Pro Ala Val Ile Phe Asp Ser Val Glu Glu Leu Asn Pro Leu Lys Gln
       310                 315                 320
   cag aaa gct ttc att ctg gac ctg ttc agg act cgg aat att gcc ata      1185
   Gln Lys Ala Phe Ile Leu Asp Leu Phe Arg Thr Arg Asn Ile Ala Ile
   325                 330                 335                 340
   atg acc att atg tct ttg ctg cta tgg atg ctg acc tca gtg ggt tac      1233
   Met Thr Ile Met Ser Leu Leu Leu Trp Met Leu Thr Ser Val Gly Tyr
                   345                 350                 355
   ttt gct ctg tct ctg gat gct cct aat tta cat gga gat gcc tac ctg      1281
   Phe Ala Leu Ser Leu Asp Ala Pro Asn Leu His Gly Asp Ala Tyr Leu
               360                 365                 370
   aac tgt ttc ctc tct gcc ttg att gaa att cca gct tac att aca gcc      1329
   Asn Cys Phe Leu Ser Ala Leu Ile Glu Ile Pro Ala Tyr Ile Thr Ala
           375                 380                 385
   tgg ctg cta ttg cga acc ctg ccc agg cgt tat atc ata gct gca gta      1377
   Trp Leu Leu Leu Arg Thr Leu Pro Arg Arg Tyr Ile Ile Ala Ala Val
       390                 395                 400
   ctg ttc tgg gga gga ggt gtg ctt ctc ttc att caa ctg gta cct gtg      1425
   Leu Phe Trp Gly Gly Gly Val Leu Leu Phe Ile Gln Leu Val Pro Val
   405                 410                 415                 420
   gat tat tac ttc tta tcc att ggt ctg gtc atg ctg gga aaa ttt ggg      1473
   Asp Tyr Tyr Phe Leu Ser Ile Gly Leu Val Met Leu Gly Lys Phe Gly
                   425                 430                 435
   atc acc tct gct ttc tcc atg ctg tat gtc ttc act gct gag ctc tac      1521
   Ile Thr Ser Ala Phe Ser Met Leu Tyr Val Phe Thr Ala Glu Leu Tyr
               440                 445                 450
   cca acc ctg gtc agg aac atg gcg gtg ggg gtc aca tcc acg gcc tcc      1569
   Pro Thr Leu Val Arg Asn Met Ala Val Gly Val Thr Ser Thr Ala Ser
           455                 460                 465
   aga gtg ggc agc atc att gcc ccc tac ttt gtt tac ctc ggt gct tac      1617
   Arg Val Gly Ser Ile Ile Ala Pro Tyr Phe Val Tyr Leu Gly Ala Tyr
       470                 475                 480
   aac aga atg ctg ccc tac atc gtc atg ggt agt ctg act gtc ctg att      1665
   Asn Arg Met Leu Pro Tyr Ile Val Met Gly Ser Leu Thr Val Leu Ile
   485                 490                 495                 500
   gga atc ctc acc ctt ttt ttc cct gaa agt ttg gga atg act ctt cca      1713
   Gly Ile Leu Thr Leu Phe Phe Pro Glu Ser Leu Gly Met Thr Leu Pro
                   505                 510                 515
   gaa acc tta gag cag atg cag aaa gtg aaa tgg ttc aga tct ggg aaa      1761
   Glu Thr Leu Glu Gln Met Gln Lys Val Lys Trp Phe Arg Ser Gly Lys
               520                 525                 530
   aaa aca aga gac tca atg gag aca gaa gaa aat ccc aag gtt cta ata      1809
   Lys Thr Arg Asp Ser Met Glu Thr Glu Glu Asn Pro Lys Val Leu Ile
           535                 540                 545
   act gca ttc tga aaaaatatct accccatttg gtgaagtgaa aaacagaaaa          1861
   Thr Ala Phe
       550
   ataagaccct gtggagaaat tcgttgttcc cactgaaatg gactgactgt aacgattgac    1921
   accaaaatga accttgctat caagaaatgc tcgtcataca gtaaactctg gatgattctt    1981
   ccagataatg tccttgcttt acaaaccaac catttctaga gagtctcctt actcattaat    2041
   tcaatgaaat ggattggtaa gatgtcttga aaacatgtta gtcaaggact ggtaaaatac    2101
   atataaagat taacactcat ttccaatcat acaaatacta tccaaataaa aataacatca    2161
   ttgtattaac gcaaatatta ggtgacaaca aaaaaaaaaa aaaaaaaaaa aaa           2214
<210> 2
   <211> 551
   <212> PRT
   <213> Homo sapiens
<400> 2
   Met Arg Asp Tyr Asp Glu Val Ile Ala Phe Leu Gly Glu Trp Gly Pro
   1               5                   10                  15
   Phe Gln Arg Leu Ile Phe Phe Leu Leu Ser Ala Ser Ile Ile Pro Asn
               20                  25                  30
   Gly Phe Asn Gly Met Ser Val Val Phe Leu Ala Gly Thr Pro Glu His
           35                  40                  45
   Arg Cys Arg Val Pro Asp Ala Ala Asn Leu Ser Ser Ala Trp Arg Asn
       50                  55                  60
   Asn Ser Val Pro Leu Arg Leu Arg Asp Gly Arg Glu Val Pro His Ser
   65                  70                  75                  80
   Cys Ser Arg Tyr Arg Leu Ala Thr Ile Ala Asn Phe Ser Ala Leu Gly
                   85                  90                  95
   Leu Glu Pro Gly Arg Asp Val Asp Leu Gly Gln Leu Glu Gln Glu Ser
               100                 105                 110
   Cys Leu Asp Gly Trp Glu Phe Ser Gln Asp Val Tyr Leu Ser Thr Val
           115                 120                 125
   Val Thr Glu Trp Asn Leu Val Cys Glu Asp Asn Trp Lys Val Pro Leu
   130 135 140
   Thr Thr Ser Leu Phe Phe Val Gly Val Leu Leu Gly Ser Phe Val Ser
   145 150 155 160
   Gly Gln Leu Ser Asp Arg Phe Gly Arg Lys Asn Val Leu Phe Ala Thr
   165 170 175
   Met Ala Val Gln Thr Gly Phe Ser Phe Leu Gln Ile Phe Ser Ile Ser
   180 185 190
   Trp Glu Met Phe Thr Val Leu Phe Val Ile Val Gly Met Gly Gln Ile
   195 200 205
   Ser Asn Tyr Val Val Ala Phe Ile Leu Gly Thr Glu Ile Leu Gly Lys
   210 215 220
   Ser Val Arg Ile Ile Phe Ser Thr Leu Gly Val Cys Thr Phe Phe Ala
   225 230 235 240
   Val Gly Tyr Met Leu Leu Pro Leu Phe Ala Tyr Phe Ile Arg Asp Trp
   245 250 255
   Arg Met Leu Leu Leu Ala Leu Thr Val Pro Gly Val Leu Cys Val Pro
   260 265 270
   Leu Trp Trp Phe Ile Pro Glu Ser Pro Arg Trp Leu Ile Ser Gln Arg
   275 280 285
   Arg Phe Arg Glu Ala Glu Asp Ile Ile Gln Lys Ala Ala Lys Met Asn
   290 295 300
   Asn Ile Ala Val Pro Ala Val Ile Phe Asp Ser Val Glu Glu Leu Asn
   305 310 315 320
   Pro Leu Lys Gln Gln Lys Ala Phe Ile Leu Asp Leu Phe Arg Thr Arg
   325 330 335
   Asn Ile Ala Ile Met Thr Ile Met Ser Leu Leu Leu Trp Met Leu Thr
   340 345 350
   Ser Val Gly Tyr Phe Ala Leu Ser Leu Asp Ala Pro Asn Leu His Gly
   355 360 365
   Asp Ala Tyr Leu Asn Cys Phe Leu Ser Ala Leu Ile Glu Ile Pro Ala
   370 375 380
   Tyr Ile Thr Ala Trp Leu Leu Leu Arg Thr Leu Pro Arg Arg Tyr Ile
   385 390 395 400
   Ile Ala Ala Val Leu Phe Trp Gly Gly Gly Val Leu Leu Phe Ile Gln
   405 410 415
   Leu Val Pro Val Asp Tyr Tyr Phe Leu Ser Ile Gly Leu Val Met Leu
   420 425 430
   Gly Lys Phe Gly Ile Thr Ser Ala Phe Ser Met Leu Tyr Val Phe Thr
   435 440 445
   Ala Glu Leu Tyr Pro Thr Leu Val Arg Asn Met Ala Val Gly Val Thr
   450 455 460
   Ser Thr Ala Ser Arg Val Gly Ser Ile Ile Ala Pro Tyr Phe Val Tyr
   465 470 475 480
   Leu Gly Ala Tyr Asn Arg Met Leu Pro Tyr Ile Val Met Gly Ser Leu
   485 490 495
   Thr Val Leu Ile Gly Ile Leu Thr Leu Phe Phe Pro Glu Ser Leu Gly
   500 505 510
   Met Thr Leu Pro Glu Thr Leu Glu Gln Met Gln Lys Val Lys Trp Phe
   515 520 525
   Arg Ser Gly Lys Lys Thr Arg Asp Ser Met Glu Thr Glu Glu Asn Pro
   530 535 540
   Lys Val Leu Ile Thr Ala Phe
   545 550
<210> 3
   <211> 3252
   <212> DNA
   <213> Homo sapiens
<220>
   <221> CDS
   <222> (222)..(1895)
   <223>
<400> 3
gcggcccagg cccgcaacct tccctggtcg tgcgccctat gtaaggccag ccgcggcagg     60
   accaaggcgg cggtgtcagc tcgcgagcct accctccgcg gacggtcttg ggtcgcctgc    120
   tgcctggctt gcctggtcgg cggcgggtgc cccgcgcgca cgcgcaaagc ccgccgcgtt    180
   ccccgacccc aggccgcgct ctgtgggcct ctgagggcgg c atg cgg gac tac gac    236
   gag gtg acc gcc ttc ctg ggc gag tgg ggg ccc ttc cag cgc ctc atc      284
   ttc ttc ctg ctc agc gcc agc atc atc ccc aat ggc ttc acc ggc ctg      332
   tcc tcc gtg ttc ctg ata gcg acc ccg gag cac cgc tgc cgg gtg ccg      380
   gac gcc gcg aac ctg agc agc gcc tgg cgc aac cac act gtc cca ctg      428
   cgg ctg cgg gac ggc cgc gag gtg ccc cac agc tgc cgc cgc tac cgg      476
   ctc gcc acc atc gcc aac ttc tcg gcg ctt ggg ctg gag ccg ggg cgc      524
   gac gtg gac ctg ggg cag ctg gag cag gag agc tgt ctg gat ggc tgg      572
   gag ttc agt cag gac gtc tac ctg tcc acc att gtg acc gag tgg aac      620
   ctg gtg tgt gag gac gac tgg aag gcc cca ctc aca atc tcc ttg ttc      668
   ttc gtg ggt gtg ctg ttg ggc tcc ttc att tca ggg cag ctg tca gac      716
   agg ttt ggc cgg aag aat gtg ctg ttc gtg acc atg ggc atg cag aca      764
   ggc ttc agc ttc ctg cag atc ttc tcg aag aat ttt gag atg ttt gtc      812
   gtg ctg ttt gtc ctt gta ggc atg ggc cag atc tcc aac tat gtg gca      860
   gca ttt gtc ctg ggg aca gaa att ctt ggc aag tca gtt cgt ata ata      908
   ttc tct acg tta gga gtg tgc ata ttt tat gca ttt ggc tac atg gtg      956
   ctg cca ctg ttt gct tac ttc atc cga gac tgg cgg atg ctg ctg gtg     1004
   gcg ctg acg atg ccg ggg gtg cta tgc gtg gca ctc tgg tgg ttc atc     1052
   cct gag tcc ccc cga tgg ctc atc tct cag gga cga ttt gaa gag gca     1100
   gag gtg atc atc cgc aag gct gcc aaa gcc aat ggg att gtt gtg cct     1148
   tcc act atc ttt gac ccg agt gag tta caa gac cta agt tcc aag aag     1196
   cag cag tcc cac aac att ctg gat ctg ctt cga acc tgg aat atc cgg     1244
   atg gtc acc atc atg tcc ata atg ctg tgg atg acc ata tca gtg ggc     1292
   tat ttt ggg ctt tcg ctt gat act cct aac ttg cat ggg gac atc ttt     1340
   gtg aac tgc ttc ctt tca gcg atg gtt gaa gtc cca gca tat gtg ttg     1388
   gcc tgg ctg ctg ctg caa tat ttg ccc cgg cgc tat tcc atg gcc act     1436
   gcc ctc ttc ctg ggt ggc agt gtc ctt ctc ttc atg cag ctg gta ccc     1484
   cca gac ttg tat tat ttg gct aca gtc ctg gtg atg gtg ggc aag ttt     1532
   gga gtc acg gct gcc ttt tcc atg gtc tac gtg tac aca gcc gag ctg     1580
   tat ccc aca gtg gtg aga aac atg ggt gtg gga gtc agc tcc aca gca      1628
   tcc cgc ctg ggc agc atc ctg tct ccc tac ttc gtt tac ctt ggt gcc      1676
   tac gac cgc ttc ctg ccc tac att ctc atg gga agt ctg acc atc ctg      1724
   aca gcc atc ctc acc ttg ttt ctc cca gag agc ttc ggt acc cca ctc      1772
   cca gac acc att gac cag atg cta aga gtc aaa gga atg aaa cac aga      1820
   aaa act cca agt cac aca agg atg tta aaa gat ggt caa gaa agg ccc      1868
   aca atc ctt aaa agc aca gcc ttc taa catcgcttcc agtaagggag            1915
   aaactgaaga ggaaagactg tcttgccaga aatggccagc ttgtgcagac tccgagtcct   1975
   tcagtgacaa aggcctttgc tgtttgtcct cttgacctgt gtctgacttg ctcctggatg   2035
   ggcacccaca ctcagaggct acatatggcc ctagagcacc accttcctct agggacactg   2095
   gggctaccta cagacaactt catctaagtc ctaactatta caatgatgga ctcagcacct   2155
   ccaaagcagt taatttttca ctagaaccag tgagatctgg aggaatgtga gaagcatatg   2215
   ctaaatgtac attttaattt tagactactt gaaaaggccc ctaataaggc tagaggtcta   2275
   agtcccccac ccctttcccc actcccctct agtggtgaac tttagaggaa aaggaagtaa   2335
   ttgcacaagg agtttgattc ttaccttttc tcagttacag aggacattaa ctggatcatt   2395
   gcttccccag ggcaggagag cgcagagcta gggaaagtga aaggtaatga agatggagca   2455
   gaatgagcag atgcagatca ccagcaaagt gcactgatgt gtgagctctt aagaccactc   2515
   agcatgacga ctgagtagac ttgtttacat ctgatcaaag cactgggctt gtccaggctc   2575
   ataataaatg ctccattgaa tctactattc ttgttttcca ctgctgtgga aacctccttg   2635
   ctactatagc gtcttatgta tggtttaaag gaaatttatc aggtgagaga gatgagcaac   2695
   gttgtctttt ctctcaaagc tgtaatgtgg gttttgtttt attgtttatt tgtttgttgt   2755
   tgtatccttt tctccttgtt atttgccctt cagaatgcac ttgggaaagg ctggttcctt   2815
   agcctcctgg tttgtgtctt tttttttttt tttttaaaca cagaatcact ctggcaattg   2875
   tctgcagctg ccactggtgc aaggccttac cagccctagc ctctagcact tctctaagtg   2935
   ccaaaaacag tgtcattgtg tgtgttcctt tcttgatact tagtcatggg aggatattac 2995
   aaaaaagaaa tttaaattgt gttcatagtc tttcagagta gctcacttta gtcctgtaac 3055
   tttattgggt gatattttgt gttcagtgta attgtcttct ctttgctgat tatgttacca 3115
   tggtactcct aaagcatatg cctcacctgg ttaaaaaaga acaaacatgt ttttgtgaaa 3175
   gctactgaag tgccttggga aatgagaaag ttttaataag taaaatgatt ttttaaatat 3235
   caaaaaaaaa aaaaaaa 3252
<210> 4
   <211> 557
   <212> PRT
   <213> Homo sapiens
<400> 4
<210> 5
   <211> 24
   <212> DNA
   <213> Homo sapiens
<400> 5
   ctgcacggaa ataactaatc tgtg 24
<210> 6
   <211> 20
   <212> DNA
   <213> Homo sapiens
<400> 6
   gagaggagct cgggttcaag 20
<210> 7
   <211> 551
   <212> PRT
   <213> Homo sapiens
<400> 7
   Met Arg Asp Tyr Asp Glu Val Ile Ala Phe Leu Gly Glu Trp Gly Pro
   1 5 10 15
   Phe Gln Arg Leu Ile Phe Phe Leu Leu Ser Ala Ser Ile Ile Pro Asn
   20 25 30
   Gly Phe Asn Gly Met Ser Val Val Phe Leu Ala Gly Thr Pro Glu His
   35 40 45
   Arg Cys Arg Val Pro Asp Ala Ala Asn Leu Ser Ser Ala Trp Arg Asn
   50 55 60
   Asn Ser Val Pro Leu Arg Leu Arg Asp Gly Arg Glu Val Pro His Ser
   65 70 75 80
   Cys Ser Arg Tyr Arg Leu Ala Thr Ile Ala Asn Phe Ser Ala Leu Gly
   85 90 95
   Leu Glu Pro Gly Arg Asp Val Asp Leu Gly Gln Leu Glu Gln Glu Ser
   100 105 110
   Cys Leu Asp Gly Trp Glu Phe Ser Gln Asp Val Tyr Leu Ser Thr Val
   115 120 125
   Val Thr Glu Trp Asn Leu Val Cys Glu Asp Asn Trp Lys Val Pro Leu
   130 135 140
   Thr Thr Ser Leu Phe Phe Val Gly Val Leu Leu Gly Ser Phe Val Ser
   145 150 155 160
   Gly Gln Leu Ser Asp Arg Phe Gly Arg Lys Asn Val Leu Phe Ala Thr
   165 170 175
   Met Ala Val Gln Thr Gly Phe Ser Phe Leu Gln Ile Phe Ser Ile Ser
   180 185 190
   Trp Glu Met Phe Thr Val Leu Phe Val Ile Val Gly Met Gly Gln Ile
   195 200 205
   Ser Asn Tyr Val Val Ala Phe Ile Leu Gly Thr Glu Ile Leu Gly Lys
   210 215 220
   Ser Val Arg Ile Ile Phe Ser Thr Leu Gly Val Cys Thr Phe Phe Ala
   225 230 235 240
   Val Gly Tyr Met Leu Leu Pro Leu Phe Ala Tyr Phe Ile Arg Asp Trp
   245 250 255
   Arg Met Leu Leu Leu Ala Leu Thr Val Pro Gly Val Leu Cys Val Pro
   260 265 270
   Leu Trp Trp Phe Ile Pro Glu Ser Pro Arg Trp Leu Ile Ser Gln Arg
   275 280 285
   Arg Phe Arg Glu Ala Glu Asp Ile Ile Gln Lys Ala Ala Lys Met Asn
   290 295 300
   Asn Thr Ala Val Pro Ala Val Ile Phe Asp Ser Val Glu Glu Leu Asn
   305 310 315 320
   Pro Leu Lys Gln Gln Lys Ala Phe Ile Leu Asp Leu Phe Arg Thr Arg
   325 330 335
   Asn Ile Ala Ile Met Thr Ile Met Ser Leu Leu Leu Trp Met Leu Thr
   340 345 350
   Ser Val Gly Tyr Phe Ala Leu Ser Leu Asp Ala Pro Asn Leu His Gly
   355 360 365
   Asp Ala Tyr Leu Asn Cys Phe Leu Ser Ala Leu Ile Glu Ile Pro Ala
   370 375 380
   Tyr Ile Thr Ala Trp Leu Leu Leu Arg Thr Leu Pro Arg Arg Tyr Ile
   385 390 395 400
   Ile Ala Ala Val Leu Phe Trp Gly Gly Gly Val Leu Leu Phe Ile Gln
   405 410 415
   Leu Val Pro Val Asp Tyr Tyr Phe Leu Ser Ile Gly Leu Val Met Leu
   420 425 430
   Gly Lys Phe Gly Ile Thr Ser Ala Phe Ser Met Leu Tyr Val Phe Thr
   435 440 445
   Ala Glu Leu Tyr Pro Thr Leu Val Arg Asn Met Ala Val Gly Val Thr
   450 455 460
   Ser Thr Ala Ser Arg Val Gly Ser Ile Ile Ala Pro Tyr Phe Val Tyr
   465 470 475 480
   Leu Gly Ala Tyr Asn Arg Met Leu Pro Tyr Ile Val Met Gly Ser Leu
   485 490 495
   Thr Val Leu Ile Gly Ile Phe Thr Leu Phe Phe Pro Glu Ser Leu Gly
   500 505 510
   Met Thr Leu Pro Glu Thr Leu Glu Gln Met Gln Lys Val Lys Trp Phe
   515 520 525
   Arg Ser Gly Lys Lys Thr Arg Asp Ser Met Glu Thr Glu Glu Asn Pro
   530 535 540
   Lys Val Leu Ile Thr Ala Phe
   545 550
<210> 8
   <211> 614
   <212> DNA
   <213> Homo sapiens
<220>
   <221> misc_feature
   <222> (184)..(184)
   <223> n at 184 can be a or t or g or c
<220>
   <221> misc_feature
   <222> (323)..(323)
   <223> n at 323 can be a or t or g or c
<220>
   <221> misc_feature
   <222> (506)..(506)
   <223> n at 506 can be a or t or g or c
<400> 8
   gcggcccagg cccgcaacct tccctggtcg tgcgccatat gtaaggccag ccgcggcagg 60
   accaaggcgg cggtgtcagc tckcgagcct accctccgcg gacggtcttg ggtckcctgc 120
   tgcctggctt gcctggtcgg cggyrggtgc cccgcgcgca cgcgcaaagc ccgccgcgtt 180
   cccngacccc aggccgcgct ctgtgggcct ctgagggcgg catgcgggac tacgacgagg 240
   tgaccgcctt cctgggcgag tgggggccct tccagcgcct catcttcttc ctgctcagcg 300
   ccagcatcat ccccaatggc ttnaccggcc tgtcctccgt gttcctgata gcgaccccgg 360
   agcaccgctg ccgggtgccg gacgccgcga acctgagcag cgcctggcgc aaccacactg 420
   tcccactgcg gctgcgggac ggccgcgagg tgccccacag ctgccgccgc taccggctcg 480
   ccaccatcgc caacttctcg gcgctngggc tggagccggg gcgcgacgtg gacctggggc 540
   agctggagca ggagagctgt ctggatggct gggagttcag tcaggacgtc tacctgtcca 600
   ccattgtgac cggg 614
<210> 9
   <211> 20
   <212> DNA
   <213> Homo sapiens
<400> 9
   accatcgcca acttctcggc 20
<210> 10
   <211> 20
   <212> DNA
   <213> Homo sapiens
<400> 10
   ctttctgctg cttcagggga 20
<210> 11
   <211> 18
   <212> DNA
   <213> Homo sapiens
<400> 11
   ccatcgccaa cttctcgg 18
<210> 12
   <211> 22
   <212> DNA
   <213> Homo sapiens
<400> 12
   aatgttgtgg gactgctgct tc 22
<210> 13
   <211> 28
   <212> DNA
   <213> Homo sapiens
<400> 13
   gaagatcttg gaaggctcag gtgggagg 28
<210> 14
   <211> 28
   <212> DNA
   <213> Homo sapiens
<400> 14
   cgacgcgtgg cagcaggcga cccaagac 28
<210> 15
   <211> 25
   <212> DNA
   <213> Homo sapiens
<400> 15
   caggcccgga accttccctg gtcgt 25
<210> 16
   <211> 25
   <212> DNA
   <213> Homo sapiens
<400> 16
   acgaccaggg aaggttccgg gcctg 25
<210> 17
   <211> 25
   <212> DNA
   <213> Homo sapiens
<400> 17
   caggcccgca accttccctg gtcgt 25
<210> 18
   <211> 25
   <212> DNA
   <213> Homo sapiens
<400> 18
   acgaccaggg aaggttgcgg gcctg 25
<210> 19
   <211> 31
   <212> DNA
   <213> Homo sapiens
<400> 19
   tcggctggaa tattcccgac ctggcagccg a 31
<210> 20
   <211> 31
   <212> DNA
   <213> Homo sapiens
<400> 20
   tcggctgcca ggtcgggaat attccagccg a 31
<210> 21
   <211> 39
   <212> DNA
   <213> Homo sapiens
<400> 21
   gactgtcctg attggaatct tcaccctttt tttccctga 39
<210> 22
   <211> 39
   <212> DNA
   <213> Homo sapiens
<400> 22
   tcagggaaaa aaagggtgaa gattccaatc aggacagtc 39
<210> 23
   <211> 400
   <212> DNA
   <213> Homo sapiens
<220>
   <221> misc_feature
   <222> (385)..(385)
   <223> n at residue 385 can be a or g or c or t
<400> 23
   gtcccgctgc cttcctaagc cgascccggg ctacctcggt cgtccccagc aggcgtggct 60
   ggcagaggcc gggcctcgcc aggtccccag gacaggcccc gcccgggcct caggtgcact 120
   cccggcccgc cccgcgccct cgcgtcccgc cccagctccg ccttcgccgg cgccgctctg 180
   cctgccagcg gggcgcgcct tgcggcccag gcccgsaacc ttccctggtc gtgcgccata 240
   tgtaaggcca gccgcggcag gaccaaggcg gcggtgtcag ctckcgagcc taccctccgc 300
   ggacggtctt gggtckcctg ctgcctggct tgcctggtcg gcggyrggtg ccccgcgcgc 360
   acgcgcaaag cccgccgcgt tcccngaccc caggccgcgc 400
<210> 24
   <211> 18
   <212> DNA
   <213> Homo sapiens
<400> 24
   taagccgagc ccgggcta 18
<210> 25
   <211> 19
   <212> DNA
   <213> Homo sapiens
<400> 25
   ccaggcccgg aaccttccc 19
<210> 26
   <211> 32
   <212> DNA
   <213> Homo sapiens
<400> 26
   cggcggtgtc agctcgcgag cctaccctcc gc 32
<210> 27
   <211> 32
   <212> DNA
   <213> Homo sapiens
<400> 27
   ggacggtctt gggtcgcctg ctgcctggct tg 32
<210> 28
   <211> 32
   <212> DNA
   <213> Homo sapiens
<400> 28
   cctggtcggc ggtaggtgcc ccgcgcgcac gc 32
<210> 29
   <211> 20
   <212> DNA
   <213> Homo sapiens
<400> 29
   gccaggttag gttccctttc 20
<210> 30
   <211> 21
   <212> DNA
   <213> Homo sapiens
<400> 30
   agcagcccaa attcttaaag g 21
<210> 31
   <211> 20
   <212> DNA
   <213> Homo sapiens
<400> 31
   gaatcacctc gctgcttttt 20
<210> 32
   <211> 19
   <212> DNA
   <213> Homo sapiens
<400> 32
   aaatgtggaa agggcatct 19
<210> 33
   <211> 20
   <212> DNA
   <213> Homo sapiens
<400> 33
   attaggcggt gtcaagagca 20
<210> 34
   <211> 20
   <212> DNA
   <213> Homo sapiens
<400> 34
   ggtcgtgcgc catatgtaag 20
<210> 35
   <211> 20
   <212> DNA
   <213> Homo sapiens
<400> 35
   gagaggagct cgggttcaag 20
<210> 36
   <211> 136
   <212> DNA
   <213> Homo sapiens
<400> 36
   gtgcttacaa cagaatgctg ccctacatcg tcatgggtag tctgactgtc ctgattggaa 60
   tcytcaccct ttttttccct gaaagtttgg gaatgactct tccagaaacc ttagagcaga 120
   tgcagaaagt gaaatg 136
<210> 37
   <211> 2136
   <212> DNA
   <213> Homo sapiens
<220>
   <221> misc_feature
   <222> (5)..(5)
   <223> n can be a or t or g or c
<220>
   <221> misc_feature
   <222> (7)..(7)
   <223> n can be a or t or g or c
<220>
   <221> misc_feature
   <222> (229)..(229)
   <223> n can be a or t or g or c
<220>
   <221> misc_feature
   <222> (324)..(324)
   <223> n can be a or t or g or c
<220>
   <221> misc_feature
   <222> (325)..(325)
   <223> n can be a or t or g or c
<220>
   <221> misc_feature
   <222> (326)..(326)
   <223> n can be a or t or g or c
<220>
   <221> misc_feature
   <222> (327)..(327)
   <223> n can be a or t or g or c
<220>
   <221> misc_feature
   <222> (328)..(328)
   <223> n can be a or t or g or c
<220>
   <221> misc_feature
   <222> (330)..(330)
   <223> n can be a or t or g or c
<220>
   <221> misc_feature
   <222> (331)..(331)
   <223> n can be a or t or g or c
<220>
   <221> misc_feature
   <222> (334)..(334)
   <223> n can be a or t or g or c
<220>
   <221> misc_feature
   <222> (569)..(569)
   <223> n can be a or t or g or c
<220>
   <221> misc_feature
   <222> (593)..(593)
   <223> n can be a or t or g or c
<220>
   <221> misc_feature
   <222> (617)..(617)
   <223> n can be a or t or g or c
<220>
   <221> misc_feature
   <222> (1063)..(1063)
   <223> n can be a or t or g or c
<220>
   <221> misc_feature
   <222> (1328)..(1328)
   <223> n can be a or t or g or c
<220>
   <221> misc_feature
   <222> (1767)..(1767)
   <223> n can be a or t or g or c
<220>
   <221> misc_feature
   <222> (2105)..(2105)
   <223> n can be a or t or g or c
<400> 37
   ccccngnttc gcgccccaat ttctaacagc ctgcctgtcc cccgggaacg ttctaacatc     60
   cttggggagc gccccagcta caagacactg tcctgagaac gctgtcatca cccgtagttg    120
   caagtttcgg agcggcagtg ggaagcatgc gggactacga cgaggtgatc gccttcctgg    180
   gcgagtgggg gcccttccag cgcctcatct tcttcctgct cagcgccanc atcatcccca    240
   atggcttcaa tggtatgtca gtcgtgttcc tggcggggac cccggagcac cgctgtcgag    300
   tgccggacgc cgcgaacctg agcnnnnncn ngcngaacaa cagtgtcccg ctgcggctgc    360
   gggacggccg cgaggtgccc cacagctgca gccgctaccg gctcgccacc atcgccaact    420
   tctcggcgct cgggctggag ccggggcgcg acgtggacct ggggcagctg gagcaggaga    480
   gctgcctgga tggctgggag ttcagccagg acgtctacct gtccaccgtc gtgaccgagt    540
   ggaatctggt gtgtgaggac aactggaang tgcccctcac cacctccctg ttnttcgtag    600
   gcgtgctcct cggctcnttc gtgtccgggc agctgtcaga caggtttggc aggaagaacg    660
   ttctcttcgc aaccatggct gtacagactg gcttcagctt cctgcagatt ttctccatca    720
   gctgggagat gttcactgtg ttatttgtca tcgtgggcat gggccagatc tccaactatg    780
   tggtagcctt catactagga acagaaattc ttggcaagtc agttcgtatt atattctcta    840
   cattaggagt gtgcacattt tttgcagttg gctatatgct gctgccactg tttgcttact    900
   tcatcagaga ctggcggatg ctgctgctgg cgctgacggt gccgggagtg ctgtgtgtcc    960
   cgctgtggtg gttcattcct gaatctcccc gatggctgat atcccagaga agatttagag   1020
   aggctgaaga tatcatccaa aaagctgcaa aaatgaacaa canagctgta ccagcagtga   1080
   tatttgattc tgtggaggag ctaaatcccc tgaagcagca gaaagctttc attctggacc   1140
   tgttcaggac tcggaatatt gccataatga ccattatgtc tttgctgcta tggatgctga   1200
   cctcagtggg ttactttgct ctgtctctgg atgctcctaa tttacatgga gatgcctacc   1260
   tgaactgttt cctctctgcc ttgattgaaa ttccagctta cattacagcc tggctgctat   1320
   tgcgaacnct gcccaggcgt tatatcatag ctgcagtact gttctgggga ggaggtgtgc   1380
   ttctcttcat tcaactggta cctgtggatt attacttctt atccattggt ctggtcatgc   1440
   tgggaaaatt tgggatcacc tctgctttct ccatgctgta tgtcttcact gctgagctct   1500
   acccaaccct ggtcaggaac atggcggtgg gggtcacatc cacggcctcc agagtgggca   1560
   gcatcattgc cccctacttt gtttacctcg gtgcttacaa cagaatgctg ccctacatcg   1620
   tcatgggtag tctgactgtc ctgattggaa tcytcaccct ttttttccct gaaagtttgg   1680
   gaatgactct tccagaaacc ttagagcaga tgcagaaagt gaaatggttc agatctggga   1740
   aaaaaacaag agactcaatg gagacanaag aaaatcccaa ggttctaata actgcattct   1800
   gaaaaaatat ctaccccatt tggtgaagtg aaaaacacaa aaataagacc ctgtggagaa   1860
   attcgttgtt cccactgaaa tggactgact gtaacgattg acaccaaaat gaaccttgct   1920
   atcaagaaat gctcgtcata cagtaaactc tggatgattc ttccagataa tgtccttgct   1980
   ttacaaacca accatttcta gagagtctcc ttactcatta attcaatgaa atggattggt   2040
   aagatgtctt gaaaacatgt tagtcaagga ctggtaaaat acatataaag attaacactc   2100
   atttnccaat catacaaata ctatccaaat aaaaat                             2136
<210> 38
   <211> 551
   <212> PRT
   <213> Homo sapiens
<400> 38
   Met Arg Asp Tyr Asp Glu Val Ile Ala Phe Leu Gly Glu Trp Gly Pro
   1 5 10 15
   Phe Gln Arg Leu Ile Phe Phe Leu Leu Ser Ala Ser Ile Ile Pro Asn
   20 25 30
   Gly Phe Asn Gly Met Ser Val Val Phe Leu Ala Gly Thr Pro Glu His
   35 40 45
   Arg Cys Arg Val Pro Asp Ala Ala Asn Leu Ser Ser Ala Trp Arg Asn
   50 55 60
   Asn Ser Val Pro Leu Arg Leu Arg Asp Gly Arg Glu Val Pro His Ser
   65 70 75 80
   Cys Ser Arg Tyr Arg Leu Ala Thr Ile Ala Asn Phe Ser Ala Leu Gly
   85 90 95
   Leu Glu Pro Gly Arg Asp Val Asp Leu Gly Gln Leu Glu Gln Glu Ser
   100 105 110
   Cys Leu Asp Gly Trp Glu Phe Ser Gln Asp Val Tyr Leu Ser Thr Val
   115 120 125
   Val Thr Glu Trp Asn Leu Val Cys Glu Asp Asn Trp Lys Val Pro Leu
   130 135 140
   Thr Thr Ser Leu Phe Phe Val Gly Val Leu Leu Gly Ser Phe Val Ser
   145 150 155 160
   Gly Gln Leu Ser Asp Arg Phe Gly Arg Lys Asn Val Leu Phe Ala Thr
   165 170 175
   Met Ala Val Gln Thr Gly Phe Ser Phe Leu Gln Ile Phe Ser Ile Ser
   180 185 190
   Trp Glu Met Phe Thr Val Leu Phe Val Ile Val Gly Met Gly Gln Ile
   195 200 205
   Ser Asn Tyr Val Val Ala Phe Ile Leu Gly Thr Glu Ile Leu Gly Lys
   210 215 220
   Ser Val Arg Ile Ile Phe Ser Thr Leu Gly Val Cys Thr Phe Phe Ala
   225 230 235 240
   Val Gly Tyr Met Leu Leu Pro Leu Phe Ala Tyr Phe Ile Arg Asp Trp
   245 250 255
   Arg Met Leu Leu Leu Ala Leu Thr Val Pro Gly Val Leu Cys Val Pro
   260 265 270
   Leu Trp Trp Phe Ile Pro Glu Ser Pro Arg Trp Leu Ile Ser Gln Arg
   275 280 285
   Arg Phe Arg Glu Ala Glu Asp Ile Ile Gln Lys Ala Ala Lys Met Asn
   290 295 300
   Asn Thr Ala Val Pro Ala Val Ile Phe Asp Ser Val Glu Glu Leu Asn
   305 310 315 320
   Pro Leu Lys Gln Gln Lys Ala Phe Ile Leu Asp Leu Phe Arg Thr Arg
   325 330 335
   Asn Ile Ala Ile Met Thr Ile Met Ser Leu Leu Leu Trp Met Leu Thr
   340 345 350
   Ser Val Gly Tyr Phe Ala Leu Ser Leu Asp Ala Pro Asn Leu His Gly
   355 360 365
   Asp Ala Tyr Leu Asn Cys Phe Leu Ser Ala Leu Ile Glu Ile Pro Ala
   370 375 380
   Tyr Ile Thr Ala Trp Leu Leu Leu Arg Thr Leu Pro Arg Arg Tyr Ile
   385 390 395 400
   Ile Ala Ala Val Leu Phe Trp Gly Gly Gly Val Leu Leu Phe Ile Gln
   405 410 415
   Leu Val Pro Val Asp Tyr Tyr Phe Leu Ser Ile Gly Leu Val Met Leu
   420 425 430
   Gly Lys Phe Gly Ile Thr Ser Ala Phe Ser Met Leu Tyr Val Phe Thr
   435 440 445
   Ala Glu Leu Tyr Pro Thr Leu Val Arg Asn Met Ala Val Gly Val Thr
   450 455 460
   Ser Thr Ala Ser Arg Val Gly Ser Ile Ile Ala Pro Tyr Phe Val Tyr
   465 470 475 480
   Leu Gly Ala Tyr Asn Arg Met Leu Pro Tyr Ile Val Met Gly Ser Leu
   485 490 495
   Thr Val Leu Ile Gly Ile Leu Thr Leu Phe Phe Pro Glu Ser Leu Gly
   500 505 510
   Met Thr Leu Pro Glu Thr Leu Glu Gln Met Gln Lys Val Lys Trp Phe
   515 520 525
   Arg Ser Gly Lys Lys Thr Arg Asp Ser Met Glu Thr Glu Glu Asn Pro
   530 535 540
   Lys Val Leu Ile Thr Ala Phe
   545 550
<210> 39
   <211> 20
   <212> DNA
   <213> Homo sapiens
<400> 39
   gtgcccagag agtcctccta 20
<210> 40
   <211> 21
   <212> DNA
   <213> Homo sapiens
<400> 40
   ttctccctaa ggcattttgg t 21
<210> 41
   <211> 26850
   <212> DNA
   <213> Homo sapiens
<220>
   <221> misc_feature
   <222> (49)..(49)
   <223> n at 49 can be a or g or c or t
<220>
   <221> misc_feature
   <222> (134)..(134)
   <223> n at 134 can be a or g or c or t
<220>
   <221> misc_feature
   <222> (546)..(546)
   <223> n at 546 can be a or g or c or t
<220>
   <221> misc_feature
   <222> (877)..(877)
   <223> n at 877 can be a or g or c or t
<220>
   <221> misc_feature
   <222> (1338)..(1338)
   <223> n at 1338 can be a or g or c or t
<220>
   <221> misc_feature
   <222> (1985)..(1985)
   <223> n at 1985 can be a or g or c or t
<220>
   <221> misc_feature
   <222> (2124)..(2124)
   <223> n at 2124 can be a or g or c or t
<220>
   <221> misc_feature
   <222> (2307)..(2307)
   <223> n at 2307 can be a or g or c or t
<220>
   <221> misc_feature
   <222> (3115)..(3115)
   <223> n at 3115 can be a or g or c or t
<220>
   <221> misc_feature
   <222> (3159)..(3159)
   <223> n at 3159 can be a or g or c or t
<220>
   <221> misc_feature
   <222> (3191)..(3191)
   <223> n at 3191can be a or g or c or t
<220>
   <221> misc_feature
   <222> (3282)..(3282)
   <223> n at 3282 can be a or g or c or t
<220>
   <221> misc_feature
   <222> (3661)..(3661)
   <223> n at 3661 can be a or g or c or t
<220>
   <221> misc_feature
   <222> (3748)..(3748)
   <223> n at 3748 can be a or g or c or t
<220>
   <221> misc_feature
   <222> (3797)..(3797)
   <223> n at 3797 can be a or g or c or t
<220>
   <221> misc_feature
   <222> (3905)..(3905)
   <223> n at 3905 can be a or g or c or t
<220>
   <221> misc_feature
   <222> (4260)..(4260)
   <223> n at 4260 can be a or g or c or t
<220>
   <221> misc_feature
   <222> (4903)..(4903)
   <223> n at 4903 can be a or g or c or t
<220>
   <221> misc_feature
   <222> (5971)..(5971)
   <223> n at 5971 can be a or g or c or t
<220>
   <221> misc_feature
   <222> (6111)..(6111)
   <223> n at 6111 can be a or g or c or t
<220>
   <221> misc_feature
   <222> (6148)..(6148)
   <223> n at 6148 can be a or g or c or t
<220>
   <221> misc_feature
   <222> (6400)..(6400)
   <223> n at 6400 can be a or g or c or t
<220>
   <221> misc_feature
   <222> (6468)..(6468)
   <223> n at 6468 can be a or g or c or t
<220>
   <221> misc_feature
   <222> (6575)..(6575)
   <223> n at 6575 can be a or g or c or t
<220>
   <221> misc_feature
   <222> (7287)..(7287)
   <223> n at 7287can be a or g or c or t
<220>
   <221> misc_feature
   <222> (8495)..(8495)
   <223> n at 8495 can be a or g or c or t
<220>
   <221> misc_feature
   <222> (9918)..(9918)
   <223> n at 9918 can be a or g or c or t
<220>
   <221> misc_feature
   <222> (9919)..(9919)
   <223> n at 9919 can be a or g or c or t
<220>
   <221> misc_feature
   <222> (9924)..(9924)
   <223> n at 9924 can be a or g or c or t
<220>
   <221> misc_feature
   <222> (9947)..(9947)
   <223> n at 9947 can be a or g or c or t
<220>
   <221> misc_feature
   <222> (10143)..(10143)
   <223> n at 10143 can be a or g or c or t
<220>
   <221> misc_feature
   <222> (10357)..(10357)
   <223> n at 10357 can be a or g or c or t
<220>
   <221> misc_feature
   <222> (10379)..(10379)
   <223> n at 10379 can be a or g or c or t
<220>
   <221> misc_feature
   <222> (10384)..(10384)
   <223> n at 10384 can be a or g or c or t
<220>
   <221> misc_feature
   <222> (10580)..(10580)
   <223> n at 10580 can be a or g or c or t
<220>
   <221> misc_feature
   <222> (10717)..(10717)
   <223> n at 10717 can be a or g or c or t
<220>
   <221> misc_feature
   <222> (10718)..(10718)
   <223> n at 10718 can be a or g or c or t
<220>
   <221> misc_feature
   <222> (10719)..(10719)
   <223> n at 10719 can be a or g or c or t
<220>
   <221> misc_feature
   <222> (10781)..(10781)
   <223> n at 10781 can be a or g or c or t
<220>
   <221> misc_feature
   <222> (11111)..(11111)
   <223> n at 11111 can be a or g or c or t
<220>
   <221> misc_feature
   <222> (11150)..(11150)
   <223> n at 11150 can be a or g or c or t
<220>
   <221> misc_feature
   <222> (11211)..(11211)
   <223> n at 11211 can be a or g or c or t
<220>
   <221> misc_feature
   <222> (11383)..(11383)
   <223> n at 11383 can be a or g or c or t
<220>
   <221> misc_feature
   <222> (11489)..(11489)
   <223> n at 11489 can be a or g or c or t
<220>
   <221> misc_feature
   <222> (11502)..(11502)
   <223> n at 11502 can be a or g or c or t
<220>
   <221> misc_feature
   <222> (11503)..(11503)
   <223> n at 11503 can be a or g or c or t
<220>
   <221> misc_feature
   <222> (11515)..(11515)
   <223> n at 11515 can be a or g or c or t
<220>
   <221> misc_feature
   <222> (11640)..(11640)
   <223> n at 11640 can be a or g or c or t
<220>
   <221> misc_feature
   <222> (11937)..(11937)
   <223> n at 11937 can be a or g or c or t
<220>
   <221> misc_feature
   <222> (13423)..(13423)
   <223> n at 13423 can be a or g or c or t
<220>
   <221> misc_feature
   <222> (14232)..(14232)
   <223> n at 14232 can be a or g or c or t
<220>
   <221> misc_feature
   <222> (14579)..(14579)
   <223> n at 14579 can be a or g or c or t
<220>
   <221> misc_feature
   <222> (15503)..(15503)
   <223> n at 15503 can be a or g or c or t
<220>
   <221> misc_feature
   <222> (15601)..(15601)
   <223> n at 15601 can be a or g or c or t
<220>
   <221> misc_feature
   <222> (15755)..(15755)
   <223> n at 15755 can be a or g or c or t
<220>
   <221> misc_feature
   <222> (15823)..(15823)
   <223> n at 15823 can be a or g or c or t
<220>
   <221> misc_feature
   <222> (16062)..(16062)
   <223> n at 16062 can be a or g or c or t
<220>
   <221> misc_feature
   <222> (16204)..(16204)
   <223> n at 16204 can be a or g or c or t
<220>
   <221> misc_feature
   <222> (16635)..(16635)
   <223> n at 16635 can be a or g or c or t
<220>
   <221> misc_feature
   <222> (16637)..(16637)
   <223> n at 16637 can be a or g or c or t
<220>
   <221> misc_feature
   <222> (16724)..(16724)
   <223> n at 16724 can be a or g or c or t
<220>
   <221> misc_feature
   <222> (17546)..(17546)
   <223> n at 17546 can be a or g or c or t
<220>
   <221> misc_feature
   <222> (18232)..(18232)
   <223> n at 18232 can be a or g or c or t
<220>
   <221> misc_feature
   <222> (18260)..(18260)
   <223> n at 18260 can be a or g or c or t
<220>
   <221> misc_feature
   <222> (18414)..(18414)
   <223> n at 18414 can be a or g or c or t
<220>
   <221> misc_feature
   <222> (18418)..(18418)
   <223> n at 18418 can be a or g or c or t
<220>
   <221> misc_feature
   <222> (18424)..(18424)
   <223> n at 18424 can be a or g or c or t
<220>
   <221> misc_feature
   <222> (20789)..(20789)
   <223> n at 20789 can be a or g or c or t
<220>
   <221> misc_feature
   <222> (21317)..(21317)
   <223> n at 21317 can be a or g or c or t
<220>
   <221> misc feature
   <222> (21696)..(21696)
   <223> n at 21696 can be a or g or c or t
<220>
   <221> misc_feature
   <222> (22199)..(22199)
   <223> n at 22199 can be a or g or c or t
<220>
   <221> misc_feature
   <222> (23399)..(23399)
   <223> n at 23399 can be a or g or c or t
<220>
   <221> misc_feature
   <222> (23712)..(23712)
   <223> n at 23712 can be a or g or c or t
<220>
   <221> misc_feature
   <222> (27712)..(27712)
   <223> n at 27712 can be a or g or c or t
<220>
   <221> misc_feature
   <222> (24038)..(24038)
   <223> n at 24038 can be a or g or c or t
<220>
   <221> misc_feature
   <222> (24565)..(24565)
   <223> n at 24565 can be a or g or c or t
<220>
   <221> misc_feature
   <222> (25073)..(25073)
   <223> n at 25073 can be a or g or c or t
<220>
   <221> misc_feature
   <222> (25419)..(25419)
   <223> n at 25419 can be a or g or c or t
<220>
   <221> misc_feature
   <222> (25420)..(25420)
   <223> n at 25420 can be a or g or c or t
<220>
   <221> misc_feature
   <222> (25426)..(25426)
   <223> n at 25426 can be a or g or c or t
<400> 41
   taattttttt aagtctatta tgctttatgc aaatgtttag atttccttnt taaaaatctg      60
   ctggccaggt taggttccct ttctgtagct ttattttcaa ctcataatat agtgactatt     120
   aaatattctt tacncaacac attatattgt aaaaatatac acttagttct gtttctatca     180
   ctaagccact actattttaa aaaggaatat cttgcattgt taagtttagc agcccaaatt     240
   cttaaaggcc ttaggaatgg tgctgtccaa taagataacc aatagctaca tgtggctact     300
   gagcacttaa aaaatgagtc tggccaggca tagtggctca cgtctgtaat tcagcatttt     360
   gggaggctga ggtgggagga ttgcttaaac ccaggagtct gagaccagcc tgggcaacac     420
   agggatgcct tgtctctgta aaataaaaaa aataaaaaaa ttagctgggc acggtggcat     480
   gtgcctgtag tcctagctac tcagcaggct gaggcaggag gcccacttaa gcttaggagt     540
   tcaggnctgc ggtgagctat gattgtgcca ctgcactcta gcctgggtga cacagtggga     600
   ccgtgtctcc aataaataaa tgaataaata aatattaaaa aataaaaagt gtggctagtc     660
   caaattgaaa tgtgctgtaa aatacacact ggattccttt tttttttttt ttcaaatcct     720
   gagaacctgt gaaacacact gaattcttaa gatttagtat gaggagggga aaaaaagatc     780
   aatgatttac atataacaat gtaaaatatc aataatttct atattgatta tatgatgaaa     840
   tgatcatgtt ctggatatac ttgattaaat ataccantaa agttaatttc atcttttatt     900
   tttacttttt aaatatgcag cattgcccaa taaaatacag gacaaccagt tacattttta     960
   ttttatatat tcctgcttgg gacataatat ttaaaaatta ttcattctat atctgaaatt    1020
   caaatttaac taggagtcct atatttttat ttgctaaatc tggcaatttt attaatatgg    1080
   ctactagaaa agttaacata acaaatgtag ctccttatcc tatggaacat tgctgcttta    1140
   gaatcacctc gctgcttttt ttttttttcc agttagcttc tgaataaact ctgcacggaa    1200
   ataactaatc tgtggggaat atttaaccct aggctaggat cgttaatcgt gaagttaaat 1260
   ttttacatct gcatgaacaa aatggcttgt tacagacgaa aatgtggaaa gggcatctgg 1320
   actagaatga aaagtcanag ctcccagcct ctctactagg gtagttaaca gtcccaggtt 1380
   caaaataatc cccgtccctc ttataagatt aggcggtgtc aagagcaact atctgtgaga 1440
   ccctgggcca gtgactttct ccttacctcc gcccccaagt aggccttgca aaaagctggg 1500
   aggggtgcgt tttcaacact ggagcttcgc ggccgctctc ccactcgctc cccgcccggc 1560
   gctagaggag cgagttcgga ctcggacccc aaggcctcga gtcccgctgc cttcctaagc 1620
   cgaccccggg ctacctcggt cgtccccagc aggcgtggct ggcagaggcc gggcctcgcc 1680
   aggtccccag gacaggcccc gcccgggcct caggtgcact cccggcccgc cccgcgccct 1740
   cgcgtcccgc cccagctccg ccttcgccgg cgccgctctg cctgccagcg gggcgcgcct 1800
   tgcggcccag gcccggaacc ttccctggtc gtgcgccata tgtaaggcca gccgcggcag 1860
   gaccaaggcg gcggtgtcag ctckcgagcc taccctccgc ggacggtctt gggtckcctg 1920
   ctgcctggct tgcctggtcg gcggyrggtg ccccgcgcgc acgcgcaaag cccgccgcgt 1980
   tcccngaccc caggccgcgc tctgtgggcc tctgagggcg gcatgcggga ctacgacgag 2040
   gtgaccgcct tcctgggcga gtgggggccc ttccagcgcc tcatcttctt cctgctcagc 2100
   gccagcatca tccccaatgg cttnaccggc ctgtcctccg tgttcctgat agcgaccccg 2160
   gagcaccgct gccgggtgcc ggacgccgcg aacctgagca gcgcctggcg caaccacact 2220
   gtcccactgc ggctgcggga cggccgcgag gtgccccaca gctgccgccg ctaccggctc 2280
   gccaccatcg ccaacttctc ggcgctnggg ctggagccgg ggcgcgacgt ggacctgggg 2340
   cagctggagc aggagagctg tctggatggc tgggagttca gtcaggacgt ctacctgtcc 2400
   accattgtga ccggggtggg tgccggcccc tgctggggct gagaccaggg ctcggaggac 2460
   ctgtcgcggt ccttgaaccc gagctcctct ctcccagatg cgcactggac gctgtcactc 2520
   cccctccccc aacggtcaac accctagcga tggagaccct ccagccaggt ggcttgggaa 2580
   cgcttcacga ggtgacctcc agccacagtg tgctcctccc tgcacaggtg gtcagtctgg 2640
   cctcccgtcc tgatggccac tttgaagagg gtaccaggaa ggtcctggcg gtccctgggc 2700
   gatgctctat ggccctgtgt gtccaggact tactctagtt ggggttgggg gtggtaagta 2760
   gcagagccag gacttgggcc aggggctatc ccgtttttcc tctagtctct tgatttcttt 2820
   ttagaagaga agaaatactt ctctttcctg aacttttaaa agttaaataa agcatgtgta 2880
   tacaactgcc tcttcccttt ttcctctagt tactccttcc cctaccgtcc acaacccaaa 2940
   aacgacaatc tggtcatgcc ctgtaagtaa ttgtttgcct tttcccatgg tcagttgtca 3000
   gtcttttttt tttttttttt tttttgagac agagtctccc tctgtcacca ggctggagcg 3060
   cagtcgtgtg ttcttggctc actgcaacct tcgcagtcgt gcgttcttgg ctcantgcaa 3120
   tcttcgcctt ccgggctcaa gtgattctcc tgcctcagnc tccgagtagc tgtgagccac 3180
   gacgcccagc naatttttgt gtttttagta gagacggggt ttcaccgtgt tggccaggat 3240
   ggtctcgatc tcttgacctc gtgatctgcc tgcttctgcc tncaaagctg ggattacaac 3300
   cgtgagccac cacgccaggc cgtcagttgt cactctttaa gatccattca tctgaagatg 3360
   ggttcagggt gacttgttga cctggaatat tttctcaggt attatgaggc aaggctgtcg 3420
   gccagattta gttaaagcat acagccttag gtcatagggt gtgggggagc ctttctcatt 3480
   tctcatcccc ttggattttc cctctgggtg gttttgtctg tcccctccga acctgttgga 3540
   gcagttgttg gagctggatg taggaacatg atgttaatga tgtatgtgtt ttgtgtcttt 3600
   tttagacact ggcactctag ctccctgaag tttcagcagc attgagtaag tagccagtga 3660
   ntagccctca ttgatagata ggctcactaa atgtgcagat gaccaattcg caggttagag 3720
   aaggcttccc agaagaggag gcctctcngt gagcctaggg tgttcagctc agcataatag 3780
   tgagctgaag gccccantgc agcagcaaga aaccacccag caggacggcg gagttcacag 3840
   agaggggaga gttcatagag agggagagtg ccacaggccc tggcacagct tcaagccctg 3900
   ctggntgttg gtgctgagcc tcccctcctg gagcctcaga ggggcttaca ggggctctgg 3960
   agatcccaac tgtgttgctt cttggcgtca tcacccttca atggagtctg agagctaccc 4020
   tgggagatcc aagtgtgtgt gcatgtgtgt gtttttcttt tttggaaatt tgatgtcccc 4080
   agcatttgga cctgctttct ccacatatat gtagtgggag tgtgaccgga gccccactgg 4140
   gatttcttag ctagtgacat agcttcagtg tccagaggca tcatggcttg acaaaagagg 4200
   cactccttgg aggtagccaa ctgtgctttc tggcccatga atccacagga aattggaacn 4260
   ttcagttgcc acaaatcctg ggtcctagcc tcacaggtgg gtgtgttacc ttcccggaag 4320
   ccagtgagta ctatgaaagc agaggctgtc cctgaggttg caggcagagg ccacagaggg 4380
   gaacatgaca caggaatccc tacaaattct acttggggct gcctaaagaa gagggaagta 4440
   gtgaagcaag aagaagcaca tggcatctct tggagtttta cattgacccc tgagggttcc 4500
   ccggcttact ctagtcactt gttcctgctt tgctgcctcc atcccacatt gggctgagtg 4560
   atggtggcat tgatgagctc ccaaaggcca gctgtgccag ggggtctgac cttatcttgc 4620
   tgccaatgtc agccttttgt tttttaatat ttagactatt tatttagctg tcttagcagt 4680
   ttcaaaggag ttatgtgccc tttcacctac ttatatgttg tcagtctttg cagggaggcc 4740
   agattaatgc ttagatcttt gttttgggct actggaatgc ttgacttgaa gttcagagct 4800
   gcttgttccc aggtgaacag ctactgctgg aagttgctgc atcaacattc taatggcttt 4860
   ttctatggcc tgttgtcttt caacccaaac ctggcctgct gancactgca tctagtccca 4920
   tgcctgctaa atgtctctaa gcctgccctc tgccccaaat catacataaa ggtgtttgta 4980
   agtacactgg tattgaatta ctagtcatat tttttccact gaagactgga acctcaggtg 5040
   tcctgtttgg atttttttaa tttgttcaag ttaaagtaca tacatgtagt accaaacttt 5100
   gtggtggatt tagatctttg cgtcttcctc aatttctgac acaattctgg atgcagaggg 5160
   aggttctcag gaaaattttt attgaatgag ttaatgaata atttaagaaa tcatctctaa 5220
   agtttgagaa ctaaagaaaa atagttcagt tcttagaagg gaaacttgag ggtggctgaa 5280
   aaggattgac tggaattttt taaaggaaat gtgactcccc ctgcccactg actggggctt 5340
   tgatgccaca tggatgtgga atgaggtgtt gggattggca gagggaatct gctagcaatt 5400
   aataaataaa taaatattgg cagggcgtgg tggctcacgc ctgtaatccc agcactttgg 5460
   gaagccaagg cagcaggtca cttgaggtcg ggagttcaag accagtctgg ccaacatggt 5520
   gaaactccat ctctactaaa aatacaaaaa ttagctgggt gtggtggcac atgcctgtat 5580
   tcccagctac ttgggagact gaagcaggag aatcgcttga acccaggagg cgggctttgc 5640
   agtgaaccga gatcgagcct ctgcactcta gcctgggcga caaagcgaga ctccatctca 5700
   aaagaataat aataatatta ataaataaaa atgatttatg aggtaaaaga gttttatgcc 5760
   cccatgttcc aggaatagtt tggtggtcca catggttctc ggctggcctc tcctctggcc 5820
   cctcagtcat ccctggggta ctggggaatt agccaaccca tcatgcagtg cttcttggcc 5880
   atggactgcc ccatctgctg gaaacctggg ttgtttctga ggttgtctgg gctgtccgct 5940
   ctttggtttc accatagctc tgtccagcct ntggacagac aggttccttg agaaacttcc 6000
   ggctgggtgt ggtggctcat gcctgtaaac ccagcacttt gggaggctga ggcaggtgga 6060
   tcacaaggca ggagttcgag accagcctgg ccaacatggt gaaacctcgt ntccgctaaa 6120
   aatacaaaaa ttagccctgc gtggtggngg gtgcctgtaa tcccagctac tcgggaggga 6180
   ggctgaggca ggagaatttc ttgaacctgg gaagtggggg ttgcagtagc caagatcgca 6240
   ccattgcatg ccagcctggg tgacaagagc aagactccgt ctcaaaaaaa aaaaaagaaa 6300
   aagaaaagaa acttccaagc tgctctgcat cgccttgctc tccacctgtc tgcttctaag 6360
   aagccctcgg cccagtcctg ggtgggactc ccactccctn cccattgtcc tggactagct 6420
   tttctatcag ccttatcttg tgtagagaca gatagtctta gaagatgnga gagccctcac 6480
   tgttatcccc aaagctgcct ggaggaaaag ccagagcaac ctgggagctg ggaccggggc 6540
   tgactctggg cagcagagac ccgagagacc tggancttga acctcactgt tacgcctttg 6600
   ttgatttctc tcactcaggg gacacacaga ccctcatcca gccttttgca gctatatggc 6660
   aaggcagaga agccacttgc ggggtcccgt ggcccactat gcacgtacat agtagacaca 6720
   tctggccatg agtggtcaga ttgagccact ctctagctag ctgacacctg tcatcctggg   6780
   tcaaatttct gacagttgac acaaagcagg gggtcaggga gccaaaaaaa aaaatggcca   6840
   ggtgtggtgg ctcttgcctg taatcccagg gctttgggag gccaaggtgg gcagataacc   6900
   tgaggtcaga gttcaagacc agcctggtca acatggcaaa accccgtctc tactaaaaat   6960
   acaaaaatta gccaggctgg tggtgcatcc ctgtagtccc agccactcag gaggctgagg   7020
   catgagaatc acttgaaccc gggaggtgga ggttgcagtg agccgagatt gtgccactgc   7080
   actccagcct gggagacaga gcaagactct gtttcaaaag aaaaaaaaga gtattctgga   7140
   gattgaagtt caggagttca gggttcatct cgactttggg cagccaagca agaactaaag   7200
   tataccaaga tgttgaaggt tgataccttt ttatttatcg attcattcac tcacctatgt   7260
   accaaagagc tcctgagcct ctcttcnata cagggggcac tgccaggagt tgtagaggat   7320
   gtgatagcaa agataggaaa taccttttct ctttgctctg acaacggtgg ggcaaggatt   7380
   catcattgat ttcagcagga ggcaggataa aatgtgtgta ggaatatagg aatacatggc   7440
   aatcagtaac atgtggtacc tagcagcatg tctgactgtt gatacggtca ggctaggtac   7500
   atcccctcag ggaagaactt ctgtcttagg ggcacacacc ctatcttttt tcccttcctg   7560
   ccaattcaca ggtaagaaca tttagtccca gggaactatg tcatctctct acttctcata   7620
   actgaaaaag cagtgccaat tatgtatgag gtataggaga cacaattctc cctcttttta   7680
   aaaatgttta atagctttat taaggtgtaa atgacataaa aactgcatgt agctaaagca   7740
   tgcaatttgg cacacatgta tctctacacc cttgaaacca tcaccactag aaaggtgcat   7800
   ttctccccag gaagaggggc aagtctaggc cctttgccag agttgctccc agattgtttt   7860
   caggttgggc ctgcattcac agctcagcca gctgaagggt gacagcatta gactcgtgac   7920
   ccaaatctta aacccacaca ttccatttta acattgatac ctgtgatcat cagccagtat   7980
   agcccatccc atgtgccagg cggaggcatc aataagctgg tctgtagcag ctttaatcac   8040
   agctggggag ccaggagcta aggaatgcta gactcctttg taaacaattt aagtaggggg   8100
   tacttagcct ggactctatg tgcttctcat ccacctcttc tcagggagat tctggccaag   8160
   tcctggaacc tactgcagtc catctggtgg ttgacagata tgtggacgga ttggcaggct   8220
   gggacccaat ctatgtttgc ccttgtgttc agttttgaga cctagcacct ttcctgatcc   8280
   tgctcacaga ccccctgcgg ccaataggaa agaagtgtta atgcatattt gcttttggag   8340
   ggcccaaagc caggcccaga gagttgtcaa gggcggtcag tggtgggtgg atggcagagt   8400
   taaccaagga gttacacacc tgcctagact aaggacaggc tggaccaagt agagagggtg   8460
   gggctaaggg agcctgagag atgctctggg gcctntctca aaatgagcac tatagtcacc   8520
   ctgtcccctg cagagattgt ctgacctggt tttaggtcac acccaacctt gccagccaag 8580
   gagtctttag aagcctgata ttgggagacc tgtcctgggg tctacaaccc cagaactcac 8640
   tgcagaagcc cacgtggatt gctagtctag ctcagccata tgggtcccca accctcacct 8700
   catgatagtc ctgtgagaaa ccgctgctga ccctttgttc atgttttcat cttttccact 8760
   ataaaagaca tgctagctgg gaaatagagc ccatcatact caagagtggc aggagccagg 8820
   tcctggcccc tgaagcttgg cctcacacac agaggccggc accctgtcat caattccctc 8880
   agctttttct ccgcctccac tcccagtcct agatttagca gccatgtgtg ggtgggggcc 8940
   actgcaggga tacttaccca cctaccagag agatggcctg tgggtgctgg cccttctgag 9000
   gctgtggagg ttggaggctg tggcagcctg ggcagtcagg ctgtggtcct cccatgttct 9060
   tgactcctgc tagtctgggc tgcctcctga ttaggggttg gatgctccag ttcttccctg 9120
   ggttggggat tgccacccta ctcccagccc atccaggttc acgcttattc caaagcggag 9180
   caccagcagt gtgcctgctg cgggagttct ccgtgtccag cctgagggtt gcctgccaac 9240
   ccctctgaga ggtgcccgga ggctgtgcgc ccacactgcc cagcagtgcg gagaagcagg 9300
   cttgtttttc cctgtcactg gcttggaaga gatgctttgt tctagggagc cgcatgtccc 9360
   cttgcctgcg ttgttggtga ggagccagca ggctccgtgg agggcaggct agcagcctgg 9420
   caccagggag gcaagggtct gagttcctag gagggtggtt gctcatgtga gaagtctgca 9480
   aaggttacta ctgagcacca tctctctgtc tgaaaaacat ttttcatttt tctgtgaacc 9540
   actaagtttc ccgtttgggc tttcttcctg cttttggctc ttgtttaggc aggcgtagcc 9600
   agatccaagc gtctggctgc ttccccatgt cttcagacct ctttttctgt tcatggtaac 9660
   tatagatgga accacacatt ggaagctgga aactcaagcg gtgcagccta ttccttaccc 9720
   caatccctgt tttacaaatg gggaaatcaa ggcacagcat ggggtgatgc ttatctgagg 9780
   ttggaagagt taatagtaga gtgggagcta aaacccagtt tcttacctcc aagctcaggg 9840
   ctttcagctg taattgagcc tagtatagtt ggtgtgcagc atcagggatt ccagctctaa 9900
   aggtcacaaa aaggaccnng gggntcattg gcccagggtg ggaaccngag cagagcaggt 9960
   ccagatggtg cactctgtgc cctggcctta gtttcttgct ggatgctttg gcccgtagag 10020
   ccccagagcc ctgcttccag aaccactcca gtgacgttca tgccaatggc ctgaacccca 10080
   ctgagcgagg gtgccctgcc tcttccacag ccctgggctc cgctcagatt tttaggagca 10140
   agngttagag gccttgcttt ctccagggtc agcatgtgga cagaacactt actctctgcc 10200
   tgtctctcct cctcaaaatg gaagcaagac agtggggcct acaatgctat gaaaaacagg 10260
   atgggaaaga agcctgctct ctgccttcct gcccaggtga gccatcacct gactaagtga 10320
   gttcacactc agagcgtgtg gggatggcag gatgttntga cttcattttc caggatgcnt 10380
   ttgntttaaa accttttaaa aagaagtgaa tgatacaccc cctttgctca tcttgcagtg 10440
   gaacctggtg tgtgaggacg actggaaggc cccactcaca atctccttgt tcttcgtggg 10500
   tgtgctgttg ggctccttca tttcagggca gctgtcagac aggtaaggtg tctgtcttct 10560
   ggagcaccag gggacctcan cactgaggaa gaagcgtgtg cctggccctt gatttcagtt 10620
   ggtagtattc tgtcagcgca gggccctgta ttttaaagaa gaggaagcta tgtctgtgat 10680
   atagactcca tgcctagtaa gaagagccaa caaatcnnnt gactccgtaa ttcttgctaa 10740
   gtaaagaaac ctgagctgtc taagctgaat gtatctgtga nccggttgac taggtaatat 10800
   gccatgattc acttctgcag tagcctggct tgcctcccct gggtcactgt gactctgtca 10860
   tgcccctgag catgggagag gttgacatca tgcacacatg cacatgtgct agattgtaga 10920
   tctgtagtag tgccacggtg tctgcctctg tagtcccaag aagaccagca ttctctctgc 10980
   aaagtgaaag gagctctcac cagccactag tggtatgaaa agcagaactc ttttgtccac 11040
   aaggctgatg ccccttagct aagtggcctg tggttttggc atttacttta tgacaggagg 11100
   gagaatagtg ntttgatcca tttcttataa gcaggttatt tgtataattn taaagctttt 11160
   aactcaagga aacattaacg gcttagagaa tcccaaaccc ctcgaaatta natgcacaat 11220
   gttagagact acatgtgagc atttttttgg agagaggtcc gtagctttca tgaagttctt 11280
   agaggggtcc attatgttgt gtcttctttc cctggagctt cagggtctgt cagagaagac 11340
   tgtgaagaga gtagcagcct tcagcaagtc cttggccaca tgncacatgt gagaacaccc 11400
   cacaaatcgg tgggttagcc ggtggaaagg agtcccagca tcttccctgg tttttaattc 11460
   ctggcctcaa gcaatcctcc ctccataanc tcccaaagtg cnnggattac aggcntgaga 11520
   caccatgcgc agccagatat tttttattgt tgttgttttt ttttggaaaa ggagtttcac 11580
   tcttgttacc caggctggag tgcaatggca cgatctcggc tcactgcaac ctctgcctcn 11640
   caggttcaag agattctcct gtctcagcct cctgagttgc tgggattaca ggcatgcacc 11700
   accatgccca gctaattttg tatttttaat agagacaagg tttctccatg ttggtcaggc 11760
   tggtctcaaa ctcccgacct caggtgatcc acccacctcg gcctcccaaa gtgctgggat 11820
   tacaggcgcg agccactgca cctgcccagc cagatttttt tttaaaaagc aggttaacct 11880
   gtttattatt cctactttac agatggagaa agtgagacag agggattaaa taacttnccc 11940
   aaggtttcac agctggcaag tggcagagtt aggatttgga cccaggtagt cttgctcctc 12000
   tattgtgtat ggactactgt tctaggtccc tgctgtccta aaacttgctt tctagcaagg 12060
   tggaatgtat taaacaacca agtgaggaag tcgttgttgt ccttggccat ggtaagagat 12120
   acagagaagt gcagggtgcc acaggagtgt ctaacagagg aggtcagggg cagcatccct 12180
   gagaaggtga agcatgagcg agagtgggaa gatgagtcga aagtagccag ctgaggggta 12240
   gagaggagaa agaacatcca ggcagggaga atagcaagtg ctaaagccgg ggctcatgaa 12300
   aaggcatggg agcaggacaa agtccgtgtg gtagaggtgc ggagagtggt gttaagatga 12360
   aggggagagg caggcagagc cctgggcaga tgagcaacca gggcttagtg gatcacagtt 12420
   aggactttgg gcttcagcca cagagcagcg gtgggctact gagatttttt aaagcaatag 12480
   tgtgacaatc agatttgtcc tttttttttt tttaaagatg ctttgacaac cttgtgaaga 12540
   aagaattgaa gggaagcaaa aggtgttgta gagagaccaa ttaaaaggtt gtcacagtag 12600
   ttcatgccag agatgatggt ggcatggcct agcatgacgg tggtagaaat ggagggaagt 12660
   ggtaagaggt aaaatcaaca agacttgccg atgggctgga tgtcggaatt gggggaagaa 12720
   agctttctgg cctgagtaac tgggtgaatg aaggtacact tctctaagac agagaatgct 12780
   ggaaaagaac cacgttcatg gatattgagt tcaatttgtg tgtactaaat ttggggtgac 12840
   tatgagaccc ctaagtggag aagtagagtg tgaagctggc tgtatggata tggtactgca 12900
   tgagaggttt tggctagaga aacacatgta ggatttgtca gcatatagaa ccctcagcag 12960
   agccccagat agggatgagg ttgcctgggg agaggggagt ggggagggga ggggggaact 13020
   gggggaggat tgtgctatgc ttagaggcca cctgagtgga catgggagat tccttctcac 13080
   ggagctaagt gacagctgcg gctcagaaga atgcccttga tgtgaaaagg agatacccca 13140
   agttcagagt agaaatgcag tgtatttttc tgggtcagct gtgtcatggg tcagcttaag 13200
   aaccttcttg ccatgtgaca caatgattac ctgaggaaag catttaagtt ccaaaaggtc 13260
   tattcccagg gaaagtggaa acaaaatttg tgagtgtgtt taggaccact ttagtctaca 13320
   gaagggctaa tatagtgttt ttcaaacctt tctaaacatt ttggccacag aactttcatt 13380
   aaagtaggat aatttaagtc tagtaaatga aatacaccct aantggctaa aagtatggct 13440
   gttctgcctg cagcccctgc cttcaattcc caatgccctg cctcaagcct gtctgtgccc 13500
   ccttggaagg cccagggccc tgtgggatgg acagcttctg agtgcattac ttctgagtaa 13560
   accagttcta aaacctaagc taagtagatt acctgaaagc aatcaattca tgccctaact 13620
   tgtctaggac atcaatgtaa ctttttatat agtataaagg tttcaccttc tttctggcct 13680
   atgaatatgt ctgaaattca agagcaatta aagtactcct gggttgtcaa agccctttat 13740
   gtaacactat tgagttatct ttatgcgtct gacttgtaag agatgcacaa ctctaggaag 13800
   aagtagactg ctgtgtcctg tttccaggta tgtgtgtgtt tgccattttg ttgacagact 13860
   ttaaaagcaa acatttctgg ccccaaccct gaactgccaa ggactggtgc tatgtaaagg 13920
   gttctctgga tctgtctctt ccctaccatc ccagggagct cttaggaagg gaaagggcat 13980
   agagattata ccagcctgcc ttgtggttag gaaccacccc ttggttggca tatagaacat 14040
   gcttgttaaa aaaaccatgc aggggaaagt agagtctact accaggcgag agtttctcaa 14100
   cctcgaccct attaacattt tgagccaaat aattttgttc tagggcattt tcctgggcat 14160
   tttagaatat ttaacaacat atctggcctc tactcattag atgccagtga accccaagtg 14220
   atggaaaaaa anaacaacaa cagaaaaaaa cctcttttat tgaggaaaaa caccaaactc 14280
   ttccacatag ttgcaagacc ttgtgcaatt tgcctcctag ccaccactgt actcttgaat 14340
   tgcacgcctg atgccaacca cactggttcc tcatgttcac catgccccct ccagccatgg 14400
   gggtgtgtgg tcttctcaga gtctgaagca ttccccaccc accccaaccc accccctgtg 14460
   gccttcttta accatgctgg ctaattcagg atccctagtt ccttatgact ttcctttaaa 14520
   acgtctacca gaaattgggg gaaaaaaagt gttattatag gattaatgtt ggtcttccnc 14580
   actatactgt gaatatcatt gagagcttgg tccctacacc ttaaatcccc catcgtcaac 14640
   tattttttcc catctcagtg tcccatgatc aaggagaccc tccctgaatg tccagttccc 14700
   caacccttac ccccagtcca gggtagcttc cttccttgtg cctctcatta acctgcatgc 14760
   cgatccttca gtgcacttga ctcagtgtgt aattgtatat tcagtagcgt gttgttagat 14820
   taaaatgtgg ttaatatgtg tttcaccagt tatactatga cactccttaa gggcagaaac 14880
   agcatctttt ttaatttatt gatatccaag tgccctctat aatagatgct caataaacat 14940
   tgaatgaaag tgggtgtcag ccagtactgg ccagactcaa actgaaccca ctgcttccca 15000
   ctagcttgac tttttcctcc tgtttgtggc actctcttta aaacaaacca aaataaaccc 15060
   aattttaaaa actttttaaa atgagcacgg atacagaaaa ccacacagaa caaatgtgta 15120
   gcttaatgaa tttttttcag agaaataacc ttatgaccac caccaagtcg agcagtagaa 15180
   ctttgctgtc cactaagaag ccctgtccat gtgccccatc ccaattacag catcctctct 15240
   ctctccccat taagtaaccg ctagcctgac tcctgtaata atcacttcct tgtgagtttt 15300
   tttagtttta ttatcgaaat atgcatcctt gacacaaatt tagtgttgcc cacttaatat 15360
   atttgatgtc ttttagtcta cttaatctat ggattctcct tctatcgcct tctatgcctt 15420
   actgattatc tatgaagaac ctgagctatt ccacctatag aatttcccag tctggatttg 15480
   ttgattgcac actgatgatg canttcagca cattcctcta tgctctgcat ttcctcaaaa 15540
   ttggcagttg gatccagaga cttgagattc aggttctgat tcaggttcag tccttttggc 15600
   nagaccatag gaagcatgca attcctgact gtctctttat gatgttaaca gtaattagta 15660
   tataatgcat agatctatta atccattggg ggctataaat ggtattattc taattttatt 15720
   accttttcat ttaaaagtta gaatactttt gtacntgata ctacctctta tctattattg 15780
   gttgctgttc acatagttta caaaggaaaa tcaggacaaa tgnttctttc tctttatttg 15840
   ccagttttca tttataatga attgtttctc tgttattctc caaatttggc agattctttt 15900
   ttaaaaaaaa tatcattatg aatgtatgga ttaaataatt gatgtatttc agtctcttgc 15960
   aatcattatc ataattgtag cattgctttt taggcaaccc tggtacccag gctgtacatt 16020
   tgtcatgggg agtggggagg gggagaaata gcatgggcac tntgagaccg agactgtccc 16080
   tggcagccag tattctggca acactgttca cacccactta ctggatggat cttgagaaag 16140
   ccccacttgg tggagcccat tcctgctgcc cttttccagc tggttatctg tcactctcct 16200
   tttnttccca ggtttggccg gaagaatgtg ctgttcgtga ccatgggcat gcagacaggc 16260
   ttcagcttcc tgcagatctt ctcgaagaat tttgagatgt ttgtcgtgct gtttgtcctt 16320
   gtaggcatgg gccagatctc caactatgtg gcagcatttg tcctgggtat ggccatcagg 16380
   ttggagttga gtacttgatc ctgtatttca ccatcatccc atcacctacc tttctggaga 16440
   caactgtgat gtccctcaag gggacagggg tttctaacaa aactagccag agcttcctgg 16500
   tgaaccttac ttacaggcag ggaaactgag ccagacatga gaccagcctg ggtccccagc 16560
   agcacaatgg cctgacttct gatttccagt tcttttctgg cctctgggct gtggctcctt 16620
   ggtcttagta cttgntngtc aatttactag gactcaccag agatcctcca tttacaaaaa 16680
   gggcctgcca ctgcacaggg ctgagccagc cccagaaaga gggnggcatg gttggaggag 16740
   gaggggctgt gactggcaag cttgctaagg tagagaaccc cttgtctgca gagcactgtg 16800
   gctggtgata tctacggaca agaataaatt gataggaagg ggctttcgtc accttcaggg 16860
   ttttaattca gagtgcacac tgcagggctt tgtctcaaat gtgccagcct gttgtcactg 16920
   agaagctgcc aggccggcct gtgtttggag gaacctgact ctagctgata aggcctttga 16980
   gttccttggg ttgtattgtt gaaagggttg ttttttcttt ttatatttaa tattctttcc 17040
   ttgaggctta agtcagcatg tgctgactta gtaatgactt cacttttaat aaattcttcc 17100
   tcatgtgagg attaaagggg gcctaccatg gcatctttag cacatggctt cagaacatgg 17160
   cgaaattttc aagagagaac tgttgcttgg gggcctgaga ggccacaggg atgtaccccc 17220
   aggagacagt cagacaggag gggttcagaa cgccatccgc tccctagcgc catgaactta 17280
   gagagagttc tcgctgtttt cttgtctgtg tattcacaaa gataccataa aaaattaata 17340
   aggaaggaac ccaaattaaa ctgctaactc gacctccctt gttttgaaca gggacagaaa 17400
   ttcttggcaa gtcagttcgt ataatattct ctacgttagg agtgtgcata ttttatgcat 17460
   ttggctacat ggtgctgcca ctgtttgctt acttcatccg agactggcgg atgctgctgg 17520
   tggcgctgac gatgccgggg gtgctntgcg tggcactctg gtggtgagtg tgaccctgtg 17580
   ccccatgtgc ccactggcag gatgatttct gtctggcctt cactagaggg cagcaacaac 17640
   ccatgaatcc ctattttgtc tcccagagac aggaagcata gattataaat tatttcagaa 17700
   tgttttctcc acactcaaaa gagccaaaac aaaacagaat cccatgacag caacagactt 17760
   gctctcagcc ctgtgctggg ttgccccaag tgtggggaaa aatagcagta gctgtgagaa 17820
   gatggggtcc agcatgccct gtaggaagtt cccaagcctc agggcaggac agtgtaggcc 17880
   ctagttctgg ctgtgtgctg ctgaagcctc atgccacagg cactggcacc aaaagcaaga 17940
   gtcctcaggg tagccacatg gaggaagcca ggctccttct gcaccaccaa ggtagaggag 18000
   ttgaacaggc agagaagagg ccattccaga ccaagagggg aacactgcag aggtgctaag 18060
   gtgggaatca ccccttgcag gtggagaagg tgagatcacc agcccaagtg gagcagagag 18120
   catttcaggg catagtggga gagtaagccg cacatcatgg ggcccagtca tgaccgaggg 18180
   tggggggcgg ctacctggtc ccagcaaggt ggaaaataat atccatagag cnctcaagtg 18240
   ccttgataaa catgctaatn tttttccttt ttttcttttc tttttctttt tttttctttt 18300
   ttttttctca gacaaagtct ctgttgccca ggctgtagtg cagtggcgtg atttccactt 18360
   actgcaacct ccgcctcccc agttcaagcg attctcatgt ctcagcctcc cgantagntg 18420
   ggantacagg tgcctgccac cgcacttggc taattttttt tgtatttctt tggagagacg 18480
   ggtttcactg cgttggccag gctggtctgg aactcctagc ctcaaatgat ccaccggcct 18540
   tggcctccta aagtgctggg attataggag tgaaccactg cacctggcca aacatgctat 18600
   tttaggtaga gtatctgact aatctgttgg ataaatcagg ggtagggtga ggagagaaga 18660
   gaagctaaaa ggccagtgca gaagcttctg ttggtgccgg ggacagggag gagagtgtag 18720
   cagggcctgg gctgacatag acatgcacag aagccaggct tccggagccc atcttgcacc 18780
   catctcctca gcccagcaga tggcaacact gctcttcaga aatggaggtg gccagccagc 18840
   atggggatgc cgtcaggggg tgcagggctc tcccattttt gtgcggtgtg gggtacacat 18900
   aagctcatcc accccaggtt attgctgcgt gtggatcagc tctttgcttc tggcttgtga 18960
   tcaccaaaca ttccacaagc tctggttctg caaccttatt cccacctatg gctgtgctct 19020
   acctggtctg tgggtctgct gttggcaggg aggcctcact gagattggac cttgtactgc 19080
   caggttcatc cctgagtccc cccgatggct catctctcag ggacgatttg aagaggcaga 19140
   ggtgatcatc cgcaaggctg ccaaagccaa tgggattgtt gtgccttcca ctatctttga 19200
   cccgagtgag gtaagcacca tgtgggtgtg ggtgagaggg acagactgac cgtgatttga 19260
   gagcagcagc acccagccct gaagtcctcc ctgctcacag cagcccagcc ctctctctgc 19320
   ccaagcccca actgcccatt ccccccatcc ccccactccc cacccccaca cgggccctgt 19380
   taacactcag aagttgagga ataggttaca gctgcctcac tcttttcacc acgggtttca 19440
   gattttcatt ttttacttcc tttctaggca atcatatatt ttaaccatta cttctaacaa 19500
   taaatactct ttttgagtaa taggcctttc ataaagtcag catttgggaa aatcattgtt 19560
   tcttatacct aaggtggctt gtcaccttac aaagctaacc ccaaacgtaa aatgtaaagc 19620
   acaaatagat ttggagttag aagtatttca tctcttgagt attagcaatt attcattaaa 19680
   aagaaaaaaa aagtgtttag tctctttctg ccctccaatg gttaattatt gcatatcatc 19740
   ttggagtcag gtcctttttg atgtccacct cttcccccta ccccacccct cccgtcagcc 19800
   ctgttctcac acaccatgac tcatttcttg gctctaccta gttcctggtt cttgcttttc 19860
   cttcccgttc cctctcctac catctctgta gcaggcagtt ttccttggtc tcgtgactat 19920
   gagaggttag aagctgtaaa tgctgcctgg tggggttctg gaatgtgtct gtggtctgac 19980
   tggaagatga ggggttgggt gtgggaacag ccacaagcag ccctgctgaa gtgtgagagg 20040
   caggcatggt tgggcttgga aaagagggaa cagttattgt agacagcgga ggccaatggc 20100
   cactgccagc cctgcagact tcccagtgag tggtggccca gcagccactg tcagcatgca 20160
   ccagaaaggg gtcctgtgcg caaaggtcag gcaggagtgt ggcagagggc ttttaagtta 20220
   ggtggttttg gggcttttta agtgaggggt caatctgggt gaatgcataa gccccactgg 20280
   catctttgag gaaatgaggc tatttcaggg gatactttca gtccaaagtt gaccttttgt 20340
   tgaacttcta actctggaaa aacaagctcc aaacctgggt ttgcttaaga aagcaacatc 20400
   agtgtgttta gacgtgtggt ttattaatgg ccttggctgt gctgaatttc ataggaagtc 20460
   actctgggtg aagctcaggt caattttcct gtttttctat ttgaattctt tttccctgga 20520
   agcacaccag taactacata gtataaggac tcaaaacatt aacttttaaa aaatatcaga 20580
   ccaataaacc acacagccag gtactctctc tgacccagag ggcagggagc caggcttcgg 20640
   gaggaatact tagaggcctc cttggaatgt ggccaccgac aggaatatgt gggggtgcag 20700
   tgaggaagct gtcagcctgg gcctctgtct tcctgtaccc ttgagggact ggtcacttac 20760
   ttttcctcat tttcattcac tctgatttnt tactgacaag gcctagggaa gttttcacag 20820
   cctaaaacac agtcagtata cttactgttc ttagaaacgt aacactcccc gacgctgaga 20880
   tgcagacagc taagatgcca gggattcaag tatgttattg tgtgctctga gtctctgacc 20940
   acctcttctt cccatacact tatgatgttg ttcctgcagt tacaagacct aagttccaag 21000
   aagcagcagt cccacaacat tctggatctg cttcgaacct ggaatatccg gatggtcacc 21060
   atcatgtcca taatgctgtg gtatgtaaaa gagacctgcc tgaggcttcc agacaaagct 21120
   tcttgaagtg gccattgggc ctcttgttta cagacatgcc tcagacaaaa ttcaaagcct 21180
   atgtcatcag agagtgaaaa ggatatgtct tgtgttagat ggaaaaaatg ggcatgtcac 21240
   aattcttaat gggatggaac ctcagaaaag gagaatgaaa acaattgtgg aggctgttgt 21300
   gggaaatatg gactctngtg gggaatctct ccagatctta agatgaatcc ttgcccaatt 21360
   tgggtcattt agttcccgtc tcctacccag ttaccgacag tggctgagga ggccaggtag 21420
   ggcttttaag aaggatctga gtgaagacac catgtcctgt aggctgcaga ggctgccagt 21480
   tactttctgg aaatgtggaa gtgggatgtg ctcctcctgg gatgtccata aacggtcctg 21540
   gagtcagggc tatagcctag atgtccttac caggttccca ctaatgaggc aaagtatgtc 21600
   agaaagggat ttgtgaatta ccagggagag gaaacatgtc caagtgcaca tcgctagctt 21660
   ttgctcagcg gccgaaccct gggattctag gcgacntctg gagcctggtg ggttagcggt 21720
   gagaagatgg gcgaggaggg cggacttcat ctcagagtcc ttattactag tctcatccag 21780
   ctttgaggca gtcagccact gtgcctactg agggagtgct atgagtcacc cgcttccaag 21840
   gaatggccca ggatccctcc aggcagttca ccattccctg agttggcctc aagacaggag 21900
   cagcatgtag cctgcaccac agacatgcaa gcctgtgatg agtcacccac ttttgtgttc 21960
   acccaggctc tcctccctgc tctggatttc ctggggactc atgcacatac tctttttatt 22020
   gtaccagctg tgtgttccac ctgcagatga gtcaaaacag tctaatccat aaaggtctgg 22080
   tttgtcaaag agtgtgggtc atcaacagag agaatgccta ctggggatgc ccaggtcagg 22140
   ggtactgcag ggcatcctga tgagaggcag tgtggcccct ccattgggag ccacctctnt 22200
   gctccacaag taccgcgggg ctggtgtcag ctgtctctga ccagcctctt cctgactggt 22260
   caccacaggt agtgtgtgag ggtctctctc caagtgtttg acctaatgtt gttccttttg 22320
   ttatcttatc ccccaaatcc tatcacacct cacttgatgt ctgcctcctg actcattctc 22380
   tagctccttc tgcagttgct ggatttgagg aggttcagct taggattttt aaagctgaaa 22440
   ggcaggttgg aatttttctt ttcaatgaag taaatctatc tgaattatac aagctttttt 22500
   gctgggacac tgtctatatg gaaggctctg agagcgcact ggcgcagggt ttacactgta 22560
   ccacttgggc tggggaaaat tatcttttga tctatgaagt aagacgcagg gttacagtta 22620
   ctgctgcctt actagtctct gcttaaagat ggtttggaat ttactgaaat aattgcattg 22680
   taaaagttgt acaggttggg aaagatgtgg atactgcttt tccagctttc ttctgcactc 22740
   tgtttcagga tgaccatatc agtgggctat tttgggtttc gcttgatact cctaacttgc 22800
   atggggacat ctttgtgaac tgcttccttt cagcgatggt tgaagtccca gcatatgtgt 22860
   tggcctggct gctgctgcaa tatttgcccc ggcgctattc catggccact gccctcttcc 22920
   tgggtggcag tgtccttctc ttcatgcagc tggtaccccc aggtagggac catgtgcatc 22980
   tatggtttgg ggtcttcact gagtctctta ctgtctacca ggctgtctca attaataaag 23040
   agaataaaat caagcccatc acagctccct tgcttatata cattcttggc ctaaaaatca 23100
   atagaaagtg tcttctgaga ctagaacact tatggcctgg gctttgaggg agtgggaaaa 23160
   agcagccatt ggggctgttg gttaatttta ctctgtaccc aagttaatgt gctcatactg 23220
   ttttccactg cagaagaaga gggaagaaat agctatccca ttcctttttt tcctggccct 23280
   gtcttcttta tttattcaac aaatagctat ggcatgccta ccatgggctg ggcactgtgc 23340
   tcggtgataa cgatacacaa gaaaacaagc caggcagaac cccaggccct catggaacnt 23400
   acaccctaga tgagaagaca gacaacaaac aagtaaataa aatgcttaat atagttcaga 23460
   ctgtgttacc ttctaggaat acaaatgaag gacaatgccg agttagttta catagtcaca 23520
   gatagtgtcc ctgaacaggg ggcagttcag tagaaatgta cataaagtga cagaaagccc 23580
   tgaaaaagtc taggagaaca ttttaggaag aagaaatggc aaaggcagcg accctgagca 23640
   ggggatgagc ctggcatgtt tgaggaggag ggagaagggg aggggccaga ccactgagag 23700
   ggcctcacag anccttagca ggattttatt tctgaaacta tcttagtatc ccacagatgg 23760
   gtgggaggta gccatttcca ataatttata gaacagttca tgggccctca tctctccctc 23820
   tccatcactg tgcccagaga cttcagtgta cctgtagatt tgggagcctc tgatggtcac 23880
   ttttgggccc atcaggctga gaacactgca cgggaacagc tccccatggg atgtggcagg 23940
   aggagcccag aactgatgta gaggctcaca gctgagctca gagtgacctt caggtcacac 24000
   atagctctcc catcagcaca gcacagagag attagaanat caactcgaga ttctgatggc 24060
   ctatgatttt tttgaggtct gagtgggagg aaagcatgaa atgagttaga actgaattct 24120
   ccattcatct aaacatcatg agttaattcc atagtgcctg cagtgtgagg ttctggggtg 24180
   acagttaatc cctgacagac atgtctttaa tgacttatag actgggaagc aggttgattg 24240
   gactattaag gagcttactc tggtggtctc caggttgagg aaagtgcatg tccttatagc 24300
   tgcaggtccc agcctccttt cagcaatcaa tttggaggga aatcttggct atagcccctt 24360
   cccccacaat aggaagtgat agaaactgac tccccaaaaa atttgggaag aaagtatgtt 24420
   tgttttgctc tcaatagctg catgccatgg gttggtacct actcctaccc tctttccttt 24480
   gcttctccag acttgtatta tttggctaca gtcctggtga tggtgggcaa gtttggagtc 24540
   acggctgcct tttccatggt ctacntgtac acagccgagc tgtatcccac agtggtgaga 24600
   aacatgggtg tgggagtcag ctccacagca tcccgcctgg gcagcatcct gtctccctac 24660
   ttcgtttacc ttggtaagtc ccatgagcca agggcacact agagcaacgg gatggaagta 24720
   ctaactggct tggagctgga ggttgcgtgt taacaggaaa acaagttcat acagtacatg 24780
   ggctccatcc agtactggat ctttggccgg gaagggttct tgtcccagtg cactggccct 24840
   cactttcaaa tggaaaacaa cctatagatt acctagaaat tgatgagaat attagagggt 24900
   ttgtttctgt tttagccatc ccaggccttc catcagagac tacaattcct ttatcctaag 24960
   aacctacaga gtggtttagg gagccagtgt gcttagttgg agaaatttct tggaatcaga 25020
   gtttaaaagg aacatgaggg gaaagatgtc catgcaagag gtctgatgaa cgnaaaatta 25080
   ttataaccta gagcactata gagtgatttt atcttgtgtg aagatccacc ccatgccatt 25140
   ttatgtagca ggtctccagt tttctcttct cagaattatg tcttcatagc acctgtggtt 25200
   tccctgcaca tccctagcca gtacctcttt agggagggtg gcacccacct gagagtactc 25260
   agagtgcttt gtgaacatgc tatgtagatc tcaaagcaag caaaagcacc ctgcctaatc 25320
   tgaaggcaga tcacatgggc tgggacacat ctgcagaggt ggaagagtta tttccatccc 25380
   tggacaagta cctcaggttc cttggaaacc caaccttgnn aaatangaat aatcagcatg 25440
   gcagaaatag gaataatcag catggcccag ctcttctcct gcaaccgccc ctttgtactc 25500
   ctcccctgca tggtggaaca ctgctgggct ctgggcatgc ctgtgccagc tctgggttct 25560
   gaaacctgtc tagatgccag attctaatct gactgctcag actgtgagag atgtgagacc 25620
   aagaaggaaa gtgatcccct tccagagtcc tgggagcata aaggggtaga tgagagacca 25680
   agtctaactg cagccctggg cctgaggctc cgtctgcttt gccataggtg cctacgaccg 25740
   cttcctgccc tacattctca tgggaagtct gaccatcctg acagccatcc tcaccttgtt 25800
   tctcccagag agcttcggta ccccactccc agacaccatt gaccagatgc taagagtcaa 25860
   agggtaagaa gacctcctct gtcagtgttg atgcactggg tctgggtctg gccaggtctc 25920
   aggagcccct cacaatagag ctactcgcaa actccctctc acagacacca tggactagtt 25980
   tagccattaa agggttgtaa atggcaaggt gcttacttat agcccatcct ctctggtctg 26040
   ttcctgtgtg gacatgtcac tatacacatc tccatggcag tagccgcact ggataactca 26100
   gaggctagaa gaaacctttc agaatctgct gcaggattct cttcccaggg aagatatcct 26160
   cagttcttgt ttgtttggag actgggaggc atctttttaa aatgtgttac tgacatattt 26220
   ttgcttgttt ttatagaatg aaacacagaa aaactccaag tcacacaagg atgttaaaag 26280
   atggtcaaga aaggcccaca atccttaaaa gcacagcctt ctaacatcgc ttccagtaag 26340
   ggagaaactg aagaggaaag actgtcttgc cagaaatggc cagcttgtgc agactccgag 26400
   tccttcagtg acaaaggcct ttgctgtttg ccctcttgac ctgtgtctga cttgctcctg 26460
   gatgggcacc cacactcaga ggctacatat ggccctagag caccaccttc ctctagggac 26520
   actggggcta cctacagaca acttcatcta agtcctaact attacaatga tggactcagc 26580
   acctccaaag cagttaattt ttcactagaa ccagtgagat ctggaggaat gtgagaagca 26640
   tatgctaaat gtacatttta attttagact acttgaaaag gcccctaata aggctagagg 26700
   tctaagtccc ccaccccttt ccccactccc ctctagtggt gaactttaga ggaaaaggaa 26760
   gtaattgcac aaggagtttg attcttacct tttctcagtt acagaggaca ttaactggat 26820
   cattgcttcc ccagggcagg agagcgcaga 26850
<210> 42
   <211> 54550
   <212> DNA
   <213> Homo sapiens
<220>
   <221> misc_feature
   <222> (351)..(351)
   <223> n can be a or t or g or c
<220>
   <221> misc_feature
   <222> (436)..(436)
   <223> n can be a or t or g or c
<220>
   <221> misc_feature
   <222> (935)..(935)
   <223> n can be a or t or g or c
<220>
   <221> misc_feature
   <222> (1725)..(1725)
   <223> n can be a or t or g or c
<220>
   <221> misc_feature
   <222> (1874)..(1874)
   <223> n can be a or t or g or c
<220>
   <221> misc_feature
   <222> (2031)..(2031)
   <223> n can be a or t or g or c
<220>
   <221> misc_feature
   <222> (2152)..(2152)
   <223> n can be a or t or g or c
<220>
   <221> misc_feature
   <222> (2297)..(2297)
   <223> n can be a or t or g or c
<220>
   <221> misc_feature
   <222> (2747)..(2747)
   <223> n can be a or t or g or c
<220>
   <221> misc_feature
   <222> (3477)..(3477)
   <223> n can be a or t or g or c
<220>
   <221> misc_feature
   <222> (3535)..(3535)
   <223> n can be a or t or g or c
<220>
   <221> misc_feature
   <222> (4035)..(4035)
   <223> n can be a or t or g or c
<220>
   <221> misc_feature
   <222> (4084)..(4084)
   <223> n can be a or t or g or c
<220>
   <221> misc_feature
   <222> (4087)..(4087)
   <223> n can be a or t or g or c
<220>
   <221> misc_feature
   <222> (4321)..(4321)
   <223> n can be a or t or g or c
<220>
   <221> misc_feature
   <222> (4374)..(4374)
   <223> n can be a or t or g or c
<220>
   <221> misc_feature
   <222> (4394)..(4394)
   <223> n can be a or t or g or c
<220>
   <221> misc_feature
   <222> (4429)..(4429)
   <223> n can be a or t or g or c
<220>
   <221> misc_feature
   <222> (4480)..(4480)
   <223> n can be a or t or g or c
<220>
   <221> misc_feature
   <222> (4482)..(4482)
   <223> n can be a or t or g or c
<220>
   <221> misc_feature
   <222> (4704)..(4704)
   <223> n can be a or t or g or c
<220>
   <221> misc_feature
   <222> (4799)..(4799)
   <223> n can be a or t or g or c
<220>
   <221> misc_feature
   <222> (4800)..(4800)
   <223> n can be a or t or g or c
<220>
   <221> misc_feature
   <222> (4801)..(4801)
   <223> n can be a or t or g or c
<220>
   <221> misc_feature
   <222> (4802)..(4802)
   <223> n can be a or t or g or c
<220>
   <221> misc_feature
   <222> (4803)..(4803)
   <223> n can be a or t or g or c
<220>
   <221> misc_feature
   <222> (4805)..(4805)
   <223> n can be a or t or g or c
<220>
   <221> misc_feature
   <222> (4806)..(4806)
   <223> n can be a or t or g or c
<220>
   <221> misc_feature
   <222> (4809)..(4809)
   <223> n can be a or t or g or c
<220>
   <221> misc_feature
   <222> (5164)..(5164)
   <223> n can be a or t or g or c
<220>
   <221> misc_feature
   <222> (5195)..(5195)
   <223> n can be a or t or g or c
<220>
   <221> misc_feature
   <222> (5207)..(5207)
   <223> n can be a or t or g or c
<220>
   <221> misc_feature
   <222> (6668)..(6668)
   <223> n can be a or t or g or c
<220>
   <221> misc_feature
   <222> (7458)..(7458)
   <223> n can be a or t or g or c
<220>
   <221> misc_feature
   <222> (7666)..(7666)
   <223> n can be a or t or g or c
<220>
   <221> misc_feature
   <222> (7687)..(7687)
   <223> n can be a or t or g or c
<220>
   <221> misc_feature
   <222> (7730)..(7730)
   <223> n can be a or t or g or c
<220>
   <221> misc_feature
   <222> (8656)..(8656)
   <223> n can be a or t or g or c
<220>
   <221> misc_feature
   <222> (9403)..(9403)
   <223> n can be a or t or g or c
<220>
   <221> misc_feature
   <222> (9598)..(9598)
   <223> n can be a or t or g or c
<220>
   <221> misc_feature
   <222> (10245)..(10245)
   <223> n can be a or t or g or c
<220>
   <221> misc_feature
   <222> (10817)..(10817)
   <223> n can be a or t or g or c
<220>
   <221> misc_feature
   <222> (10829)..(10829)
   <223> n can be a or t or g or c
<220>
   <221> misc_feature
   <222> (10895)..(10895)
   <223> n can be a or t or g or c
<220>
   <221> misc_feature
   <222> (11001)..(11001)
   <223> n can be a or t or g or c
<220>
   <221> misc_feature
   <222> (11049)..(11049)
   <223> n can be a or t or g or c
<220>
   <221> misc_feature
   <222> (11063)..(11063)
   <223> n can be a or t or g or c
<220>
   <221> misc_feature
   <222> (11067)..(11067)
   <223> n can be a or t or g or c
<220>
   <221> misc_feature
   <222> (11192)..(11192)
   <223> n can be a or t or g or c
<220>
   <221> misc_feature
   <222> (11294)..(11294)
   <223> n can be a or t or g or c
<220>
   <221> misc_feature
   <222> (11336)..(11336)
   <223> n can be a or t or g or c
<220>
   <221> misc_feature
   <222> (11644)..(11644)
   <223> n can be a or t or g or c
<220>
   <221> misc_feature
   <222> (12434)..(12434)
   <223> n can be a or t or g or c
<220>
   <221> misc_feature
   <222> (12531)..(12531)
   <223> n can be a or t or g or c
<220>
   <221> misc_feature
   <222> (12630)..(12630)
   <223> n can be a or t or g or c
<220>
   <221> misc_feature
   <222> (12796)..(12796)
   <223> n can be a or t or g or c
<220>
   <221> misc_feature
   <222> (13123)..(13123)
   <223> n can be a or t or g or c
<220>
   <221> misc_feature
   <222> (13365)..(13365)
   <223> n can be a or t or g or c
<220>
   <221> misc_feature
   <222> (13988)..(13988)
   <223> n can be a or t or g or c
<220>
   <221> misc_feature
   <222> (13991)..(13991)
   <223> n can be a or t or g or c
<220>
   <221> misc_feature
   <222> (14048)..(14048)
   <223> n can be a or t or g or c
<220>
   <221> misc_feature
   <222> (14136)..(14136)
   <223> n can be a or t or g or c
<220>
   <221> misc_feature
   <222> (14346)..(14346)
   <223> n can be a or t or g or c
<220>
   <221> misc_feature
   <222> (14842)..(14842)
   <223> n can be a or t or g or c
<220>
   <221> misc_feature
   <222> (14889)..(14889)
   <223> n can be a or t or g or c
<220>
   <221> misc_feature
   <222> (14901)..(14901)
   <223> n can be a or t or g or c
<220>
   <221> misc_feature
   <222> (15119)..(15119)
   <223> n can be a or t or g or c
<220>
   <221> misc_feature
   <222> (15815)..(15815)
   <223> n can be a or t or g or c
<220>
   <221> misc_feature
   <222> (15879)..(15879)
   <223> n can be a or t or g or c
<220>
   <221> misc_feature
   <222> (16767)..(16767)
   <223> n can be a or t or g or c
<220>
   <221> misc_feature
   <222> (16829)..(16829)
   <223> n can be a or t or g or c
<220>
   <221> misc_feature
   <222> (16832)..(16832)
   <223> n can be a or t or g or c
<220>
   <221> misc_feature
   <222> (16932)..(16932)
   <223> n can be a or t or g or c
<220>
   <221> misc_feature
   <222> (16971)..(16971)
   <223> n can be a or t or g or c
<220>
   <221> misc feature
   <222> (16982)..(16982)
   <223> n can be a or t or g or c
<220>
   <221> misc_feature
   <222> (16983)..(16983)
   <223> n can be a or t or g or c
<220>
   <221> misc_feature
   <222> (16984)..(16984)
   <223> n can be a or t or g or c
<220>
   <221> misc_feature
   <222> (16986)..(16986)
   <223> n can be a or t or g or c
<220>
   <221> misc_feature
   <222> (17598)..(17598)
   <223> n can be a or t or g or c
<220>
   <221> misc_feature
   <222> (17653)..(17653)
   <223> n can be a or t or g or c
<220>
   <221> misc_feature
   <222> (18106)..(18106)
   <223> n can be a or t or g or c
<220>
   <221> misc_feature
   <222> (18190)..(18190)
   <223> n can be a or t or g or c
<220>
   <221> misc_feature
   <222> (18242)..(18242)
   <223> n can be a or t or g or c
<220>
   <221> misc_feature
   <222> (18266)..(18266)
   <223> n can be a or t or g or c
<220>
   <221> misc_feature
   <222> (18277)..(18277)
   <223> n can be a or t or g or c
<220>
   <221> misc_feature
   <222> (18292)..(18292)
   <223> n can be a or t or g or c
<220>
   <221> misc_feature
   <222> (18319)..(18319)
   <223> n can be a or t or g or c
<220>
   <221> misc_feature
   <222> (18348)..(18348)
   <223> n can be a or t or g or c
<220>
   <221> misc_feature
   <222> (18379)..(18379)
   <223> n can be a or t or g or c
<220>
   <221> misc_feature
   <222> (18995)..(18995)
   <223> n can be a or t or g or c
<220>
   <221> misc_feature
   <222> (19091)..(19091)
   <223> n can be a or t or g or c
<220>
   <221> misc_feature
   <222> (19112)..(19112)
   <223> n can be a or t or g or c
<220>
   <221> misc_feature
   <222> (19809)..(19809)
   <223> n can be a or t or g or c
<220>
   <221> misc_feature
   <222> (20072)..(20072)
   <223> n can be a or t or g or c
<220>
   <221> misc_feature
   <222> (20604)..(20604)
   <223> n can be a or t or g or c
<220>
   <221> mise_feature
   <222> (20752)..(20752)
   <223> n can be a or t or g or c
<220>
   <221> misc_feature
   <222> (20874)..(20874)
   <223> n can be a or t or g or c
<220>
   <221> misc_feature
   <222> (21039)..(21039)
   <223> n can be a or t or g or c
<220>
   <221> misc feature
   <222> (21552)..(21552)
   <223> n can be a or t or g or c
<220>
   <221> misc_feature
   <222> (21655)..(21655)
   <223> n can be a or t or g or c
<220>
   <221> misc_feature
   <222> (21656)..(21656)
   <223> n can be a or t or g or c
<220>
   <221> misc_feature
   <222> (21657)..(21657)
   <223> n can be a or t or g or c
<220>
   <221> misc_feature
   <222> (21676)..(21676)
   <223> n can be a or t or g or c
<220>
   <221> misc_feature
   <222> (21724)..(21724)
   <223> n can be a or t or g or c
<220>
   <221> misc_feature
   <222> (21731)..(21731)
   <223> n can be a or t or g or c
<220>
   <221> misc_feature
   <222> (21745)..(21745)
   <223> n can be a or t or g or c
<220>
   <221> misc_feature
   <222> (21920)..(21920)
   <223> n can be a or t or g or c
<220>
   <221> misc_feature
   <222> (21956)..(21956)
   <223> n can be a or t or g or c
<220>
   <221> misc_feature
   <222> (22100)..(22100)
   <223> n can be a or t or g or c
<220>
   <221> misc_feature
   <222> (22102)..(22102)
   <223> n can be a or t or g or c
<220>
   <221> misc_feature
   <222> (22160)..(22160)
   <223> n can be a or t or g or c
<220>
   <221> misc_feature
   <222> (22184)..(22184)
   <223> n can be a or t or g or c
<220>
   <221> misc_feature
   <222> (22208)..(22208)
   <223> n can be a or t or g or c
<220>
   <221> misc_feature
   <222> (22267)..(22267)
   <223> n can be a or t or g or c
<220>
   <221> misc_feature
   <222> (22327)..(22327)
   <223> n can be a or t or g or c
<220>
   <221> misc_feature
   <222> (22328)..(22328)
   <223> n can be a or t or g or c
<220>
   <221> misc_feature
   <222> (22329)..(22329)
   <223> n can be a or t or g or c
<220>
   <221> misc_feature
   <222> (22330)..(22330)
   <223> n can be a or t or g or c
<220>
   <221> misc_feature
   <222> (22331)..(22331)
   <223> n can be a or t or g or c
<220>
   <221> misc_feature
   <222> (22348)..(22348)
   <223> n can be a or t or g or c
<220>
   <221> misc_feature
   <222> (22385)..(22385)
   <223> n can be a or t or g or c
<220>
   <221> misc_feature
   <222> (22423)..(22423)
   <223> n can be a or t or g or c
<220>
   <221> misc_feature
   <222> (22683)..(22683)
   <223> n can be a or t or g or c
<220>
   <221> misc_feature
   <222> (23339)..(23339)
   <223> n can be a or t or g or c
<220>
   <221> misc_feature
   <222> (23423)..(23423)
   <223> n can be a or t or g or c
<220>
   <221> misc_feature
   <222> (23463)..(23463)
   <223> n can be a or t or g or c
<220>
   <221> misc_feature
   <222> (23469).(23469)
   <223> n can be a or t or g or c
<220>
   <221> misc_feature
   <222> (23499)..(23499)
   <223> n can be a or t or g or c
<220>
   <221> misc_feature
   <222> (24198)..(24198)
   <223> n can be a or t or g or c
<220>
   <221> misc_feature
   <222> (24522)..(24522)
   <223> n can be a or t or g or c
<220>
   <221> misc_feature
   <222> (25144)..(25144)
   <223> n can be a or t or g or c
<220>
   <221> misc_feature
   <222> (25458)..(25458)
   <223> n can be a or t or g or c
<220>
   <221> misc_feature
   <222> (25470)..(25470)
   <223> n can be a or t or g or c
<220>
   <221> misc_feature
   <222> (25842)..(25842)
   <223> n can be a or t or g or c
<220>
   <221> misc_feature
   <222> (26054)..(26054)
   <223> n can be a or t or g or c
<220>
   <221> misc_feature
   <222> (26088)..(26088)
   <223> n can be a or t or g or c
<220>
   <221> misc_feature
   <222> (26519)..(26519)
   <223> n can be a or t or g or c
<220>
   <221> misc_feature
   <222> (26954)..(26954)
   <223> n can be a or t or g or c
<220>
   <221> misc_feature
   <222> (26975)..(26975)
   <223> n can be a or t or g or c
<220>
   <221> misc_feature
   <222> (27016)..(27016)
   <223> n can be a or t or g or c
<220>
   <221> misc_feature
   <222> (27079)..(27079)
   <223> n can be a or t or g or c
<220>
   <221> misc_feature
   <222> (27092)..(27092)
   <223> n can be a or t or g or c
<220>
   <221> misc_feature
   <222> (27093)..(27093)
   <223> n can be a or t or g or c
<220>
   <221> misc_feature
   <222> (27672)..(27672)
   <223> n can be a or t or g or c
<220>
   <221> misc_feature
   <222> (27788)..(27788)
   <223> n can be a or t or g or c
<220>
   <221> misc_feature
   <222> (28202)..(28202)
   <223> n can be a or t or g or c
<220>
   <221> misc_feature
   <222> (29025)..(29025)
   <223> n can be a or t or g or c
<220>
   <221> misc_feature
   <222> (29232)..(29232)
   <223> n can be a or t or g or c
<220>
   <221> misc_feature
   <222> (29325)..(29325)
   <223> n can be a or t or g or c
<220>
   <221> misc_feature
   <222> (29352)..(29352)
   <223> n can be a or t or g or c
<220>
   <221> misc_feature
   <222> (29355)..(29355)
   <223> n can be a or t or g or c
<220>
   <221> misc_feature
   <222> (29617)..(29617)
   <223> n can be a or t or g or c
<220>
   <221> misc_feature
   <222> (29618)..(29618)
   <223> n can be a or t or g or c
<220>
   <221> misc_feature
   <222> (29619)..(29619)
   <223> n can be a or t or g or c
<220>
   <221> misc_feature
   <222> (29623)..(29623)
   <223> n can be a or t or g or c
<220>
   <221> misc_feature
   <222> (29628)..(29628)
   <223> n can be a or t or g or c
<220>
   <221> misc_feature
   <222> (29635)..(29635)
   <223> n can be a or t or g or c
<220>
   <221> misc_feature
   <222> (29680)..(29680)
   <223> n can be a or t or g or c
<220>
   <221> misc_feature
   <222> (29693)..(29693)
   <223> n can be a or t or g or c
<220>
   <221> misc_feature
   <222> (29796)..(29796)
   <223> n can be a or t or g or c
<220>
   <221> misc_feature
   <222> (29816)..(29816)
   <223> n can be a or t or g or c
<220>
   <221> misc_feature
   <222> (29823)..(29823)
   <223> n can be a or t or g or c
<220>
   <221> misc_feature
   <222> (29844)..(29844)
   <223> n can be a or t or g or c
<220>
   <221> misc_feature
   <222> (29923)..(29923)
   <223> n can be a or t or g or c
<220>
   <221> misc_feature
   <222> (29928)..(29928)
   <223> n can be a or t or g or c
<220>
   <221> misc_feature
   <222> (29964)..(29964)
   <223 > n can be a or t or g or c
<220>
   <221> misc_feature
   <222> (29984)..(29984)
   <223> n can be a or t or g or c
<220>
   <221> misc_feature
   <222> (30017)..(30017)
   <223> n can be a or t or g or c
<220>
   <221> misc_feature
   <222> (30024)..(30024)
   <223> n can be a or t or g or c
<220>
   <221> misc_feature
   <222> (30034)..(30034)
   <223> n can be a or t or g or c
<220>
   <221> misc_feature
   <222> (30037)..(30037)
   <223> n can be a or t or g or c
<220>
   <221> misc_feature
   <222> (30793)..(30793)
   <223> n can be a or t or g or c
<220>
   <221> misc_feature
   <222> (30976)..(30976)
   <223> n can be a or t or g or c
<220>
   <221> misc_feature
   <222> (31273)..(31273)
   <223> n can be a or t or g or c
<220>
   <221> misc_feature
   <222> (32695)..(32695)
   <223> n can be a or t or g or c
<220>
   <221> misc_feature
   <222> (32850)..(32850)
   <223> n can be a or t or g or c
<220>
   <221> misc_feature
   <222> (32854)..(32854)
   <223> n can be a or t or g or c
<220>
   <221> misc_feature
   <222> (33053)..(33053)
   <223> n can be a or t or g or c
<220>
   <221> misc_feature
   <222> (33070)..(33070)
   <223> n can be a or t or g or c
<220>
   <221> misc_feature
   <222> (33549)..(33549)
   <223> n can be a or t or g or c
<220>
   <221> misc_feature
   <222> (33571)..(33571)
   <223> n can be a or t or g or c
<220>
   <221> misc_feature
   <222> (33894)..(33894)
   <223> n can be a or t or g or c
<220>
   <221> misc_feature
   <222> (34106)..(34106)
   <223> n can be a or t or g or c
<220>
   <221> misc_feature
   <222> (34395)..(34395)
   <223> n can be a or t or g or c
<220>
   <221> misc_feature
   <222> (34621)..(34621)
   <223> n can be a or t or g or c
<220>
   <221> misc_feature
   <222> (34640)..(34640)
   <223> n can be a or t or g or c
<220>
   <221> misc_feature
   <222> (34641)..(34641)
   <223> n can be a or t or g or c
<220>
   <221> misc_feature
   <222> (34645)..(34645)
   <223> n can be a or t or g or c
<220>
   <221> misc_feature
   <222> (34646)..(34646)
   <223> n can be a or t or g or c
<220>
   <221> misc_feature
   <222> (34649)..(34649)
   <223> n can be a or t or g or c
<220>
   <221> misc_feature
   <222> (34656)..(34656)
   <223> n can be a or t or g or c
<220>
   <221> misc_feature
   <222> (34707)..(34707)
   <223> n can be a or t or g or c
<220>
   <221> misc_feature
   <222> (34737)..(34737)
   <223> n can be a or t or g or c
<220>
   <221> misc_feature
   <222> (34746)..(34746)
   <223> n can be a or t or g or c
<220>
   <221> misc_feature
   <222> (35432)..(35432)
   <223> n can be a or t or g or c
<220>
   <221> misc_feature
   <222> (35625)..(35625)
   <223> n can be a or t or g or c
<220>
   <221> misc_feature
   <222> (35802)..(35802)
   <223> n can be a or t or g or c
<220>
   <221> misc_feature
   <222> (36086)..(36086)
   <223> n can be a or t or g or c
<220>
   <221> misc_feature
   <222> (36269)..(36269)
   <223> n can be a or t or g or c
<220>
   <221> misc_feature
   <222> (36736)..(36736)
   <223> n can be a or t or g or c
<220>
   <221> misc_feature
   <222> (36783)..(36783)
   <223> n can be a or t or g or c
<220>
   <221> misc_feature
   <222> (37338)..(37338)
   <223> n can be a or t or g or c
<220>
   <221> misc_feature
   <222> (37456)..(37456)
   <223> n can be a or t or g or c
<220>
   <221> misc_feature
   <222> (37457)..(37457)
   <223> n can be a or t or g or c
<220>
   <221> misc_feature
   <222> (37914)..(37914)
   <223> n can be a or t or g or c
<220>
   <221> misc_feature
   <222> (38619)..(38619)
   <223> n can be a or t or g or c
<220>
   <221> misc_feature
   <222> (38664)..(38664)
   <223> n can be a or t or g or c
<220>
   <221> misc_feature
   <222> (38787)..(38787)
   <223> n can be a or t or g or c
<220>
   <221> misc_feature
   <222> (38789)..(38789)
   <223> n can be a or t or g or c
<220>
   <221> misc_feature
   <222> (38791)..(38791)
   <223> n can be a or t or g or c
<220>
   <221> misc_feature
   <222> (38808)..(38808)
   <223> n can be a or t or g or c
<220>
   <221> misc_feature
   <222> (38811)..(38811)
   <223> n can be a or t or g or c
<220>
   <221> misc_feature
   <222> (38813)..(38813)
   <223> n can be a or t or g or c
<220>
   <221> misc_feature
   <222> (38815)..(38815)
   <223> n can be a or t or g or c
<220>
   <221> misc_feature
   <222> (38817)..(38817)
   <223> n can be a or t or g or c
<220>
   <221> misc_feature
   <222> (38819)..(38819)
   <223> n can be a or t or g or c
<220>
   <221> misc_feature
   <222> (38820)..(38820)
   <223> n can be a or t or g or c
<220>
   <221> misc_feature
   <222> (38821)..(38821)
   <223> n can be a or t or g or c
<220>
   <221> misc_feature
   <222> (38822)..(38822)
   <223> n can be a or t or g or c
<220>
   <221> misc_feature
   <222> (38823)..(38823)
   <223> n can be a or t or g or c
<220>
   <221> misc_feature
   <222> (38825)..(38825)
   <223> n can be a or t or gore
<220>
   <221> misc_feature
   <222> (38826)..(38826)
   <223> n can be a or t or g or c
<220>
   <221> misc_feature
   <222> (38827)..(38827)
   <223> n can be a or t or g or c
<220>
   <221> misc_feature
   <222> (38832)..(38832)
   <223> n can be a or t or g or c
<220>
   <221> misc_feature
   <222> (38834)..(38834)
   <223> n can be a or t or g or c
<220>
   <221> misc_feature
   <222> (38835)..(38835)
   <223> n can be a or t or g or c
<220>
   <221> misc_feature
   <222> (38836)..(38836)
   <223> n can be a or t or g or c
<220>
   <221> misc_feature
   <222> (38838)..(38838)
   <223> n can be a or t or g or c
<220>
   <221> misc_feature
   <222> (38840)..(38840)
   <223> n can be a or t or g or c
<220>
   <221> misc_feature
   <222> (38842)..(38842)
   <223> n can be a or t or g or c
<220>
   <221> misc_feature
   <222> (38850)..(38850)
   <223> n can be a or t or g or c
<220>
   <221> misc_feature
   <222> (38852)..(38852)
   <223> n can be a or t or g or c
<220>
   <221> misc_feature
   <222> (38854)..(38854)
   <223> n can be a or t or g or c
<220>
   <221> misc_feature
   <222> (38856)..(38856)
   <223> n can be a or t or g or c
<220>
   <221> misc_feature
   <222> (38858)..(38858)
   <223> n can be a or t or g or c
<220>
   <221> misc_feature
   <222> (38862)..(38862)
   <223> n can be a or t or g or c
<220>
   <221> misc_feature
   <222> (38864)..(38864)
   <223> n can be a or t or g or c
<220>
   <221> misc_feature
   <222> (38873)..(38873)
   <223> n can be a or t or g or c
<220>
   <221> misc_feature
   <222> (38875)..(38875)
   <223> n can be a or t or g or c
<220>
   <221> misc_feature
   <222> (38877)..(38877)
   <223> n can be a or t or g or c
<220>
   <221> misc_feature
   <222> (39427)..(39427)
   <223> n can be a or t or g or c
<220>
   <221> misc_feature
   <222> (39428)..(39428)
   <223> n can be a or t or g or c
<220>
   <221> misc_feature
   <222> (39435)..(39435)
   <223> n can be a or t or g or c
<220>
   <221> misc_feature
   <222> (39436)..(39436)
   <223> n can be a or t or g or c
<220>
   <221> misc_feature
   <222> (39439)..(39439)
   <223> n can be a or t or g or c
<220>
   <221> misc_feature
   <222> (39440)..(39440)
   <223> n can be a or t or g or c
<220>
   <221> misc_feature
   <222> (39441)..(39441)
   <223> n can be a or t or g or c
<220>
   <221> misc_feature
   <222> (39443)..(39443)
   <223> n can be a or t or g or c
<220>
   <221> misc_feature
   <222> (39445)..(39445)
   <223> n can be a or t or g or c
<220>
   <221> misc_feature
   <222> (39446)..(39446)
   <223> n can be a or t or g or c
<220>
   <221> misc_feature
   <222> (39447)..(39447)
   <223> n can be a or t or g or c
<220>
   <221> misc_feature
   <222> (39448)..(39448)
   <223> n can be a or t or g or c
<220>
   <221> misc_feature
   <222> (39626)..(39626)
   <223> n can be a or t or g or c
<220>
   <221> misc_feature
   <222> (39649)..(39649)
   <223> n can be a or t or g or c
<220>
   <221> misc_feature
   <222> (39671)..(39671)
   <223> n can be a or t or g or c
<220>
   <221> misc_feature
   <222> (39961)..(39961)
   <223> n can be a or t or g or c
<220>
   <221> misc_feature
   <222> (40088)..(40088)
   <223> n can be a or t or g or c
<220>
   <221> misc_feature
   <222> (40602)..(40602)
   <223> n can be a or t or g or c
<220>
   <221> misc_feature
   <222> (40653)..(40653)
   <223> n can be a or t or g or c
<220>
   <221> misc_feature
   <222> (41601)..(41601)
   <223> n can be a or t or g or c
<220>
   <221> misc_feature
   <222> (41796)..(41796)
   <223> n can be a or t or g or c
<220>
   <221> misc_feature
   <222> (41850)..(41850)
   <223> n can be a or t or g or c
<220>
   <221> misc_feature
   <222> (41863)..(41863)
   <223> n can be a or t or g or c
<220>
   <221> misc_feature
   <222> (41887)..(41887)
   <223> n can be a or t or g or c
<220>
   <221> misc_feature
   <222> (41984)..(41984)
   <223> n can be a or t or g or c
<220>
   <221> misc_feature
   <222> (42114)..(42114)
   <223> n can be a or t or g or c
<220>
   <221> misc_feature
   <222> (42436)..(42436)
   <223> n can be a or t or g or c
<220>
   <221> misc_feature
   <222> (42630)..(42630)
   <223> n can be a or t or g or c
<220>
   <221> misc_feature
   <222> (42631)..(42631)
   <223> n can be a or t or g or c
<220>
   <221> misc_feature
   <222> (42632)..(42632)
   <223> n can be a or t or g or c
<220>
   <221> misc_feature
   <222> (42652)..(42652)
   <223> n can be a or t or g or c
<220>
   <221> misc_feature
   <222> (42715)..(42715)
   <223> n can be a or t or g or c
<220>
   <221> misc_feature
   <222> (42850)..(42850)
   <223> n can be a or t or g or c
<220>
   <221> misc_feature
   <222> (42902)..(42902)
   <223> n can be a or t or g or c
<220>
   <221> misc_feature
   <222> (42946)..(42 946)
   <223> n can be a or t or g or c
<220>
   <221> misc_feature
   <222> (43373)..(43373)
   <223> n can be a or t or g or c
<220>
   <221> misc_feature
   <222> (43408)..(43408)
   <223> n can be a or t or g or c
<220>
   <221> misc_feature
   <222> (43409)..(43409)
   <223> n can be a or t or g or c
<220>
   <221> misc_feature
   <222> (43512)..(43512)
   <223> n can be a or t or g or c
<220>
   <221> misc_feature
   <222> (43657)..(43657)
   <223> n can be a or t or g or c
<220>
   <221> misc_feature
   <222> (43765)..(43765)
   <223> n can be a or t or g or c
<220>
   <221> misc_feature
   <222> (43777)..(43777)
   <223> n can be a or t or g or c
<220>
   <221> misc_feature
   <222> (43825)..(43825)
   <223> n can be a or t or g or c
<220>
   <221> misc_feature
   <222> (43905)..(43905)
   <223> n can be a or t or g or c
<220>
   <221> misc_feature
   <222> (43934)..(43934)
   <223> n can be a or t or g or c
<220>
   <221> misc_feature
   <222> (44380)..(44380)
   <223> n can be a or t or g or c
<220>
   <221> misc_feature
   <222> (44422)..(44422)
   <223> n can be a or t or g or c
<220>
   <221> misc_feature
   <222> (44559)..(44559)
   <223> n can be a or t or g or c
<220>
   <221> misc_feature
   <222> (44790)..(44790)
   <223> n can be a or t or g or c
<220>
   <221> misc_feature
   <222> (45124)..(45124)
   <223> n can be a or t or g or c
<220>
   <221> misc_feature
   <222> (45125)..(45125)
   <223> n can be a or t or g or c
<220>
   <221> misc_feature
   <222> (45155)..(45155)
   <223> n can be a or t or g or c
<220>
   <221> misc_feature
   <222> (45157)..(45157)
   <223> n can be a or t or g or c
<220>
   <221> misc_feature
   <222> (45158)..(45158)
   <223> n can be a or t or g or c
<220>
   <221> misc_feature
   <222> (45172)..(45172)
   <223> n can be a or t or g or c
<220>
   <221> misc_feature
   <222> (45468)..(45468)
   <223> n can be a or t or g or c
<220>
   <221> misc_feature
   <222> (45714)..(45714)
   <223> n can be a or t or g or c
<220>
   <221> misc_feature
   <222> (45987)..(45987)
   <223> n can be a or t or g or c
<220>
   <221> misc_feature
   <222> (46036)..(46036)
   <223> n can be a or t or g or c
<220>
   <221> misc_feature
   <222> (46042)..(46042)
   <223> n can be a or t or g or c
<220>
   <221> misc_feature
   <222> (46056)..(46056)
   <223> n can be a or t or g or c
<220>
   <221> misc_feature
   <222> (46090)..(46090)
   <223> n can be a or t or g or c
<220>
   <221> misc_feature
   <222> (46094)..(46094)
   <223> n can be a or t or g or c
<220>
   <221> misc_feature
   <222> (46110)..(46110)
   <223> n can be a or t or g or c
<220>
   <221> misc_feature
   <222> (46112)..(46112)
   <223> n can be a or t or g or c
<220>
   <221> misc_feature
   <222> (46114)..(46114)
   <223> n can be a or t or g or c
<220>
   <221> misc_feature
   <222> (46121)..(46121)
   <223> n can be a or t or g or c
<220>
   <221> misc_feature
   <222> (46134)..(46134)
   <223> n can be a or t or g or c
<220>
   <221> misc_feature
   <222> (46144)..(46144)
   <223> n can be a or t or g or c
<220>
   <221> misc_feature
   <222> (46146)..(46146)
   <223> n can be a or t or g or c
<220>
   <221> misc_feature
   <222> (46149)..(46149)
   <223> n can be a or t or g or c
<220>
   <221> misc_feature
   <222> (46176)..(46176)
   <223> n can be a or t or g or c
<220>
   <221> misc_feature
   <222> (46177)..(46177)
   <223> n can be a or t or g or c
<220>
   <221> misc_feature
   <222> (46200)..(46200)
   <223> n can be a or t or g or c
<220>
   <221> misc_feature
   <222> (46223)..(46223)
   <223> n can be a or t or g or c
<220>
   <221> misc_feature
   <222> (46447)..(46447)
   <223> n can be a or t or g or c
<220>
   <221> misc_feature
   <222> (46713)..(46713)
   <223> n can be a or t or g or c
<220>
   <221> misc_feature
   <222> (46886)..(46886)
   <223> n can be a or t or g or c
<220>
   <221> misc_feature
   <222> (46906)..(46906)
   <223> n can be a or t or g or c
<220>
   <221> misc_feature
   <222> (46987)..(46987)
   <223> n can be a or t or g or c
<220>
   <221> misc_feature
   <222> (47006)..(47006)
   <223> n can be a or t or g or c
<220>
   <221> misc_feature
   <222> (47406)..(47406)
   <223> n can be a or t or g or c
<220>
   <221> misc_feature
   <222> (47442)..(47442)
   <223> n can be a or t or g or c
<220>
   <221> misc_feature
   <222> (47729)..(47729)
   <223> n can be a or t or g or c
<220>
   <221> misc_feature
   <222> (47851)..(47851)
   <223> n can be a or t or g or c
<220>
   <221> misc_feature
   <222> (48512)..(48512)
   <223> n can be a or t or g or c
<220>
   <221> misc_feature
   <222> (48051)..(48051)
   <223> n can be a or t or g or c
<220>
   <221> misc_feature
   <222> (48512)..(48512)
   <223> n can be a or t or g or c
<220>
   <221> misc_feature
   <222> (50116)..(50116)
   <223> n can be a or t or g or c
<220>
   <221> misc_feature
   <222> (50789)..(50789)
   <223> n can be a or t or g or c
<220>
   <221> misc_feature
   <222> (50790)..(50790)
   <223> n can be a or t or g or c
<220>
   <221> misc_feature
   <222> (50791)..(50791)
   <223> n can be a or t or g or c
<220>
   <221> misc_feature
   <222> (50793)..(50793)
   <223> n can be a or t or g or c
<220>
   <221> misc_feature
   <222> (50794)..(50794)
   <223> n can be a or t or g or c
<220>
   <221> misc_feature
   <222> (50795)..(50795)
   <223> n can be a or t or g or c
<220>
   <221> misc_feature
   <222> (50796)**..**(50796)
   <223> n can be a or t or g or c
<220>
   <221> misc_feature
   <222> (50797)..(50797)
   <223> n can be a or t or g or c
<220>
   <221> misc_feature
   <222> (50799)..(50799)
   <223> n can be a or t or g or c
<220>
   <221> misc_feature
   <222> (50801)..(50801)
   <223> n can be a or t or g or c
<220>
   <221> misc_feature
   <222> (50802)..(50802)
   <223> n can be a or t or g or c
<220>
   <221> misc_feature
   <222> (50803)..(50803)
   <223> n can be a or t or g or c
<220>
   <221> misc_feature
   <222> (50805)..(50805)
   <223> n can be a or t or g or c
<220>
   <221> misc_feature
   <222> (50806)..(50806)
   <223> n can be a or t or g or c
<220>
   <221> misc_feature
   <222> (50807)..(50807)
   <223> n can be a or t or g or c
<220>
   <221> misc_feature
   <222> (50808)..(50808)
   <223> n can be a or t or g or c
<220>
   <221> misc_feature
   <222> (50809)..(50809)
   <223> n can be a or t or g or c
<220>
   <221> misc_feature
   <222> (50811)..(50811)
   <223> n can be a or t or g or c
<220>
   <221> misc_feature
   <222> (50812)..(50812)
   <223> n can be a or t or g or c
<220>
   <221> misc_feature
   <222> (50813)..(50813)
   <223> n can be a or t or g or c
<220>
   <221> misc_feature
   <222> (50814)..(50814)
   <223> n can be a or t or g or c
<220>
   <221> misc_feature
   <222> (50816)..(50816)
   <223> n can be a or t or g or c
<220>
   <221> misc_feature
   <222> (50817)..(50817)
   <223> n can be a or t or g or c
<220>
   <221> misc_feature
   <222> (50819)..(50819)
   <223> n can be a or t or g or c
<220>
   <221> misc_feature
   <222> (50820)..(50820)
   <223> n can be a or t or g or c
<220>
   <221> misc_feature
   <222> (50822)..(50822)
   <223> n can be a or t or g or c
<220>
   <221> misc_feature
   <222> (50824)..(50824)
   <223> n can be a or t or g or c
<220>
   <221> misc_feature
   <222> (50937)..(50937)
   <223> n can be a or t or g or c
<220>
   <221> misc_feature
   <222> (51301)..(51301)
   <223> n can be a or t or g or c
<220>
   <221> misc_feature
   <222> (51327)..(51327)
   <223> n can be a or t or g or c
<220>
   <221> misc_feature
   <222> (51896)..(51896)
   <223> n can be a or t or g or c
<220>
   <221> misc_feature
   <222> (52444)..(52444)
   <223> n can be a or t or g or c
<220>
   <221> misc_feature
   <222> (52666)..(52666)
   <223> n can be a or t or g or c
<220>
   <221> misc_feature
   <222> (53747)..(53747)
   <223> n can be a or t or g or c
<220>
   <221> misc_feature
   <222> (54085)..(54085)
   <223> n can be a or t or g or c
<220>
   <221> misc_feature
   <222> (54153)..(54153)
   <223> n can be a or t or g or c
<220>
   <221> misc_feature
   <222> (54251)..(54251)
   <223> n can be a or t or g or c
<220>
   <221> misc_feature
   <222> (54520)..(54520)
   <223> n can be a or t or g or c
<400> 42
   catgagcaga aggcagatga cagctgccat aatggaaaat agtatttcct cataaatttc     60
   cttgatctag gccagttctc aggctcagag cctattgatt taaggaaagg ccaagccttt    120
   ttatagaaga gccctccaat gctgccaaag gtatacaaga taattatttc cccaatcttt    180
   ccacatgaga gatctgtggc catttatcat agtaactctg gtcaggggga agaaaaagaa    240
   ccagactttc aaaggctatt gggtacagag tatgagctgg catcagtgct ggggggaaac     300
   actatcatgg tccccccgtg acagaggctc atgaaagcca ggtaaatgga ntactcttgc    360
   aaacagatcc agaagtctga agatccacac tgtactaatt tccttatccc taagtaaata    420
   actaggacag atgttntagc aactggcagt acactaacat ttactcccta ttttattaag    480
   agccattaag gtaggaaggg ccaaatgaaa gcccctgaca cttaccctca cctttcttac    540
   tgatttatta aatgagaagt aaaaccacat cctgggtggt attgcagaga ttaatttcaa    600
   catcaaatat tttcttctca atatccttca gtcaggagga tgaaaaactg tttgctttta    660
   tgtaggaata atagcagtac atattcactc tcttgcctct gggctatgtt agttctcctc    720
   tctgcacaac acagtatcta gcgactttga ttgtctcaat attccattga acatcatggt    780
   agtccactgt attgatgacc taatgctaat ttgacctagt aagcaagaag aagcaagaac    840
   tctagatgtc ctagtaaaga cacatgtatg ccaaaggata gcagataaaa atatgaagat    900
   tcaggacatt catattgttc aacttttttc ttacnttttt gttctagaat aagttaaatg     960
   gatgtaaaag tagacaaact agtaaaacga atcccttgaa ccactattca cagcccatat   1020
   tatttcatct atatccccac caacttcttc tatcccatat tattttgaaa caaatgggta   1080
   aagttatcag ggatcagtag tcatgccagg ttatcccctg taaagtaaag acaagttatt   1140
   gagctctgta tcctccaaca ctaaggcata gtgcttggtg gagtattcct ctgacccatc   1200
   tatttcgtgc ccctgaaggc tttcagtttc gggtgaaacc cagagcagga gagagctgta   1260
   cagtgtgtcc aactagagta caagcttctc taccatttgg ttcatattat ccagcagata   1320
   caatggggct agaggcatac atgatgaata aggctacagg gcagagtctc tgacatgccc   1380
   taataaaaga gtttcagcac agatctctaa agttctggag caaggccatg cagcagagag 1440
   atattcgact tttgaaaagc agctgtttgg ccccaataaa aaccaagagc ctagcaatgg 1500
   gacatcaagg gtctatgcaa ccagagatac ccattgtaag ccaggtattg tcagagtcta 1560
   ccaagtcgta agattaggca ggtttaagcc aggcatggtg gctcacacct gtaatcccgg 1620
   cactttggga ggaagctgag ggcagagaat tacttgagcc caggaggcaa catggcaaaa 1680
   ccccatctct aaaaaataaa gaaaaaaaaa ttagctgggt gtggnggtgt ttgcctgtag 1740
   tcccagcaac ctgggaggct gaagtagaag gattgcttga gcctagggag gttgaggctg 1800
   cagtgagctg tgattgtgcc actgcactcc agcctgggtg acagactgag aatctctctc 1860
   aaaaaaaaaa aaangcagac ttatcagcaa tccatcatat aataatagaa caggaatggt 1920
   aaattcagga tcaggactaa gcaggtcaat agggtgcatg taagtcaaca aaaggtggct 1980
   cagactccta tgacacctac ctcagctatc ggctatacca acccttctgt nttagctcag 2040
   acctacagcc ttagtaggta gttctctgta accaactgat ggaggggaaa atagccctta 2100
   ctcacagatg gattagcttt agacattgat actagttaaa aaagaaaaaa anaaaaagag 2160
   attgttcctg taacatagct ccatttaggg ggtagcccta aacaactttg aagggaaatt 2220
   cccccagtgg gctaagctgt aagcagtata cttgttcatt cattttgtat ggaggacaaa 2280
   acaattccag atagagnttt acatacactt gtgagcaatg gcaaatagtc gagtggtacc 2340
   tgttcagccc tcacttatgt gcaatctgga agtgcaagca gttgaccaat ggagacagaa 2400
   ctaggaaaca aatacctcct cattctatat ggctctttag aagcccccag tgggattaaa 2460
   cctcagtaac ccacagcaat ggggattgag acaaggctga ggtgaggaaa aagagcaagg 2520
   cacttgtctc tggtgcaaat ttaaggaggc accaagaaat tcagtaacca agattaatta 2580
   tattttaatg caatattttt taaaatagaa attaatgcaa atacccatga tgaacgcaat 2640
   atcaaaattt tcaataaaga caggctcagt attgctaatt ttccatttat tcttaggctc 2700
   cactagggct ccacacaaca ctgttactga tcttggtcat cgccaanaac catctcaatt 2760
   tcacagtcta tgttggcttt acctccctct ctgttcactc tcctccctag tccctcactt 2820
   ctgttttatg gtatcattac tcagaataaa cgaccttata tacgttcttg tctccaactt 2880
   tgctctatta tatggggtgg agtagggaga tgaaacccaa gctaagtaag ataatgacca 2940
   taatatatca ttcgaagaag gatacttgtg aaagtaaagg gaggctctat taataatttc 3000
   actggattaa taggcataaa tcagagctgt cctagcacat caggatgtat agtcttatta 3060
   caggtaggaa actgaggttc caagggggtg ggaagtcctc ctaagaggag gggtcagact 3120
   ggaagctaca caaaaagagg cagaaatgtc cctccgcctc ttaatcggag cctggggaaa 3180
   caaacacact cgtgcaaaat ctccaaagag gtgagctctt caccctgtca attttatctg 3240
   cctcgtgaac cccagggctc aggctccagg cccgggctag ctcacacaac agcagaaggg 3300
   ccttaggtct gggcactgag ccaaaaaaag ggaggctgtg gtccctgctt tggttaccct 3360
   gccgggtctg agcgccaaat cggatctcct acttctctct ccctccctcc ctctgttcct 3420
   ggactggggg caggcccttc tgctaacaga aagccttcca gtcggagaaa gctctgncag 3480
   cctgccgcga taaagggccc tgggggcgtg aggagagcaa ggggctcaaa atcanaaccc 3540
   agcgtgacga cagtgcctcc atgactgtgc tcccgcaatg cgcaggctcc atgtcctggc 3600
   gcccgcccgc ccatggaccc ggcgggggct tccaggctgg gctcagccat tacgccggcg 3660
   tgcgggggag gaaactcgcc tcccgggcac tcggttgtct cctgcccccg cccctccctc 3720
   cgatccgggc ccatctctga cgtagtgtga ccttgctcat cccttccagg ctgtgggcct 3780
   gttttccctg tgcaagatga gggtcctggc tgtcctgagg acgctgtccg ggcgccgcca 3840
   ggggtgaccg aattcagctc tgctaggact gttgggaaat gagctccctg tcggcgtgtg 3900
   ccagccgcct gcgcgaggcg ccacaggaga gggcgcgctc ctgtcgctct gccgccccca 3960
   gaagtttccc gggaaccgac tccactgact cgcccctccg cgccccacgc gtggcagcct 4020
   aagctcagcc tccanattgg aggagaccgc ggagggaacc ctgctggggt ctgggcccgg 4080
   ggcnacncgg cccgagcaga tcgagggccg acccctccga gaactcgctc tctggcctcg 4140
   gctcctccct tgcgcccgcc ctcccacgtg gggcccaggt ctgggaatca gcgctcgggg 4200
   gtggctgggg acaaccgaga acgagcttct tccccggcac gcgggcggaa tggctgagcc 4260
   cagcctggaa gcccccgtca ggtccttggg ggcgggcggg cgcgcgaagc acagggcgga 4320
   nacagccggg agcccagcct cccgggctgg gccgccctcc ccttccccgc gccnggccgg 4380
   ggatgggggt gtgntcccaa gtgtacagtg gcatcaagct cagcgcganc tcccgggaac 4440
   gctccaacgc cttcagcctg tttcccagga acggtccccn gnttcgcgcc ccaatttcta 4500
   acagcctgcc tgtcccccgg gaacgttcta acatccttgg ggagcgcccc agctacaaga 4560
   cactgtcctg agaacgctgt catcacccgt agttgcaagt ttcggagcgg cagtgggaag 4620
   catgcgggac tacgacgagg tgatcgcctt cctgggcgag tgggggccct tccagcgcct 4680
   catcttcttc ctgctcagcg ccancatcat ccccaatggc ttcaatggta tgtcagtcgt 4740
   gttcctggcg gggaccccgg agcaccgctg tcgagtgccg gacgccgcga acctgagcnn 4800
   nnncnngcng aacaacagtg tcccgctgcg gctgcgggac ggccgcgagg tgccccacag 4860
   ctgcagccgc taccggctcg ccaccatcgc caacttctcg gcgctcgggc tggagccggg 4920
   gcgcgacgtg gacctggggc agctggagca ggagagctgc ctggatggct gggagttcag 4980
   ccaggacgtc tacctgtcca ccgtcgtgac cgaggtgggt gccaggccga gaccgttgac 5040
   ccgggagtgc ctgaccctcc ctctgcgtca gccccccttg agactccctg cgcagtgccc 5100
   gggtcagcgc tcccctcccc ctcaaacctg ctgttcatac ttccagcagc gggtgtgcac 5160
   cccnagaaag aataggactc acgcgaggcg cattnctggg tcgtttntgt ccaataagga 5220
   ggtgatggag aaaggagttt gtcaccaact gtcttttcat ttccgatttt cacaacaaat 5280
   gaagtttcct aagaacctag atgttgcgtt gggggaagcg cagcccaggc tacccagacg 5340
   cactgcctga gtcaccttcc tgccaggcat gggaggaggg gtgtgaggga ccttgttact 5400
   gggcagggcg gggccaaatc ttgtctgttc cctggccaca gacttctgga ggcatcagga 5460
   ggtgtactgc cgtaccatat acttgtgagg tttggggtcc tttcctgagg ccccagtttc 5520
   tctaagacag cagatggcct cctgaggctc attgggtgca aggaagcaca gaaggcgaaa 5580
   ccccagctgg aagccctggc cctcagggac ctctctgagc acagggtgag ttggcaggca 5640
   ggggctgtag cccctcagca ccctgcgtgg ttagtggcag gttttctaag ttccaggaat 5700
   gaggatgcac acacacttct cccgcttttg ctgcttcaca tctggtaagg acctgggtca 5760
   ggatacacct ttcttctgag gagagcatcc tcatggagct gttgcctgct attggtcagg 5820
   caggtaggat ttccctgctg ctagtccctc ctctgcctca tgctcctccc aagaaaaacc 5880
   tgagtggcct ctgctagtcc cttatccctt tccctcagca cggtatagcg gtcatcagac 5940
   attgtgtctg gccttgtgct gagcactaag gtgtggacat ctggtgtgtg gagcctaccc 6000
   tcagatgccc atggggcaca ctggctgtgt tactgagaag gctaacatta accctggtta 6060
   gcaatagggt tctggaaccc aaattcaagt cctgccttgc ttgctatgtg acctgggata 6120
   aataaatcac cctctctgtg ggacaatagg tggaggaaat actccaagaa ggggtgatat 6180
   gattttagct gattctgaaa atgtgagcaa gacctgtcta ggcaagagta tgggaaattc 6240
   aggtaatgga attctcatat atcggagtgg ctggaactca agggatgaca gatatgacaa 6300
   atgagactag aaatggccac aagggctagt tcatagtggc cttctatgta tactgaggag 6360
   ctgactttat tcggaggaca gtgagaaaat agtaagcatt ctaaggccag gttcgtgctt 6420
   ttggaaagag ctcactctag ctgctatgat aaacaagaag accagtgagg aagttttgaa 6480
   actctcctct gcaagagaat ggtggccagc caggcgtggt ggttgtcgaa tctgtggcac 6540
   ctgtgggaag tgggctcagc ccagggactg cctttggatc ccccagcatt ggcaccatac 6600
   ctaccctggg ccctaaggtg gccatgcttc tctggatttg cttccctagc aggggctcta 6660
   gtgcttgncc actccctgcc cacagcatgg cctgggagca gctgagactg ggggccagct 6720
   catcccgcgt cgattcctgg aagtgttatc agtgcctgtt atggaggctg gacccatgag 6780
   tggcagcctt ccctggcagc tgggctgacc tgtctgcttt tccattgctc gctggttttg 6840
   ttcactgtag ggcgtgaggg gtgagtagct gctggcctcc aagtccatag ctactcatgt 6900
   tgtacgctgt tcacagggac ctctaaggat gtacatcatc acacattcac acacatgcac 6960
   caggatttgc tttttttggc agcttttccc ttcctggctt cctttttgag tggtggaagt 7020
   aaaataaaaa gcaactaggg gctgggcaca gtggctcacg cctgtaatcc cagtactttg 7080
   agaggctgag gcgggcagat tacttgaggt caggagtttg agaccagcct ggccaacatg 7140
   gtgaaacccc gtctctacta aaaatacaaa aattagctgg gagtggtggt gcacccctgt 7200
   agtcccagct actcgggagg ctaaggcagg agaatcactt caacctggga ggcggaggtt 7260
   gcagtgagct gagatcacac cacagcactc cagcctgggt gacagagcca gactgtgtct 7320
   caaaacaaac aaacaaacaa caacaacaac aaaaaactgg gctctggtgg ttgggaggag 7380
   gagggaagga agcaagtacc agggtaagca ggatggatgg atggctctcc cccagagggg 7440
   cggcagcaca cagagttntg gagtcagact cagtagaggg ccagttttga ctccactgcc 7500
   aaccacctgg ctgactccag gcaggttaca tcactgatga aagcctcagt ttccttgtct 7560
   ataaattggg ggtacaagcg atgaaaagag gctcaacatc actaatcact agggaaatgc 7620
   aaatgaaaat cataaggagg taccactttg tacccttaaa aatagntact acaaaaagaa 7680
   aaaaaanaca cccagaaatc cactttggga ggccgaggca ggaggattgn tagggggcag 7740
   gagttcaaga cagcactggg caacatagta agaccccatc tctacaaaaa ataaataaaa 7800
   aattagccag gcatgagggc atgtgactgt agttccagct tctcaggagg ctggggcagg 7860
   aggatcactt gagcccaaga gtttgaggtt gcagtgagct atgaccatat ctctgcactc 7920
   cagcctgggt gacagggcaa gaccccacct ctaaaaaagt gttgttgttg tttttaaaca 7980
   cagaaaataa caagtgttag tgaggatgta gagaaattga aacccttgtt cattcctagt 8040
   gggagtgtaa aatggtgcag ccactatgga aaataatgtg gtggtcccta aaaaaattaa 8100
   aaatagaatc accatatgat ccagcaatac taattctgga tatatgtcta aaataattga 8160
   aacagggttt caaagagata tttgtacacc atgtctatgg tagcattatt cacaatagtc 8220
   aaaaggtgaa agcaacccaa gtgtccttcc acagacaaat ggataaacat aatgtggtat 8280
   atgcgtacaa tggacgatta ttcagcttta aaaaggaagg acatgctaca acgtggataa 8340
   accttgagga gactacgctt agtgaaataa gccagtcaca agacaaatta ttttctgatt 8400
   tcactacagg agtagtcaca ctcacagaag cagaaagtaa atggtggttg ccagggactg 8460
   gaagaaggaa ggagtaggga gtcgttgttt catgggtata gagtttcagt tttgtaagat 8520
   gaaagtagtg ttggagattg attgcacaac agtgtgaacg cttctgaact gtacacttaa 8580
   aaatggctaa gatggtaaac tgtatattat gtgtacttta tctcagtttt caaaatgggg 8640
   ataataagag aagccncctt atctgagttg ttgggaggac tcagtgagat aatgccccat 8700
   gccccatccg gtggtggcca gcacagagtg tggcaccagc aaagagtgtg gcaccagcaa 8760
   atgggcttcc taggtggtgg tgaggagtgg taaaggcaga agctcagcac cttctttgcc 8820
   ttctccactg gctggggaca gtgggcatgg tgggagctgt tctaaagtcc aggttgtggc 8880
   ctgtgtaata ggtgatggga cttgctggag gctgagcccc tctccccaca gtggcagttc 8940
   caagcctcag agaatagaac acaagtccaa acccatactg acttggccag tgccagtgta 9000
   cagggccaag cccagggccc ctgagaaggt tctctgcatc ccagttggca caacacaggt 9060
   agtgggactg tcctcagatt gtgtctttgc cttatactgc cgtgccagga tctcccaatg 9120
   ggcatatctt tggggacaga caagtctgcc tgaactgccc cccaacacct cttcacagga 9180
   ggacagggtt agccaggaac caaggatggc atgctgtgct gtagaaatgc ctttcatagt 9240
   atgccaggag gtagcaataa ggtgtctgag ttggggtttg cgtgagtgca ttcgtctgtg 9300
   tgtgtacatg tgtataaggt agtttccttc ttcaggggtt gattgtgagg tccaagtttt 9360
   tcaacctgtg cagttactaa gagtcaaatt atttttcgtt ttnttttatt ttattttatt 9420
   ttattttatt tttttgagat ggagtttctt tcttcgttct tgttgcccag ggtggaggag 9480
   tgcaatggtg ccatctcggc tcactgcaac ctctgcctcc ttggttcaag cgattctcct 9540
   gcctcagcct cccgagtagc tgggattaca ggggcctgct accatgcccg gctagttntt 9600
   tgtttgtttg tttgtttgtt ttttgtattt ttagtagaga cagggtttca ccatgttggc 9660
   caggctggtc tcgaactcct gacctaaggt gatccacctg cctcggcctc ccaaagtgtt 9720
   gggattacag gcgtgagcca ccgcgcctgg cgattatttt ttaactttat tttgaaataa 9780
   cttttgattt agagagaagt ttgaaaatag tacagagatt tcacatatac ccttcatcta 9840
   gctttctctg atgttaacaa tttaaatacg cgttatgcat ttatccaaac caggaaatta 9900
   acattggtta caatagtaat aactgaacta cagactttgt ttggatttca ccagtttttc 9960
   tactactgcc ctttttctat cgtaggatcc catccaaaat cccatgttgt atttagttgt 10020
   catgtctcag tctccaatct gtgattgttc cttagtcttt ccttatcttt catgatcttg 10080
   acatttttat gagtattttg atgaatgctc ctcagtttgg gttggcatga tttttctcac 10140
   aattagattg cgttatgcac tttttggtaa tgatacctta gaagtgatgc attctctggg 10200
   catcatacca gtgggtacat gatgctgatg tgtcttatta ctggngatgt taaccttgat 10260
   catttgtcaa gttggtgtct gtggaatttc tctgctgtaa aggtactatt ttttcctctg 10320
   tcactaataa atatcttaag ggaggttctt cgagattata caaaatcctg tgtctcctca 10380
   aatttttatt cactgatttt agcattcatc actaggtctt gactgaaaca attattactg 10440
   cagtttgcct agtgatgatt ttgcattttc tgattccttc aacatttatt aattggaatt 10500
   cttctgagaa gaagagcttc ccctccacat ctatttatta atgtatgcaa ttgttaactt 10560
   atatcagtac aggttcatac atacttattt tatttcatca gttataaact aatgctatcc 10620
   atctttattt tgttgctcca gttattctag ctcaaaccaa gttatttttc actttaagga 10680
   tagagaatgt agatatccag ggtaagggcc tttatctgtc tctgcagtca gggctagagg 10740
   agtctgtgtt tacccctgcg ggaggagtgc cagtcacagc tcgttgctaa gttttccatc 10800
   aggcagcagt tgagcangaa cgcaagcang gactggtggg acctgggttg ggactgaagc 10860
   ttctgttggg gaaggaagtg ccccaagcaa cccanagtgt ctgatgctgg ggtcctcatc 10920
   cctgagacct actgcagggg tcagatctca gtctccatgg gcatggccct tcatatatag 10980
   ccctcagggc caagacctca nagagaagag gccctgcgtg tgaggaaacc cggcagcctc 11040
   ctgccctgng gctaaaggag cangaanggc aacaaatgct ctgctctgga gagggcctaa 11100
   gttcatctgc agggccacag ggcaagcagc agaggcaatc ctggctcgtg gccaggggct 11160
   aagcatgggc tggcctttgc ctcaagcatg tnctcttctc ccatggagca tcattagggt 11220
   cttgaccttc ccatcagtcc atgggctggg aaaactgagg cagtcacctc taagctggat 11280
   accacttctg ccangtcttg tgctccagag gccctggggg aaccttgtgt gttaangacc 11340
   agccttcttt gcaggccacc agtgatctaa gacagggctg ctgccacacc gtagatcctc 11400
   ctgtcctctt ctgcagctgg gctgcctcct ctaggaaccc tagtggagct gctggtagcc 11460
   cactggtagt ggagttgatg gagcctccag caaagggcct ggacctctgg tggcactagc 11520
   cttgtgcttt cctgggaact ggggtcaggg cctgaacagc agtaggactt actgattccc 11580
   cacccataac ccagaggcga gccaggttat gtggcgaagg ataaggcctc ttcccctgct 11640
   aacngggaac ttctgggagg cactgagagc agcaagcaag ggaataatga aacagactaa 11700
   ccctcacatg cggctgccac tatgtgggac ttcacatagc ggctctcaag gatgcacctc 11760
   ccctggggtc tgggtaggcc ccgacaaaag gggaaaacct tccgtgatag gcaggtttgt 11820
   ggagtttcta tcaggcctgc aggctgccct gggacattgc tcacgggtgt ggagactgtc 11880
   aagatagcct gaatattaac caagttgatc atccaagtcc atactcagac ctgccctctg 11940
   gcagattcca aaggggataa ggtgtggggg cctctggctc cagccagccc ggcgagactt 12000
   ctatttccag tcaatgcttc agaaagtaga tcttgccaga gagcacatag aggagactca 12060
   ggggcttccc cttgctctgg cctgaatgtg cttccctgtt tgtttatcag ctgcctcacc 12120
   ccaaactgtt tgcctctctg ctgtgttcct ctgacaacag ccaccaggat ctcagaatat 12180
   gcttttgttc agaggctgct tcacctgcac ctcctgccaa gtttctggat aaacagactt 12240
   tgtccatccc tcttggtggg cagcagaact ctcagacaga gtccaaggtg gcattttgac 12300
   aaaaacaagg ctttgacagg actggtcagg gggagctgca ggtaccttgg gcctctagaa 12360
   ggactgtaat tcctccctta tggcaggctg cattcaattt aggtgtaaga ctaatacagg 12420
   tgtgccttgt tttntaaagc tgtgttgtgg ttttatgcat gtctctggag tccaaaagaa 12480
   aggcaaggat ccccatttgg aatgaagaag gtgtggagag aatggacgtt ntggggaaag 12540
   gctggaatga cccacatgct ctagcaagaa gcacagacag tccaaggctg gtggctcctg 12600
   ctcagcattt gcaagactgg agggcaggtn caggcggcaa cttccagagc ttcccctcag 12660
   tgcttggtga ctggcacaga cacgatgcca tttgctcatg ttcccctaat acagactggt 12720
   agagctgtgg tggatccatt gtcaggatgc aaggggcaca gccaggtccc tgagtgaccc 12780
   ttctccttcc cacagnccct ccactcccat ccctatcttc ccactcacac tcactggttc 12840
   ttctgctttg gtttagcctc caatgatcac agaactcatc agtgaggctt ttccatctgt 12900
   ggtagctttt ctaatataaa gaaagaaaga tactgagaac attttaaggc ttagcagcct 12960
   tctggcttag ttgtttgtaa atgaaaatat gagtagaaga attaaacagc caaaaagata 13020
   acccacaaaa tgggagaaaa tattgacaaa tcatatatct gataaaggat ttgtatctag 13080
   aatataaaaa taactctgat aactcagcaa taaaaagaca acntgattta aaaatggata 13140
   aaatcatttg cccattttgg agaatagatg ttttgccaaa gaaatacaaa tcaattagca 13200
   catgaaaaga ttctgagact tcgaagttag cagtgtgagg agcgccagcg ccatagacca 13260
   gctcctcagt gaaacagcca taactggtga aaatacacac acacacacac acatttagag 13320
   tctctggaag ttgtcctaag ggcatatatt tattcaagaa agtcnactat accttggcaa 13380
   gaacagtgaa gtctatgata tttgagtcat aactccttca gccctcccct tccccttcct  13440
   cccagcacaa gaaaaatgga agttccactc taggtgggta tggccaagaa aatactgttc 13500
   tctctcccat cagcttccaa ggaagggcta cagcagccca ccaagaggga caaccaatgc 13560
   ccatccccca cccatccctg tattctctta gctccaaacc acagaaggta aaatcctcgt 13620
   gagtgcaacc aggggatcat aggttcccat cctccaccca ccgcctattc atatagtaga 13680
   gactctactc caggtgccac aagttgagaa tattagagcc ttgattgccc ttatcccagc 13740
   tcattcatac ggcagagatt ccacaccaag ctcccacttc cactcagtgt ctgactcata 13800
   aagtaggggt gtcattctga gataaatggg ccgctttccc tgcctacctc tctacaacag 13860
   gagctcagat attttgccca gcggggagag ataatccaaa agaacagagt tctaaagctg 13920
   tccccacagg aacgaacttt atttgaaata gagtgtgtga aaggacagcc taagattgct 13980
   ctcaaganca ntggagaatt tagtggtaag caactaaaag gattctggta actccattag 14040
   agcaacanat gaacccataa tgcagctagt ttaccaggaa aaaacagaaa acaaacaaac 14100
   aaacaaaaca agaaagagac agctaagagc agcccntctg gagtcaaaac aaaccttata 14160
   gatttgcctc aaaaactacc actgcaaaga ggcttgacgt caactggatt agactgtggg 14220
   acaatatatg cccccagggc attattgaaa tgaatgaggc aactggctag caattagtga 14280
   ggctaacagc tgggtgtgat accagcagac acagacagct taatagaaag atcagactgg 14340
   cgtggnggct catgcctgta atcctagcac tttgggaggc caaggcgggt ggatcacctg 14400
   aggccgggag tttgagacca gcctggctaa tgtggggaaa ccccatctct actaaaaata 14460
   caaaaattag ctgggcatgg tggcaggtcc ctgtaatccc agctactcag gaggccgagg 14520
   caggagaatt gctggaaccc aggaggtgga ggttgcagtg agccgagatc atgccactgt 14580
   actctagctt gggcaacaga gtgagacttc atctcggaaa agaagaagaa gaaagatcag 14640
   ggaaatgggg gtggaagaat gtgacagtac ctaatttcta caatatatta tctaaaatgt 14700
   ccagttttta accagaaatt acgagatatg cagagaaaca gaaaaatatg acctatacat 14760
   ggggggaaaa cgtgggcaac agtaactgcc tgagagggtc cacatgtcag attttaacag 14820
   actttaaggc ataaggcagc cnttataaat atgttcaaag aatgaaagga agtcatgctt 14880
   ttaaaagtnt gatgacaata ntgcatcaaa tagagaatag caataaagag agaaaaatta 14940
   catttatatt tttaaaagct gagccagaca tgatggcatg tgcctatagt cccagttact 15000
   caggaggctg aggtaggagg attgcttaag cccaggagtt caaccccagc ctgggcaaca 15060
   gaaagagacc ttgtctctta aacaaacaaa acaactgaat agaacttctg gagtaaaant 15120
   atacaataat tgaaatttaa aagtaactca agggccttaa cagaagattt gaacaggcaa 15180
   aagaaagaat cagcaaactt gaagatagag gattggaaat tatgcaatcc aaagaacaga 15240
   gaggaaaaag ggaataaaga aaatgtatag agtctcagag aaatggggaa caccattaaa 15300
   catagcaata tatgcataat agaattacta ggagaggaga gagagagaaa agggcagaaa 15360
   aaatgctcaa agaaataatg actgaaaact tcccaaattt ggtggaaaac attaatctac 15420
   acctttcaga agctcaacaa actccaaata gaatgatcac aaagagatcc acaccctgat 15480
   atatcatggt aaaactgctt aaataaaaaa agagacaaaa acgaagggaa aattttgaaa 15540
   gcagcaaata tatgactagg tacatacaag agaactccaa taagatcaac gtttgacttc 15600
   tcattagaaa ccataaagga cagaaggcag tggagtggca tattctaagt gctgaaggaa 15660
   aaaagaaact gtcaaccaag aatcatgcat ccaacaaaac tgtctttcaa aactgagata 15720
   aagaaattca aaaatacaca aaacttgaga gaattgtttc tagcagtgat gtcttacaag 15780
   aaatattaaa ggaggcactt cagaaataaa tttanccaaa aatttgcaaa atgtatactt 15840
   taaaaactat aaaatgtcat tgaaagaaat tccagaagnc cttaacaagt ggaaaagctt 15900
   cccatgttct gttcatggag cagaagactt attgttagta tggcagtgct ctccaaattg 15960
   atcaatagat tcaatgcaaa agatgttcaa cattagttat tagagaaatg caatcaaaac 16020
   cacaatgaga tacctcttta cacccactac aatgactaaa attaaagaca cacaataaca 16080
   agtactggta ggaatgtgga gaaattgaaa ccctcattca ttgctgttgg gattgtaaaa 16140
   tggtgtagcc actttggaaa gcagattagg agttctgcaa aaggttgagt atatacttac 16200
   cgtatgaccc aacaatttca ctactatgta tataccccaa agaaatgaaa acatgtacac 16260
   acaaaatgtt gtacatgaat gttcacagca gcattattca tagtagccaa aaagtggaaa 16320
   caacccaact acttatcaac tgatgaacag atcattgaaa acgtggtaga cccaaaccgt 16380
   gaaatatata tatatatata tatatatata tatatatagt tgttgttgtt gttgttgttg 16440
   agatggagtc tcactttgtt gcccaggctg gagtgcagtg gtgtgatctc agctcactgc 16500
   aacctccacc tccaggctca agcaactctc ctgcctcagt ctcccaagta gctgggatta 16560
   caggcacgtg ccaccgtgcc cagctaattt ttgcattttt agtagagaca gggtttcacc 16620
   atgttaccca ggctggtctt gaactcctga cctcaggtga cccacccgcc tcggcctccc 16680
   aaagtgctgg gattacaggt gtgacccacc gcacccagcc caaaccatgg catattatta 16740
   aaccataaaa agtaatgaag tgctganaca ctgcacaatg acatggatgg acttagaaaa 16800
   ggagctaagt ataagaagcc agtcataana cnctatatat tgtatgattc catttatatg 16860
   aaatgtccag aataggcaaa ttcatagaga cagaaagtag attagtggtt tccaggggct 16920
   agaggaagta gngaatgagg agtgactgct aacaggtatg ggttttcttt naagggtgat 16980
   gnnnantcta aaatcagatc attgctgatg gttgctgaag tctgtgaata tactaaaaat 17040
   gattgatttg tatactttaa aagagtaaat gttatggtgt ataaatcata tctcaataaa 17100
   gttgttacaa aaaagtaatt aactagccat gtttgttttt gtttttcttt gttttgtttt 17160
   ttcatattgt caaggaattc ccagctttaa gtcacaatca agtgacaaag atagaactgg 17220
   ggtggatgta tagtccatta ttaactacta cttcttactt ttctctagaa taaaggataa 17280
   ctatgagagg atactataga aatcaatctt ttattcttaa aaggaccatt gctaattttt 17340
   cactcacagt catctctggg attgaattcc atttatcaaa tcccttttat aagagctttt 17400
   ctagctttta gtaatggact ggaataattt ttatttctta gaatttcttt ctcttagtca 17460
   gatttaagtg taacctgctt tgcttattcg tccatgtgtt ccaagtcatg attttgctca 17520
   ggacaaataa aatgctctac tagtatgagc ctggtagaaa gggaactacc atttaatgag 17580
   gcctgtaatg tgtcagangc tccactgata tttcccatcc aatgcacaca acaaacctag 17640
   gagcaggtat tantgtcatc tttttgaagg tgaagaagca ggctaataga ggtgaatgga 17700
   ttgcccaaca tcacacagtc caaagttgca gaccccgaga aagcctgtgt tctttgctga 17760
   acaccaggct accttccagc gttctcagtt ccgtgcaggt cagacggggt tcacttatct 17820
   ttatggtcct ttatagctct ggtaaaaact aaaactgcca gactttggct acttggagag 17880
   ttagttcaag gctgagggtg attttccaag tgcaatgcgt gatactcctc agcccaagaa 17940
   ccacattttc caggctgatt ttctatgact cctaattaat tgttcaatat tcattcaaca 18000
   atttgttaag cacccactgt gtgcctagca ctgtgcaaac actagtcatc taaaatatga 18060
   ttatacggat tattcaggtt ttagaactct gtccttctgc tggagnatgg tgaggagggg 18120
   gtgcctcttt cctctctagc caaagagcaa gggtgaagtg gcccagaaca gcgcccaccc 18180
   cacccagggn tgctggcctc atgcatgcac atgtgcacat ctgtcctgta aagtatgctt 18240
   gnccttgagc tttgtgaggg cagggnccat gtctttnttg tgccctacac anagcctggc 18300
   ctggagcagg taggtgctna atgtcagttg aatcaatgat taagcaantt tttttttttt 18360
   tttttttttt ttttttttna ccaattcact aggagaactt gggctggttg ctttattgct 18420
   ctgtgccttc atttcctcat ttgtaaaaca aggataagag tacccatctc acatgccaac 18480
   cctgagatcc cctgagataa tacacggcac tcagtgagca ctccacacac tgttactgtc 18540
   tttcccatta ctccctttac tatagggagc catgggaagc ggcacatcct tctatgccaa 18600
   gagtgacctc ctatccccta ttaccttgtc acctggattt ttgtttctta aattagagct 18660
   gaaggccatg cggcccagct aaatggagct cactctgctc tgttgtgaga accttggtct 18720
   gggaccctgc tgatgggaca gacatagtaa ccccgggagg ccccatcaga ggggcaggga 18780
   gaggggaggt aagctatgtt tcagaaatgt taggatttga cttgaaagca cacccccaaa 18840
   gcaatgccag ccagctcctt accaggacat ctgccattag cctctgagct ggtcatgtgt 18900
   atgtatgtgt gtgtgcatgt atgtacaaac atgtgcaggg acccacaggg acaggtctgt 18960
   cccatcaagg aaaacactgt ctcccactgg gcccnccatg tcactgttaa ccacagggag 19020
   gttttgtggc cctggctagc ggagacccaa gacatatccc tcaaacctga agggcccagg 19080
   ggcaggcagg nccggtttcc taagaaaagc cntttctaaa ggacccctct taggactggc 19140
   agctgattta ctggactcta gcccttcgct ccaggtagaa cttcatctgc caatcctgtg 19200
   tcctaatacc aggcccctca cagctttcca aaaagacact tgcggcacca taactcccca 19260
   aaggagagcc cagccctaga atcaaccatg ggcagccatg cagtctagct gccttcccct 19320
   gctggttgta agcagggtct ccaaaggcag atggcacccc tcatgggctt ggggcaaacg 19380
   gccactgcgt gtgcatggct cttcctgtgc tgcatgatgc tgacagggct tcttgggccc 19440
   ctcttcaggg agggaatggg accgaaagcc atcgatgcag agcctacagg aaggagaatg 19500
   atgggagatg gccctcacct cacccccctt cccagtgtcc ccattcagaa ctcctgcatc 19560
   tgcttcattt cctggaggcc aggtttgatc ccctgccagg gccagcttca tgggcatgca 19620
   actgtgcagt cacacagagc gccacacttg gtttcaatgc tttgccattg ctgtattgaa 19680
   attcttaata atttttgacc aaggagttct gcattttcag tttgccctgg accttgcaaa 19740
   ttaagtagcc agttcccagt cctacccttt gctcatgcaa tgcctggctc cacatctaac 19800
   agccttccnt tcctttctat ctctaaatcc tatctatcct gccctaaggc aaagtccaga 19860
   cagaaaaata tttgacagac tacaaaggag agcacattat atttgaaaga gcttttctgc 19920
   tcaactgcag atggaatgat gatggttggc atgttaatgc ctctaccaat cttgagttct 19980
   tcttggctca gtctgaggct aaaggctgtg atggaggcct tctactccta gtcctgggta 20040
   attgtctcct ggtatcacat tccttgaaga antgtagggc atcatattta ataagctcat 20100
   tttgtcctgc cttagggagt ataggtccaa agcaggacac tggtacaaga ttgggttctg 20160
   aggcccaagt aggtatctat catgctcatt tacttgctat gacttcagac aaatcccatt 20220
   ctcacagtct cagtttaatc ttctgcaaaa tgggtcacac cacatcatga ggctttgggc 20280
   ctcaattgct catctctcta agtcagaagc agaacccaag cttttatctg aagctttgga 20340
   caattatttt tggagggaga tggccatgaa tgcaggcaca cgtcctgttt accacttccg 20400
   gtctatggac tggcagaaca cattaatctg gccatacagt tttagaggat tcatttccct 20460
   ttctcctgaa gcccatgcaa gagccccagg cgaggcaatc ctgctctggg tacagtctga 20520
   gatttatcct tggattgtgc ctgagcagca gagcgctaac ttcctggccc gagttgtttt 20580
   tcttctgcag agctgtgttg ggcnttgcct gtggccagca tttgagcact gtgacaggcg 20640
   gcctcatgaa tcttgctatt gtctctagga caggactatt ctcgaggaca ttatcctcag 20700
   catcacagag tggctccttt gtggaagaag tacataggag acacttcttt cngatctcag 20760
   tattcagaac aactgtgctt ctgcgaataa gcagactact tcggatactg taacccttct 20820
   gagagctgag ctgctgggat acgcagagcc agttttggag ccccgtctgg caancaggca 20880
   ggcccgtgga caggatagtc tagggtcagt cttaccccag gcttggtttt tgcaacttcc 20940
   ctcctcttta ttttccattt tccccaattc ccttagcttt ttgttttaca tttttctctt 21000
   gtgtgtgtct ttataaacac ctctgtctgc tgcagaatna ggtgtgatat aaatatgtga 21060
   gtttgtgctt aaatccacca cactttcaag ggagttgtga ggattcagaa ggaattgttt 21120
   ggcgaatgca gtaaaagcag ccccatccaa gatggagcta gggctgggtg ggaggcaggg 21180
   gtctttcctc cagactggat catctcgcca cagacagcag cccccaaaac ccaagcccaa 21240
   agtgctcatt ggcatctgtt tgtttattta tttgttttta ataaatgaaa ggtcactaaa 21300
   cacactttga aatttttgtc acctcccttg agatgttgct taattgactt aagtcaaact 21360
   gaaactctgg ctgaaactca ttttgtagat gcactggcag ggacttgtta gggcagcagc 21420
   atcccacaga ccctagggaa tgggagggac cacacttctg gaaagactgg atgggaagga 21480
   gggtgtaggg atgtaggagg ggagctaggg agccctggag aggagtaagt aggccccaga 21540
   acgatggggc cncgaatcag gccacaagct tggcctaggc gtgagtggac cagggaccac 21600
   atgatttctc agaggggcct gctcagaaaa taaagtcaca gagctcctgg aaccnnntgc 21660
   acttttcaac aagggnaaag aaattttacg cctggaccaa gtggccatgc cctctgccct 21720
   gccntttggg nttttcaaac acctnccctc cgcactaggc tggcttctga agatgcagtg 21780
   ccacctttac tttggactgc ctcacagctg aggggtcacc agtcacagcc atggccttgg 21840
   ctggcttctt gctatgtaat gcgagcggag taggagcatc accctccctt tccaaagagg 21900
   atcctgagtt tcagggaggn cagtgacctg ccccaggtta ctctccccag tcccgncaca 21960
   ggccagtcaa acccagggct gcaaggccta tgccctttgt acaaaggcaa tatcctggcc 22020
   caggggagga gagtctgccc gccagccgtg ctaatattcc ctcagagctg ggctggggtt 22080
   gaggtctgcc tcagggttgn gntcacgctt catgctgtct tccttggcag tggaatctgg 22140
   tgtgtgagga caactggaan gtgcccctca ccacctccct gttnttcgta ggcgtgctcc 22200
   tcggctcntt cgtgtccggg cagctgtcag acaggtaagc acatgggagg ggaggaaggt 22260
   gggatgntgc cccttgtcaa tcactgccta tcttggggct ggactgaatt cagggtattt 22320
   ctacccnnnn naagggagga ccctcagncc actgattcag tgataggagg agccccgatg 22380
   tctcntattc accccgccct cagtcccctt cacctggaca canttttgtt ccattctccc 22440
   agcctcatgc tcccagggct ttggccagtg gtggggaggg gccactgacc ccactggaaa 22500
   gtggggtcct cataccacag actccttgac aaaacatgac gctgccatca ccactgccct 22560
   ccctgctcca ccaccacctc aggggtaggg gtggatggca caggatctgt ctgcaaacag 22620
   ccagcagcca gacagtgagc cccaactgtg cacacactgc tgctgtggct tggaccctgg 22680
   gcnaggcact ggggccacag agctgatgct ggctctgacc ctcatagagc ttacaggcct 22740
   gaggaggcag catcacagca ccaggagaca gtattccaca ggcccctgta tgtgctgggt 22800
   gaccagtgca ggaaggtggg ggtactaaat ggcaatgctg gggaaagcct cccagcccaa 22860
   gccaacagcc agagacatga gtttggaaaa tggggcggga aaggctgttc tgctcacacc 22920
   tgtgctccat tctatccact ggtcactacc agaatgtgaa gataatactt aaaacaactt 22980
   cagtgtcacc aacagacatt gctgaaagct acaccttccc acagagcagt ggtttgcaat 23040
   ttttgttgca cgctactcat ctgggaaact ttcccaaatc ctaataccca aactgtatcc 23100
   caaaatcttg ggaggtgggg tgctggcatc agtatttttt ccaagctctc cgggtgattc 23160
   caagtgcagc caaggttaaa aaccacttgc agagtgactc ttagttctgg atttacagaa 23220
   ttttcatgtt gctgattcaa gaaatatctg caatctgtgc tgtacccact ttccagaaat 23280
   gtgctggtag catctatctg tggctccttc taaagagcat atttccacaa ggtccttgng 23340
   tacactgctc catgcttttg agacatggtg tctccacagg aagctgagaa tctcaaaaga 23400
   tatctcctga gcagatggat gcntgagacc tcaccaagga ggaagatggg aggatgtgac 23460
   agngaaaana gtctgccaga gccctgaaaa aacactaant ctgcattgat gcctgactca 23520
   gggcttgcaa cacaccttgg caacctacac atctcatgtt ttgtgttata ctgcattctc 23580
   taggtttggc aggaagaacg ttctcttcgc aaccatggct gtacagactg gcttcagctt 23640
   cctgcagatt ttctccatca gctgggagat gttcactgtg ttatttgtca tcgtgggcat 23700
   gggccagatc tccaactatg tggtagcctt catactaggt aggaatggct tctgggacat 23760
   ggggtgcttc cctctaaccc tctgaaaggc ccagaaagag gaaatcattg ggccattggg 23820
   ctgtgcttcc aaagcctttg gatatatgtg tcttgggagg gagccgtagc cacagctctt 23880
   ggagcaagtc aggggcaggg caggcactcg cagggagcca cctctgtgct gagccctgaa 23940
   atgtggtgga gaagaagagc aggagccatt aagaaccagc tgcacaacag ctgagacaca 24000
   ttctctggag cacagggctt aagagaggca cagaggagga cagtttaggg gaagggcagc 24060
   ctgaggatgt ctttgatctg agttgtgtag gcttgagact tcttcctggt tgcttgtgaa 24120
   gagacttagg gtagtcactg tggccagtac acaggtgggg aggctcatgt cagacagggg 24180
   gagttcagtg ccagcccncc atcactgggc ctatctggcc tcaccctgag ttagagccca 24240
   ccccagagcc tatcagctaa gcacaggaca agcctcccca cactgatagc cccatctcct 24300
   gtcaggctgc atgaggaaga ctcatcagtt acaaggtcat gaagaggtcc ctggggccct 24360
   cagcagcttt tcctgggagg gagttaggtg agctccaggc gagttgatga gggtcatcca 24420
   caggagtgag agagtgtgtt ctcttgagcc cagcgaatgg gcaaatgaga gaagcagagg 24480
   tgaggggtat ccctgggaca gtagacacca gttcccatca tntctagggt gataatatat 24540
   tctaggtggc tcagtaccat cccaatttgt ccctattgtc acagtgtcat tattaataat 24600
   actcctttaa tagaagtcta acgattcttc ttgcctcaaa agtccgaatc tggttaggtg 24660
   actctgattg ctttttaaga gtctgaagga ctcgttggct ttggagatgc tgcactgtct 24720
   taattgatgg tgtgtacacc ttctgtcaag ttgccaggat ggaaatattc caactctact 24780
   cctttaatta tttgcgaaga gccccgtgcc atgggctcag cctcagtgtc tgcagagggg 24840
   agaagggagg agacaaggtg ggaagaacat gctggagcca catggtccca gcaggtagca 24900
   cactcttcag caggccccag cgggaggagg ctgctcccct tcctgcaggg ggaaggagcg 24960
   gggcaagatt ttcttgaaga tcatttgtgt agagggcaca gggagcttca ggccagtgtt 25020
   gttggaaact ggaggttgga ggaacttaag atagaaattg caacctccta gtccccagtg 25080
   gctccaagaa cacaaggcaa agcctattgt cttcatgttc aaagtcccca gaatccatct 25140
   tccntgtgac gatcctccct aagcctgaaa aacccaacat gtggctctgc ttgggccaga 25200
   cacataccaa aagccaaaca ggcctcccca aaacggagga ctctgaccag caggcccata 25260
   tcccaggagg acctgaagga ccatctggaa cagaagggta gagagtcacg gtgaagccag 25320
   agaagctctc tccgtgggaa tgggaacaag gtgggacttg tgccaggtgg tggcatcccc 25380
   ggttctatag aatgggcagc actgagatct gcctttttag tggcacaggg tgattctgga 25440
   gcctccaagc ctcccctntg tgagcgggtn aggcagggag agcatacccc aggcttccta 25500
   caggaaaggg ctgccctccc ccgccggctc tgacactatc atctactctc ctcccagttc 25560
   ccatgcagca tgtggtccca catgcagggt gaagaagggt aagagggcca aggggaaagt 25620
   aaggccatgg aagatcagtc aagccagcca gtcagtaggt aagaccctgg gtgcagcagc 25680
   ctcgctatca gctgggatcc aggcctctca gggcttgcta tcagcctggg gccaggcctg 25740
   ttatagggtg ctctgggagc ctcttcatgg tagttttact ccattgtgct ctgcaggaaa 25800
   ttactggctt ttgtatcctg gtatgagact cgttgtgttg gnttctcagg tctgaaagtt 25860
   taagtgtccc tgaaaaccag cctgctctgg aatggctgga cgccccagtc agcacctggg 25920
   gttgggggac acagtacaaa ggcaaggtgg ctgctagctt ccctacagtg cagcagtgct 25980
   ctctggtgtt tctgggataa aatagctgaa ttccaggttg tatttgacta aaataaacca 26040
   aagggtgtgg tctnggctga ggagtttggt aggtgccttc gcttccancc tctggcccta 26100
   aggaagggtt ggcaaagctc tgcatgataa tttcacatta acttgggaag ccaaattact 26160
   cccactgtca aagtagtgca gaaatgagtc cttgttttgt gctgctttta aaaaatgatc 26220
   aaggctgcca cagtcgtagc agactagaaa agagaaagaa agaacagtgc taccctacac 26280
   agcataaagc agacatgcct ggaagtgatg gtgttgccgg aatgatgctg gtttaggagg 26340
   aaatgaccac tcgggaccaa ggacactaac cctacccacg ttccagctcc cctcacaatc 26400
   tcctactgtt gcccagaaat aatgccctcc acctgtcaat agccttctgg gtagaatgcc 26460
   atcagtatcc tctcttatcc cccataacta gctgcttaaa cacaaaaagt gtttctgtnt 26520
   ttaataacac agaaaaaact cttgactgat tcattaattc agtatttttc agtacttcta 26580
   acatcaacct ccatgtgaca tttctttggg catgctgaac catttaaagc tttgcttcct 26640
   ctgatgtcta gaggttcctc cttttgatag gtgtgtaagt gatggcatct tcctttacaa 26700
   ctctggctac caggaagctc aggcaaattt gaaatttaac tacatcaatg atgcagcttt 26760
   taccagccac atatatatat attttttact gtttttaaat tgaacagttg aaatataatt 26820
   ttgtcttcgc ctgtttagga tgccgtaaca aaatcctata ggctgggtgg tttaaacaac 26880
   caaaatttat tttccacagt tgtggagggt agaagtccaa gatcaaagtg ccagcagggt 26940
   tggtgtctgg tganggttct cccattgggt tgcanatggc tgccttctcc ctaagtcctc 27000
   acatggtctt tcctcngcgc aagtgctcag aaagagggaa agcaagaaag agagagtgag 27060
   ccatagagag ctcactctnt ggtgtctctt cnngtaagaa cactaatcct ataccctatc 27120
   aggtcagggg atcaatgtat gaataataga gggacacaaa cattcagtcc atgacagatt 27180
   tgcataccat gatattcaca cttttaaagt gtataattca gtggtgttta gcatattcac 27240
   aaagttgtac agccatcaac actatctaac tcctcatcac cctgaaaaga aaccctatac 27300
   gaattagcag tcatttccct cttcccctcg cccctggcaa ccactaattt actttctgcc 27360
   tttaatggat ttccctattc cgcacatttg atataaacag aatcatatat tatgtggcct 27420
   ttgtgactga ctccacttgt tttcaaggtt gatccatgtt gtaacatgaa ttagtattcc 27480
   actggatgaa gacaacacat ttcggtcatc cattcatcag tcattgaaca cctgggtgtt 27540
   tctactttgg ggctgttatg aataatgcta tgaacattgg agaataagta tttttgtgga 27600
   tatatgtttt taattctctt gggtataagg taggacctct gctgggtcct atggtgattc 27660
   tatatttaac cntttgagga actgccaaac tgttttcaaa atagtcagct gcgtattttt 27720
   gatcttttca ccaaggcaca actttttact tttctttata tcttacttta atgttaccat 27780
   tattttantg tatttaaatt tgaagttgcc tcaaaatctc caagggatga gctggcatat 27840
   aaatacgtaa acatgatgca gaaatttgct ggcatacctt atttgcattg caccgtaaga 27900
   gggaagaact ggggagaccc agggaagagc aagaacttac tcctgtctat tcacaaggag 27960
   ctcactgcct ggttgtgcct ctgcagccaa tgcagctgtc actgtggaca tggggacatg 28020
   ggtcaaggga gtggggcaca tattgctggg tatacacaga gataggaaat gttacttttg 28080
   actggagtga ttggtgcaga cagaggggaa gccctgagca ggggaacatc tgttaagccc 28140
   tgaagaggag ttctgtcttc cactgggcct ttttggatca aaagcattgt actttccatc 28200
   antcccctga catgtagcac atttcaactg cttgatcagg ataaagaata actattactg 28260
   agcacctgcc acatgccaga caccctccta ggcactttgc atacatgatc tcagtcttca 28320
   taataatatc tccatgaaag acggcttatc acagtcactc tagtagcaac tcagcatcaa 28380
   ggaacccagg ttctccccgt gccagatgga ggacactcct agcccatggt tcctgggtct 28440
   cgcctcatat cctggcagct gcactacagt cttgtttctt gagctggggt ctactgagag 28500
   cctatgttgg tcatttatga aggtgcatgt cctgcctctg tgtggaatgg gcccagcggt 28560
   tcctggggca ggtactgctc tacatggaag gcagcctggc cctctctgag tcactccctc 28620
   ttggagagag tgctcagatt cagaggggtg tgacaggtgg tgggcaatac tcctagggct 28680
   atgctggccc tcatcctagc caggtcatgt ccctttggct tagatgtcaa agtttcactc 28740
   ccaggagaaa ctcagaagcc cagccagacc aagaccaacc aggctttgcc ctagaagcaa 28800
   agcaggcaga agaggcagat agagcatagg gggccaggac agggatcccc cgtcagcaca 28860
   gcaggagtca ctgctgtgtc atctgtttca ctgccacatg gaaagctcat ggcagctgca 28920
   ggccataaaa gatctgtgta atcagggttc tctcctagca ttactcagag gggtctggct 28980
   ggcttccagt gacaagccaa ggaagggaaa atgtaaatcc acttntgaaa gggggataag 29040
   gtgggtggat cacctgaggt caggagttca agaccagcct ggccaacatg gcaaaacccc 29100
   gtctctacta aaaatacaaa aattagccgg gcatggtggc aagcgcctgt agtcctagct 29160
   acttgggagg ctgaggcagg aaaatcgctt gaacccagga ggaagaggtt acagtaagct 29220
   gagattgcac cnctgcactc tggcctaggc aacagagcac aagtctgtct caaaaaaaaa 29280
   aaaaaaagaa aaaaaagaaa aactgtaaaa atgacagcag gatanggcct tacccatctc 29340
   ccctcatgtc tntantggac tctcagttga agaactgctt tataaggcca agtcatgccc 29400
   ccagtaattt ttttcttgga aggtagacca gtatccatag agcaaataag gcttaaatcc 29460
   tggtgcacca cttattatgt gcatgaattt aagcaattgg ctttaactct gaagtctcag 29520
   ctaattatgt gtaaagtagg aggaatatta gcttttgttt tagtttttgt ttttgtttga 29580
   gatggagttt cgctcttgtt gcccagtctg gagtgcnnng gcntgatntc ggctnactgc 29640
   aacctctgcc tcctgggttc aagcgattat cctgtctcan cctcccgaat agntgggatt 29700
   acaggtgcct gccaccatgc ctggctaatt tttgtattct tagtagagac aggtttcatc 29760
   atattggtca ggctggtctc aaactcctga cctcangtga tctgcctgcc ttggcntccc 29820
   tantgctggg attacaggca taanccaccg tgcctggcct ggaatattag tttttatata 29880
   actggtgtga agggtcaaag agataatata tgtaaacact tancctgnaa cctgtctcaa 29940
   agtacctact caaaaaaatg ctanctgtga agatggtgat cctntttaag gaagggtgac 30000
   tgcctaaaag agagcanaaa gtangactaa aaangantta tttcaatttg taccatccat 30060
   gctgtccaca ggaaggcaaa gagagagacc tacaaagtct ctgtccccaa catgcactct 30120
   gcaaagttat ataactgttc tggtctgaga cccatgctta gagagggaga ttatccagga 30180
   acccagtagt ataacttctc ttttcttaac aaggtcatga aggtaggaga aagctcctct 30240
   ggcctcacaa ttctacaaga ttctcacttg atcctgcgcc ctttctcttt tccaaaatca 30300
   aaccctctgc cactgaatct ctgcttaaga ataaagcacc ttctatttat ttccacatca 30360
   caatcacggc caaggggttt agaaaccagt gcatagcagg aaggtcatta atgaggtcag 30420
   aaaagacctg gaaatcatac tcatgatcct caaagaagtc aggccctatc cttggaaatg 30480
   ggtttatagg ctgagcaatg ctctgggtgg ttagggcaat tgtctccaac atgggatgca 30540
   ggaggcacaa agtactggct gtttctatca gtccaggttt ttaaatggcc tcttgagagg 30600
   gacccactga ggcagatctc tcagttatga acagtgaagg ggaatgagtg gggtgggatg 30660
   ggaacagata ggtggtaaaa tgagaaacca gccaacatag taattttgct gcttctctgg 30720
   aattagctct taatccctta gcctgtgcct agggcatata aacatgactg agaaaatctc 30780
   tcttaaaagg cangcatgaa tgacctttga attatctgtt ttgtatcttc caggccactt 30840
   ctctatagca tgggcagatc cccaacaaaa ggcagagcca ttgccattgg tcacatctcc 30900
   gccccaggtg tagtgaacca agggcagact ctgtggaaag ggcccagagc tccgtgacaa 30960
   gagaaggagc tttgtncttg gactcccatc tggtgccatc tctgctcact gtggcactga 31020
   gcagagcaga ggccaatagt cgcctgcatg tcctttccca aaaagcagtg cccgcaccaa 31080
   gcagtcatgg gcctttctgc tgttcaggga aagcactgtg agttcaatgg gtgtgtggac 31140
   aggtctaggg ctgctgcctg caggggcagc agaacagcgc ctgcttttct ttccttggtc 31200
   caaatggact gtggactgtg tctgcctggg ctgatctctg ggcacagcat gctgcaggaa 31260
   ggcctgtctg tgnacctcca gagccagctc actccaactg atttctcagg gctcaggcat 31320
   gggtaagtgt gccctgaggt cttggaaatg atgaactctc cactgcttcc tgatgggcca 31380
   atgtctgttt atttacagtt cagaactgtt ggaaaggcta cacacacctt gaccccacat 31440
   ggcccctcag aagagccact gcttgtctcc tgaaatgctc tctctgcttc tcatgttggg 31500
   gctcttattt tggaaatgtg gcattctcag aagggattag ctcttggggc tgggcatgat 31560
   atttttatga catggaatgg ccttaaaata agttggccag gcgcggtggc tcatgactgt 31620
   aatcccaaca ttttgggagg ccaaggcagg tagatcactt gaggtcagga gttcaagacc 31680
   agcctggcca acatggcgaa actctgtctc tactaaaaat acaaaaatta gccaggcgtg 31740
   gtggtgcacg cctgtagttc cagctgttcg gaaggctgaa gtgggaaggt cacttgagct 31800
   tgggaggcag aggttgcaat gagctgagat cacgccactg cactgcagcc cgggcaacag 31860
   agggagactt tgtctcaaaa ataataataa taataataaa ttaattaaaa ataaataaat 31920
   aaatctatgg ctccattaaa atctcataga aattcaacct tctataagtt gccaagtctt 31980
   ggatcacgtc ccaccttcct ctaccctgac catgcctgct ctgggccatg aggcaaatgc 32040
   ctttcccctt ttctaagtgg tgatagtttg aactctaact gccacataat gatataaaaa 32100
   gcaaatatta aagtgagtta atatgctaat actcctccct tgttttgaac aggaacagaa 32160
   attcttggca agtcagttcg tattatattc tctacattag gagtgtgcac attttttgca 32220
   gttggctata tgctgctgcc actgtttgct tacttcatca gagactggcg gatgctgctg 32280
   ctggcgctga cggtgccggg agtgctgtgt gtcccgctgt ggtggtgagt gtgactcgtc 32340
   cccagacagg ccctctgctg cgggtcagca cacctggaac acacctggag cctgatgctg 32400
   acctagcctg ggcttgcatg acctccacgg aactcgcgga ggccagcttc caagccggga 32460
   gagtttttgc ctcattgtgg gtgggcctgt gtgctggagt ttgagtctcc attgtcctaa 32520
   aaagcacatt ctcaggattc caatctcttc cttgatacca gccactcctg tctgttgcta 32580
   cttgttattt gtggagctgg tggtgttgga ttttgttgtt tttaactctg ccccctgctc 32640
   tttcatcact tttctctctc tagaaaaatc tcactctatc caactaatgt aattnttcaa 32700
   gcttccctat catataacga ttagcatcta tgaaagggaa gcctatttat tagcactatg 32760
   atagtgtgcc tatttatctg ctatgtctcg aagagtgaga aaattacttt ttatttaata 32820
   aattgttaga gaaatgtcct cttctctccn cccncaacct cccaactcag ttttaacaca 32880
   acagttatag gaataacaaa gtttgaagat tctggcctca gtcttgccca atatgactat 32940
   ctgggggatt tcaggtgaaa gaataaatta aaaatcatag aacaaggtgt cagacatacc 33000
   ctctttttgg agatttcctg ttcctttgct ttttcatatt taagcaagag aanaggagat 33060
   tccttccatn ggtgggtctg gttctgtctc tctctcattc tctttctctc tcattaattc 33120
   ttctttgttt gatttcatta tatcaaagtc aaatcttcca gcactgactg gtttcaccga 33180
   actgcataac ttctacctgc ttacacataa cacaagcttc tttgaccatt ttttggaact 33240
   cacaatttag actagacctc atggttgccc tgtcctttgg atacctcctg actgggaagc 33300
   tgtctaggcc tttatcttac tgcagctgtt aggcagtcat agaggttgac aggcaggcca 33360
   cctgtctgac gagatagcgc aggtcaggtg ggctcactct aagcccctgt attctcatga 33420
   cctgtaagag tgctgtgatt tactagtgta aattattggc atgaagtgtg cagggggagt 33480
   tgacagtttg ttaaaggtta tgtattattc tgatttgcaa cattcctctg gaaccctgtc 33540
   tgaggaccng gaaacccggc ctgaggagca ntgaataagg gctggccatt atctacatag 33600
   ctgtgcctcc aaggaaagca tcccagactt acaagagcag agcaattttc tgaatgagag 33660
   taattatcat catcatcatc tgtccttctc tccacaactc ccttgctagc tgaatttatt 33720
   ggaaaagatg ttagtgacta aggttctctc aagacacatg acaagattat tcaaaggttc 33780
   ttttctgcct cttcagctga atggttttaa taaactatgt tctctggctc tagttgtcat 33840
   tttgacctaa gagaccagta ctttcaaaat gttataatta gccaggtatg gtgnttcaca 33900
   cctgtaatcc cagcactttg ggaggccaag gtgggaggat cactttagcc cagcagttca 33960
   aaaccagcct gggcaacatg gcaaaaccct gtctgtacta aaaataaaaa attagctggg 34020
   tgcagtggtg agcgcctgta gtcccagcta cttaggaggc agaggtggga gggtcccttg 34080
   agcctgggag ttcaagatta cagtgngcta tatgatcatg ccactgcact ccagcccaga 34140
   tagaatgaga tgctgtctca aaaaaaaagt tataatcaat ctgattaagg tatgattcta 34200
   tcagagagaa agagagagag agaataacaa tataaagcct ctgatgggct gtacagagat 34260
   tagtagggat gggatctttg cctcgggacc atatgtcaca gcgaggaacg aacagctctg 34320
   atccattagc cctccagagt cagcctccag agtttccagc agagaaggtc catcccacag 34380
   ggagcctgct cactntgccc taaggagagc tttttcttct attccccagg agctggtcca 34440
   tgaatgctcc tggctgtggg catactgtac cagggacagg acttgcccca gacagaggcc 34500
   agcctgggac cagcactgac aagctgagtg ctccccctga acatttacca gtggtcttac 34560
   cattttccta taaaagaaaa tagctttctt ctgaacatcc catccttgca ggtgggtctt   34620
   nccaaaggga tgctcatcan nctannggnt ggggtntgga gggatgctta gagctgacat   34680
   ggaccctgcc gcctgctcag tatgagncaa gctctccagc aacacctcac tgctgtntag 34740
   tctganttca atctacagag agattatagt gtgctacctc atttggcact ccattgactt 34800
   ttgaaatgat cagctagatt tgagattatg tgcaccaaaa aaaaatcaaa gcaattcaaa 34860
   actagagata ccagtggccc tgagtacagt gggctagaaa tgggaggtaa acctacatag 34920
   aggcactgct ggtggtttgg gggacttttt atgtcccaag tttcctttaa aaggcttggc 34980
   agtaagtggc cacaggtgtg aatggagtat gtgtataggc ggtggggatg gggtagggaa 35040
   gcaggccata gatgcaatgt gaggaaagct atgcaatttc tagctgatta aacattaaga 35100
   atctgaggcc agtggtctgt agaaaaaaag gtaggatatt tctaagcata atctggttgg 35160
   agaaagatac tggccccatc catgaggtat atcgtggctg tgtagtaaga tctctaattc 35220
   tgcactaata tagtagccac cagccacatg cagctattta agttaaaatt aattaaaagt 35280
   aaataaaatt gaattttcat ttcttcaatc acactagctc aatagctaca tgtggttagt 35340
   gggtgctgtc ttagtacaga tacagaacat ttcagaagtt tctatcggac agcacagttc 35400
   taattactga gagtcagtga cctaacatta tngtagttcc tatagggacc cactaaaggc 35460
   atattctgaa aaatgcaaac taggctcatt tttcaactca tgtgctatta agctggagag 35520
   gggacctgct ggttggtcat ttgttttgct ctgagctaat tcctagtgtg ggtttggggc 35580
   caattaacta cacaggactc tgtgtctgct acttataatt tgttntctag gctaagtaag 35640
   tacagatgct ctttgactta taatggggtt acatcccaat aagcccattg taaattgaga 35700
   atattctagg tcaatacacc taacctactg aggtaggagg ccagacacga ctccagaggc 35760
   agggcttgga cactggacca aattgaggac cagctaaaac anagccaggg cagaagacgc 35820
   tttccataag acacgcccac ccgtgtgcca tgtcagttta ctgttgccat gacaacactc 35880
   aggctttatt gcccctttcc atggcaatga cccaatgacc caaaaattac taccctttcc 35940
   ctagaaattt ctgcataaac tgacctttca tctacatgta attaaaagta gttataaata 36000
   tgagtgcaaa actgtcctga gctgctactc tctgcctgtg gggcagccct gctctgcagg 36060
   agcagtcacg gagctgtaac attgcntctt caataaagct gttttcttcc acctccagtt 36120
   tgcccttgaa ttctttcctg ggcaaagcca agaaccctca tgggccaaac cccactctgg 36180
   agcttgcctg ccctgcatcc ctaccaaata tcatagctca gcctgaccta ccttaaacat 36240
   gctcagaaca cttacattag cctacaatng ggcaaaattt tctaacacaa aacctatttt 36300
   ataattaagt gttgaatatt tcatgtaatt tattaaatac tgtactgaaa gtgaacaaca 36360
   gaatggctgt atgtatgagc actcaaagta aggtttctac tcaatgcata tcatttttct 36420
   cctatagtag aaaaatcata agtcgaacca tcataagtta gggaccatct gtactgacaa 36480
   taggtaagga aaatgagagc tgacaggaga atggctaaaa gattttgaga aataaaaatt 36540
   tagaaagaat agaagcccta ttatttgcaa ctgtgtttga gcatcagcta taaatgggaa 36600
   tagactatgt aatttaaaac aataagcttt tttagacatt aacaaaaaga caacattggg 36660
   gctcatctcc caaaagctgg tttgggcact cgctgatctg ggaatctgtt gaaagccatg 36720
   ggggccccac acaggngggt gtcagtgccc tcactttctc ttttgactaa aggaaacgat 36780
   cangaacaca ggtgccctgg ccatcctttt cccaataaac tgtgaacatc accagctggg 36840
   acaccagcaa ttctccttag gatggaccca ctgcaaagac agtctcttct ctaggggaac 36900
   ttatagtcta gtagctatgt ttcttttatg agccacacta cctataagtt ttggatgaac  36960
   ttctccagag ccctggctca aacatgccca tagttaatgt caccccttat ggtctggtcc  37020
   tggtctcaag cacaactgat gatcggtctt ataatttgat cagaagtttg aaacttgaag 37080
   gtggttttat ttattcctat gctccaatag aaatcttatg tggactccct ttaaaaagac 37140
   aaactagaat tttacaaaat taaaactatt tgggggaaac tcagatttaa tagtatccag 37200
   ccctgctgtt gtgaaaaatt attaaatatt aaaaataaat tataggttca ttcctgaatc 37260
   tccccgatgg ctgatatccc agagaagatt tagagaggct gaagatatca tccaaaaagc 37320
   tgcaaaaatg aacaacanag ctgtaccagc agtgatattt gattctgtgg aggtaagcat 37380
   ttgcagatgt ttcctcttga gatcagctat gcattcttga ttttatggaa tttaacttaa 37440
   ttcaatattt gttaanngcc cactatgtac caggcattgc actaggcctt aggaacacaa 37500
   tcatgattaa attctaagat aaaatccacc ttaaagggct gtttataaga attaaatgca 37560
   ataatatatg taaactgctt agcaaatagc aagcgttcat taaatattag tttgctttat 37620
   tttgcaagtt tatcatcaac tttctattgg cattgctcca aaccactgac acagctatgt 37680
   actgatgatt tttaaatgtc cagattaagt ggaaaaatat gtttcagggc tagtccaccc 37740
   tgggttctcc actgtaccct gaatcttacc acagactgct gaggaagcta tgcatgtctt 37800
   tagtctcact ctgtgtcatg tctagaacct taaggcctga aagcacaggc ttgaggattc 37860
   acccttgaaa gctggtggcc acaaggaaaa gctgatattc tgtggaagca gccncacctc 37920
   ctgacagttc gtgaggatat gcagtgtgcc atgccctgtg ctggctccta gaggaaataa 37980
   gaaaagacac agcccaaatt ctaaaggaac atagaatgta actaagattg gcaagaggat 38040
   tagataaaaa gataaagaat ggcataaggg aggaaatggt aagtatccaa gtagagacac 38100
   tgtgtgccag caatacagag aaagatagca gggtggccca tggctggagg caggtccaag 38160
   ccagggagga cagcaggact cacttaaatg agtggagagg atggggcagg gcattcctgt 38220
   ctaggagata ggtaggagtc tgctcaggac acctaaaaaa gactgtggtg gcttcagtaa 38280
   cattatacat gccctgcacc aaagcatgta gtacagaaag cagaactctg tagaggacgg 38340
   tgggcatgcc actgctcacc cccacggctt ccccagtccc tggccttcag gaccttttcc 38400
   agaagcagat actctggcta tggctagagg cttagactgg ccatgaactc aagctctcag 38460
   agggctgcca ggacaaaaca tcccttaatc ctttgccctt caaatcaaga agtccatttc 38520
   aagtctcagt caatttaaat ggataaaaca acaacaacaa caacaacatt gggagtgtct 38580
   aacacttctg cccttcaggg ctgtgctggg gtgtttatng gtatttatca gctaggtggc 38640
   ctgattccta tctgtgaaac ttanagtcta gttgtaggga tctacctatg tggatgacag 38700
   caatcataaa atagatatga gatatatagt tatggacaaa taagaatata gttcagtgtt 38760
   aaaacacatg gttctggcag tgccttnana nacacacaca cacacacnca nanananann 38820
   nnnannnata tntnnntntn tntatatatn tntntntnta tntntatata tanananaca 38880
   cactttaaaa aaattttgag tctcactgtt ttgctcaggc tagagtgcag tggtacaatt 38940
   acagctcaat gtagcctcga cctcgacctt ctgggctcaa gcaatcctcc cacctcaacc 39000
   tcccaagctg ggactacagg cacttgccac caaacccagc taacattttg tatttttttt 39060
   tgtagagaca gggtttcact atgttgccca ggctggtctc aaactcctgg gctcaagcaa 39120
   tcctcctacc tcagcctccc ataatgctag gattacagat gtgagccatc attctcagcc 39180
   tgacagtgcc ttctttcttc ctgccatgat gcacatctag cctgaactgg acaaacccat 39240
   taagggaaag ggggcttgcg ctttcctgag gcctggtcat ctcccctgct cacctcccgt 39300
   aggatcctct ttaggaggat ctgcagagcc ctgtgccagc aaatgcaccc tatttagaat 39360
   gatattgaag agcctaccaa atgccaggca ctgtgttctg ctcattgctc atgctttttt 39420
   ttttttnnaa aaaannaann nangnnnnag cgaagttaag taacttgccc aggatcacac 39480
   agatgttaaa ctgggatttg tgacctagtt ctcccaggca ccaaagtaca tgctcttaac 39540
   aactgtgatt tttcacttca tgttacaggc agtagcatta acttggatag gaaaagatat 39600
   agagtagagt attgtcttag aaagcnatct caagcatcct tggcttcana agaagcacct 39660
   tcaacctgct nctggtacct gatccaaaga cattttggtg gctccaccca ggccagggac 39720
   aaccactgga tacaagcata ttcttgttct gaggtttcca atcctggctg ctcatcacaa 39780
   tcacccaagt gctttaagaa tacagattct caggcctctg tttaaaagag tcgaagtagg 39840
   caagcccagg gaggaacctg gcaacctgca tttaagaagg gccccgtgga accaccaagt 39900
   attagcacag gactggtcca caggtcctgt ctgtgtgcac caccctggaa ccctccaaga 39960
   ngatgtctgg ttcccactag tttatgcagt ggctttgctt tgttataata ccagggttat 40020
   tctgctgggg gcatgttctg gggctctaca atccaaatag tgcaagagag aaaaggcaat 40080
   gtggctgnga gctgtgtaag tgtctgatca ttcctagaaa aaaggacact cacatttgga 40140
   gaggacctgc tgtgaaggca ggcctggcac tcccacttct tcataataaa aactcctcca 40200
   tcagtctctt actgcgcatg cactgtaggg ccacaaagtc acaaagtcct ggtacttcat 40260
   ccctgagaga ttccccacct cagagcctaa taacttttat gttagtttca attctaatca 40320
   gcttggttac cttgattcat aactcatagc aagttaatat cagcaaatta aaagcaaaag 40380
   aataggccgg gcgcagtggc tcacacctgt aatcccagca ctttgggaag ctgaggtgga 40440
   tggatcacct gaagtcagga gtttgagacc agcctggcca atatggcgaa accccatctc 40500
   tactaaaaat acaaaaatta gccaggtgtg gtggcaggtg cctgtaatcc cagctactca 40560
   ggaggctgag gcaggggaat tgcttgaacc tgggaggcgg angttgcagt gagctgagat 40620
   cgcgccactg tactccagcc tgggcaacaa gantgaaact ccctctcaaa aaacaaacaa 40680
   gaaaataaat ataaataaaa gtaaaagagt ataaacccat tgatagcagt aaggatttgt 40740
   cactgaccgc tgatggctag cctcttttcc tcaatgatac agtaataata ataatagttt 40800
   aaatttactg gacatttacc atggttcaag cactgtgcta agctatatat atatgatctc 40860
   atttaatctc tcaactccca attaatatac ccattttata tagcagtaag ctgaagttca 40920
   gagaggatcg ttacttgccc aaggcccgcc agtaaactga agtctgttct gatgctaaat 40980
   gactacaaca agagaagtgt aaaaagcaag gctgggtaat cccagcactt tgggaggcca 41040
   aagtgggcag actgcttgag gccaggagtt tgagaccaac ctggccaaca tggtgaaacc 41100
   cagtctctac aaaaaatgca aaaattagcc agatgcggtg gtgctcgcct gtaatcccag 41160
   ctactcagga ggctgagagg caggagaatc gcttgaacct gggaggtgga ggttgcagtg 41220
   agctgagatc acaccactgc actccagcct gggccacaga gctagactcc atctaaaaaa 41280
   aacaaacaaa taaacagaga agtgtaaaaa gcagacgtgg cctcaaggaa gggactacat 41340
   ggccctaccc ttcaataggc agtcctgagg atcacagatc agtgtggtaa atgacatcca 41400
   ttctctgact gtcagtcaga gaaaggctat gttagccaac aggctttgtc taacgagaca 41460
   gggaaggggg tgttatatat ttaaaaggct tctgtgtaaa atggactaca aacattttaa 41520
   agtgtcagag taagtgtcat atatgtagat gccctattgc ctccaatcac cagacttcta 41580
   agcatagtca aggtgaagtg ngagttgcat gcataagttt tccccatgca tcatagccaa 41640
   agatacttcc ttactacctc ttaatctcat ggttatttgc attattttgg ttacaggagc 41700
   taaatcccct gaagcagcag aaagctttca ttctggacct gttcaggact cggaatattg 41760
   ccataatgac cattatgtct ttgctgctat ggtaantaat aagtgacctg gaaatgcaga 41820
   tatccagcac ataagtatgc aactgatttn cttaactcag agnaacatta tgacaacgac 41880
   tgggttntcc tgaggaaaaa ttaagattat aaaattctaa attattttga aatgcctcta 41940
   ttcagttcat gggactagat attgaaaaat gtcactgggc gcantggctt acgcctgtaa 42000
   ttccagcact ttgggaggcc aaggtgggca gatcacctga ggtcaggagt tcgagacgaa 42060
   tctagccaac atggtgaaac cccgtctcta ctaaaaatac aaaaattacc tggncatggt 42120
   ggcacatacc tgtaatccca gctacttggg aggctgaggc aggagaatca cttgaaccca 42180
   ggaggtggag gttccagtga gccttgatcg cactactgta ctccagcctg ggcagcagag 42240
   caagactcca tctaaaaaaa aaaaagtcac agggtacagt atttaagtct gcagtactgg 42300
   gacccaaagg gaaagcattc ccttccccct cctaaagtta tgggattgag atcattgatg 42360
   tccctgggta atggttccct ccccaccaaa aattatgaga ttaagactaa tgatgtccgt 42420
   gttgcctgag gcaganatgc taattcagct cctgcttcct gaagctgacc tggcaattga 42480
   acactgaggc tagaataacc tagatcccca ttgcctggat tggagccttg atcagctttt 42540
   tacacgtgcc aatagcaaac taacaagtgg ctttcaggcg cagaaatact gaattggctt 42600
   tatagattag atttaataat ttatctgccn nnaaaaaagg aaaaaagaaa antcctactc 42660
   tatgatggca gcacacacac acacacacac acacacacac acacacacga tgatngtcag 42720
   aactggtaga tttaggctgc agctaatcta agtttatttt taagggccat cctacttggc 42780
   aattagaaga gatcaatgac agagcttatg cgtctgaatt aagcaagtgt gtcctaggta 42840
   catgggcttn ttagttctta catttcagcc tatatccctg atgcaaatgt tcttgggtag 42900
   tngtgctact aagctaataa aaaattagtc actagttcta tctaanctac tcctattact 42960
   aaatgggaaa atcactaaga tcccttgagc atacttattt gttgtgttta tgaattagtc 43020
   cttgtattat ctaatttggg tctagtatgt gtaaaaagat ccactagtgt tcctgaagtc 43080
   atttttcaga tatcctctgc ttctcagtca tgttaataat aagcaaattt ctacacaggg 43140
   gtgtctgcat agtgcagttg taacaaaaac tcaggttcag tcactcacag cttgcagcgt 43200
   ccaattaaca agagcaaagt ctgatgtaag gaaagtgact ttttattcca aagctagctt 43260
   agggagaaag gtatcggctt cctgcctgaa gggtaccacc gctcctcttt ttgaagcgga 43320
   aagcaggcac ttctaaaagg caggggagga cgcgagcagg tgagggatca ganggctagc 43380
   ttgctgcctt atctactggg cagttgannt ggtgccttca tgggcagaaa caggttgtat 43440
   aggtggccaa aaactctagc aggcatactt tgagttggaa attgactgat atcgcttgag 43500
   gaaatctggg cngtgagagt tccactctgg agcttctaag cacatagtta gatgaatttg 43560
   ccctgcaggg agtgtccagt gaagaggagg ttaaaaggct ataattgcac ttctaaagag 43620
   ctaagtagaa agtgcagaaa aggaggaaag agaaaangag agagaaaata aactatctct 43680
   tagaaaaatg ggggtactca gttacatagt tatttttcca cagagaagct tctttttttt 43740
   atttatgttt ttattttttt gaganagagt ttcgctntgt cacccagcct ggagtgcaat 43800
   ggtgcgatct cagctcactg caacnttcgc ctcctgggtt caagcaattc tcctgtctca 43860
   gcctcccaag tagctgggat tataggcaca tgccaccacc atgcntggct aatttttgtc 43920
   ttttaagtag aganggggtt tcaccatgtt ggccaggctg gtctcaaact cctgacctca 43980
   ggtgatttgc ctgcctcggt ctcccaaagt gctgggattt caggcatgag ccaccacgca 44040
   tggcccacag agaagcttct acatggaagt taactataat gacagaatat acagactgca 44100
   gtctgtgata ctttgaagga ttaggggaca ttcactcttt tccctaaaga gaatcagaat 44160
   ttattctaag tgaatcccac agtatgaccc ttgtatctct tttttgttaa agaatgaaat 44220
   ctggatttta tgttcactga tgtgactaat attttttctt catataatag ctctaagaga 44280
   ttgaggaggt acaaactatt caggaaatag gaaaacttga aatcaactag ctgagaaaag 44340
   ttattgttga atattggtat atctagacta gcactgtccn atagaacttt ctgcagtgac 44400
   ggaaatgttt catatctatg tngttcaata tagtagccac tagctacacg tggctattga 44460
   gcacttgaag tgtggctagt gtaactaagg aactgaattt gtaatcttat ttaattttaa 44520
   tttattcaaa tttaagtagc caatgagggt agtggctant tataagacag caagatatag 44580
   agaatttctt gaccatcata aaattttaga gcgattcaca ccatcccttt gtcattttta 44640
   ccttcttctt tcaggatgct gacctcagtg ggttactttg ctctgtctct ggatgctcct 44700
   aatttacatg gagatgccta cctgaactgt ttcctctctg ccttgattga aattccagct 44760
   tacattacag cctggctgct attgcgaacn ctgcccaggc gttatatcat agctgcagta 44820
   ctgttctggg gaggaggtgt gcttctcttc attcaactgg tacctgtggg taagaagtta 44880
   accaagatga acagcttacc agaaaagacc cacatattga ctaggctgct tctgagtcac 44940
   tgctccatgt ttattcagcc tttacatcat aagccattca taagtcaatt agacaggtga 45000
   ttgctcacta cgtatgttgt ttcctgtgtt caaaacaact accccaaaca gaagagacag 45060
   tctcagagaa gcagaaagca ccaaagagga agagtaactc tcccagtcat ccccttcaca 45120
   gaannactca cctaacatct ccctttaact catantnntg attcctaatt tntggctact 45180
   ctcaaacatt ttccctgggt gtatattcaa gtattacagt gagtaggaca tgtcggttcc 45240
   aggatacatt cagtggtttt ctggtctgaa tctttaagta gttaaatagc acacaaaagg 45300
   atatatgctt cccttttccc cttgtgtctg acgttctcat aatattaaaa tgtcactaaa 45360
   ttattgatag taatagaaat acatatttct taataataag aaagaggata gaaaggattg 45420
   gtcctcctct ttgaagataa aacttaacta tattaacatg ctaatagntt acccatgaaa 45480
   ctgttttaac aaacaaaaga caagtggaca aaggtcactg aatgcattaa tgacctagaa 45540
   taacagtaga acaaagtaca aagccacccc tcacatgagc tccatctttg tccatgccca 45600
   aatattagga catttgaaga gaaaatgcta ttttgggaat atatttatgt taataaaagt 45660
   actcccactg aagcaaaaag gacaataaaa ataactgata taagaaagta tcanttctaa 45720
   aagcaccatt gtttataccg ggtctctttt ccagattatt acttcttatc cattggtctg 45780
   gtcatgctgg gaaaatttgg gatcacctct gctttctcca tgctgtatgt cttcactgct 45840
   gagctctacc caaccctggt caggaacatg gcggtggggg tcacatccac ggcctccaga 45900
   gtgggcagca tcattgcccc ctactttgtt tacctcggtg agctgcatct tgtgcatttg 45960
   ttcttccttt aaattagttt tcaatgntaa aagtaagatt ttcattgtga aaacgtcaaa 46020
   tagcatagaa tgtacnggaa anaaagtaca agttcncctc tcctctccca ggaggagctc 46080
   tagaaagccn atcncaaact ggtgagtagn gntncagaca ntttgttttg cacnacatca 46140
   tgtncncang tatgtatata attttttaaa cacacnngca actcaggcgt ggtggctcan 46200
   gcatgtaatc ccagcacttt ggnaggccaa ggtaggtgga tcacctgagg ccaggagttc 46260
   aagaccagcc tggccagcat ggtgaaaccc tgtctctact aaaaataaaa aaattagcca 46320
   ggtgtgctgg caggtgcctg taatcccggc tactctggag gctgaggtag gagaattgct 46380
   tgaacccagg aggcagaggc tgcagtgagt caagattgca ccactgcact ccaacctggg 46440
   caacaanagc aaaactctgt ctcaaaaata aataaaaata ataaaaaata aaaagaataa 46500
   acacacaaat aacattatat atgttaaaac tttttaaact taacattgta tcagaatatc 46560
   cttccaaata gttacatatt gctcctttac cttcttttta aataggtacc tccttttaaa 46620
   aatgtatata agtacatttt aaaataagta tatgtaagta taatctacag atagattcac 46680
   agaagtaaaa ttcctggagc aaagagtgcc gtntaaaaaa ttatatatat atgtgtgtgt 46740
   gtacataatg ttgtgcaaaa caaaatgtct gtagggctac tcaccaaaca gttcatgttg 46800
   gttttctagg gatcctcctg ggagagggga ggtgaacttg tacttttttc cagtacattc 46860
   catgccattt gaagttttca caatgnaaaa tcttactttt atactngaaa cacataattt 46920
   atttaaccag gctccaatga atggaggaca tgtaggtcat ttccaggttt ttgctattac 46980
   aaataancat tacaacgaac attctnccta tgctcctaca ccctttgtga atttacttac 47040
   gtatatttgg atttacaggt agattcatag aagtaaaatt cctggagcaa agagagccat 47100
   taaaaaattt catagatatt cccaaattga actctacata ttgtgccaat tataatctaa 47160
   ttattaatag gtaagagtgg ttttcaataa tctcaccaac aataaagatt acctaattct 47220
   tttttttttt tttttttctg agacagggtc tcactctgga gtgtagtggt gcgatcctag 47280
   attcccagct cactgcagcc tcaatccccc aggctcaaga gatcctccca cctcagactc 47340
   ccaactagct ggaactacag gcatgcacac cgtcaagccc agctaatttt tttttttttc 47400
   tggtanagac gggattttgc cagtttgccc aggctggttt cnaactcctg ggctcaagtg 47460
   atccacccgc ctcaacctcc caaagtgctg ggattacagg tttgagccac agcacctggc 47520
   ctaatttttt aatcattggt tatctgttat gtgaaaaatg gatatcattg ttactttcat 47580
   tcctatttat taattatgaa tgaggttgag catgtttttg tgtatttttt ggccatttta 47640
   tttatttttc agtgaactga ctgctcatgt tctttgcccc tttttttttt tttggagatg 47700
   gagtctcact ctgtcaccca ggctggagnt acagtggcgc aatctcagct cactgcaacc 47760
   tccgccgcct gggttcaatc aattctcctg cctcaacctc ctgagttgct gggactacag 47820
   gcgcatgcca ccatgtccgg ctaatttttt nttttttttt gtatttttag tagagatggg 47880
   gtttcatcat attggtcagg ctggtctcaa acccctgacc tcaggtgatc cacctgcctt 47940
   ggcctcccaa agtgctggga taacagacat gagccactgt gcctggcctt ctttgcccat 48000
   tctttaatgg tgtatatcgg gcaaaattca gccctgataa ttcacatagg nttcttttct 48060
   attttcccta agtgccagct ggtctgagaa ataaagggac agagtacaaa agagagaaat 48120
   tttaaagcta ggtgtccggg ggagacatca catattggca ggttccatga tgccccctga 48180
   gccataaaac cagcaagttt ttattagtga tttcaaaagg ggagggagtg tacgaatagg 48240
   gtgtaggtca cagagatcac gtgcttcaca aggtaataga atatcacaag gcaaatggag 48300
   gcagggtgag atcacaggac cacaggaccg gggcaaaatt aaaattgcta atgaagtttt 48360
   gggcacgcat tgtcattgat aacatcttat caggaaacag ggtttgagag cagacaaccg 48420
   gtctgaccaa aatttattag gtgggaattt cctcatccta acaagcctgg gagtgctacg 48480
   ggagactggg gcttatttca tccctacagc tncaaccgta aaagacagct gcccccaaag 48540
   tggccatttt agaggcctac cctcagggac gaattgtctt tctcagggat gttccttgct 48600
   gagaaaaaga attcagcgat atttctccca tttacttttg aaagaagaga aatatggctc 48660
   tgttctgccc gactcaccag cggtcagagt ttaaggttat ctctcttgtt ccctgaacat 48720
   tgctgttatc ctgttctttt ttcaaggtgc ccagatttcg tattgttcaa acacacatgc 48780
   tctacaaaca atttgtgcag ttaacgcaat catcacaggg tcctgaggcg acatacatcc 48840
   tcctcagctt atgaagatga cgggattaag agattaaaga caggcatagg aaatcacaag 48900
   ggtgttgatt ggggaagtga taagtgtcca tgaaatcttc acaatttatg ttcagagatt 48960
   gcagtaaaga caggcgtaag aaattataaa agtattaatt tggggaacta ataaatgtcc 49020
   atgaaatctt cacaatccac attcttctgc catggcttca gccagtccct cctttcgggg 49080
   tccctaactt cctgcaacag gtgtattcat ctttttcttt ttgacttgtg caaactcttt 49140
   gcaatggaag aaattagccc cttgtatata tgttgtaaat atttttccta gtttaccatt 49200
   tgtattttca ctttatgata tctttgtaat gaagacaatt ttagttttta tatactcaaa 49260
   ggtgtcaatc tgaaatgaaa ctacagaggt gtttcagcgg catgttcaat ctgatgtagg 49320
   atctgacacc ccatgcctgg tcctcattaa ggactctact ggtgagaaat gcagacacac 49380
   ctcatcaatt gggcaattgg ttacctactg gtacacacgt acacacacac acacacacac 49440
   acacacacct gaaaacatgt atgaattctc aggaaaggta taatccaaac ccttacagag 49500
   aaagagaaac attttcctaa caaacatatt ctgcacagtt gccctgtgcc acacctgaga 49560
   tagcagatta tacagagtgg tgttattttt ccctttgtcc ttccctgtac tttcaaacta 49620
   tttcaaacta tgctcagatg gtccagtggt cagacactgg tacataagac agggagtcca 49680
   agagtctcct actccacaca gctactcctg ttagtgacac agaggcctca gagaagctgc 49740
   aataacaata ttttccttat gttttgggtc aaatgaaatc catgggtgtg ctgttcctct 49800
   gtaagctctg tatgtataca ggggcaaagc aagatgagga tctgtttttc tcctgtgtga 49860
   gtaaagcttc acaaagagat tacatattaa cccaagacat gtaagtgctc caaaaagaag 49920
   ccaaatccag gcacacagtg cctttacaga atgtgcctga tacttataat ttactgattt 49980
   aatctgaagt gaacataata cttagttgtt tttttttttt tttttttttt ttttgagtct 50040
   catagccctg tggacctaac tttccactgc agcctactag tttgggcaaa agcagagtca 50100
   tgccagacct atcatngcca aaacctatcc ctgaaacatc ctggttaacc tccttctcct 50160
   tgatgtcctc ccatccccca tttccaatta cctccacctt aagaggagaa ctctggtagg 50220
   caaagaactc taccaaagga acatctgtca cacatgactg agtagtagct tagaggatac 50280
   cagttgacag gaaagaatga aaagccttat gataatgcaa aaggattata gacatgcaca 50340
   atgtcatctg ccaaaaagaa actgatattt tgcttacttt tttttctctg aaaatgttca 50400
   gatttaaatc cattctctta tactgttcac caacttcaca aaatgatgct caagagtgcc 50460
   cagagagtcc tcctatctga ttgatgttct tatgtcccgg gctttacagg tgcttacaac 50520
   agaatgctgc cctacatcgt catgggtagt ctgactgtcc tgattggaat cytcaccctt 50580
   tttttccctg aaagtttggg aatgactctt ccagaaacct tagagcagat gcagaaagtg 50640
   aaatggtaag taggactttt aacaaaatga taccaaaatg ccttagggag aataagcatg 50700
   gaagaatagc taggaaggtt ctgaaataga agagtaataa gtagagacta gctctatcag 50760
   ataataaagc atattgttgg ctgggcacnn ngnnnnncnc nnntnnnnnc nnnncnntnn 50820
   gngnggctga ggcaggtgga tcacctgagg tcaggaattt gagaccagcc tggccaacat 50880
   agtgaaacct catctttact aaaaatacaa aaaattagct gggtgtggtg gcagtcncat 50940
   gtaatcccag ctactcagga ggctgaggca ggagaactgc ttgacccagg aggcagaggt 51000
   tgcagtgagc tgagatcaca ccactgcact ccagcctggg caacaagagc aaaactccgt 51060
   ctcaaaaaaa ataaataaat aataggccag gcgcggtggc acatgcctgt aatcccaaca 51120
   ctttgggagg ctgaggcagg cggatcatga ggtcaagaga tcgagaccat cctggccaac 51180
   atggtgaaac cctgtctcta ctaaaaatac aaaaattagc tgggcatggt ggcacacacc 51240
   tgtagtccca gctactcagg aggctgaggt aggagaattg cttgaacccg ggaggtggag 51300
   nttgcagtga gccaacatca tgccacngca ctccagactg ggcaacagag ctagactctg 51360
   tctcaaaaat aataataata gtaataaagc atattgttga gcaatagtaa ttaaaacaat 51420
   gtgataatgg tataggaata gaaaccaatg gaataggata gagttcagga taaaacccaa 51480
   gaaaatataa aaattcagta tgtaataaaa agtagcaaag tcaaggggga atagttattc 51540
   agtaaaaggt attagaacat tcagctagac attttgaata aattaaaaag ttgtctcttt 51600
   aaaaaatgca ttggctgggc atggtggctc acacctgtaa tcccagcact tagggagatt 51660
   gaggcaggca gattgcttga ggtcaggagt tcgagaacag tctgtccaac atggtgaaac 51720
   cccatctcta ctaaaaatac aaaaattagc caggcatggt ggcgcacccc tgtaatccca 51780
   gctcgcggga ggctgaggca ggagaatcgc ttgaaccagg gtggtggatg ttgcagtgag 51840
   ccgaaatcgc gccactacac tccagtctgc gtgacagagc gagactccgt ctccanaaaa 51900
   aaaacacatc aagataaaat ccagacagtt aaaaaattta ggcctaaaaa tgtaaaatca 51960
   taaatatacc aggaggaaac aaacattttt ataaccttag agaagatctt actgagcata 52020
   acataaaatc aaagaaccat aaaggaacag attgatatat ctgattgtac attttttaaa 52080
   ttattggcaa ggcaaaaaga aaccacccac aaagacaaaa gtcaataagc caggagaaaa 52140
   tatttgctac acaaaaccta aacataaggc taatatactt aatatacaaa aagctcatag 52200
   taaccagtga gaaaaagaca aacaattcag taggaaaacc agcaaaggac aagaacagac 52260
   agtttacaga agaagtcact tcagtgtcca atatatacat gaaaaaagtt atttgtccac 52320
   attaataagg aaatgcaaaa ataaaacaat tcaacatctt tttttggctt atgagattgc 52380
   gcaagggttt ttcttcttga cgcaaaccaa ttctgacacc tattgagtgt cctataatgc 52440
   agtncaattt tgacaatacc taaatttagc atcagactcc acaggtgtaa agactcagtc 52500
   ccacaatact gccttgcttc acttgccact cacaaatgtc aggtccccag gttacccaca 52560
   tttctctctg actttgctac caagtctggg gttccaacaa atcccctcct tgggtttgat 52620
   tatttgctag aatgactcac agcactcaga acactggcca ctagcngttt cttacaaagg 52680
   atacaaatga acagccagat gaagaggtac atacagagag gtctggaagg gtcctgagtg 52740
   cagaggttct gtccctacgg agttaaggtg cactgccctc ctggcatgtg gatgtggtca 52800
   ccaactcgga agttctccaa accccatcat ttagaggttt tcatggaggt ttagtcatgt 52860
   tggcatgatg gattattaag tctatcccca gcctctctcc tctccccaga ggttccaggc 52920
   ttctaatcca ggcttagtct tctggtgacc agcccccacc ctgaagctat ctaagggccc 52980
   agccaagggt tgcctcatta gaacaaaaga agcttttatc actccctatc actccagaaa 53040
   ttataagaga tttaggatct ctatgttagg agccagggac aaagaccagt atctttttat 53100
   gacaccacag caaacattta aaagattagt ggtatttagg agaagtttag agtttcgagt 53160
   aacactgcct ggctttaaat cccaaatcta ctgcaaaaat ttgggcatat cacttaatat 53220
   tcactaagct tcagtattcc catctgctat atgggatatt tcatacggct attttgaggt 53280
   tataaaagat aaagtgttcc cagtgcctgg ccttgataaa ggtaactctg catatgaatt 53340
   aattctgatc cctgacagaa catttatagc aaaaagtgcc cattgttgat aagttgtttt 53400
   cttctaatga tagaactact tcagtatcaa ctttaattgc caagaatttc atgcctccct 53460
   ctctatcctt tgcttgggaa aactgtgaaa gagtttaaat tctggcttct atgcttaatt 53520
   aagaaagtag cctcagatgt ttaaaagcat aaaatattag atctacacca tgaggttctc 53580
   attagagttc aaatagttac ttgttcattg attggagaaa tatttttcaa tgtcaatttg 53640
   gatggagcat tttgaggagg aaagtcttga atatttaatt ttccttagca tctattttat 53700
   tttcaggttc agatctggga aaaaaacaag agactcaatg gagacanaag aaaatcccaa 53760
   ggttctaata actgcattct gaaaaaatat ctaccccatt tggtgaagtg aaaaacacaa 53820
   aaataagacc ctgtggagaa attcgttgtt cccactgaaa tggactgact gtaacgattg 53880
   acaccaaaat gaaccttgct atcaagaaat gctcgtcata cagtaaactc tggatgattc 53940
   ttccagataa tgtccttgct ttacaaacca accatttcta gagagtctcc ttactcatta 54000
   attcaatgaa atggattggt aagatgtctt gaaaacatgt tagtcaagga ctggtaaaat 54060
   acatataaag attaacactc atttnccaat catacaaata ctatccaaat aaaaataaca 54120
   tcattgtatt aacgcaaata ttaggtgaca acnaatgtgt gtgtatgtgt gtgtgtttgt 54180
   acatgtggaa gtgaaccagc acagtcaaaa ttatagtagt ttctgaagtg aaccaaatga 54240
   ttatatgcag nattcctatc cagaaaacct tccacactcc tttgagaggg agtgatcact 54300
   gttagaggaa aaaacaaacc actttttacc ctgtactgtc acactcaacc cacaatactt 54360
   ctgtgaccag atgtggtggg gggcttctca tacaccaagt aattctccag cagataccaa 54420
   tggggtgttc tctaatagaa ttaagctctg accctatctg cggggagata gtgtcagatc 54480
   ccacaggttg tgtgctctgt ccctcttcag atgccattcn aacatccaag ccacacgcac  54540
   ctctgaccta                                                          54550